# EUROPÄISCHE PATENTSCHRIFT

(11) **EP 2 754 660 B1**
(45) Veröffentlichungstag und Bekanntmachung des Hinweises auf die Patenterteilung: **02.11.2016**
(21) Anmeldenummer: 14001269.1
(22) Anmeldetag: 29.04.2008
(51) Int. Cl.: A61K 31/506, A61K 31/5355, A61K 31/55, A61K 31/5377, A61P 35/00, A61K 31/501, A61K 9/00, C07D 453/02, A61K 45/06, C07D 405/14, C07D 417/10, C07D 409/14, C07D 451/06, C07D 417/14, C07D 401/14, C07D 403/10, C07D 401/10, A61K 9/28, A61K 9/08

(54) **Pyridazinonderivate**
Pyridazinone derivatives
Dérivés de pyridazinone

(30) Priorität: 12.07.2007 DE 102007032507
(43) Veröffentlichungstag der Anmeldung: 16.07.2014
(62) Teilanmeldung aus: 08758359.7
(73) Patentinhaber: Merck Patent GmbH, 64293 Darmstadt (DE)
(72) Erfinder: Dorsch, Dieter, 64372 Ober-Ramstadt (DE); Stieber, Frank, 64683 Einhausen (DE); Schadt, Oliver, 63517 Rodenbach (DE); Blaukat, Andree, 64342 Seeheim-Jugenheim (DE)

(56) Entgegenhaltungen:
- WO-A-2007/065518

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Arzneimittel, enthaltend Verbindungen bei denen die Hemmung, Regulierung und/oder Modulation der Signaltransduktion von Kinasen, insbesondere der Tyrosinkinasen und/oder Serin/Threonin-Kinasen eine Rolle spielt.

Die vorliegende Erfindung betrifft insbesondere Arzneimittel, enthaltend Verbindungen bei denen die Hemmung, Regulierung und/oder Modulation der Signaltransduktion von Met-Kinase eine Rolle spielt.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Rolle der Rezeptortyrosinkinase Met bei der menschlichen Onkogenese, sowie die Möglichkeit der Inhibierung der HGF(hepatocycte growth factor)-abhängigen Met-Aktivierung wird von S. Berthou et al. in Oncogene, Vol. 23, Nr. 31, Seiten 5387-5393 (2004) beschrieben. Der dort beschriebene Inhibitor SU11274, eine Pyrrol-Indolin-Verbindung, ist potentiell zur Krebsbekämpfung geeignet.

Ein anderer Met-Kinase-Inhibitor zur Krebstherapie ist von J.G. Christensen et al. in Cancer Res. 2003, 63(21), 7345-55 beschrieben. Von einem weiterem Tyrosinkinase-Inhibitor zur Krebsbekämpfung berichten H. Hov et al. in Clinical Cancer Research Vol. 10, 6686-6694 (2004). Die Verbindung PHA-665752, ein Indolderivat, ist gegen den HGF-Rezeptor c-Met gerichtet. Weiter wird dort berichtet, daß HGF und Met erheblich zum malignen Prozess verschiedener Krebsformen, wie z.B. multipler Myeloma, betragen.

Die Synthese von kleinen Verbindungen, die die Signaltransduktion der Tyrosinkinasen und/oder Serin/Threonin-Kinasen, insbesondere der Met-Kinase spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Im einzelnen betrifft die vorliegende Erfindung Verbindungen der Formel I, die die Signaltransduktion der Met-Kinase hemmen, regulieren und/oder modulieren, Zusammensetzungen, die diese Verbindungen enthalten, sowie Verfahren zu ihrer Verwendung zur Behandlung von Met-Kinasebedingten Krankheiten und Leiden wie Angiogenese, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Feste Tumore, insbesondere schnell wachsende Tumore, können mit Met-Kinasehemmern behandelt werden. Zu diesen festen Tumoren zählen die Monozytenleukämie, Hirn-, Urogenital-, Lymphsystem-, Magen-, Kehlkopfund Lungenkarzinom, darunter Lungenadenokarzinom und kleinzelliges Lungenkarzinom.

Die vorliegende Erfindung richtet sich auf Verfahren zur Regulation, Modulation oder Hemmung der Met-Kinase zur Vorbeugung und/oder Behandlung von Erkrankungen im Zusammenhang mit unregulierter oder gestörter Met-Kinase-Aktivität. Insbesondere lassen sich die Verbindungen der Formel I auch bei der Behandlung gewisser Krebsformen einsetzen. Weiterhin können die Verbindungen der Formel I verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die Verbindungen der Formel I zur Isolierung und zur Untersuchung der Aktivität oder Expression von Met-Kinase verwendet werden. Außerdem eigenen sie sich insbesondere zur Verwendung in diagnostischen Verfahren zu Erkrankungen im Zusammenhang mit unregulierter oder gestörter Met-Kinase-Aktivität.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszenz nachweisbar (Ross et al., 2002, Biochem. J.).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK

Andere Pyridazinderivate sind als MET-Kinase-Inhibitoren in WO 2007/065518 beschrieben.

Thiadiazinone sind in DE19604388, WO2003/037349 WO2007/057093 bzw. WO2007/057092 beschrieben.

Dihydropyridazinone zur Krebsbekämpfung sind in WO 03/037349 A1 beschrieben.

Andere Pyridazine zur Behandlung von Krankheiten des Immunsystems, ischämischer und entzündlicher Erkrankungen kennt man aus EP 1 043 317 A1 und EP 1 061 077 A1.

In EP 0 738 716 A2 und EP 0 711 759 B1 sind andere Dihydropyridazinone und Pyridazinone als Fungizide und Insektizide beschrieben. Andere Pyridazinone sind als cardiotonische Agenzien in US 4,397,854 beschrieben.

In JP 57-95964 sind andere Pyridazinone offenbart.

Die Verwendung anderer MET-Kinase Inhibitoren zur Krebsbekämpfung ist in der WO 2007/075567 beschrieben.

### ZUSAMMENFASSUNG DER ERFINDUNG

Die Erfindung betrifft Arzneimittel, enthaltend mindestens eine Verbindung der Formel I 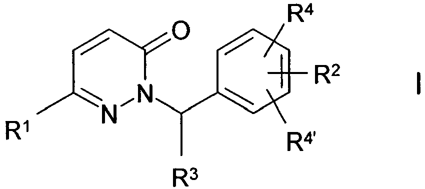 worin
- R¹: Ar oder Het,
- R^{2 2}: einen ungesättigten, gesättigten oder aromatischen 6-gliedrigen Heterocyclus mit 1 bis 4 N-, O- und/oder S-Atomen, der ein-, zwei- oder dreifach durch N=CR³N(R³)₂, SR³, NO₂, CN, COOR³, CON(R³)₂, NR³COA, NR³SO₂A, SO₂N(R³)₂, S(O)mA, [C(R³)₂]ₙN(R³)2, [C(R³)_{2]n}Het, O[C(R³)₂]ₚOR³, O[C(R³)_{2]n}N(R³)₂, O[C(R³)₂]ₙCΞC[C(R³)₂]nN(R³)₂, O[C(R³)₂]ₙN⁺O-(R³)₂, O[C(R³)₂]ₙHet, S[C(R³)₂]ₙN(R³)₂, S[C(R³)₂]ₙHet, NR³[C(R³)₂]ₙN(R³)₂, NR³[C(R³)₂]ₙHet, NHCON(R³)₂, NHCONH[C(R³)₂]ₙN(R³)₂, NHCONH[C(R³)₂]ₙHet, NHCO[C(R³)₂]ₙN(R³)₂, NHCO[C(R³)₂]ₙHet, CON(R³)₂, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, COA, CH=CH-COOR³ und/oder CH=CH-N(R³)₂ substituiert ist,
- R³: H oder A,
- R⁴, R⁴': jeweils unabhängig voneinander H, Hal, A, OR³, CN, COOR³, CON(R³)₂, NR³COA, NR³SO₂A, SO₂N(R³)₂ oder S(O)mA,
- Ar: unsubstituiertes oder ein-, zwei- oder dreifach durch Hal, A, [C(R³)₂]ₙOR³, [C(R³)₂]ₙN(R³)₂, SR³, NO₂, CN, COOR³, CON(R³)₂, NR³COA, NR³SO₂A, SO₂N(R³)₂, S(O)mA, CO-Het, Het, O[C(R³)₂]ₙN(R³)2, O[C(R³)₂]ₙHet, NHCOOA, NHCON(R³)₂, NHCOO[C(R³)₂]ₙN(R³)₂, NHCOO[C(R³)₂]ₙ-Het, NHCONH[C(R³)₂]ₙN(R³)2, NHCONH[C(R³)₂]ₙHet, OCONH[C(R³)₂]ₙN(R³)₂, OCONH[C(R³)₂]ₙHet, CONR³[C(R³)₂]ₙN(R³)₂, CONR³[C(R³)_{2]n}Het und/oder COA substituiertes Phenyl, Naphthyl oder Biphenyl,
- Het: einen ein-, zwei- oder dreikernigen gesättigten, ungesättigten oder aromatischen Heterocyclus mit 1 bis 4 N-, O- und/oder S-Atomen, der unsubstituiert oder ein-, zwei-, drei-, vier- oder fünffach durch Hal, A, [C(R³)₂]ₙOR³, [C(R³)₂]ₙN(R³)₂, SR³, NO₂, CN, COOR³, CON(R³)₂, NR³COA, NR³SO₂A, SO₂N(R³)₂, S(O)mA, CO-Het¹, [C(R³)₂]ₙHet¹, O[C(R³)₂]ₙN(R³)₂, O[C(R³)₂]ₙHet¹, NHCOOA, NHCON(R³)₂, NHCOO[C(R³)₂]ₙN(R³)₂, NHCOO[C(R³)₂]ₙHet¹, NHCONH[C(R³)₂]ₙN(R³)₂, NHCONH[C(R³)₂]ₙHet¹, OCONH[C(R³)₂]ₙN(R³)2, OCONH[C(R₃)₂]ₙHet¹, CO-Het¹, CHO, COA, =S, =NH, =NA und/oder =O (Carbonylsauerstoff) substituiert sein kann, und wobei ein Ringstickstoff oxidiert sein kann,
- Het¹: einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
- A: unverzweigtes oder verzweigtes Alkyl mit 1-10 C-Atomen, worin 1-7 H-Atome durch F ersetzt sein können und/oder worin eine oder zwei nicht-benachbarte CH₂-Gruppen durch O, NH, S, SO, SO₂ und/oder durch CH=CH-Gruppen ersetzt sein können, oder cyclisches Alkyl mit 3-7 C-Atomen,
- Hal: F, Cl, Br oder l,
- m: 0, 1 oder 2,
- n: 0, 1, 2, 3 oder 4,
- p: 1, 2, 3 oder 4
bedeuten,
sowie ihre pharmazeutisch verwendbaren Solvate, Salze, Tautomere und Stereoisomere, einschließlich deren Mischungen in allen Verhältnissen, sowie gegebenenfalls Träger- und/oder Hilfsstoffe, wobei 0,5 mg bis 1 g einer Verbindung der Formel I enthalten ist.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I sowie ihrer pharmazeutisch verwendbaren Salze, Solvate, Tautomeren und Stereoisomeren, dadurch gekennzeichnet, daß man
a) eine Verbindung der Formel II 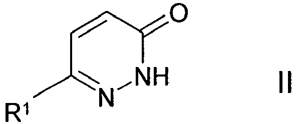 worin R¹ die in Anspruch 1 angegebene Bedeutung hat,
   mit einer Verbindung der Formel III 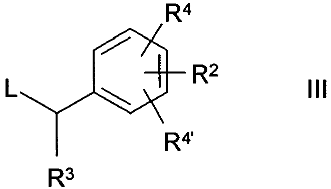 worin R², R³, R⁴ und R^{4'} die in Anspruch 1 angegebenen Bedeutungen haben und
   L Cl, Br, l oder eine freie oder reaktionsfähig funktionell abgewandelte OH-Gruppe bedeutet,
   umsetzt,
   oder
b) einen Rest R² in einen anderen Rest R² umwandelt, indem man
   i) einen Oxadiazolrest in einen Pyrimidinylrest überfphrt,
   ii) eine Aminogruppe acyliert oder alkyliert,
   iii) eine Hydroxygruppe verethert,
   oder
c) daß man sie aus einem ihrer funktionellen Derivate durch Behandeln mit einem solvolysierenden oder hydrogenolysierenden Mittel in Freiheit setzt,
   und/oder
   eine Base oder Säure der Formel I in eines ihrer Salze umwandelt.

Vor- und nachstehend haben die Reste R¹, R², R³, R⁴, R^{4'} die bei der Formel I angegebenen Bedeutungen, sofern nicht ausdrücklich etwas anderes angegeben ist.

A bedeutet Alkyl, dieses ist unverzweigt (linear) oder verzweigt, und hat 1, 2, 3, 4, 5, 6, 7, 8, 9 oder 10 C-Atome. Abedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cyclisches Alkyl (Cycloalkyl) bedeutet vorzugsweise Cyclopropyl, Cyclobutyl, Cylopentyl, Cyclohexyl oder Cycloheptyl.

Ar bedeutet z.B. O-, m- oder p-Tolyl, O-, m- oder p-Ethylphenyl, O-, m- oder p-Propylphenyl, O-, m- oder p-Isopropylphenyl, O-, m- oder p-tert.-Butylphenyl, O-, m- oder p-Hydroxyphenyl, O-, m- oder p-Nitrophenyl, O-, m-oder p-Aminophenyl, O-, m- oder p-(N-Methylamino)-phenyl, O-, m- oder p-(N-Methylaminocarbonyl)-phenyl, O-, m- oder p-Acetamidophenyl, O-, m-oder p-Methoxyphenyl, O-, m- oder p-Ethoxyphenyl, O-, m- oder p-Ethoxycarbonylphenyl, O-, m- oder p-(N,N-Dimethylamino)-phenyl, O-, m- oder p-(N,N-Dimethylaminocarbonyl)-phenyl, O-, m- oder p-(N-Ethylamino)-phenyl, O-, m- oder p-(N,N-Diethylamino)-phenyl, O-, m- oder p-Fluorphenyl, O-, m-oder p-Bromphenyl, O-, m- oder p- Chlorphenyl, O-, m- oder p-(Methylsulfonamido)-phenyl, O-, m- oder p-(Methylsulfonyl)-phenyl, O-, m- oder p-Methylsulfanylphenyl, O-, m- oder p-Cyanphenyl, O-, m- oder p-Carboxyphenyl, O-, m- oder p-Methoxycarbonylphenyl, o-, m- oder p-Formylphenyl, O-, m- oder p-Acetylphenyl, O-, m- oder p-Aminosulfonylphenyl, O-, m- oder p-(Morpholin-4-ylcarbonyl)-phenyl, O-, m- oder p-(Morpholin-4-ylcarbonyl)-phenyl, O-, m- oder p-(3-Oxo-morpholin-4-yl)-phenyl, O-, m- oder p-(Piperidinyl-carbonyl)-phenyl, O-, m- oder p-[2-(Morpholin-4-yl)ethoxy]-phenyl, O-, m- oder p-[3-(N,N-Diethylamino)propoxy]-phenyl, O-, m- oder p-[3-(3-Diethylaminopropyl)-ureido]-phenyl, O-, m- oder p-(3-Diethylaminopropoxy-carbonylamino)-phenyl, weiter bevorzugt 2,3-, 2,4-, 2,5-, 2,6-, 3,4-oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet weiterhin vorzugsweise unsubstituiertes oder ein-, zwei- oder dreifach durch A, Hal, CN, S(O)ₘA, NR³COA, CON(R³)₂, O[C(R³)₂]ₙN(R³)₂, [C(R³)₂]ₙOR³, CONR³[C(R³)₂]ₙN(R³)2 und/oder CONR³[C(R³)₂]ₙHet substituiertes Phenyl, Naphthyl oder Biphenyl.

Het bedeutet, ungeachtet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4-oder 5-Pyrazolyl, 2-, 4- oder 5-Oxazolyl, 3-, 4- oder 5-lsoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-lsochinolyl, 3-, 4-, 5-, 6-, 7-oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl, Aza-bicyclo[3.2.1]octyl oder Dibenzofuranyl.

Die heterocyclischen Reste können auch teilweise oder vollständig hydriert sein.

Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3-oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7-oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydro-benzofuranyl, 2,3-Dihydro-2-oxo-furanyl, 3,4-Dihydro-2-oxo-1*H-*chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

Het bedeutet vorzugsweise unsubstituiertes oder ein-, zwei-, drei-, vier- oder fünffach durch A, CHO, COOR³, CON(R³)₂, [C(R³)₂]ₙHet¹, [C(R³)₂]ₙOR³, [C(R³)₂]ₙN(R³)2, O[C(R³)₂]ₙHet¹ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl, Azabicyclo[3.2.1]octyl, Aza-bicyclo[2.2.2]octyl, Imidazolidinyl, Azepanyl oder Benzo[2,1,3]thiadiazolyl,
und wobei ein Ringstickstoff oxidiert sein kann.

Het¹ bedeutet vorzugsweise unsubstituiertes oder ein- oder zweifach durch A und/oder =O (Carbonylsauerstoff) substituiertes Pyrrolidin, Piperidin, Piperazin oder Morpholin.

R¹ bedeutet vorzugsweise Ar oder Benzo[2,1,3]thiadiazolyl.

Der ungesättigte, gesättigte oder aromatische 6-gliedrige Heterocyclus mit 1 bis 4 N- und/oder O-Atomen in der Bedeutung für R² hat z.B. folgende Bedeutungen Pyrimidin, Pyridazin, Pyridin, [1,3]Oxazinan, Morpholin, Piperidin, Piperazin, 1,4-Dihydropyridin, 1,2,3,4-Tetrahydro-6-pyridin, Tetrahydropyran, 1,4-Dioxan, 1,3-Dioxan, Hexahydropyridazin oder Hexahydro-pyrimidin.

R² bedeutet vorzugsweise ein-, zwei- oder dreifach durch N=CR³N(R³)₂, CN, COOR³, [C(R³)₂]ₙN(R³)₂, [C(R³)₂]ₙHet, O[C(R³)₂]ₚOR³, O[C(R3)₂]ₙN(R³)2, O[C(R³)₂]ₙCΞC[C(R³)₂]ₙN(R³)₂, O[C(R³)₂]ₙN⁺O⁻(R³)₂, O[C(R³)₂]ₙHet, NR³[C(R³)₂]ₙN(R³)₂, NR³[C(R³)₂]ₙHet,
[C(R³)₂]ₙNHCO[C(R³)₂]ₙN(R³)₂, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ und/oder CH=CH-N(R³)₂ substituiertes Pyrimidinyl, Pyridazinyl, Pyridinyl, [1,3]Oxazinanyl, Morpholinyl, Piperidinyl oder Piperazinyl.

R³ bedeutet vorzugsweise H, Methyl, Ethyl oder Propyl.

R⁴, R^{4'} bedeuten vorzugsweise H.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Arzneimitteln, enthaltend Verbindungen der Formel I, können durch die folgenden Teilformeln la bis II ausgedrückt werden, die der Formel I entsprechen und worin die nicht näher bezeichneten Reste die bei der Formel I angegebene Bedeutung haben, worin jedoch
- in la R²: einen ungesättigten, gesättigten oder aromatischen 6-gliedrigen Heterocyclus mit 1 bis 4 N- und/oder O-Atomen, der ein-, zwei- oder dreifach durch N=CR³N(R³)₂, CN, COOR³, [C(R³)₂]ₙN(R³)₂, [C(R³)₂]ₙHet, O[C(R³)₂]pOR³, O[C(R³)₂]ₙN(R³)₂, O[C(R³)₂]ₙCΞC[C(R³)₂]ₙN(R³)₂, O[C(R³)₂]ₙN⁺O⁻(R³)₂, O[C(R³)₂]ₙHet, NR³[C(R³)₂]ₙN(R³)₂, NR³[C(R³)₂]ₙHet, [C(R³)₂]ₙNHCO[C(R³)₂]ₙN(R³)2, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ und/oder CH=CH-N(R³)₂ substituiert ist,
bedeutet;
- in Ib Ar: unsubstituiertes oder ein-, zwei- oder dreifach durch A, Hal, CN, S(O)ₘA, NR³COA, CON(R³)₂, O[C(R³)₂]ₙ-N(R³)₂, [C(R³)₂]ₙOR³, CONR³[C(R³)₂]ₙN(R³)₂ und/oder CONR³[C(R³)₂]ₙHet substituiertes Phenyl, Naphthyl oder Biphenyl,
bedeutet;
- in Ic R⁴, R^{4'}: H bedeutet;
- in Id Het: einen ein-, zwei- oder dreikernigen gesättigten, ungesättigten oder aromatischen Heterocyclus mit 1 bis 4 N-, O- und/oder S-Atomen, der unsubstituiert oder ein-, zwei-, drei-, vier- oder fünffach durch A, CHO, COOR³, CON(R³)₂, [C(R³)₂]ₙHet¹, [C(R³)₂]ₙOR³, [C(R³)₂]ₙN(R³)₂, O[C(R³)₂]ₙHet¹ und/oder =O (Carbonylsauerstoff) substituiert sein kann, und wobei ein Ringstickstoff oxidiert sein kann,
bedeutet;
- in le Het¹: einen einkernigen gesättigten Heterocyclus mit 1 bis 2 N und/oder O-Atomen, der ein- oder zweifach durch A und/oder =O (Carbonylsauerstoff) substituiert sein kann,
bedeutet;
- in If A: unverzweigtes oder verzweigtes Alkyl mit 1-8 C-Atomen, worin 1-7 H-Atome durch F ersetzt sein können,
bedeutet;
- in Ig R¹: Ar oder Benzo[2,1,3]thiadiazolyl
bedeutet;
- in Ih R³: H, Methyl, Ethyl oder Propyl,
bedeutet;
- in li R²: ein-, zwei- oder dreifach durch N=CR³N(R³)₂, CN, COOR³, [C(R³)₂]ₙN(R³)₂, [C(R³)₂]ₙHet, O[C(R³)₂]ₚOR³, O[C(R³)2]ₙN(R³)₂, O[C(R³)₂]ₙCΞC[C(R³)₂]ₙN(R³)₂, O[C(R³)₂]ₙN⁺O⁻(R³)2, O[C(R³)₂]ₙHet, NR³[C(R³)₂]ₙN(R³)₂, NR³[C(R³)₂]ₙHet, [C(R³)₂]ₙNHCO[C(R³)₂]ₙN(R³)₂, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ und/oder CH=CH-N(R³)₂ substituiertes Pyrimidinyl, Pyridazinyl, Pyridinyl, [1,3]Oxazinanyl, Morpholinyl, Piperidinyl oder Piperazinyl,
bedeutet;
- in Ij Het: unsubstituiertes oder ein-, zwei-, drei-, vier- oder fünffach durch A, CHO, COOR³, CON(R³)₂, [C(R³)₂]ₙHet¹, [C(R³)₂]ₙOR³, [C(R³)₂]ₙN(R³)₂, O[C(R³)₂]ₙHet¹ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl, Aza-bicyclo[3.2.1 ]octyl, Aza-bicyclo[2.2.2]octyl, Imidazolidinyl, Azepanyl oder Benzo[2,1,3]thiadiazolyl, und wobei ein Ringstickstoff oxidiert sein kann,
bedeutet;
- in Ik Het¹: unsubstituiertes oder ein- oder zweifach durch A und/oder =O (Carbonylsauerstoff) substituiertes Pyrrolidin, Piperidin, Piperazin oder Morpholin,
bedeutet;
- in II R¹: Ar oder Het,
- R²: ein-, zwei- oder dreifach durch N=CR³N(R³)₂, CN, COOR³, [C(R³)₂]ₙN(R³)₂, [C(R³)₂]ₙHet, O[C(R³)₂]ₚOR³, O[C(R3)₂]ₙN(R³)₂, O[C(R³)₂]ₙCΞC[C(R³)₂]ₙN(R³)₂, O[C(R³)₂]ₙN⁺O⁻(R³)2, O[C(R³)₂]ₙHet, NR³[C(R³)₂]ₙN(R³)₂, NR³[C(R³)₂]ₙHet, [C(R³)₂]ₙNHCO[C(R³)₂]ₙN(R³)₂, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ und/oder CH=CH-N(R³)₂ substituiertes Pyrimidinyl, Pyridazinyl, Pyridinyl, [1,3]Oxazinanyl, Morpholinyl, Piperidinyl oder Piperazinyl,
- R³: H, Methyl, Ethyl oder Propyl,
- R⁴, R⁴': H,
- Ar: unsubstituiertes oder ein-, zwei- oder dreifach durch A, Hal, CN, S(O)ₘA, NR³COA, CON(R³)₂, O[C(R³)₂]ₙ-N(R³)₂, [C(R³)₂]ₙOR³, CONR³[C(R³)₂]ₙN(R³)₂ und/oder CONR³[C(R³)₂]ₙHet substituiertes Phenyl, Naphthyl oder Biphenyl,
- Het: unsubstituiertes oder ein-, zwei-, drei-, vier- oder fünffach durch A, CHO, COOR³, CON(R³)₂, [C(R³)₂]ₙHet¹, [C(R³)₂]ₙOR³, [C(R³)₂]ₙN(R³)₂, O[C(R³)₂]ₙHet¹ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl, Aza-bicyclo[3.2.1]octyl, Aza-bicyclo[2.2.2]octyl, Imidazolidinyl, Azepanyl oder Benzo[2,1,3]thiadiazolyl, und wobei ein Ringstickstoff oxidiert sein kann,
- Het¹: unsubstituiertes oder ein- oder zweifach durch A und/oder =O (Carbonylsauerstoff) substituiertes Pyrrolidin, Piperidin, Piperazin oder Morpholin,
- A: unverzweigtes oder verzweigtes Alkyl mit 1-8 C-Atomen, worin 1-7 H-Atome durch F ersetzt sein können,
- Hal: F, Cl, Br oder I,
- m: 0, 1 oder 2,
- n: 0, 1, 2, 3 oder 4,
- p: 1, 2, 3 oder 4
bedeuten;
sowie ihre pharmazeutisch verwendbaren Salze, Solvate, Tautomere und Stereoisomere, einschließlich deren Mischungen in allen Verhältnissen, sowie gegebenenfalls Träger- und/oder Hilfsstoffe, wobei 0,5 mg bis 1 g einer Verbindung der Formel I enthalten ist.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsverbindungen der Formeln II und III sind in der Regel bekannt. Sind sie neu, so können sie aber nach an sich bekannten Methoden hergestellt werden.

Die verwendeten Pyridazinone der Formel II werden, wenn nicht käuflich erhältlich, in der Regel nach W. J. Coates, A. McKillop, Synthesis, 1993, 334-342 hergestellt.

Verbindungen der Formel I können vorzugsweise erhalten werden, indem man eine Verbindung der Formel II mit einer Verbindung der Formel III umsetzt.

In den Verbindungen der Formel III bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Die Umsetzung erfolgt in der Regel in Gegenwart eines säurebindenden Mittels vorzugsweise einer organischen Base wie DIPEA, Triethylamin, Dimethylanilin, Pyridin oder Chinolin.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Besonders bevorzugt ist Acetonitril, Dichlormethan und/oder DMF.

Vorzugsweise erfolgt die Umsetzung einer Verbindung der Formel II mit einer Verbindung der Formel III, worin L OH bedeutet, in einer Mitsunobu-Reaktion durch Zugabe von z.B. Triphenylphosphin und eines Dialkylazodicarboxylats. Als Lösungsmittel ist THF bevorzugt.

Es ist ferner möglich, eine Verbindung der Formel I in eine andere Verbindung der Formel I umzuwandeln, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxy schutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlor säure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Die Tritylgruppe wird zum Schutz der Aminosäuren Histidin, Asparagin, Glutamin und Cystein eingesetzt. Die Abspaltung erfolgt, je nach gewünschtem Endprodukt, mit TFA / 10% Thiophenol, wobei die Tritylgruppe von allen genannten Aminosäuren abgespalten wird, bei Einsatz von TFA / Anisol oder TFA / Thioanisol wird nur die Tritylgruppe von His, Asn und Gln abgespalten, wogegen sie an der Cys-Seitenkette verbleibt. Die Pbf (Pentamethylbenzofuranyl)-gruppe wird zum Schutz von Arg eingesetzt. Die Abspaltung erfolgt z.B. mit TFA in Dichlormethan.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine. Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Besonders bevorzugt sind Hydrochlorid, Dihydrochlorid, Hydrobromid, Maleat, Mesylat, Phosphat, Sulfat und Succinat.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-ÖI-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unter schiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.

Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.

Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.

Ebenfalls umfasst ist die Verwendung der Verbindungen der Formel I und/oder ihre physiologisch unbedenklichen Salze und Solvate zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die vorliegende Erfindung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.

Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.

Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I können an Patienten zur Behandlung von Krebs, insbesondere schnell wachsenden Tumoren, verabreicht werden.

Gegenstand der Erfindung ist somit die Verwendung von Verbindungen der Formel I, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt ist hierbei die Met-Kinase.

Bevorzugt ist die Verwendung von Verbindungen der Formel I, sowie ihrer pharmazeutisch verwendbaren Salze, Solvate und Stereoisomere, einschließlich deren Mischungen in allen Verhältnissen,
zur Herstellung eines Arzneimittels zur Behandlung von Krankheiten, die durch Inhibierung der Tyrosinkinasen durch die Verbindungen nach Anspruch 1 beeinflußt werden.

Besonders bevorzugt ist die Verwendung zur Herstellung eines Arzneimittels zur Behandlung von Krankheiten, die durch Inhibierung von Met-Kinase durch die Verbindungen nach Anspruch 1 beeinflußt werden. Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren der Lunge, des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens und/oder des Kehlkopfs.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (Cl 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-ανβ3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und In-vivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson |
| | Tetraplatin | Matthey) |
| | Ormiplatin | BBR-3464 (Hoffrnann-La |
| | Iproplatin | Roche) |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La |
| | Idatrexate | Roche) |
| | | Ethinylcytidin (Taiho ) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour- |
| | Irinotecan (CPT-11) | Ipsen) |
| | 7-Ethyl-10- | TAS-103 (Taiho) |
| | hydroxycamptothecin | Elsamitrucin (Spectrum) |
| | Topotecan | J-107088 (Merck & Co) |
| | Dexrazoxanet | BNP-1350 (BioNumerik) |
| | (TopoTarget) | CKD-602 (Chong Kun |
| | Pixantron (Novuspharrna) | Dang) |
| | Rebeccamycin-Analogon | KW-2170 (Kyowa Hakko) |
| | (Exelixis) | |
| | BBR-3576 (Novuspharrna) | |
| | | |
| Antitumor-Antibiotika | Dactinomycin (Actinomycin | Amonafid |
| | D) | Azonafid |
| | Doxorubicin (Adriamycin) | Anthrapyrazol |
| | Deoxyrubicin | Oxantrazol |
| | Valrubicin | Losoxantron |
| | Daunorubicin | Bleomycinsulfat |
| | (Daunomycin) | (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem |
| | Cyanomorpholino- | Pharmaceuticals) |
| | doxorubicin | |
| | Mitoxantron (Novantron) | |
| | | |
| Antimitotische | Paclitaxel | SB 408075 |
| Mittel | Docetaxel | (GlaxoSmithKline) |
| | Colchicin | E7010 (Abbott) |
| | Vinblastin | PG-TXL (Cell |
| | Vincristin | Therapeutics) |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner- | D 24851 (ASTA Medica) |
| | Lambert) | ER-86526 (Eisai) |
| | Cemadotin (BASF) | Combretastatin A4 (BMS) |
| | RPR 109881A (Aventis) | Isohomohalichondrin-B |
| | TXD 258 (Aventis) | (PharmaMar) |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | !DN-5109 (Indena) |
| | Vinflunin (Fabre) | AVLB (Prescient |
| | Auristatin PE (Teikoku | NeuroPharma) |
| | Hormone) | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| Aromatase- | Aminoglutethimid | Exemestan |
| Inhibitoren | Letrozol | Atamestan (BioMedicines) |
| | Anastrazol | YM-511 (Yamanouchi) |
| | Formestan | |
| | | |
| Thymidylatsynthase-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter |
| | Glufosfamid (Baxter | International) |
| | International) | Apaziquon (Spectrum |
| | Albumin + 32P (Isotope | Pharmaceuticals) |
| | Solutions) | 06-Benzylguanin |
| | Thymectacin (NewBiotics) | (Paligent) |
| | Edotreotid (Novartis) | |
| | | |
| Farnesyltransferase-Inhibitoren | Arglabin (NuOncology | Tipifarnib (Johnson & |
| | Labs) | Johnson) |
| | lonafarnib (Schering- | Perillylalkohol (DOR |
| | Plough) | BioPharma) |
| | BAY-43-9006 (Bayer) | |
| | | |
| Pumpen- | CBT-1 (CBA Pharma) | Zosuquidar-Trihydrochlorid |
| Inhibitoren | Tariquidar (Xenova) | (Eli Lilly) |
| | MS-209 (Schering AG) | Biricodar-Dicitrat (Vertex) |
| | | |
| Histonacetyltransferase-Inhibitoren | Tacedinalin (Pfizer) | Pivaloyloxymethylbutyrat |
| | SAHA (Aton Pharma) | (Titan) |
| | MS-275 (Schering AG) | Depsipeptid (Fujisawa) |
| | | |
| Metalloproteinase-Inhibitoren | Neovastat (Aeterna | CMT -3 (CollaGenex) |
| | Laboratories) | BMS-275291 (Celltech) |
| Ribonucleosidred | Marimastat (British | Tezacitabin (Aventis) |
| uktase-Inhibitoren | Biotech) | Didox (Molecules for |
| | Galliummaltolat (Titan) | Health) |
| | Triapin (Vion) | |
| | | |
| TNF-alpha-Agonisten / Antagonisten | Virulizin (Lorus | Revimid (Celgene) |
| | Therapeutics) | |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor-Agonisten | Fenretinid (Johnson & | Alitretinoin (Ligand) |
| | Johnson) | |
| | LGD-1550 (Ligand) | |
| | | |
| Immunmodulatoren | Interferon | Dexosom-Therapie |
| | Oncophage (Antigenics) | (Anosys) |
| | GMK (Progenics) | Pentrix (Australian Cancer |
| | Adenokarzinom-Impfstoff | Technology) |
| | (Biomira) | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe | MGV (Progenics) |
| | (CTL Immuno) | !3-Alethin (Dovetail) |
| | Melanom-Impfstoff (CTL | CLL-Thera (Vasogen) |
| | Immuno) | |
| | p21-RAS-Impfstoff | |
| | (GemVax) | |
| | | |
| Hormonelle und | Östrogene | Prednison |
| antihormonelle | konjugierte Östrogene | Methylprednisolon |
| Mittel | Ethinylöstradiol | Prednisolon |
| | Chlortrianisen | Aminoglutethimid |
| | Idenestrol | Leuprolid |
| | Hydroxyprogesteron- | Goserelin |
| | caproat | Leuporelin |
| | Medroxyprogesteron | Bicalutamid |
| | Testosteron | Flutamid |
| | Testosteronpropionat | Octreotid |
| | Fluoxymesteron | Nilutamid |
| | Methyltestosteron | Mitotan |
| | Diethylstilbestrol | P-04 (Novogen) |
| | Megestrol | 2-Methoxyöstradiol |
| | Tamoxifen | (EntreMed) |
| | Toremofin | Arzoxifen (Eli Lilly) |
| | Dexamethason | |
| | | |
| Photodynamische | Talaporfin (Light Sciences) | Pd-Bacteriopheophorbid |
| Mittel | Theralux | (Yeda) |
| | (Theratechnologies) | Lutetium-Texaphyrin |
| | Motexafin-Gadolinium | (Pharmacyclics) |
| | (Pharmacyclics) | Hypericin |
| | | |
| Tyrosinkinase-Inhibitoren | Imatinib (Novartis) | Kahalid F (PharmaMar) |
| | Leflunomid | CEP- 701 (Cephalon) |
| | (Sugen/Pharmacia) | CEP-751 (Cephalon) |
| | ZDI839 (AstraZeneca) | MLN518 (Millenium) |
| | Erlotinib (Oncogene | PKC412 (Novartis) |
| | Science) | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKl166 (Novartis) | IMC-1C11 (ImClone) |
| | GW2016 | |
| | (GlaxoSmithKline) | |
| | EKB-509 (Wyeth) | |
| | EKB-569 (Wyeth) | |
| Verschiedene | SR-27897 (CCK-A- | BCX-1777 (PNP-Inhibitor, |
| Mittel | Inhibitor, Sanofi- | BioCryst) |
| | Synthelabo) | Ranpirnase |
| | Tocladesin (cyclisches- | (Ribonuclease-Stimulans, |
| | AMP-Agonist, Ribapharm) | Alfacell) |
| | Alvocidib (CDK-Inhibitor, | Galarubicin (RNA- |
| | Aventis) | Synthese-Inhibitor, Dong- |
| | CV-247 (COX-2-lnhibitor, | A) |
| | Ivy Medical) | Tirapazamin |
| | P54 (COX-2-Inhibitor, | (Reduktionsmittel, SRI |
| | Phytopharm) | International) |
| | CapCell™ (CYP450- | N-Acetylcystein |
| | Stimulans, Bavarian | (Reduktionsmittel, |
| | Nordic) | Zambon) |
| | GCS-100 (gal3- | R-Flurbiprofen (NF- |
| | Antagonist, | kappaB-lnhibitor, Encore) |
| | GlycoGenesys) | 3CPA (NF-kappaB- |
| | G17DT-lmmunogen | Inhibitor, Active Biotech) |
| | (Gastrin-Inhibitor, Aphton) | Seocalcitol (Vitamin-D- |
| | Efaproxiral (Oxygenator, | Rezeptor-Agonist, Leo) |
| | Allos Therapeutics) | 131-I-TM-601 (DNA- |
| | PI-88 (Heparanase- | Antagonist, |
| | Inhibitor, Progen) | TransMolecular) |
| | Tesmilifen (Histamin- | Eflornithin (ODC-Inhibitor, |
| | Antagonist, YM | ILEX Oncology) |
| | BioSciences) | Minodronsäure |
| | Histamin (Histamin-H2- | (Osteoclasten-Inhibitor, |
| | Rezeptor- Agonist, Maxim) | Yamanouchi) |
| | Tiazofurin (IMPDH- | Indisulam (p53-Stimulans, |
| | Inhibitor, Ribapharm) | Eisai) |
| | Cilengitid (Integrin- | Aplidin (PPT-Inhibitor, |
| | Antagonist, Merck KGaA) | PharmaMar) |
| | SR-31747 (IL-1- | Rituximab (CD20- |
| | Antagonist, Sanofi- | Antikörper, Genentech) |
| | Synthelabo) | Gemtuzumab (CD33- |
| | CCI-779 (mTOR-Kinase- | Antikörper, Wyeth Ayerst) |
| | Inhibitor, Wyeth) | PG2 (Hämatopoese- |
| | Exisulind (PDE-V-Inhibitor, | Verstärker, |
| | Cell Pathways) | Pharmagenesis) |
| | CP-461 (PDE-V-Inhibitor, | Immunol™ (Triclosan- |
| | Cell Pathways) | Oralspülung, Endo) |
| | AG-2037 (GART-Inhibitor, | Triacetyluridin (Uridin- |
| | Pfizer) | Prodrug, Wellstat) |
| | WX-UK1 | SN-4071 (Sarkom-Mittel, |
| | (Plasminogenaktivator- | Signature BioScience) |
| | Inhibitor, Wilex) | TransMID-107™ |
| | PBI-1402 (PMN-Stimulans, | (Immunotoxin, KS |
| | ProMetic LifeSciences) | Biomedix) |
| | Bortezomib (Proteasom- | PCK-3145 (Apoptose- |
| | Inhibitor, Millennium) | Förderer, Procyon) |
| | SRL-172 (T-Zell- | Doranidazol (Apoptose- |
| | Stimulans, SR Pharma) | Förderer, Pola) |
| | TLK-286 (Glutathion-S- | CHS-828 (cytotoxisches |
| | Transferase-Inhibitor, | Mittel, Leo) |
| | Telik) | trans-Retinsäure |
| | PT-100 (Wachstumsfaktor- | (Differentiator, NIH) |
| | Agonist, Point | MX6 (Apoptose-Förderer, |
| | Therapeutics) | MAXIA) |
| | Midostaurin (PKC-Inhibitor, | Apomin (Apoptose- |
| | Novartis) | Förderer, ILEX Oncology) |
| | Bryostatin-1 (PKC- | Urocidin (Apoptose- |
| | Stimulans, GPC Biotech) | Förderer, Bioniche) |
| | CDA-II (Apoptose- | Ro-31-7453 (Apoptose- |
| | Förderer, Everlife) | Förderer, La Roche) |
| | SDX-101 (Apoptose- | Brostallicin (Apoptose- |
| | Förderer, Salmedix) | Förderer, Pharmacia) |
| | Ceflatonin (Apoptose- | |
| | Förderer, ChemGenex) | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel I wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Nati. Cancer Inst. 52:413-427; Nicosia et al., In Vitro 18:538- 549).

### Messung der Met Kinase Aktivität

Die Met Kinase wird laut Herstellerangaben (Met, active, Upstate, Katalog-Nr. 14-526) zum Zweck der Proteinproduktion in Insektenzellen (Sf21; S. frugiperda) und der anschließenden affinitätschromatographischen Aufreinigung als "N-terminal 6His-tagged" rekombinantes humanes Protein in einem Baculovirus-Expressionsvektor exprimiert.

Zur Messung der Kinase-Aktivität kann auf verschiedene zur Verfügung stehender Meßsysteme zurückgegriffen werden. Beim Scintillation-Proximity- (Sorg et al., J. of. Biomolecular Screening, 2002, 7, 11-19), dem FlashPlate-Verfahren oder dem Filterbindungstest wird die radioaktive Phosphorylierung eines Proteins oder Peptids als Substrat mit radioaktiv markiertem ATP (³²P-ATP, ³³P-ATP) gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-Antikörper bindet nur das phosphorylierte Substrat. Diese Bindung ist mit einem zweiten Peroxidase-konjugierten Antikörper durch Chemilumineszenz nachweisbar (Ross et al., 2002, Biochem. J.).

### Flashplate-Verfahren (Met Kinase):

Als Testplatten dienen 96-well Flashplate^{R} Mikrotiterplatten der Firma Perkin Elmer (Kat.-Nr. SMP200). In die Assay Platte werden die Komponenten der unten beschriebenen Kinasereaktion pipettiert. Die Met Kinase und das Substrat poly Ala-Glu-Lys-Tyr, (pAGLT, 6:2:5:1). werden mit radioaktiv markiertem ³³P-ATP in An- und Abwesenheit von Testsubstanzen in einem Gesamtvolumen von 100 µl bei Raumtemperatur 3 Std. inkubiert. Die Reaktion wird mit 150 µl einer 60mM EDTA-Lösung abgestoppt. Nach Inkubation für weitere 30 min bei Raumtemperatur werden die Überstände abgesaugt und die Wells dreimal mit je 200 µl 0,9% NaCl-Lösung gewaschen. Die Messung der gebundenen Radioaktivität erfolgt mittels eines Szintillationsmessgerätes (Topcount NXT, Fa. Perkin-Elmer).

Als Vollwert wird die Inhibitor-freie Kinasereaktion verwendet. Dieser sollte ca. im Bereich von 6000-9000 cpm liegen. Als pharmakologischer Nullwert wird Staurosporin in einer Endkonzentration von 0,1 mM verwendet. Eine Bestimmung der Hemmwerte (IC50) erfolgt unter Verwendung des Programms RS1_MTS ().

### Kinase-Reaktionsbedingungen pro well:

30 µl Assaypuffer
10 µl zu testende Substanz in Assaypuffer mit 10 % DMSO
10 µl ATP (Endkonzentration 1 µM kalt, 0,35 µCi ³³P-ATP)
50 µl Gemisch Met Kinase/Substrat in Assaypuffer;
   (10 ng Enzym/well, 50 ng pAGLT/well)

### Verwendete Lösungen:

- Assay-Puffer:
   50 mM HEPES
   3 mM Magnesiumchlorid
   3 µM Natrium orthovanadat
   3 mM Mangan (II) chlorid
   1 mM Dithiothreitol (DTT)
   pH= 7,5 (einzustellen mit Natriumhydroxid)
- Stopp-Lösung:
   60 mM Titriplex III (EDTA)
- ³³P-ATP: Perkin-Elmer;
- Met Kinase: Upstate, Kat.-Nr. 14-526, Stock 1 µg/10 µl; spez.

### Aktivität 954 U/mg;

- Poly-Ala-Glu-Lys-Tyr, 6:2:5:1: Sigma Kat.-Nr. P1152

### In vivo-Tests (FIG. 1/1)

Experimenteller Ablauf: Weibliche Balb/C Mäuse (Züchter: Charles River Wiga) waren bei der Ankunft im Alter von 5 Wochen. Sie wurden 7 Tage lang an unsere Haltungsbedingungen akklimatisiert. Anschließend wurden jeder Maus 4 Millionen TPR-Met / NIH3T3 - Zellen in 100 µl PBS (ohne Ca++ und Mg++) subkutan im Beckenbereich injiziert. Nach 5 Tagen wurden die Tiere in 3 Gruppen randomisiert, so dass jede Gruppe von 9 Mäusen ein mittleres Tumorvolumen von 110 µl (Spanne: 55 - 165) hatte. Der Kontrollgruppe wurden 100 µl Vehikel (0,25 % Methylzellulose / 100 mM Acetatpuffer, pH 5.5), den Behandlungsgruppen wurden 200 mg/kg "A56" bzw. "A91" gelöst im Vehikel (Volumen ebenfalls 100 µl / Tier) per Schlundsonde täglich verabreicht. Nach 9 Tagen hatten die Kontrollen ein mittleres Volumen von 1530 µl und der Versuch wurde beendet.
Messung des Tumorvolumens: Die Länge (L) und Breite (B) wurde mit einer Schubleere gemessen und das Tumorvolumen nach der Formel LxBxB/2 berechnet.
Haltungsbedingungen: je 4 bzw. 5 Tiere pro Käfig, Fütterung mit kommerziellem Mäusefutter (Fa. Sniff).

Die Verbindungen "A18" und "A22" weisen eine überzeugende antitumorale Wirkung auf.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS):
   EI (Elektronenstoß-Ionisation) M⁺
   FAB (Fast Atom Bombardment) (M+H)⁺
   ESI (Electrospray lonization) (M+H)⁺
   APCI-MS (atmospheric pressure chemical ionization - mass spectrometry) (M+H)⁺.
Massenspektrometrie
   (MS): EI (Elektronenstoß-Ionisation) M⁺
   FAB (Fast Atom Bombardment) (M+H)⁺
   ESI (Electrospray lonization) (M+H)⁺
   APCI-MS (atmospheric pressure chemical ionization - mass spectrometry) (M+H)⁺.

### HPLC/MS-Analysen

erfolgen auf einer Säule 3 µ Silica-Rod, mit einem 210-sekündlichen Gradienten von 20 bis 100% Wasser/Acetonitril / 0.01% Trifluoressigsäure, bei 2,2 ml/min Fluss, und Detektion bei 220 nm.

### HPLC-Analysen (Methode A)

Säule: Chromolith RP18e 100*3 mm
Fluß: 2 ml/min
Solvent A: H₂O + 0,1 % Trifluoressigsäure
Solvent B: Acetonitril + 0,1 % Trifluoressigsäure
Gradient 5 min
0-4 min: 99:1 -> 1:99
4-5 min: 1:99 - 1:99

### HPLC-Analysen (Methode B)

Säule: Chromolith RP18e 100*3 mm
Fluß: 4 ml/min
Solvent A: H₂O + 0,05 % HCOOH
Solvent B: Acetonitril + 10 % Solvent A
Gradient 8 min
0-1 Min: 99:1 -> 99:1
1-7 min: 99:1 - 1:99
7-8 min: 1:99 → 1:99

### HPLC-Analyse (Methode C)

Flußrate: 2 ml/min
99:01 - 0:100 Wasser + 0.1%(Vol.) TFA : Acetonitril + 0.1%(Vol.) TFA 0.0 bis 0.2 min: 99:01
0.2 bis 3.8 min: 99:01---> 0:100
3.8 bis 4.2 min: 0:100
Säule: Chromolith Performance RP18e; 100 mm lang, Innendurchmesser 3 mm, Wellenlänge: 220nm
Retentionszeit Rt. in Minuten [min].

### Beispiele zur Herstellung der Pyridazinon-Ausgangsverbindungen

Die Pyridazinone werden in der Regel nach Verfahren aus W. H. Coates, A. McKillop, Synthesis 1993, S. 334, hergestellt.

Exemplarisch hierfür ist die Synthese von 3-(6-Oxo-1,6-dihydropyridazin-3-yl)-benzonitril:

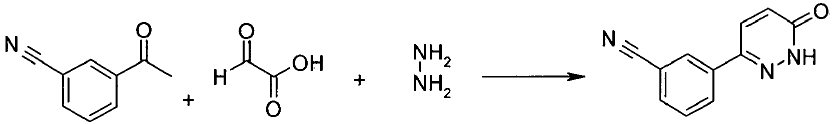

In eine Lösung von 1278 g (8.80 mol) 3-Acetylbenzonitril in 1.5 I Essigsäure wird portionsweise 927 g (10.6 mol) Glyoxylsäure-Monohydrat eingetragen. Die entstandene Lösung wird 18 Stunden auf 95° C erhitzt. Man lässt auf 30° C abkühlen und gibt nacheinander 7 I Wasser und 899 ml (18.5 mol) Hydraziniumhydroxid zu. Das Reaktionsgemisch wird 4 Stunden bei 95° C gerührt. Man lässt auf 60°C abkühlen, saugt den entstandenen Niederschlag ab und wäscht ihn mit 5 I Wasser und 2 l Aceton. Der Rückstand wird in 5 I Aceton zum Sieden erhitzt und heiß abgesaugt. Der Rückstand wird mit 5 I Essigsäure versetzt und 2 Stunden unter Rühren auf 90° C erhitzt. Man lässt auf Raumtemperatur abkühlen, saugt ab und wäscht mit Aceton. Der Rückstand wird nochmal mit 5 I Essigsäure auf 90° C erhitzt, auf Raumtemperatur gekühlt, abgesaugt und der Rückstand mit Aceton gewaschen. Der Rückstand wird im Vakuum getrocknet: 3-(6-Oxo-1,6-dihydro-pyridazin-3-yl)-benzonitril als beige Kristalle; ESI 198.

Einige Pyridazinone können in Anlehnung an A. J. Goodman et al, Tetrahedron 55 (1999), 15067-15070 hergestellt werden. Examplarisch hierfür ist die Alternativsynthese von 3-(6-Oxo-1,6-dihydro-pyridazin-3-yl)-benzonitril:

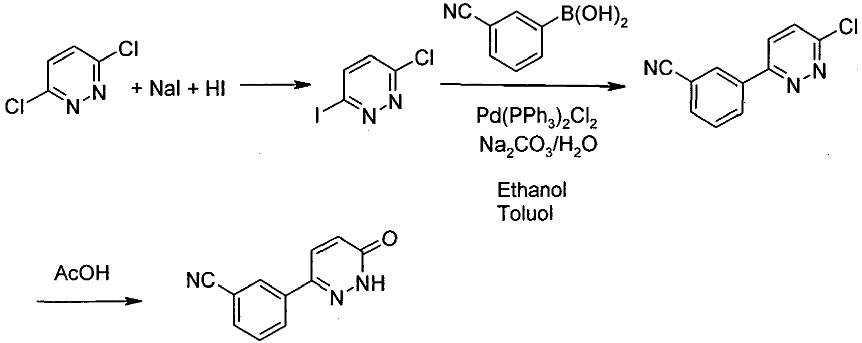

Zu einem Gemisch von 5.0 I Wasser und 11.3 I 57%iger wässriger lodwasserstoffsäure (75.2 mol) werden bei Raumtemperatur portionsweise 2.70 kg (18.0 mol) Natriumiodid gegeben. Anschließend werden zu der auf 20°C gehaltenen Lösung portionsweise 2.00 kg (13.4 mol) 3,6-Dichlorpyridazin gegeben. Das Reaktionsgemisch wird 18 Stunden bei 20° C gerührt. Das Reaktionsgemisch wird mit 10 I tert.-Butylmethylether und 4 I Wasser versetzt. Die organische Phase wird abgetrennt, mit Wasser und wässriger Natriumsulfit-Lösung gewaschen. Die organische Phase wird eingeengt, mit Heptan versetzt, der entstandene Feststoff abgesaugt und mit Heptan gewaschen. Der Rückstand wird im Vakuum getrocknet: 3-Chlor-6-iod-pyridazin als farblose blättchenförmige Kristalle; ESI 241.

Eine unter Stickstoff gehaltene Lösung von 240 mg (1.00 mmol) 3-Chlor-6-iod-pyridazin in 1 ml Toluol wird mit einer Lösung von 212 mg (2.0 mmol) Natriumcarbonat in 1 ml Wasser versetzt und das Gemisch auf 80° C erhitzt. Dazu werden 7.0 mg (0.010 mmol) Bis(triphenylphosphin)-palladium(II)-chlorid gegeben und anschließend eine Lösung von 147 mg (1.00 mmol) 3-Cyan-benzolboronsäure zugetropft. Das Reaktionsgemisch wird 18 Stunden bei 80° C gerührt. Das Reaktionsgemisch wird auf Raumtemperatur abgekühlt, mit Wasser versetzt, abgesaugt und mit Wasser gewaschen. Der Rückstand wird im Vakuum getrocknet: 3-(6-Chlorpyridazin-3-yl)-benzonitril als farblose Kristalle; ESI 216.

Eine Suspension von 85 mg (0.396 mol) 3-(6-Chlorpyridazin-3-yl)-benzonitril in 0.5 ml Essigsäure wird auf 80° C erhitzt und 24 Stunden bei dieser Temperatur gerührt. Das Reaktionsgemisch wird auf Raumtemperatur abgekühlt, mit Wasser versetzt und abgesaugt. Der Rückstand wird mit Wasser gewaschen und im Vakuum getrocknet: 3-(6-Oxo-1,6-dihydro-pyridazin-3-yl)-benzonitril als farblose Kristalle.

Nach diesem Verfahren werden vorzugsweise folgende Pyridazinone hergestellt:

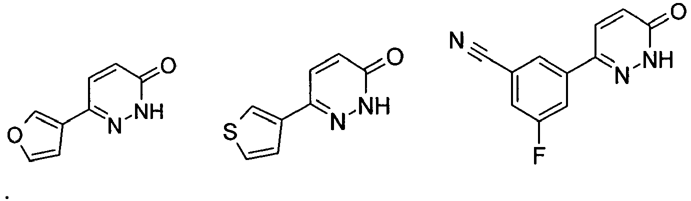

Einige Pyridazinone werden nach folgendem Verfahren hergestellt. Examplarisch hierfür ist die Synthese von 6-(1-Methyl-1 H-pyrazol-4-yl)-2H-pyridazin-3-on:

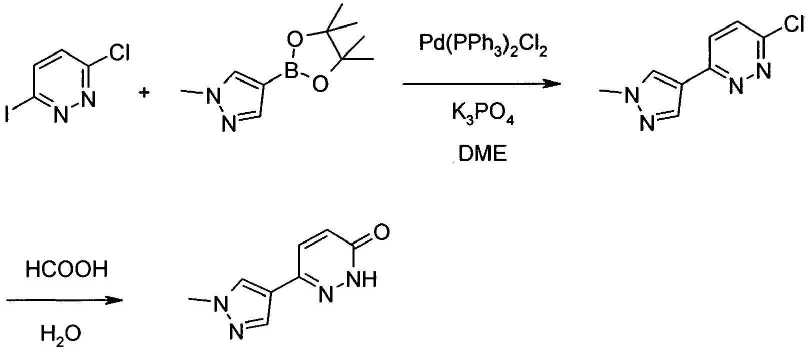

Eine Lösung von 815 g (3.39 mol) 3-Chlor-6-iod-pyridazin in 3.8 l 1,2-Dimethoxyethan wird mit 705 g (3.39 mol) 1-Methyl-1 H-pyrazol-4-boronsäure-pinacolester und 1.44 kg Trikaliumphosphat-Trihydrat versetzt. Die entstandene Suspension wird unter Stickstoff und unter Rühren auf 80° C erhitzt und 59.5 g (85 mmol) Bis(triphenylphosphin)-palladium(II)-chlorid zugegeben. Das Reaktionsgemisch wird 3 Stunden bei 80° C gerührt. Man lässt auf Raumtemperatur abkühlen und gibt 9 I Wasser hinzu. Der entstandene Niederschlag wird abgesaugt, mit Wasser gewaschen und im Vakuum getrocknet: 3-Chlor-6-(1-methyl-1 H-pyrazol-4-yl)-pyridazin als braune Kristalle; ESI 195.

Eine Suspension von 615 g (2.90 mol) 3-Chlor-6-(1-methyl-1 H-pyrazol-4-yl)-pyridazin in einem Gemisch aus 1.86 I Ameisensäure und 2.61 l Wasser wird unter Rühren auf 80° C erhitzt und 28 Stunden bei dieser Temperatur gerührt. Das Reaktionsgemisch wird auf Raumtemperatur abgekühlt, mit etwas Aktivkohle versetzt und abgesaugt. Das Filtrat wird unter Eiskühlung mit 40%iger wässriger Natronlauge auf einen pH-Wert von 7 gebracht und 16 h bei 6° C belassen. Der entstandene Niederschlag wird abgesaugt, mit Wasser gewaschen und im Vakuum getrocknet: 6-(1-Methyl-1 H-pyrazol-4-yl)-2H-pyridazin-3-on als farblose Kristalle; ESI 177.

Nach diesem Verfahren werden vorzugsweise folgende Pyridazinone hergestellt:

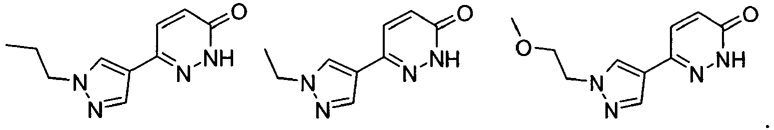

Herstellung von 6-(5-Methyloxazol-2-yl)-2H-pyridazin-3-on:

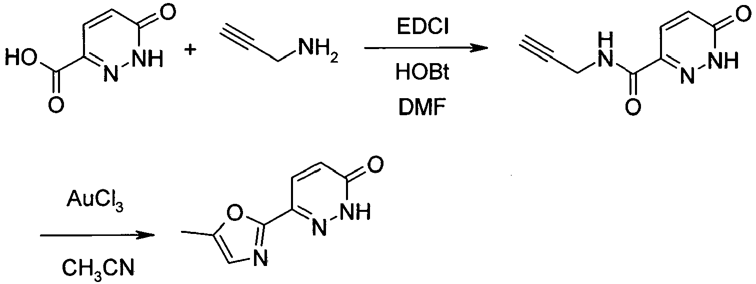

Eine Lösung von 10.0 g (69.2 mmol) 6-Oxo-1,6-dihydro-pyridazin-3-carbonsäure-Monohydrat und 3.85 g (69.2 mmol) Propargylamin in 200 ml DMF wird mit 10.6 g (69.2 mmol) 1-Hydroxybenztriazol-Hydrat und 17.3 g N-(3-Dimethylaminopropyl)-N'-ethylcarbodiimid-hydrochlorid versetzt und die resultierende Lösung 18 Stunden bei Raumtemperatur gerührt. Das Reaktionsgemisch wird zwischen Wasser und Dichlormethan verteilt. Die organische Phase wird mit gesättiger Natriumhydrogencarbonat-Lösung gewaschen, über Natriumsulfat getrocknet und eingedampft: 6-Oxo-1,6-dihydro-pyridazin-3-carbonsäure-prop-2-inylamid als farblose Kristalle; ESI 178.

Eine Lösung von 3.69 g (20.5 mmol) 6-Oxo-1,6-dihydro-pyridazin-3-carbonsäure-prop-2-inylamid in 41 ml Acetonitril wird mit 622 mg (2.05 mmol) Gold(III)chlorid versetzt und 3 Tage bei Raumtemperatur gerührt. Es werden weitere 622 mg (2.05 mmol) Gold(III)chlorid zugegeben und 7 Tage bei Raumtemperatur gerührt. Das Reaktionsgemisch wird eingedampft und an einer Kieselgelsäule mit Dichlormethan/Methanol als Laufmittel chromatographiert: 6-(5-Methyloxazol-2-yl)-2H-pyridazin-3-on als gelbliche Kristalle; ESI 178.

Herstellung von 6-[4-(5-Methyl-[1,2,4]oxadiazol-3-yl)-phenyl]-2H-pyridazin-3-on:

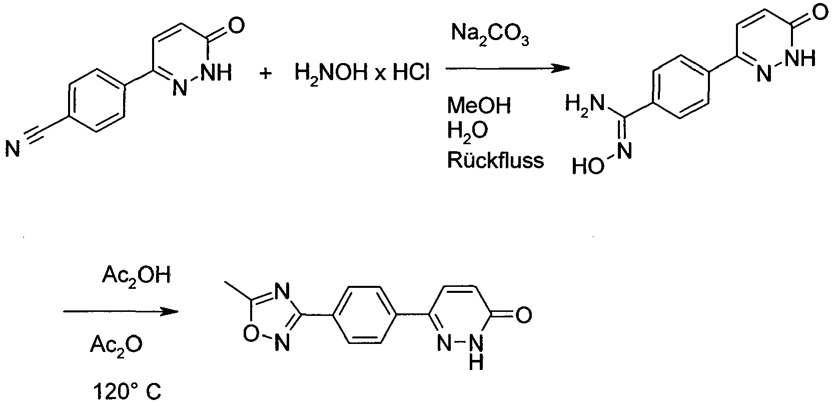

Herstellung von 4-(6-Oxo-1,6-dihydro-pyridazin-3-yl)-piperazin-1-carbonsäure-tert.-butylester:

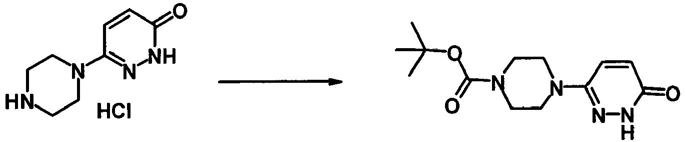

1 g (4.62 mmol) 6-Piperazin-1-yl-2H-pyridazin-3-on Hydrochlorid (Eur. J. Med. Chem. 1992, 27, 545-549) werden in 10 ml THF suspendiert und mit 1.34 ml (9.69 mmol) Triethylamin und 1.09 ml (5.08 mmol) Di-*tert.-*butyldicarbonat versetzt. Es wird 15 h bei RT gerührt und das Lösungsmittel entfernt. Der Rückstand wird mit Ethylacetat und Wasser versetzt. Ein weißer Feststoff bleibt ungelöst. Der Rückstand wird abgesaugt und mit Wasser und Ethylacetat gewaschen und im Vakuum getrocknet: Ausbeute 0.9 g; HPLC: Rt. = 2.27 min (Methode B); HPLC-MS: 281 (M+H).

### Herstellung von 6-(5-Methyl-[1,2,4]oxadiazol-3-yl)-2H-pyridazin-3-on

1. 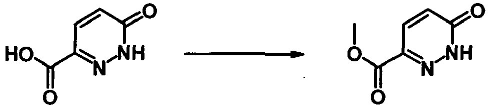
   20 g (125 mmol) 6-Oxo-1,6-dihydropyridazine-3-carbonsäure Hydrat werden in 400 ml Methanol suspendiert und unter Eiskühlung langsam mit 10.7 ml (147 mmol) Thionylchlorid versetzt. Die Suspension wird 15 h bei 70°C gerührt, wobei sich alles löst. Das Reaktionsgemisch wird auf ca. 100 ml eingeengt, wobei ein weißer Niederschlag ausfällt. Dieser Niederschlag wird abgesaugt und mit Methanol gewaschen und im Vakuum getrocknet. Ausbeute 19.2 g; HPLC: Rt. = 1.27 min (Methode B); HPLC-MS: 155 (M+H).
2. 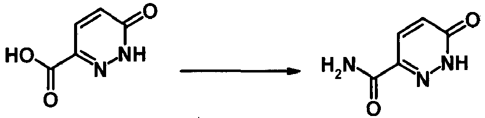
   19.27 g (125 mmol) 6-Oxo-1,6-dihydro-pyridazine-3-carbonsäure-methylester werden in 300 ml ammoniakalischem Methanol gelöst und 16 h bei Raumtemperatur gerührt. Das Lösungsmittel wird entfernt und der Rückstand ohne weitere Aufarbeitung weiter umgesetzt; Ausbeute 16.5 g.
3. 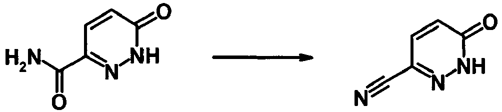
   15 g (108 mmol) 6-Oxo-1,6-dihydro-pyridazine-3-carbonsäure-amid werden in 200 ml Dichlormethan suspendiert. Die Suspension wird auf 0°C eingekühlt und anschließend werden 45 ml Pyridin sowie 18 ml (129 mmol) Trifluoressigsäureanhydrid zugetropft. Das Gemisch rührt man 5 Tage bei RT. Die Suspension wird mit 400ml Wasser versetzt und mit 3 x 300 ml DCM extrahiert. Die vereinigten organischen Phasen werden mit Natriumsulfat getrocknet und zum Rückstand eingeengt. Es bildet sich ein Niederschlag im Filtrat. Dieser Niederschlag wird abgesaugt, mit Wasser gewaschen und im Vakuum getrocknet. Die wässrige Phase wird mit Natriumchlorid gesättigt und mit 3 x 300 ml Ethylacetat extrahiert. Die organische Phase wird getrocknet und eingeengt. Alle 3 Fraktionen werden vereinigt und ohne weitere Aufreinigung weiter umgesetzt; Ausbeute: 14.3 g; GC-MS: 121 (M⁺).
4. 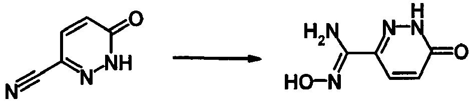
   1 g (8.26 mmol) 6-Oxo-1,6-dihydro-pyridazin-3-carbonitril und 2.87 g (41.3 mmol) Hydroxylammoniumchlorid werden in 200 ml Ethanol suspendiert und mit 5.7 ml (41.3 mmol) Triethylamin versetzt. Das Reaktionsgemisch wird 5 Tage bei Raumtemperatur gerührt. Das Lösungsmittel wird entfernt und der Rückstand wird mit Wasser verrührt, filtriert und getrocknet; Ausbeute: 754 mg, rotbrauner Feststoff; LC-MS: 155 (M+H).
5. 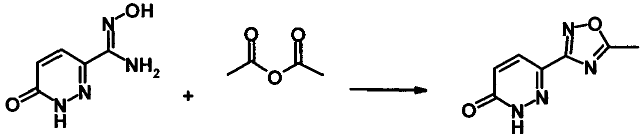
   375 mg (2.43 mmol) N-Hydroxy-6-oxo-1,6-dihydro-pyridazin-3-carboxamidin werden mit 2.8 ml Eisessig, 2.3 ml Essigsäureanhydrid und 200 µl Pyridin versetzt und 15 h bei 90 °C gerührt. Beim Abkühlen des Reaktionsgemisches fälltl ein Niederschlag aus, der abgesaugt, mit Wasser gewaschen und im Vakuum getrocknet wird; Ausbeute: 253 mg, gelber Feststoff; HPLC: Rt. = 1.51 min; LC-MS: 179 (M+H).

### Beispiel 1 (Vergleich)

Die Herstellung von 2-[3-(5-Methylpyrimidin-2-yl)-benzyl]-6-(3,4,5-trifluorphenyl)-2H-pyridazin-3-on ("A1") erfolgt analog nachstehendem Schema 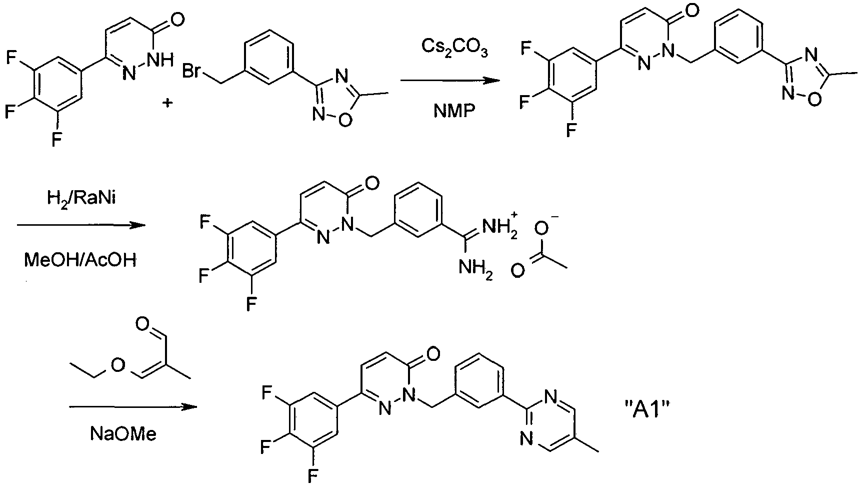
1.1 Eine Lösung von 4.52 g (20 mmol) 6-(3,4,5-Trifluorphenyl)-2H-pyridazin-3-on und 5.06 g (20 mmol) 3-(3-Brommethyl-phenyl)-5-methyl-[1,2,4]oxadiazol (hergestellt nach W. W. K. R. Mederski et al, Tetrahedron 55, 1999, 12757-12770) in 40 ml 1-Methylpyrrolidinon (NMP) wird mit 6.52 g (20 mmol) Caesiumcarbonat versetzt und die entstandene Suspension 18 Stunden bei Raumtemperatur gerührt. Das Reaktionsgemisch wird mit Wasser versetzt, der entstandene Niederschlag abfiltriert, mit Wasser gewaschen und getrocknet. Das Rohprodukt wird aus 2-Propanol umkristallisiert: 6-(3,4,5-Trifluorphenyl)-2-[3-(5-methyl-[1,2,4]oxadiazol-3-yl)-benzyl]-2H-pyridazin-3-on als leicht gelbliche Kristalle; ESI 399.
1.2 Eine Lösung von 6.00 g (14.9 mmol) 6-(3,4,5-Trifluorphenyl)-2-[3-(5-methyl-[1,2,4]oxadiazol-3-yl)-benzyl]-2H-pyridazin-3-on in 60 ml Methanol wird mit 2 ml Essigsäure, 2 ml Wasser und 6 g Raney-Nickel versetzt und 44 Stunden bei Raumtemperatur und Normaldruck hydriert. Das Reaktionsgemisch wird filtriert und das Filtrat eingedampft. Der kristalline Rückstand wird in tert.-Butyl-methylether aufgekocht. Man lässt abkühlen, saugt ab und wäscht mit tert.-Butylmethylether. Der Rückstand wird im Vakuum getrocknet: und abkühlen lassen. 3-[6-Oxo-3-(3,4,5-trifluorphenyl)-6H-pyridazin-1-ylmethyl]-benzamidinium-Acetat als farblose Kristalle; ESI 359.
   Analog wird hergestellt 3-[6-Oxo-3-(3,5-difluorphenyl)-6H-pyridazin-1-ylmethyl]-benzamidinium-Acetat, farblose Kristalle; ESI 341.
1.3 Eine Suspension von 4.18 g (10.0 mmol) 3-[6-Oxo-3-(3,4,5-trifluorphenyl)-6H-pyridazin-1-ylmethyl]-benzamidinium-Acetat in 40 ml Methanol wird mit 1.31 ml (11.0 mmol) 3-Ethoxymethacrolein und 2.04 ml (11.0 mmol) einer 30%igen Natriummethanolat-Lösung in Methanol versetzt und 18 Stunden auf 50° C erhitzt. Man lässt abkühlen, saugt den entstandenen Niederschlag ab, wäscht ihn mit Methanol und trocknet ihn im Vakuum: 2-[3-(5-Methylpyrimidin-2-yl)-benzyl]-6-(3,4,5-trifluorphenyl)-2H-pyridazin-3-on ("A1") als farblose Kristalle; ESI 409;
   ¹H-NMR (DMSO-d₆): δ [ppm] = 2.32 (s, 3H), 5.45 (s, 2H), 7.16 (d, J = 9.5 Hz, 1H), 7.52 (m, 2H), 7.90 (m, 2H), 8.13 (d, J = 9.5 Hz, 1H), 8.30 (dt, J₁ 7.5 Hz, J₂ = 1.5 Hz, 1H), 8.46 (t, J = 1. 5 Hz, 1H), 8.75 (s, 2H).

Durch analoge Umsetzung des Benzamidinium-Acetats mit 4-Trimethylsilyl-3-butin-1-on mit Kaliumcarbonat/Acetonitril bei 120°C in der Mikrowelle erhält man die nachstehenden Verbindungen
6-(3,5-Difluorphenyl)-2-[3-(5-methyl-pyrimidin-2-yl)-benzyl]-2H-pyridazin-3-on ("A2"), ESI 391;
2-[3-(4-Methyl-pyrimidin-2-yl)-benzyl]-6-(3,4,5-trifluor-phenyl)-2H-pyridazin-3-on ("A3"), ESI 409.

Durch durch Erhitzen des Benzamidinium-Acetats mit Malondialdehyd-bisdimethylacetal auf 175° C erhält man in analoger Weise die Verbindung
2-(3-Pyrimidin-2-yl-benzyl)-6-(3,4,5-trifluor-phenyl)-2H-pyridazin-3-on ("A4"), ESI 395.

### Beispiel 2 (Vergleich)

Die Herstellung von 4-{3-[3-(3,5-Difluor-phenyl)-6-oxo-6H-pyridazin-1-ylmethyl]-phenyl}-morpholin-3-on ("A5") erfolgt analog nachstehendem Schema 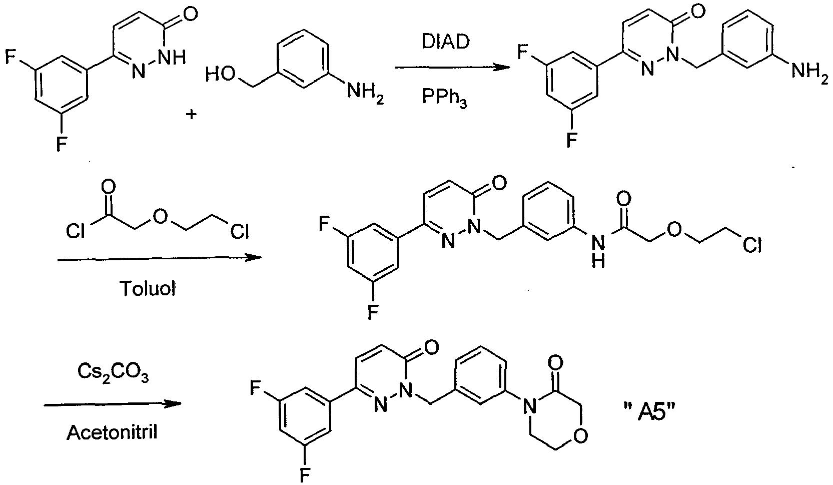
2.1 Eine unter Stickstoff gehaltene Suspension von 3.12 g (15.0 mmol) 6-(3,5-Difluorphenyl)-2H-pyridazin-3-on in 80 ml THF wird mit 2.83 g (22.5 mmol) 3-Aminobenzylalkohol und 5.96 g (22.5 mmol) Triphenylphosphin versetzt und 30 Minuten bei Raumtemperatur gerührt. Die Suspension wird auf 0° C abgekühlt und 4.65 ml (22.5 mmol) Diisopropylazodicarboxylat (DIAD) zugetropft. Das Reaktionsgemisch wird 18 Stunden bei Raumtemperatur gerührt. Das Reaktionsgemisch wird eingedampft und der Rückstand in 50 ml Isopropanol erhitzt und abkühlen lassen. Der entstandene Niederschlag wird abgesaugt, mit Isopropanol und tert.-Butylmethylether gewaschen und im Vakuum getrocknet: 2-(3-Aminobenzyl)-6-(3,5-difluorphenyl)-2H-pyridazin-3-on als farblose Kristalle; ESI 314.
2.2 Eine Suspension 313 mg (1.00 mmol) von 2-(3-Aminobenzyl)-6-(3,5-difluorphenyl)-2H-pyridazin-3-on in 2 ml Toluol wird mit 235 mg (1.5 mmol) (2-Chlorethoxy)-acetylchlorid versetzt und 18 Stunden zum Sieden erhitzt. Man lässt abkühlen, saugt den entstandenen Niederschlag ab, wäscht ihn mit tert.-Butylmethylether und trocknet ihn im Vakuum: 2-(2-Chlorethoxy)-N-{3-[3-(3,5-difluorphenyl)-6-oxo-6H-pyridazin-1-ylmethyl]-phenyl}-acetamid als farblose Kristalle; ESI 434.
2.3 Eine Lösung von 339 mg (0.78 mmol) 2-(2-Chlorethoxy)-N-{3-[3-(3,5-difluorphenyl)-6-oxo-6H-pyridazin-1-ylmethyl]-phenyl}-acetamid in 2 ml Acetonitril wird mit 509 mg (1.56 mmol) Caesiumcarbonat versetzt und 18 Stunden bei Raumtemperatur gerührt. Das Reaktionsgemisch wird filtriert und das Filtrat eingedampft. Der Rückstand wird in tert.-Butyl-methylether aufgenommen, abgesaugt und mit tert.-Butylmethylether gewaschen: 4-{3-[3-(3,5-Difluorphenyl)-6-oxo-6H-pyridazin-1-ylmethyl]-phenyl}-morpholin-3-on ("A5") als farblose Kristalle; ESI 398.

Durch analoge Umsetzung der Anilinderivate mit 3-Chlorpropylchlorformiat erhält man die nachstehenden Verbindungen
3-{3-[3-(3,5-Difluor-phenyl)-6-oxo-6H-pyridazin-1-ylmethyl]-phenyl}-[1,3]oxazinan-2-on 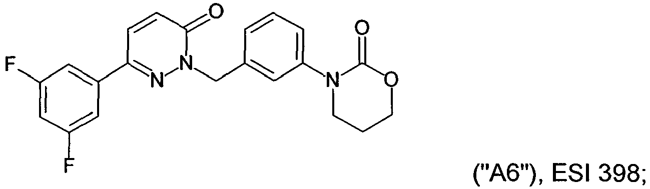 3-{3-[6-Oxo-3-(3,4,5-trifluor-phenyl)-6H-pyridazin-1-ylmethyl]-phenyl}-[1,3]oxazinan-2-on ("A7"), ESI 416.

### Beispiel 3 (Vergleich)

Die Herstellung von 1-{3-[3-(3,5-Difluorphenyl)-6-oxo-6H-pyridazin-1-ylmethyl]-phenyl}-3-methyl-6H-pyridazin-6-on ("A8") erfolgt analog nachstehendem Schema 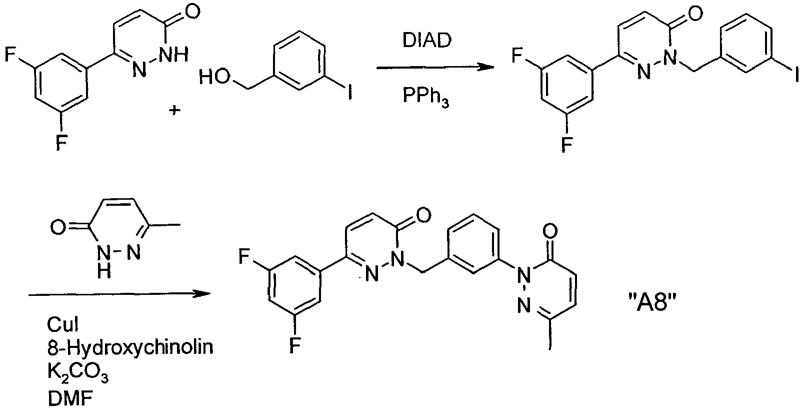
3.1 Eine unter Stickstoff gehaltene Suspension von 2.92 g (14.0 mmol) 6-(3,5-Difluorphenyl)-2H-pyridazin-3-on in 100 ml THF wird mit 5.03 g (21.1 mmol) 3-Iodbenzylalkohol und 5.55 g (20.9 mmol) Triphenylphosphin versetzt und 30 Minuten bei Raumtemperatur gerührt. Die Suspension wird auf 0° C abgekühlt und 4.33 ml (20.9 mmol) Diisopropylazodicarboxylat zugetropft. Das Reaktionsgemisch wird 1.5 Stunden bei Raumtemperatur gerührt. Das Reaktionsgemisch wird eingedampft und der Rückstand in 50 ml Isopropanol erhitzt und abkühlen lassen. Der entstandene Niederschlag wird abgesaugt, mit Isopropanol und Petrolether gewaschen und im Vakuum getrocknet: 6-(3,5-Difluorphenyl)-2-(3-iodbenzyl)-2H-pyridazin-3-on als farblose Kristalle; ESI 425.
3.2 Eine Lösung von 212 mg (0.50 mmol) 6-(3,5-Difluorphenyl)-2-(3-iodbenzyl)-2H-pyridazin-3-on und 55.1 mg (0.5 mmol) 6-Methylpyridazin-3(2H)-on in 2 ml DMF wird mit 14.3 mg (0.08 mmol) Kupfer(I)-iodid, 76 mg (0.55 mmol) Kaliumcarbonat und 11 mg (0.08 mmol) 8-Hydroxychinolin versetzt und 24 Stunden auf 120° C erhitzt. Man lässt das Reaktionsgemisch abkühlen, versetzt es mit 10%iger wässriger Ammoniaklösung und Ethylacetat. Der entstandene Niederschlag wird abgesaugt, mit Wasser gewaschen und getrocknet. Der Rückstand wird in Ethylacetat aufgekocht, abgesaugt und mit Ethylacetat gewaschen. Der Rückstand wird im Vakuum getrocknet: 1-{3-[3-(3,5-Difluorphenyl)-6-oxo-6H-pyridazin-1-ylmethyl]-phenyl}-3-methyl-6H-pyridazin-6-on ("A8") als bräunliche Kristalle; ESI 407.

### Beispiel 4 (Vergleich)

Die Herstellung von 6-(3,5-Difluorphenyl)-2-[3-(5-methylpyridin-2-yl)-benzyl]-2H-pyridazin-3-on ("A9") erfolgt analog nachstehendem Schema 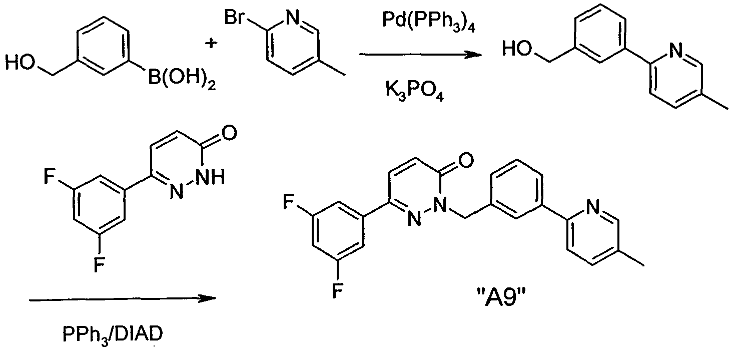
4.1 Zu einer unter Stickstoff gehaltenen Suspension von 849 mg (4.0 mmol) Trikaliumphosphat, 344 mg (2.0 mmol) 2-Brom-5-methylpyridin und 304 mg (2.0 mmol) 3-Hydroxymethylbenzolboronsäure in 12 ml Dioxan und 1 ml Wasser werden 92 mg (0.08 mmol) Tetrakis(triphenylphosphin)-palladium gegeben und das Gemisch 18 Stunden unter Rühren zum Sieden erhitzt. Das Reaktionsgemisch wird auf Raumtemperatur abgekühlt und zwischen Wasser und Ethylacetat verteilt. Die organische Phase wird über Natriumsulfat getrocknet, eingedampft und der Rückstand an einer Kieselgelsäule mit Dichlormethan/Methanol als Laufmittel chromatographiert: [3-(5-Methyl-pyridin-2-yl)-phenyl]-methanol als gelbliches Öl; ESI 200.
4.2 Eine Lösung von 88 mg (0.44 mmol) [3-(5-Methyl-pyridin-2-yl)-phenyl]-methanol, 138 mg (0.66 mmol) 6-(3,5-Difluorphenyl)-2H-pyridazin-3-on und 174 mg (0.66 mmol) Triphenylphosphin in 3.5 ml THF wird mit 134 mg (0.66 mmol) Diisopropylazodicarboxylat versetzt. Das Reaktionsgemisch wird 18 Stunden bei Raumtemperatur gerührt. Es wird eingedampft und der Rückstand an einer Kieselgelsäule mit Dichlormethan / Methanol als Laufmittel chromatographiert: 6-(3,5-Difluorphenyl)-2-[3-(5-methylpyridin-2-yl)-benzyl]-2H-pyridazin-3-on ("A9") als farblose Kristalle; ESI 390.

### Analog erhält man die Verbindungen

6-(3,5-Difluor-phenyl)-2-[3-(5-methoxy-pyridin-2-yl)-benzyl]-2H-pyridazin-3-on

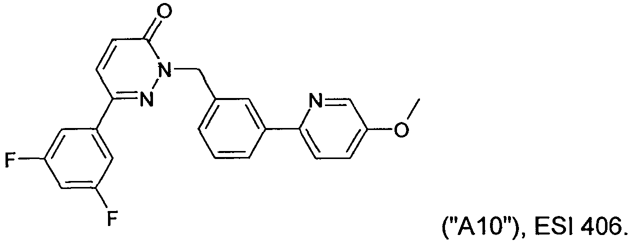

### Beispiel 5

Die Herstellung von 2-[3-(5-Aminopyridin-2-yl)-benzyl]-6-(3,5-difluorphenyl)-2H-pyridazin-3-on ("A11") und von 6-(3,5-Difluor-phenyl)-2-{3-[5-(4-methyl-piperazin-1-yl)-pyridin-2-yl]-benzyl}-2H-pyridazin-3-on ("A12") erfolgt analog nachstehendem Schema 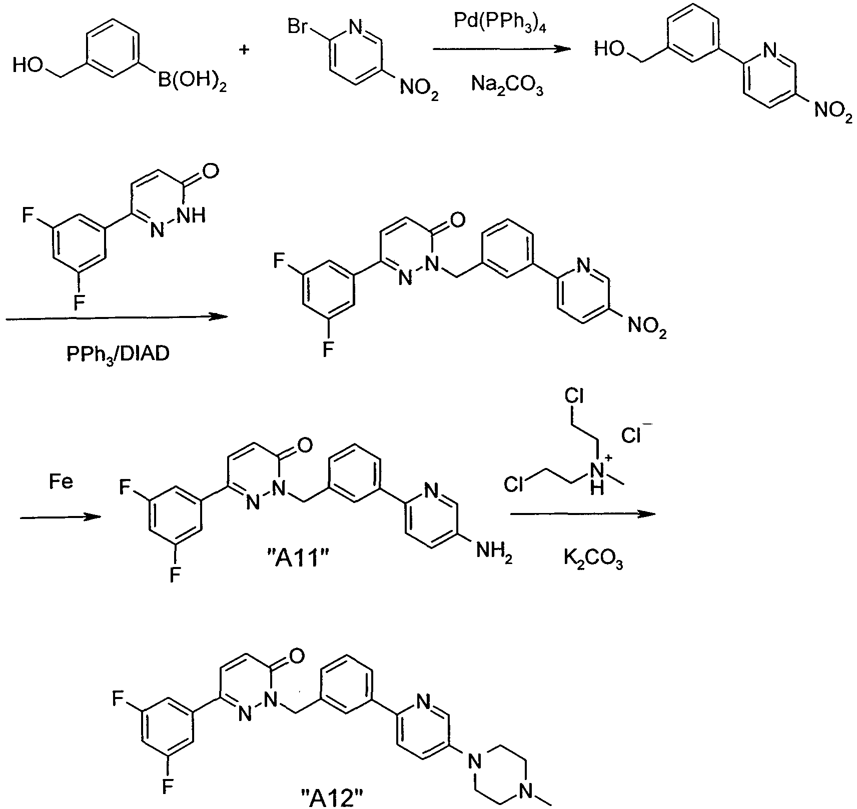
5.1 Eine unter Stickstoff gehaltene Suspension von 3.69 g (18.2 mmol) 2-Brom-5-nitropyridin, 840 mg (0.73 mmol) Tetrakis(triphenylphosphin)-palladium und 3.55 g (33.4 mmol) Natriumcarbonat in 133 ml Toluol wird zum Sieden erhitzt. Dann wird eine Lösung von 5.07 g (32.7 mmol) 3-(Hydroxymethyl)-benzolboronsäure in 133 ml Toluol zugetropft und das Reaktionsgemisch 18 Stunden zum Sieden erhitzt. Das Reaktionsgemisch wird mit Wasser versetzt. Die organische Phase wird abgetrennt und die wässrige mehrmals mit Toluol extrahiert. Die vereinigten organischen Phasen werden über Natriumsulfat getrocknet und eingedampft. Der Rückstand wird an einer Kieselgelsäule mit Dichlormethan/Methanol chromatographiert: [3-(5-Nitropyridin-2-yl)-phenyl]-methanol als gelbe Kristalle; ESI 231.
5.2 Zu einer Lösung von 3.37 g (14.7 mmol) [3-(5-Nitropyridin-2-yl)-phenyl]-methanol, 4.58 g (22.0 mmol) 6-(3,5-Difluorphenyl)-2H-pyridazin-3-on und 5.77 g (22.0 mmol) Triphenylphosphin in 120 ml THF werden 4.46 g (22.0 mmol) Diisopropylazodicarboxylat zugetropft und das Reaktionsgemisch 18 Stunden bei Raumtemperatur gerührt. Der entstandene Niederschlag wird abgesaugt, mit THF gewaschen und im Vakuum getrocknet: 6-(3,5-Difluorphenyl)-2-[3-(5-nitro-pyridin-2-yl)-benzyl]-2H-pyridazin-3-on als gelbliche Kristalle; ESI 421.
5.3 Eine Suspension von 420 mg (1.00 mmol) 6-(3,5-Difluorphenyl)-2-[3-(5-nitro-pyridin-2-yl)-benzyl]-2H-pyridazin-3-on in 4 ml Ethanol wird mit 220 µl 2N Salzsäure versetzt, auf 95° C erhitzt und auf Raumtemperatur gekühlt. 402 mg (7.2 mmol) Eisenpulver wird zugegeben und das Reaktionsgemisch 1 Stunde bei 85° C und 17 Stunden bei 60° C gerührt. Das Reaktionsgemisch wird filtriert und das Filtrat zwischen Wasser und Ethylacetat verteilt. Die organische Phase wird nacheinander mit Natriumhydrogencarbonat-Lösung, Natriumcarbonat-Lösung und NatriumchloridLösung gewaschen, über Natriumsulfat getrocknet und eingedampft: 2-[3-(5-Aminopyridin-2-yl)-benzyl]-6-(3,5-difluorphenyl)-2H-pyridazin-3-on ("A11") als bräunlicher Schaum; ESI 391.
5.4 Die letzte Stufe wird analog Beispiel 9.3 durchgeführt. Man erhält 6-(3,5-Difluor-phenyl)-2-{3-[5-(4-methyl-piperazin-1-yl)-pyridin-2-yl]-benzyl}-2H-pyridazin-3-on ("A12").

### Beispiel 6

Die Herstellung von 6-(3,5-Difluorphenyl)-2-[3-(4-piperazin-1-yl-pyrimidin-2-yl)-benzyl]-2H-pyridazin-3-on ("A13") erfolgt analog nachstehendem Schema 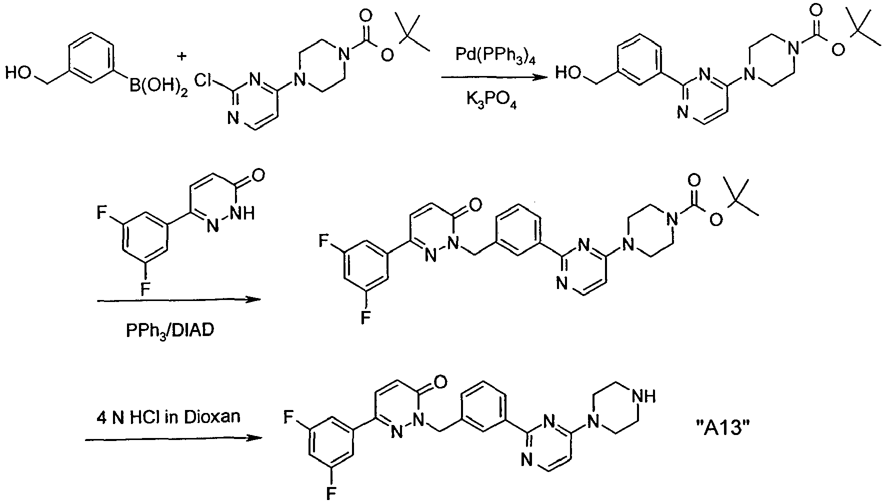
6.1 Zu einer unter Stickstoff gehaltenen Suspension von 849 mg (4.0 mmol) Trikaliumphosphat, 598 mg (2.0 mmol) 4-(2-Chlorpyrimidin-4-yl)-piperazin-1-carbonsäure-tert.-butylester (hergestellt nach WO03/104225) und 304 mg (2.0 mmol) 3-Hydroxymethylbenzolboronsäure in 12 ml Dioxan und 1 ml Wasser wird eine Katalysatorlösung, die durch Umsetzung von von 56 mg (0.08 mmol) Bis(triphenylphosphin)-palladium(II)-chlorid und 3.0 mg (0.08 mmol) Natriumborhydrid in 0.4 ml THF bei 55° C hergestellt wurde, zugegeben. Das Reaktionsgemisch wird 18 Stunden bei 97° C gerührt. Das Reaktionsgemisch wird abgekühlt und zwischen Wasser und Ethylacetat verteilt. Die organische Phase wird über Natriumsulfat getrocknet, eingedampft und der Rückstand an einer Kieselgelsäule mit Dichlormethan/Methanol als Laufmittel chromatographiert: 4-[2-(3-Hydroxymethylphenyl)-pyrimidin-4-yl]-piperazin-1-carbonsäure-tert-butylester als gelblicher Feststoff; ESI 371.
6.2 Eine Lösung von 144 mg (0.388 mmol) 4-[2-(3-Hydroxymethyl-phenyl)-pyrimidin-4-yl]-piperazin-1-carbonsäure-tert.-butylester, 122 mg (0.582 mmol) 6-(3,5-Difluorphenyl)-2H-pyridazin-3-on und 153 mg (0.582 mmol) Triphenylphosphin in 3 ml THF wird mit 118 mg (0.582 mmol) Diisopropylazodicarboxylat versetzt. Das Reaktionsgemisch wird 18 Stunden bei Raumtemperatur gerührt. Es wird eingedampft und der Rückstand an einer Kieselgelsäule mit Dichlormethan/Methanol als Laufmittel chromatographiert: 4-(2-{3-[3-(3,5-Difluor-phenyl)-6-oxo-6H-pyridazin-1-ylmethyl]-phenyl}-pyrimidin-4-yl)-piperazin-1-carbonsäure-tert.-butylester als gelbliches Öl; ESI 561.
6.3 Eine Lösung von 81 mg (0.14 mmol) 4-(2-{3-[3-(3,5-Difluor-phenyl)-6-oxo-6H-pyridazin-1-ylmethyl]-phenyl}-pyrimidin-4-yl)-piperazin-1-carbonsäure-tert.-butylester in 1 ml Dioxan wird mit 1.3 ml 4 N HCl in Dioxan versetzt und 18 Stunden bei Raumtemperatur belassen. Das Reaktionsgemisch wird zwischen Wasser und Ethylacetat verteilt. Die wässrige Phase wird mit 1 N NaOH auf einen pH von 14 gebracht und mit Ethylacetat extrahiert. Die organische Phase wird über Natriumsulfat getrocknet und eingedampft: 6-(3,5-Difluorphenyl)-2-[3-(4-piperazin-1-yl-pyrimidin-2-yl)-benzyl]-2H-pyridazin-3-on("A13") Hydrochlorid als farbloser amorpher Feststoff; ESI 461.

### Analog wird nachstehende Verbindung erhalten

6-(3,5-Difluorphenyl)-2-{3-[4-(methyl-piperidin-4-yl-amino)-pyrimidin-2-yl]-benzyl}-2H-pyridazin-3-on

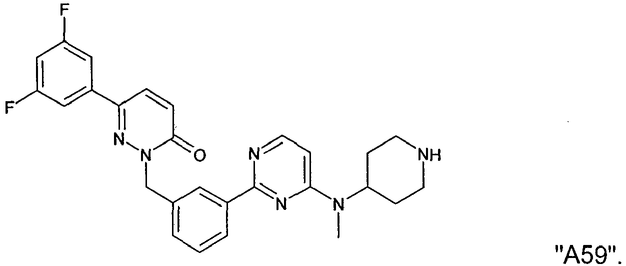

### Beispiel 7

Die Herstellung von 6-(3,5-Difluorphenyl)-2-{3-[5-(4-methylpiperazin-1-ylmethyl)-pyrimidin-2-yl]-benzyl}-2H-pyridazin-3-on ("A14") erfolgt analog nachstehendem Schema 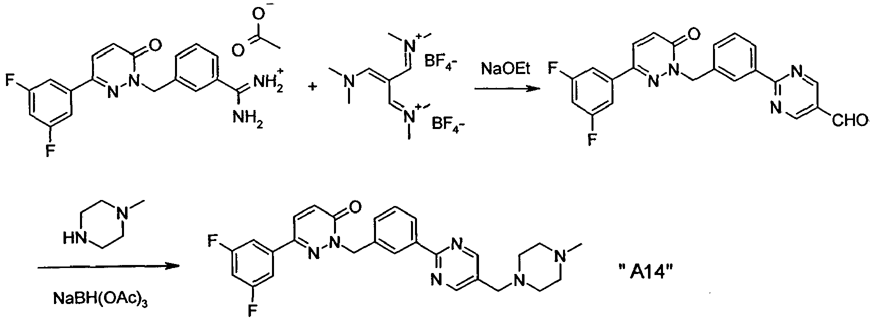
7.1 Zu einer unter Stickstoff gehaltenen Suspension von 4.00 g (10.0 mmol) 3-[6-Oxo-3-(3,5-difluorphenyl)-6H-pyridazin-1-ylmethyl]-benzamidinium-Acetat und 4.64 g (13.0 mmol) 2-Dimethylaminomethylen-1,3-bis-(dimethylimmonio)-propan-bistetrafluoroborat (hergestellt nach P. J. Coleman et al., J. Med. Chem. 2004, 47, 4829-4837) in 280 ml Ethanol werden 12.0 ml (31.5 mmol) einer 20%igen Lösung von Natriumethanolat in Ethanol gegeben und 2 Stunden zum Sieden erhitzt. Das Reaktionsgemisch wird abgekühlt, im Vakuum eingedampft und mit Wasser digeriert. Der entstandene Niederschlag wird abgesaugt und mit Wasser gewaschen. Der Rückstand wird an einer Kieselgelsäule mit Dichlormethan / Methanol chromatographiert: 2-{3-[3-(3,5-Difluorphenyl)-6-oxo-6H-pyridazin-1-ylmethyl]-phenyl}-pyrimidin-5-carbaldehyd als farblose Kristalle; ESI 405.
7.2 Eine Suspension von 472 mg (1.17 mmol) 2-{3-[3-(3,5-Difluorphenyl)-6-oxo-6H-pyridazin-1-ylmethyl]-phenyl}-pyrimidin-5-carbaldehyd in 5 ml Dichlormethan wird nacheinander mit 166 µl 1-Methylpiperazin, 495 mg (2.34 mmol) Natriumtriacetoxyborhydrid und 67 µl Essigsäure gegeben und das Reaktionsgemisch 42 Stunden bei Raumtemperatur gerührt. Das Reaktionsgemisch wird zwischen Dichlormethan und 1 N NaOH verteilt. Die organische Phase wird abgetrennt, über Natriumsulfat getrocknet und eingedampft. Der Rückstand wird an einer Kieselgelsäule mit Dichlormethan / Methanol als Laufmittel chromatographiert: 6-(3,5-Difluorphenyl)-2-{3-[5-(4-methylpiperazin-1-ylmethyl)-pyrimidin-2-yl]-benzyl}-2H-pyridazin-3-on ("A14") als farblose Kristalle; ESI 489;
   ¹H-NMR (CDCl₃): δ [ppm] = 2.29 (s, 3H), 2.48 (m, 8H), 3.54 (s, 2H), 5.50 (s, 2H), 6.86 (tt, J₁ = 8.8 Hz, J₂ = 2.3 Hz, 1H), 7.04 (d, J = 9.5 Hz, 1H), 7.34 (m, 2H), 7.47 (t, J = 7.8 Hz, 1H), 7.58 (d, J = 9.5 Hz, 1H), 7.58 (m, 1H), 8.38 (dt, J₁ = 7.8 Hz, J₂ = 1 Hz, 1 H), 8.64 (t, J = 1 Hz, 1 H), 8.74 (s, 2H).

### Analog erhält man nachstehende Verbindung

6-(3,5-Difluor-phenyl)-2-[3-(5-dimethylaminomethyl-pyrimidin-2-yl)-benzyl]-2H-pyridazin-3-on

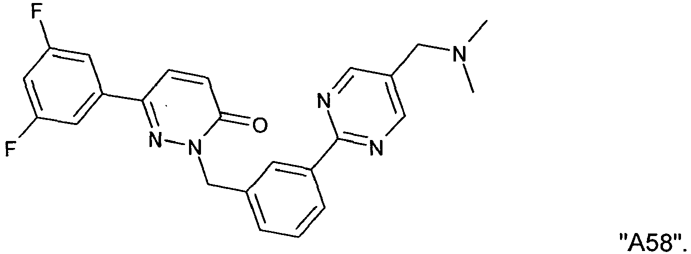

### Beispiel 8 (Vergleich)

Die Herstellung von 3-{1-[3-(5-Methylpyrimidin-2-yl)-benzyl]-6-oxo-1,6-dihydro-pyridazin-3-yl}-benzonitril ("A15") erfolgt analog nachstehendem Schema 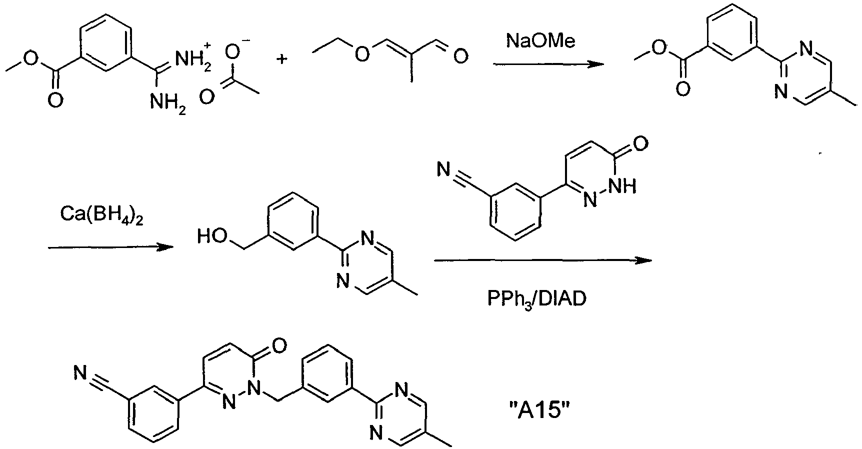
8.1 Eine Suspension von 2.41 g (10.0 mmol) 3-Carbamimidoylbenzoesäuremethylester-Acetat (Herstellung siehe Beispiel 37) in 40 ml Methanol wird mit 1.31 ml (11.0 mmol) 3-Ethoxymethacrolein und 2.04 ml (11.0 mmol) einer 30%igen Lösung von Natriumethanolat in Methanol versetzt und die resultierende Lösung 18 Stunden bei 50° C gerührt. Das Reaktionsgemisch wird im Vakuum eingeengt und mit Wasser versetzt. Der entstandene Niederschlag wird abgesaugt, mit Wasser gewaschen und im Vakuum getrocknet: 3-(5-Methylpyrimidin-2-yl)-benzoesäure-methylester als farblose Kristalle; ESI 229.
8.2 Zu einer Suspension von 400 mg (10.6 mmol) Natriumborhydrid in 20 ml THF werden 600 mg (5.41 mmol) gepulvertes Calciumchlorid gegeben und das Gemisch 1.5 Stunden bei Raumtemperatur gerührt. Zu dieser Suspension wird unter Rühren eine Lösung von 751 mg (3.29 mmol) 3-(5-Methylpyrimidin-2-yl)-benzoesäuremethylester in 10 ml THF zugetropft und 18 Stunden bei Raumtemperatur gerührt. Das Reaktionsgemisch wird 10 ml 1 N NaOH, Wasser und Dichlormethan versetzt und filtriert. Die organische Phase des Filtrats wird abgetrennt, über Natriumsulfat getrocknet und eingedampft. Der Rückstand wird an einer Kieselgelsäule mit Dichlormethan/Methanol als Laufmittel chromatographiert: [3-(5-Methylpyrimidin-2-yl)-phenyl]-methanol als farbloser Feststoff; ESI 201.
8.3 Zu einer Suspension von 98.6 mg (0.50 mmol) 3-(6-Oxo-1,6-dihydro-pyridazin-3-yl)-benzonitril, 100 mg (0.50 mmol) [3-(5-Methylpyrimidin-2-yl)-phenyl]-methanol und 197 mg (0.75 mmol) Triphenylphosphin in 3 ml THF werden 147 µl (0.75 mmol) Diisopropyldiazodicarboxylat zugetropft und die resultierende Lösung 18 Stunden bei Raumtemperatur gerührt. Das Reaktionsgemisch wird im Vakuum eingedampft und der Rückstand mit 2-Propanol versetzt. Der entstandene Niederschlag wird abgesaugt und an einer Kieselgelsäule mit Dichlormethan / Methanol als Laufmittel chromatographiert: 3-{1-[3-(5-Methylpyrimidin-2-yl)-benzyl]-6-oxo-1,6-dihydro-pyridazin-3-yl}-benzonitril ("A15") als gelblicher Feststoff; ESI 380; ¹H-NMR (DMSO-d₆): δ [ppm] = 2.31 (s, 3H), 5.46 (s, 2H), 7.16 (d, J = 9.7 Hz, 1 H), 7.51 (m, 2H), 7.72 (t, J = 8.0 Hz, 1 H), 7.93 (dt, J₁ = 7.5 Hz, J₂ = 1 Hz, 1H), 8.17 (d, J = 9.7 Hz, 1H), 8.25 (dt, J₁ = 7.8 Hz, J₂ = 1 Hz, 1H), 8.30 (dt, J₁ = 6.8 Hz, J₂ = 1.6 Hz, 1 H), 8.37 (t, J = 1.6 Hz, 1 H), 8.46 (bs, 1 H), 8.75 (s, 2H).

### Analog erhält man die nachstehenden Verbindungen

6-Benzo[1,2,5]thiadiazol-5-yl-2-[3-(5-methyl-pyrimidin-2-yl)-benzyl]-2H-pyridazin-3-on, ESI 413,

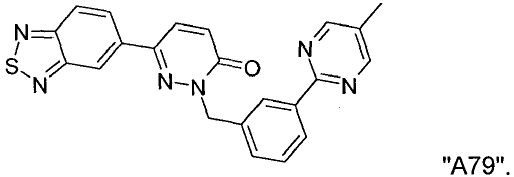

### Beispiel 9

Die Herstellung von
N'-(2-{3-[3-(3,5-Difluorphenyl)-6-oxo-6H-pyridazin-1-ylmethyl]-phenyl}-pyrimidin-5-yl)-N,N-dimethyl-formamidin ("A16"),
2-[3-(5-Aminopyrimidin-2-yl)-benzyl]-6-(3,5-difluorphenyl)-2H-pyridazin-3-on ("A17") und
6-(3,5-Difluorphenyl)-2-{3-[5-(4-methylpiperazin-1-yl)-pyrimidin-2-yl]-benzyl}-2H-pyridazin-3-on ("A18")
erfolgt analog nachstehendem Schema 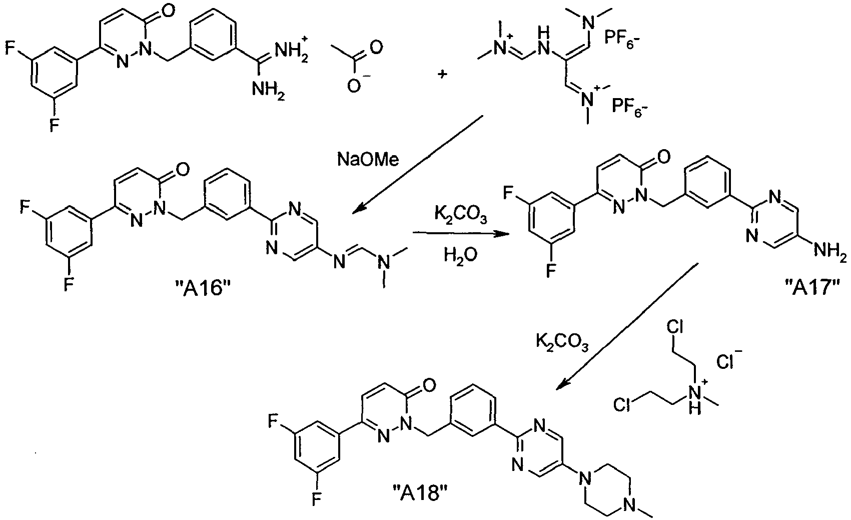
9.1 Zu einer unter Stickstoff gehaltenen Suspension von 20.0 g (50.0 mmol) 3-[6-Oxo-3-(3,5-difluorphenyl)-6H-pyridazin-1-ylmethyl]-benzamidinium-Acetat und 24.4 g (50.0 mmol) ({2-Dimethylamino-1-[dimethylimmoniomethyl]-vinylamino)-methylene)-dimethyl-ammonium-di-hexafluorophosphat in 20 ml Methanol wird eine Natriummethanolat-Lösung, die durch Auflösen von 3.45 g (150 mmol) Natrium in 35 ml Methanol hergestellt wurde, zugetropft. Das Reaktionsgemisch wird langsam auf 60° C erwärmt und 20 Minuten bei dieser Temperatur gerührt. Das Reaktionsgemisch wird auf Raumtemperatur abgekühlt und zwischen Wasser und Dichlormethan verteilt. Die organische Phase wird über Natriumsulfat getrocknet und eingedampft. Der Rückstand wird in Methanol aufgenommen, abgesaugt, der Rückstand mit Ether gewaschen und im Vakuum getrocknet: N'-(2-{3-[3-(3,5-Difluorphenyl)-6-oxo-6H-pyridazin-1-ylmethyl]-phenyl}-pyrimidin-5-yl)-N,N-dimethyl-formamidin ("A16") als farblose Kristalle; ESI 447.
9.2 Zu einer Lösung von 19.1 g (137 mmol) Kaliumcarbonat in 380 ml Wasser werden 190 ml Dioxan und 17.4 g (39.0 mmol) N'-(2-{3-[3-(3,5-Difluorphenyl)-6-oxo-6H-pyridazin-1-ylmethyl]-phenyl}-pyrimidin-5-yl)-N,N-dimethyl-formamidin gegeben. Das Reaktionsgemisch wird 3 Tage zum Sieden erhitzt und anschließend auf Raumtemperatur abgekühlt. Der entstandene Niederschlag wird abgesaugt, mit Wasser gewaschen und im Vakuum getrocknet: 2-[3-(5-Aminopyrimidin-2-yl)-benzyl]-6-(3,5-difluorphenyl)-2H-pyridazin-3-on ("A17") als farblose Kristalle; ESI 392.
9.3 Eine unter Stickstoff gehaltene Lösung von 587 mg (1.5 mmol) 2-[3-(5-Amino-pyrimidin-2-yl)-benzyl]-6-(3,5-difluorphenyl)-2H-pyridazin-3-on in 2 ml 1-Methylpyrrolidon wird mit 501 mg (2.55 mmol) Bis-(2-chlorethyl)-methyl-ammonium-chlorid versetzt und das Reaktionsgemisch 32 Stunden auf 130° C erhitzt. Das Reaktionsgemisch wird abgekühlt, mit Dichlormethan versetzt und filtriert. Das Filtrat wird im Vakuum eingedampft und der Rückstand an einer Kieselgelsäule mit Dichlormethan/Methanol chromatographiert. Die Produkt enthaltenden Fraktionen werden vereinigt, eingedampft und der Rückstand aus Methanol umkristallisiert. Dieses Material wird in Methanol suspendiert, mit Chlorwasserstoff in Diethylether ins Hydrochlorid überführt und das Hydrochlorid mit Diethylether ausgefällt: 6-(3,5-Difluorphenyl)-2-{3-[5-(4-methylpiperazin-1-yl)-pyrimidin-2-yl]-benzyl}-2H-pyridazin-3-on ("A18") Hydrochlorid als farblose Kristalle; ESI 475;
   ¹H-NMR (DMSO-d₆): δ [ppm] = 2.81 (d, J = 3.3 Hz, 3H), 3.19 (m 2H), 3.30 (m, 2H), 3.50 (m, 2H), 4.05 (m, 2H), 5.43 (s, 2H), 7.14 (d, J = 9.5 Hz, 1H), 7.35 (tt, J₁ = 8.8 Hz, J₂ = 2.3 Hz, 1 H), 7.47 (m, 2H), 7.66 (m, 2H), 8.15 (d, J = 9.5 Hz, 1 H), 8.22 (m, 1 H) 8.34 (bs,1 H), 8.65 (s, 2H), 11.0 (bs, 1 H).

### Analog werden die nachstehenden Verbindungen erhalten

6-(3,5-Difluor-phenyl)-2-[3-(5-piperazin-1-yl-pyrimidin-2-yl)-benzyl]-2H-pyridazin-3-on ("A19") Hydrochlorid, ESI 461,
¹H-NMR (d₆-DMSO): δ [ppm] = 3.25 (m, 4H), 3.59 (m, 4H), 5.44 (s, 2H), 7.16 (d, J = 10 Hz, 1H), 7.37 (tt, J₁ = 9.2 Hz, J₂ = 2 Hz, 1H), 7.47 (m, 2H), 7.67 (m, 2H), 8.16 (d, J = 10 Hz, 1H), 8.22 (m, 1H), 8.35 (bs, 1H), 8.65 (s, 2H), 9.38 (bs, 2H);
2-{3-[5-(4-Methyl-piperazin-1-yl)-pyrimidin-2-yl]-benzyl}-6-(3,4,5-trifluor-phenyl)-2H-pyridazin-3-on ("A20"), Hydrochlorid, ESI 493;
2-{3-[5-(Piperazin-1-yl)-pyrimidin-2-yl]-benzyl}-6-(3,4,5-trifluorphenyl)-2H-pyridazin-3-on ("A65");
N'-(2-{3-[3-(3,4,5-Trifluorphenyl)-6-oxo-6H-pyridazin-1-ylmethyl]-phenyl}-pyrimidin-5-yl)-N,N-dimethyl-formamidin ("A76"), ESI 465;
2-[3-(5-Aminopyrimidin-2-yl)-benzyl]-6-(3,4,5-trifluorphenyl)-2H-pyridazin-3-on ("A82"), ESI 410.

### Beispiel 10

Die Herstellung von
6-(3,5-Difluorphenyl)-2-[3-(5-hydroxypyrimidin-2-yl)-benzyl]-2H-pyridazin-3-on ("A21") und 6-(3,5-Difluorphenyl)-2-{3-[5-(3-dimethylamino-propoxy)-pyrimidin-2-yl]-benzyl}-2H-pyridazin-3-on ("A22")
erfolgt analog nachstehendem Schema 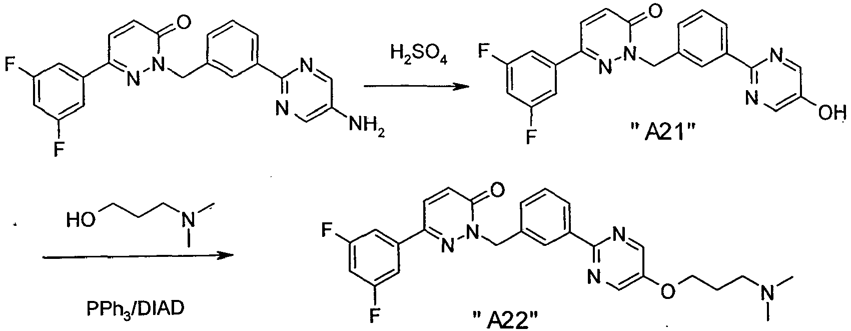
10.1 Eine Suspension von 4.76 g (12.2 mmol) 2-[3-(5-Aminopyrimidin-2-yl)-benzyl]-6-(3,5-difluorphenyl)-2H-pyridazin-3-on in einem Gemisch von 5.40 ml konzentrierter Schwefelsäure und 44 ml Wasser wird 4 Stunden zum Sieden erhitzt. Das Reaktionsgemisch wird auf Raumtemperatur abgekühlt, mit eiskaltem Wasser verdünnt und mit konz. wässrigem Ammoniak alkalisiert. Der Niederschlag wird abgesaugt, mit Wasser gewaschen und getrocknet. Das Rohprodukt wird aus Methanol umkristallisiert: 6-(3,5-Difluorphenyl)-2-[3-(5-hydroxypyrimidin-2-yl)-benzyl]-2H-pyridazin-3-on ("A21") als farblose Kristalle; ESI 393.
10.2 Eine unter Stickstoff gehaltene Suspension von 215 mg (0.55 mmol) 6-(3,5-Difluorphenyl)-2-[3-(5-hydroxypyrimidin-2-yl)-benzyl]-2H-pyridazin-3-on in 5 ml THF wird mit 98.3 µl (0.82 mmol) 3-(Dimethylamino)-1-propanol, 218 mg (0.82 mmol) Triphenylphosphin versetzt und im Eisbad gekühlt. 170 µl (0.82 mmol) Diisopropylazodicarboxlat werden zugetropft und das Reaktionsgemisch 2 Stunden bei Raumtemperatur gerührt. Das Reaktionsgemisch wird im Vakuum eingedampft und der Rückstand an einer Kieselgelsäule mit Dichlormethan/Methanol als Laufmittel chromatographiert. Die Produkt enthaltenden Fraktionen werden vereinigt und eingedampft. Dieses Material wird in Aceton gelöst, mit Chlorwasserstoff in Diethylether ins Hydrochlorid überführt und das Hydrochlorid mit Diethylether ausgefällt: 6-(3,5-Difluorphenyl)-2-{3-[5-(3-dimethylamino-propoxy)-pyrimidin-2-yl]-benzyl}-2H-pyridazin-3-on ("A22") Hydrochlorid als farblose Kristalle; ESI 478;
   ¹H-NMR (DMSO-d₆): δ [ppm] = 2.21 (m, 2H), 2.78 (d, J = 5 Hz, 6H), 3.22 (m 2H), 4.31 (t, J = 6 Hz, 2H), 5.44 (s, 2H), 7.14 (d, J = 9.5 Hz, 1 H), 7.35 (tt, J₁ = 8.8 Hz, J₂ = 2.3 Hz, 1H), 7.49 (m, 2H), 7.66 (m, 2H), 8.15 (d, J = 9.5 Hz, 1 H), 8.24 (m, 1 H) 8.38 (bs,1 H), 8.65 (s, 2H), 10.7 (bs, 1 H).

### Analog werden folgende Verbindung erhalten

6-(3,5-Difluor-phenyl)-2-{3-[5-(1-methyl-piperidin-4-yloxy)-pyrimidin-2-yl]-benzyl}-2H-pyridazin-3-on

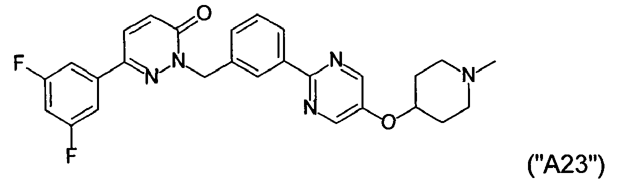

Hydrochlorid, ESI 490;
6-(3,5-Difluor-phenyl)-2-{3-[5-(3-pyrrolidin-1-yl-propoxy)-pyrimidin-2-yl]-benzyl}-2H-pyridazin-3-on 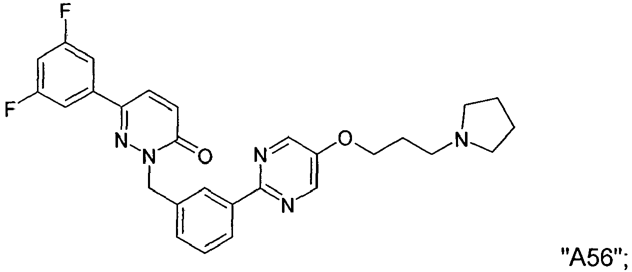 6-(3,5-Difluor-phenyl)-2-(3-{5-[2-(4-methyl-piperazin-1-yl)-ethoxy]-pyrimidin-2-yl}-benzyl)-2H-pyridazin-3-on, Hydrochlorid, 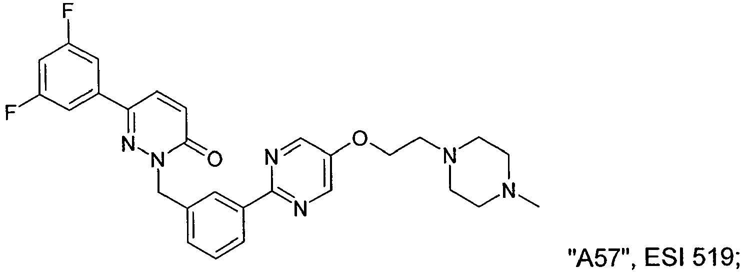 2-{3-[5-(3-Dimethylamino-propoxy)-pyrimidin-2-yl]-benzyl}-6-(3,4,5-trifluor-phenyl)-2H-pyridazin-3-on 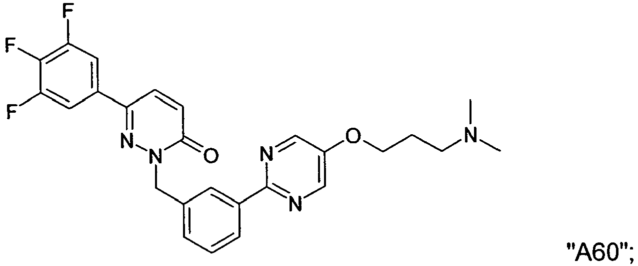 2-{3-[5-(2-Dimethylamino-ethoxy)-pyrimidin-2-yl]-benzyl}-6-(3,5-difluor-phenyl)-2H-pyridazin-3-on ("A64") Hydrochlorid, ESI 464;
6-(3,5-Difluor-phenyl)-2-{3-[5-(1-methyl-piperidin-4-ylmethoxy)-pyrimidin-2-yl]-benzyl}-2H-pyridazin-3-on, ESI 504, 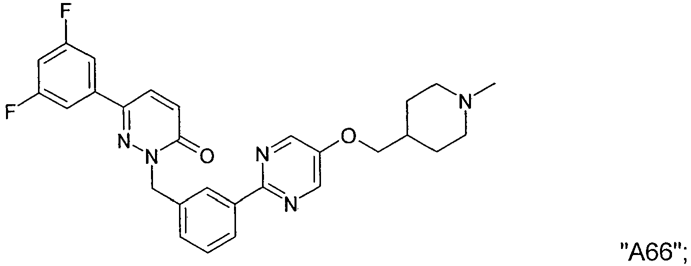 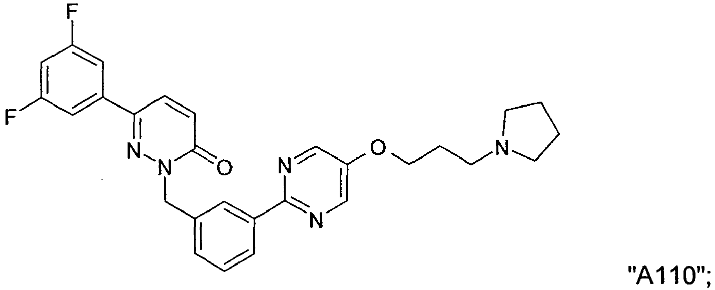 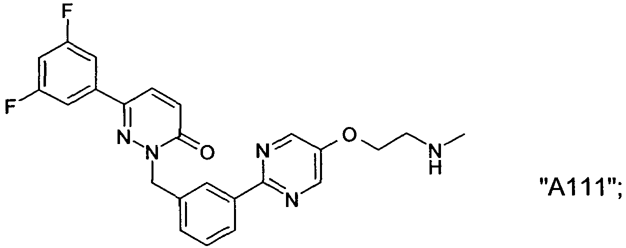 Hydrochlorid, ESI 450 (Vorstufe BOC-geschützte Verbindung);
6-(3,5-Difluor-phenyl)-2-{3-[5-(piperidin-4-yloxy)-pyrimidin-2-yl]-benzyl}-2H-pyridazin-3-on Hydrochlorid, ESI 476 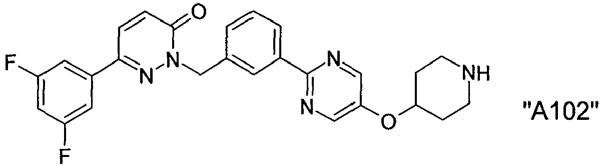 (Vorstufe BOC-geschützte Verbindung),
¹H-NMR-Spektrum von "A102" Hydrochlorid:
¹H-NMR (d₆-DMSO): δ [ppm] = 1.94 (m, 2H), 2.19 (m, 2H), 3.08 (m, 2H), 3.26 (m, 2H), 4.89 (m, 1H), 5.44 (s, 2H), 7.15 (d, J = 10 Hz, 1H), 7.36 (tt, J₁ = 9.2 Hz, J₂ = 2 Hz, 1H), 7.50 (m, 2H), 7.66 (m, 2H), 8.16 (d, J = 10 Hz, 1 H), 8.24 (m, 1 H), 8.37 (bs, 1 H), 8.71 (s, 2H), 9.11 (bs, 1 H), 9.19 (bs, 1H).

### Beispiel 11

Die Herstellung von 6-(3,5-Difluor-phenyl)-2-{3-[5-(3-dimethylamino-propoxy)-pyridin-2-yl]-benzyl}-2H-pyridazin-3-on ("A24"), ESI 477, erfolgt analog nachstehendem Schema

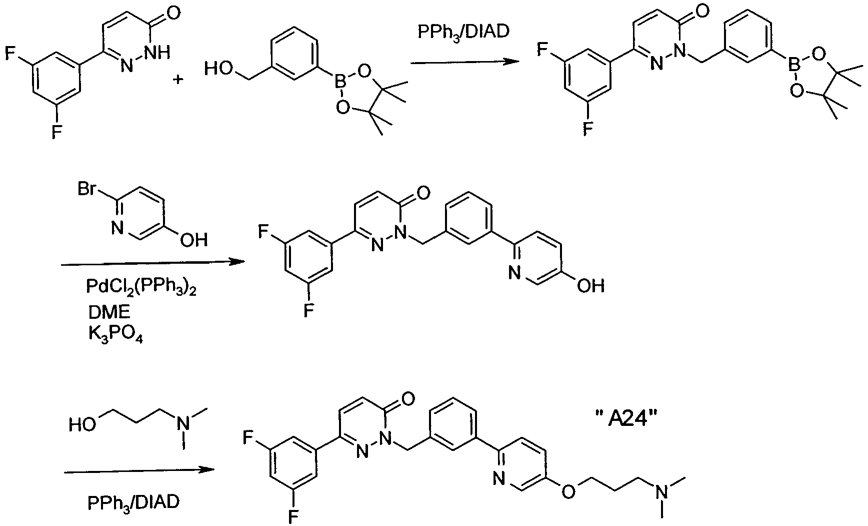

### Beispiel 12

Die Herstellung von 2-[3-(5-Brompyrimidin-2-yl)-benzyl]-6-(3,5-difluorphenyl)-2H-pyridazin-3-on ("A25") und 6-(3,5-Difluorphenyl)-2-{3-[5-(1-methyl-1H-pyrazol-4-yl)-pyrimidin-2-yl]-benzyl}-2H-pyridazin-3-on ("A26") erfolgt analog nachstehendem Schema 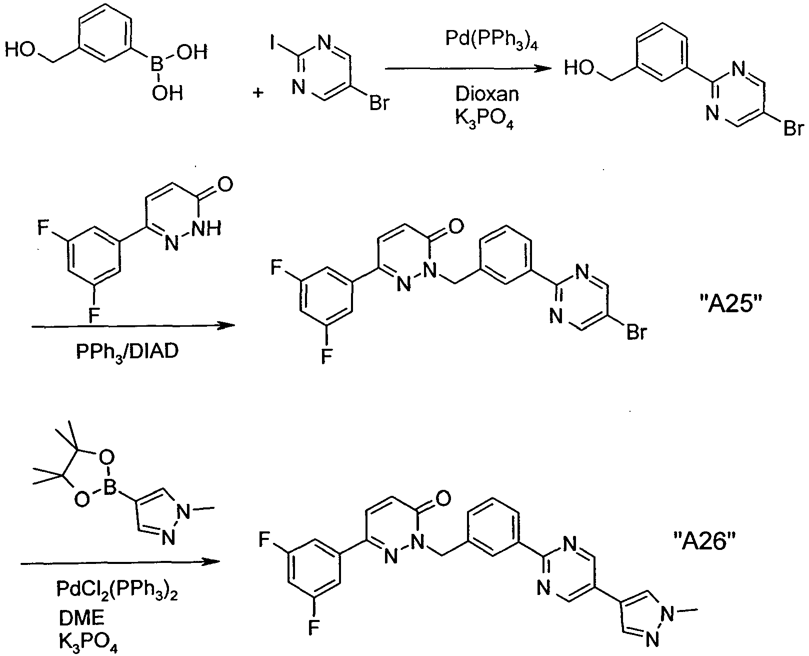
12.1 Eine unter Stickstoff gehaltene Lösung von 6.11 g (21.5 mmol) 5-Brom-2-lodpyrimidin, 3.91 g (25.7 mmol) 3-(Hydroxymethyl)-benzolboronsäure und 9.11 g (42.9 mmol) Trikaliumphosphat-Trihydrat in 120 ml Dioxan und 14 ml Wasser wird mit 750 mg (0.65 mmol) Tetrakis(triphenylphosphin)-palladium versetzt und 18 Stunden bei 90° C gerührt. Das Reaktionsgemisch wird auf Raumtemperatur abgekühlt, mit tert.-Butyl-methylether und Wasser versetzt und über Kieselgur filtriert. Die organische Phase des Filtrats wird abgetrennt, über Natriumsulfat getrocknet und eingedampft. Der Rückstand wird an einer Kieselgelsäule mit Dichlormethan/Methanol als Laufmittel chromatographiert: [3-(5-Brompyrimidin-2-yl)-phenyl]-methanol als hellgelbe Kristalle; ESI 265,267.
12.2 Zu einer Suspension von 2.76 g (13.2 mmol) 6-(3,5-Difluorphenyl)-2H-pyridazin-3-on, 2.49 g (8.83 mmol) [3-(5-Brompyrimidin-2-yl)-phenyl]-methanol, und 3.47 g (13.2 mmol) Triphenylphosphin in 30 ml THF werden 2.60 ml (13.2 mmol) Diisopropylazodicarboxylat zugetropft und das Reaktionsgemisch 18 Stunden bei Raumtemperatur gerührt. Das Reaktionsgemisch wird im Vakuum eingedampft, in 2-Propanol aufgenommen, zum Sieden erhitzt und abkühlen lassen. Der entstandene Niederschlag wird abgesaugt, mit 2-Propanol gewaschen und erneut aus 2-Propanol umkristallisiert: 2-[3-(5-Brompyrimidin-2-yl)-benzyl]-6-(3,5-difluorphenyl)-2H-pyridazin-3-on ("A25") als farblose Kristalle; ESI 455,457;
   ¹H-NMR (DMSO-d₆): δ [ppm] = 5.45 (s, 2H), 7.14 (d, J = 9.5 Hz, 1H), 7.35 (tt, J₁ = 8.8 Hz, J₂ = 2.3 Hz, 1H), 7.53 (t, J = 7.5 Hz, 1H), 7.59 (m, 1H), 7.66 (m, 2H), 8.14 (d, J = 9.5 Hz, 1 H), 8.29 (m, 1 H), 8.38 (bs,1 H), 9.06 (s, 2H).
12.3 Eine unter Stickstoff gehaltene Lösung von 455 mg (1.00 mmol) 2-[3-(5-Brompyrimidin-2-yl)-benzyl]-6-(3,5-difluorphenyl)-2H-pyridazin-3-on und 229 mg (1.10 mmol) 1-Methyl-4-(4,4,5,5-tetramethyl-[1,3,2]dioxaborolan-2-yl)-1 H-pyrazol in 10 ml 1,2-Dimethoxyethan wird mit 425 mg (2.0 mmol) Trikaliumphosphat-Trihydrat und 56.2 mg (0.08 mmol) Bis(triphenylphosphin)-palladiumchlorid versetzt und 18 Stunden bei 80° C gerührt, wobei sich ein grauer Niederschlag bildet. Das Reaktionsgemisch wird mit Wasser verdünnt und filtriert. Der Rückstand wird an einer Kieselgelsäule mit Dichlormethan / Methanol als Laufmittel chromatographiert: 6-(3,5-Difluorphenyl)-2-{3-[5-(1-methyl-1H-pyrazol-4-yl)-pyrimidin-2-yl]-benzyl}-2H-pyridazin-3-on ("A26") als farblose Kristalle; ESI 457;
   ¹H-NMR (DMSO-d₆): δ [ppm] = 3.80 (s, 3H), 5.44 (s, 2H), 7.13 (d, J = 9.5 Hz, 1H), 7.29 (tt, J₁ = 8.8 Hz, J₂ = 2.3 Hz, 1H), 7.50 (m, 2H), 7.64 (m, 2H), 8.05 (s, 1 H), 8.14 (d, J = 9.5 Hz, 1 H), 8.32 (m, 1 H), 8.35 (s, 1 H), 8.45 (bs,1H), 9.11 (s, 2H).

### Analog werden nachstehende Verbindungen erhalten:

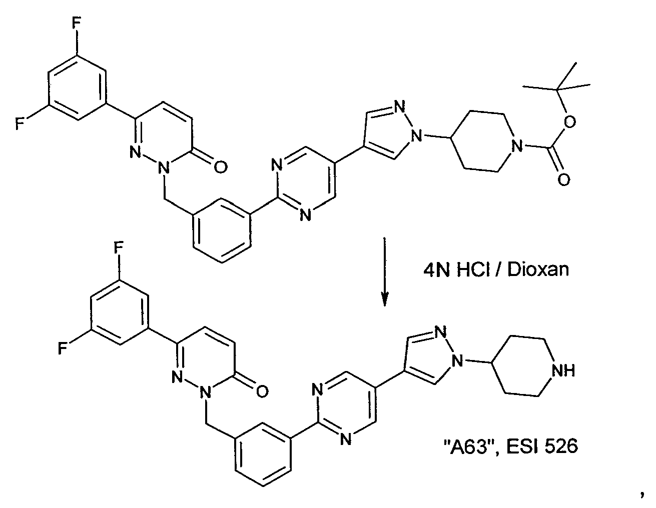

"A63": ¹H-NMR (DMSO-d₆): δ [ppm] = 1.81 (m, 2H), 2.01 (m, 2H), 2.15 (bs, 1 H), 2.61 (m, 2H), 3.06 (m, 2H), 4.24 (m, 1 H), 5.47 (s, 2H), 7.16 (d, J = 9.5 Hz, 1 H), 7.36 (tt, J₁ = 8.8 Hz, J₂ = 2.3 Hz, 1 H), 7.53 (m, 2H), 7.68 (m, 2H), 8.10 (s, 1H), 8.16 (d, J = 9.5 Hz, 1H), 8.33 (m, 1H), 8.46 (bs,1H), 8.48 (s, 1 H), 9.14 (s, 2H);

| Verbindung Nr. | Name und/oder Struktur | ESI [M+H]⁺ | HPLC (Rt. in min) Methode |
|---|---|---|---|
| | | | |
| "A103" | 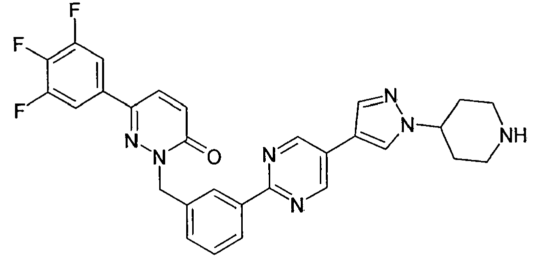 | | |
| "A104" | 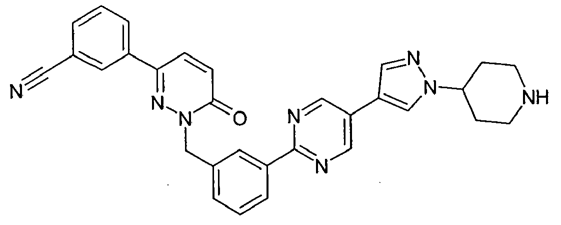 | | |
| "A105" | 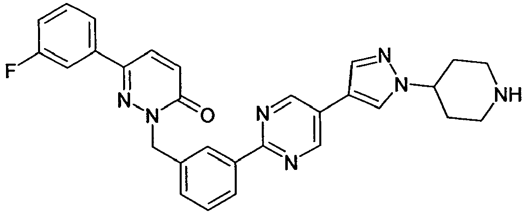 | | |
| "A106" | 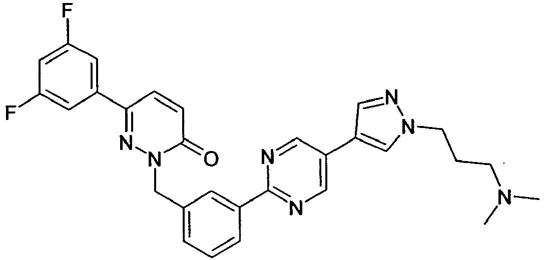 | | |
| "A107" | 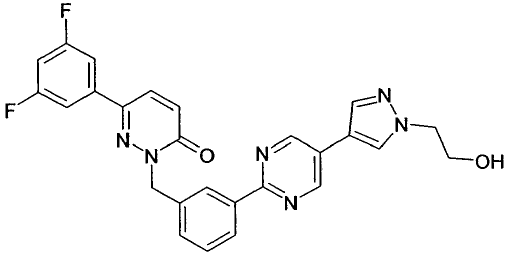 | | |
| "A108" | 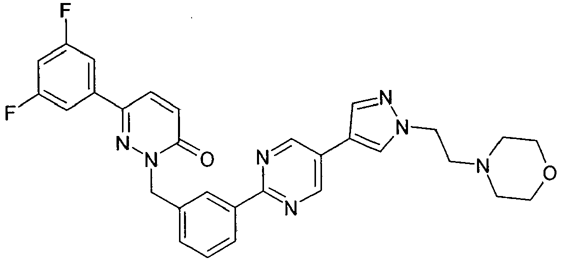 | | |
| "A109" | 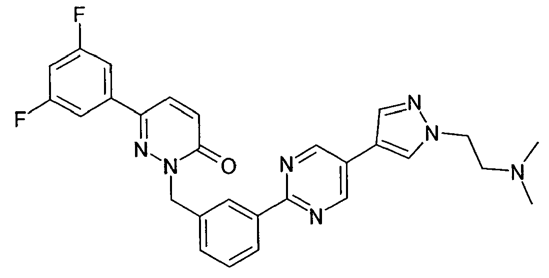 | | |

### Beispiel 13

Die Herstellung von 6-(3,5-Difluor-phenyl)-2-{3-[6-(4-methyl-piperazin-1-yl)-pyridazin-3-yl]-benzyl}-2H-pyridazin-3-on ("A27") und 6-(3,5-Difluorphenyl)-2-{3-[6-(3-dimethylamino-propoxy)-pyridazin-3-yl]-benzyl}-2H-pyridazin-3-on ("A28") erfolgt analog nachstehendem Schema

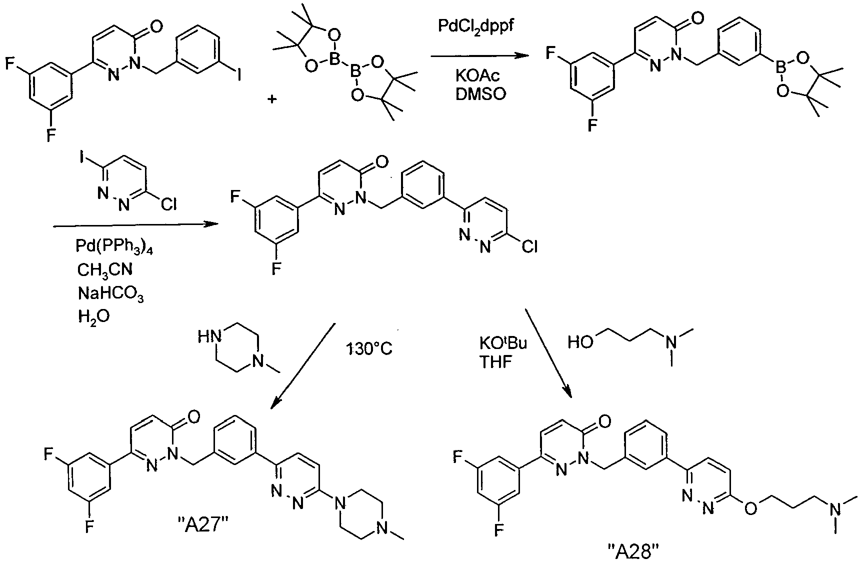

### Analog werden nachstehende Verbindungen erhalten

6-(3,5-Difluor-phenyl)-2-{3-[6-(3-dimethylamino-propylamino)-pyridazin-3-yl]-benzyl}-pyridazin-3-on ("A67") Hydrochlorid, ESI 477, 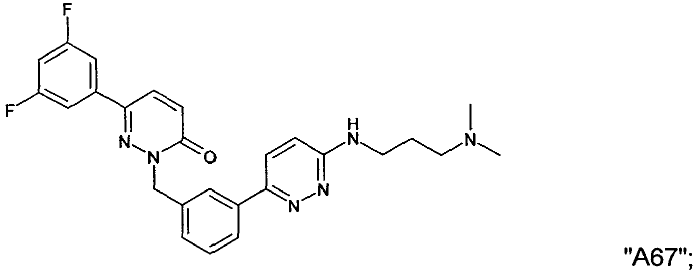 6-(3,5-Difluor-phenyl)-2-{3-[6-(2-dimethylamino-ethylamino)-pyridazin-3-yl]-benzyl}-pyridazin-3-on ("A68") Hydrochlorid, ESI 463;
6-(3,5-Difluor-phenyl)-2-{3-[6-(4-dimethylamino-butylamino)-pyridazin-3-yl]-benzyl}-pyridazin-3-on ("A69");
6-(3,5-Difluor-phenyl)-2-{3-[6-(1-methyl-piperidin-4-ylamino)-pyridazin-3-yl]-benzyl}-pyridazin-3-on

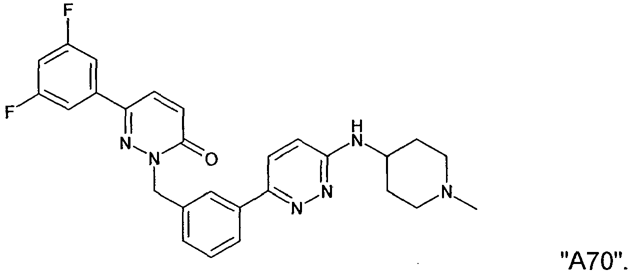

### Beispiel 14

Die Herstellung von 2-{3-[6-Oxo-3-(3,4,5-trifluor-phenyl)-6H-pyridazin-1-ylmethyl]-phenyl}-pyrimidin-5-carbonsäureethylester ("A29") erfolgt analog nachstehendem Schema

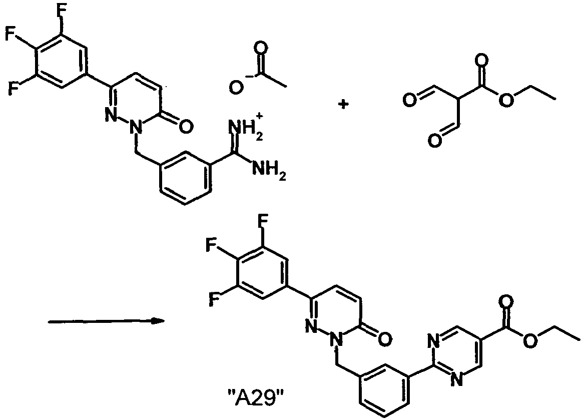

4.7 g (11.23 mmol) 3-[6-Oxo-3-(3,4,5-trifluorphenyl)-6H-pyridazin-1-ylmethyl]-benzamidinium-Acetat werden in 40 ml Pyridin suspendiert und mit 2.4 g (16.85 mmol) 2-Formyl-3-oxo-propionsäureethylester (hergestellt entsprechend S.H. Berz et al., Journal of Organic Chemistry 1982, 47, 2216) versetzt und 4 h bei 80°C gerührt. Anschließend werden nochmals 500 mg (3.47 mmol) 2-Formyl-3-oxo-propionsäure-ethylester zugegeben und 1 h bei 80°C gerührt. Das Reaktionsgemisch wird in 400 ml Wasser eingerührt, der Niederschlag wird abgesaugt, mehrmals mit Wasser gewaschen und im Trockenschrank getrocknet. Ausbeute: 4.43 g "A29" (76 %), Rt. = 3.58 min (Methode B), ESI 467.

### Analog erhält man die Verbindungen

2-{3-[3-(3,5-Difluor-phenyl)-6-oxo-6H-pyridazin-1-ylmethyl]-phenyl}-pyrimidin-5-carbonsäure-ethylester

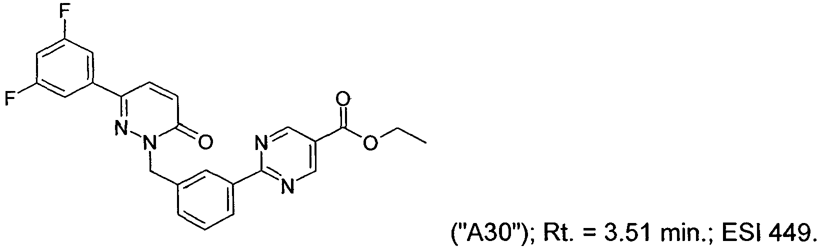

Herstellung von 6-(3,5-Difluor-phenyl)-2-[3-(5-hydroxymethyl-pyrimidin-2-yl)-benzyl]-2H-pyridazin-3-on ("A101")

1 g (2.43 mmol) 2-{3-[3-(3,5-Difluor-phenyl)-6-oxo-6H-pyridazin-1-ylmethyl]-phenyl}-pyrimidin-5-carbaldehyd [Herstellung Beispiel 7] wird in 15 mol Ethanol und 15 ml THF suspendiert. Das Reaktionsgemisch wird auf 5°C gekühlt, 374 mg (9.89 mmol) Natriumborhydrid wird zugegeben und innerhalb von 30 min auf Raumtemperatur gebracht.

Das Reaktionsgemisch wird auf ein Gemisch aus Eis/Wasser/1 N HCl (1:1:1) Gemisch gegossen. Das ausgefallene Produkt wird abgesaugt und im Trockenschrank getrocknet.
Ausbeute: 960 mg, weißer Feststoff "A101", ESI 407.

### Beispiel 15

Die Herstellung von 2-{3-[6-Oxo-3-(3,4,5-trifluor-phenyl)-6H-pyridazin-1-ylmethyl]-phenyl}-pyrimidin-5-carbonsäure ("A31") erfolgt analog nachstehendem Schema

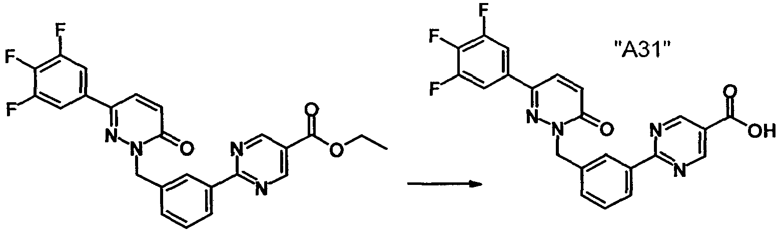

3.4 g (7.29 mmol) 2-{3-[6-Oxo-3-(3,4,5-trifluor-phenyl)-6H-pyridazin-1-ylmethyl]-phenyl}-pyrimidin-5-carbonsäureethylester werden in 300 ml THF und 30 ml Wasser gelöst und mit 713 mg (29.2 mmol) Lithiumhydroxid versetzt. Das Reaktionsgemisch wird 4 h refluxiert, auf Raumtemperatur abgekühlt und das organische Lösungsmittel wird am Rotationsverdampfer abdestilliert. Der Rückstand wird mit 300 ml Wasser und 30 ml THF versetzt, und zu dieser Lösung wird langsam unter Rühren konz. HCl bis zur stark sauren Reaktion zugetropft. Der gebildetet Niederschlag wird abgesaugt, mit viel Wasser gewaschen und im Vakuumtrockenschrank getrocknet.
Ausbeute: 2.87 g "A31", Rt. = 3.06 min (Methode B), ESI 439.

### Analog erhält man die Verbindung

2-{3-[3-(3,5-Difluor-phenyl)-6-oxo-6H-pyridazin-1-ylmethyl]-phenyl}-pyrimidin-5-carbonsäure ("A32"), ESI 421.

### Beispiel 16

Die Herstellung von 2-{3-[6-Oxo-3-(3,4,5-trifluor-phenyl)-6H-pyridazin-1-ylmethyl]-phenyl}-pyrimidin-5-carbonsäure(2-dimethylamino-ethyl)-amid ("A33") erfolgt analog nachstehendem Schema

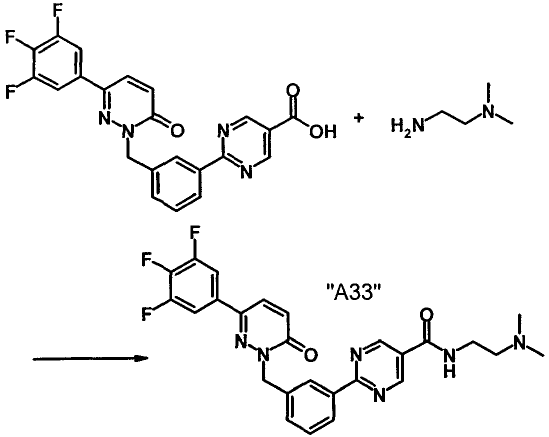

150 mg (0.334 mmol) 2-{3-[6-Oxo-3-(3,4,5-trifluor-phenyl)-6H-pyridazin-1-ylmethyl]-phenyl}-pyrimidin-5-carbonsäure werden in 2 ml DMF gelöst und mit 75 µl (0.67 mmol) 4-Methylmorpholin, 97 mg (0.50 mmol) EDCI und 60 mg (0.43 mmol) HOBt versetzt. Es werden 47 µl (0.43 mmol) *N,N*-Dimethylaminoethylendiamin zugegeben und das Reaktionsgemisch wird 18 h bei Raumtemperatur gerührt. Die Reaktionslösung wird direkt mittels präparativer HPLC getrennt.
Ausbeute: 124 mg "A33" Trifluoracetat; Rt. = 2.63 (Methode B); ESI 509.

### Analog werden die nachstehenden Verbindungen erhalten

| Verbindung Nr. | Name und/oder Struktur | ESI [M+H]⁺ | HPLC (Rt. in min) Methode |
|---|---|---|---|
| "A34" | 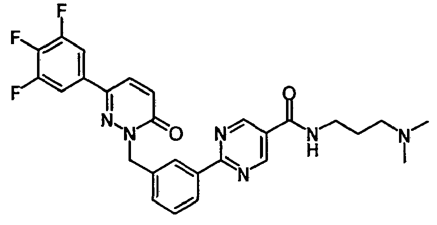 | 523 | 2.64 B |
| | Trifluoracetat | | |
| "A35" | 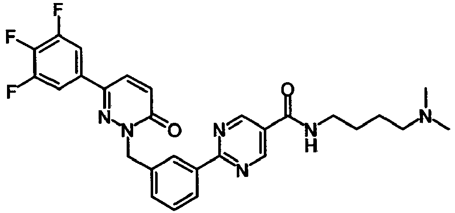 | 537 | 2.66 B |
| | Trifluoracetat | | |
| "A36" | 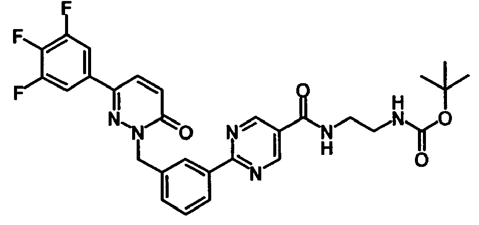 | 581 | 3.28 B |
| "A37" | 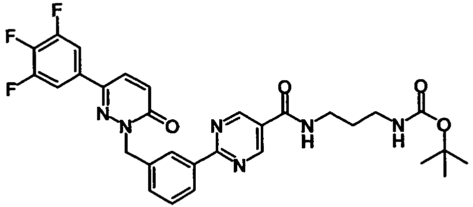 | 595 | 3.34 B |
| "A38" | 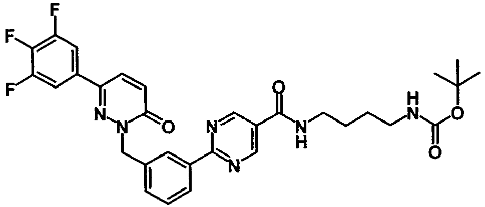 | 609 | 3.36 B |
| "A39" | 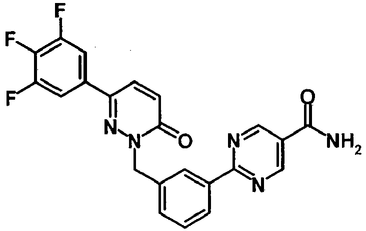 | 438 | 2.90 B |
| "A40" | 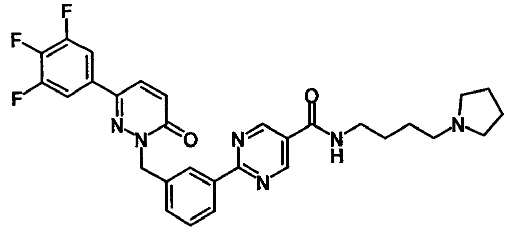 | 562 | 2.72 B |
| | Trifluoracetat | | |
| "A41" | 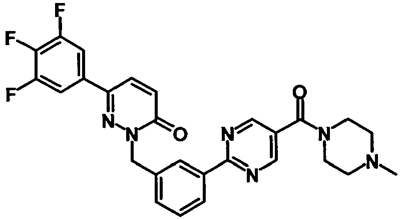 | 521 | 2.63 B |
| | Trifluoracetat | | |
| "A42" | 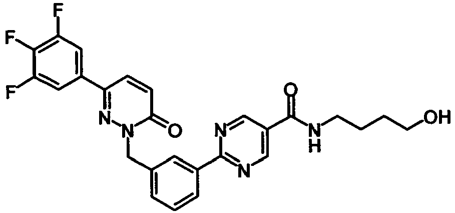 | 510 | 2.91 B |
| "A43" | 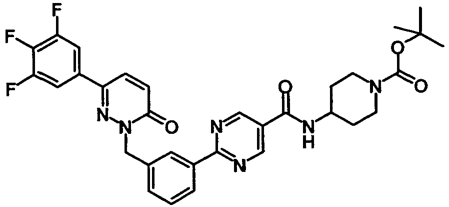 | 621 | 3.48 B |
| "A44" | 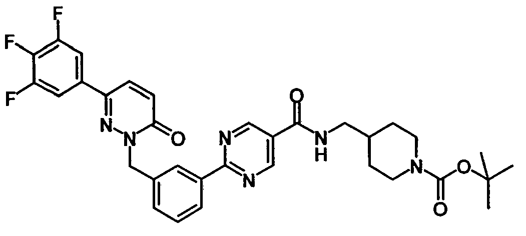 | 657 (M+Na) | 3.51 B |
| "A44a" | aus "A44" mit TFA/Dioxan: | | |
| | 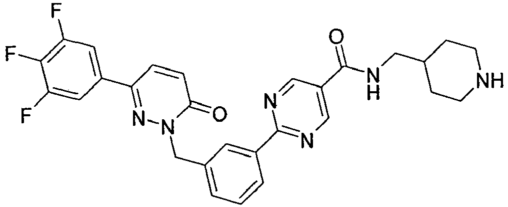 | 535 | |
| | Trifluoracetat | | |
| "A45" | 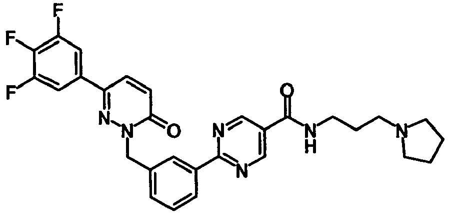 | 549 | 2.68 B |
| | Trifluoracetat | | |
| "A46" | 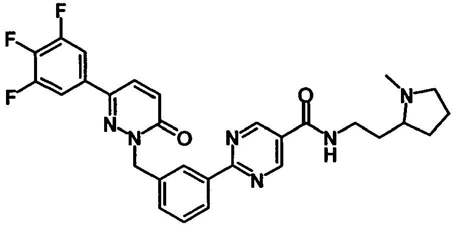 | 549 | 2.68 B |
| | Trifluoracetat | | |
| "A47" | 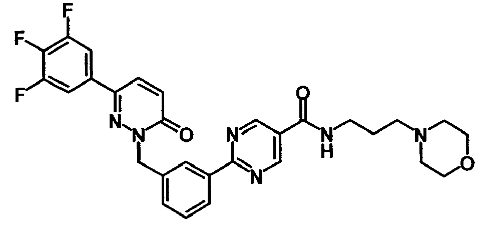 | 565 | 2.65 B |
| | Trifluoracetat | | |
| "A48" | 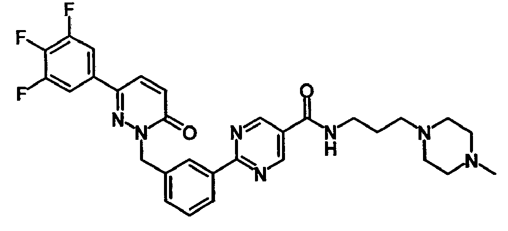 | 578 | 2.50 B |
| | Trifluoracetat | | |
| "A49" | 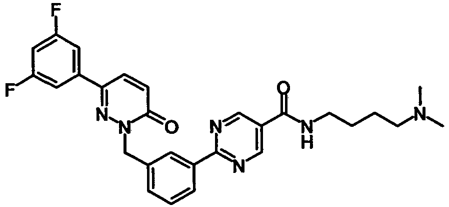 | 519 | 2.60 B |
| | Trifluoracetat | | |
| "A50" | 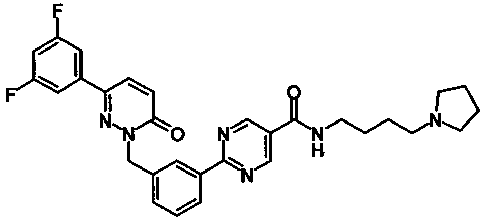 | 544 | 2.64 B |
| | Trifluoracetat | | |
| "A71" | 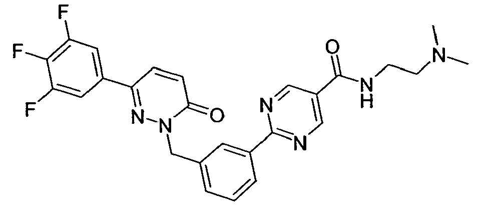 | 509 | |
| | Trifluoracetat | | |
| "A72" | 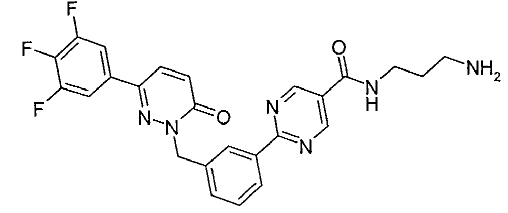 | 495 | |
| | Trifluoracetat (erhältlich aus Boc-geschützter Vorstufe) | | |
| "A73" | 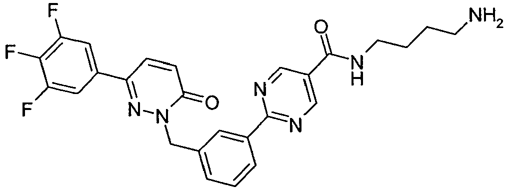 | 509 | |
| | Trifluoracetat (erhältlich aus Boc-geschützter Vorstufe) | | |
| "A74" | 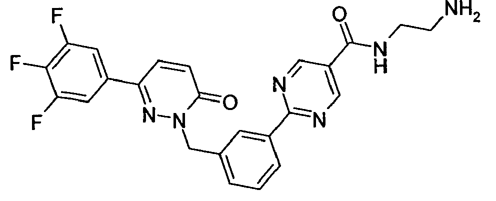 | 481 | |
| | Trifluoracetat (erhältlich aus Boc-geschützter Vorstufe) | | |
| "A84" | 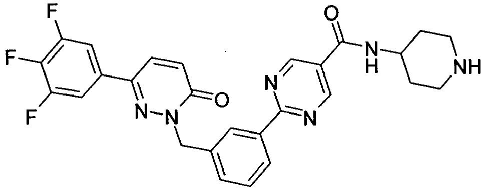 | 521 | |
| | Trifluoracetat (erhältlich aus Boc-geschützter Vorstufe) | | |
| "A90" | 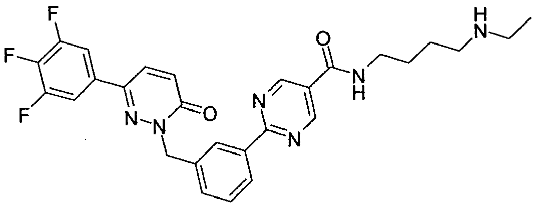 | 537 | |
| | Trifluoracetat (erhältlich aus Boc-geschützter Vorstufe) | | |
| "A92" | 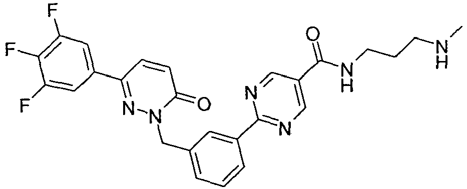 | 509 | |
| | Trifluoracetat (erhältlich aus Boc-geschützter Vorstufe) | | |

### Beispiel 17

Die Herstellung von 2-{3-[6-Oxo-3-(3,4,5-trifluor-phenyl)-6H-pyridazin-1-ylmethyl]-phenyl}-pyrimidin-5-carbonsäure-[2-(1H-imidazol-4-yl)-ethyl]-amid ("A51") erfolgt analog nachstehendem Schema

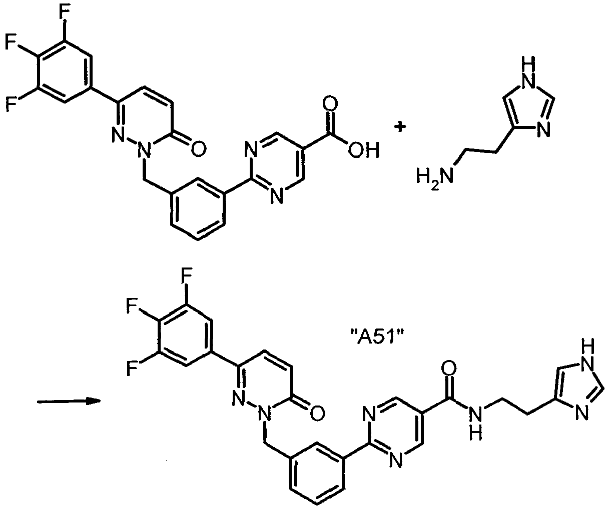

150 mg (0.334 mmol) 2-{3-[6-Oxo-3-(3,4,5-trifluor-phenyl)-6H-pyridazin-1-ylmethyl]-phenyl}-pyrimidin-5-carbonsäure werden in 2 ml DMF gelöst und mit 75 µl (0.67 mmol) 4-Methylmorpholin, 97 mg (0.50 mmol) EDCI und 60 mg (0.43 mmol) HOBt versetzt. Es werden 51 mg (0.44 mmol) Histamin zugegeben und das Reaktionsgemisch 18 h bei Raumtemperatur gerührt. Das Reaktionsgemisch wird mit Wasser versetzt, der entstandene Niederschlag wird abgesaugt. Der Rückstand wird mit Acetonitril versetzt, erneut abgesaugt und der Rückstand im Vakuum getrocknet.
Ausbeute: 127 mg "A51", Rt. = 2.63 min (Methode B), ESI 532.

### Analog werden die nachstehenden Verbindungen erhalten

| Verbindung Nr. | Name und/oder Struktur | ESI [M+H]⁺ | HPLC (Rt. in min) Methode |
|---|---|---|---|
| "A52" | 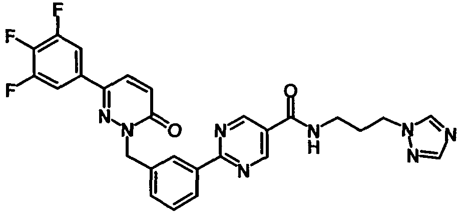 | 547 | 2.85 B |
| "A53" | 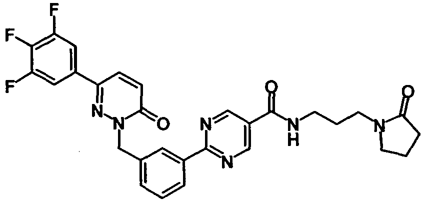 | 563 | 2.99 B |

### Beispiel 18

Die Herstellung von 2-[3-(5-Chlor-pyrimidin-2-yl)-benzyl]-6-(3,4,5-trifluor-phenyl)-2H-pyridazin-3-on ("A54") erfolgt analog nachstehendem Schema

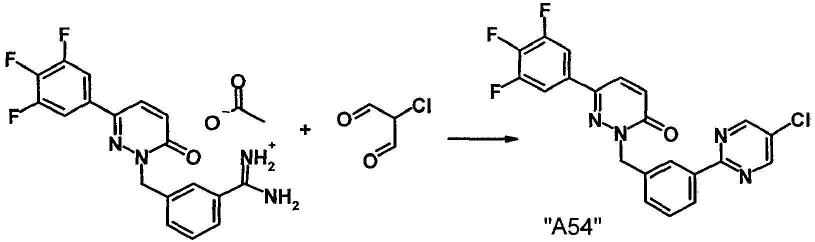

250 mg (0.60 mmol) 3-[6-Oxo-3-(3,4,5-trifluorphenyl)-6H-pyridazin-1-ylmethyl]-benzamidinium-Acetat werden in 3 ml Pyridin suspendiert, mit 74 mg (0.66 mmol) 2-Chlormalonaldehyd versetzt und 15 h bei 90°C gerührt. Das Reaktionsgemisch wird eingedampft und der Rückstand mittels präparativer HPLC gereinigt. Man erhält "A54".

### Beispiel 19 (Vergleich)

Die Herstellung von 2-[3-(4-Methyl-pyrimidin-2-yl)-benzyl]-6-(3,4,5-trifluor-phenyl)-2H-pyridazin-3-on ("A3") erfolgt analog nachstehendem Schema

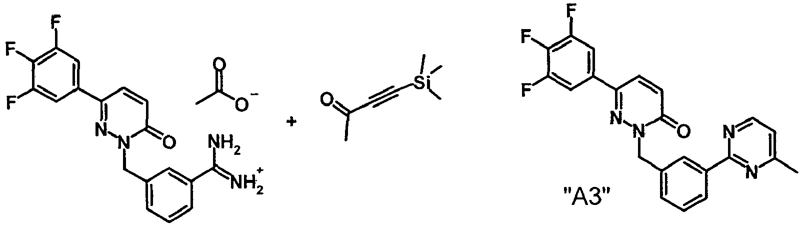

150 mg (0.36 mmol) 3-[6-Oxo-3-(3,4,5-trifluorphenyl)-6H-pyridazin-1-ylmethyl]-benzamidinium-Acetat und 99.5 mg (0.72 mmol) Kaliumcarbonat werden in 5 ml Acetonitril suspendiert, mit 42 mg (0.3 mmol) 4-Trimethylsilyl)-3-butyn-2-on versetzt und 45 min bei 120°C in einem Mikrowellenreaktor erhitzt (Emrys Optimizer). Das Reaktionsgemisch wird filtriert, eingedampft und der Rückstand mittels präparativer HPLC gereinigt.
Ausbeute: 16 mg "A3", weißer Feststoff, Rt. = 3.38 min (Methode B), ESI-MS: 408.

### Beispiel 20 (Vergleich)

Die Herstellung von 2-(3-Pyrimidin-2-yl-benzyl)-6-(3,4,5-trifluor-phenyl)-2H-pyridazin-3-on ("A4") erfolgt analog nachstehendem Schema

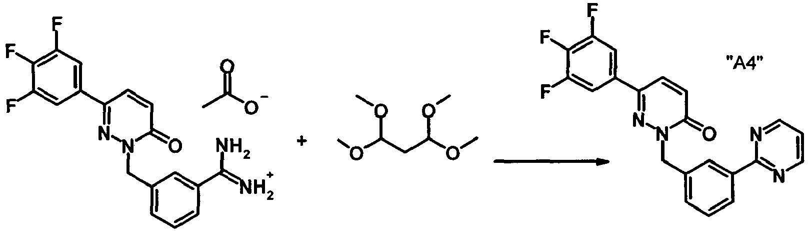

150 mg (0.36 mmol) 3-[6-Oxo-3-(3,4,5-trifluorphenyl)-6H-pyridazin-1-ylmethyl]-benzamidinium-Acetat und 1.1 ml (6.6 mmol) 1,1,3,3-Tetramethoxy-propan werden 1 h bei 175°C gerührt. Das Reaktionsgemisch wird direkt mittels präparativer HPLC gereinigt.
Ausbeute: 23 mg "A4", weißer Feststoff; Rt. = 3.28 min (Methode B); ESI-MS: 395.

### Beispiel 21

Die Herstellung von 4-{1-[3-(5-Methyl-pyrimidin-2-yl)-benzyl]-6-oxo-1,6-dihydro-pyridazin-3-yl}-N-(3-piperidin-1-yl-propyl)-benzamid ("A55") erfolgt analog nachstehendem Schema

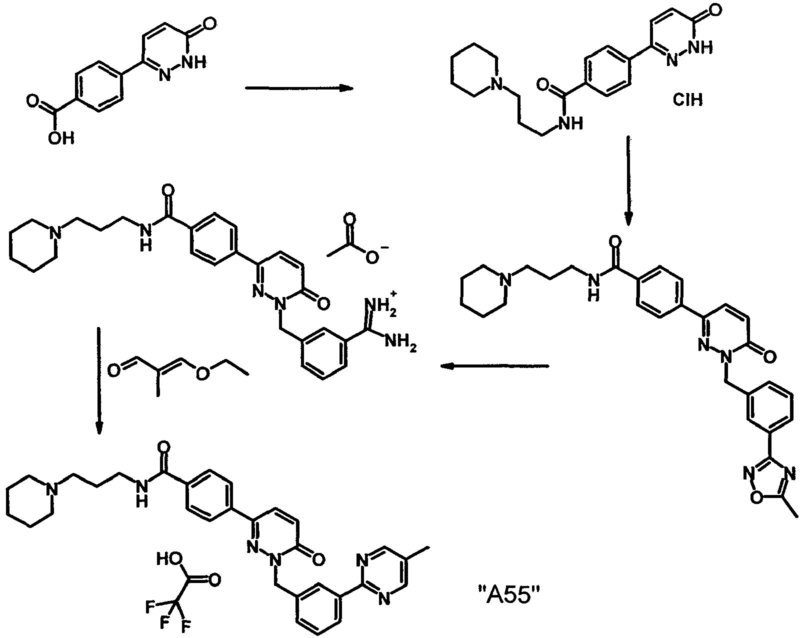

1.5 g (6.94 mmol) 4-(6-Oxo-1,6-dihydro-pyridazin-3-yl)-benzoesäure (Herstellung laut DE 10010422) werden in 20 ml DMF gelöst, mit 1.18 g (8.33 mmol) 3-Piperidino-propylamin, 1.56 ml (13.9 mmol) 4-Methylmorpholin, 2.7 g (13.9 mmol) EDCI und 967 mg (6.94 mmol) HOBt versetzt und 18 h bei Raumtemperatur gerührt. Das DMF wird abdestilliert, der Rückstand mit 2 M NaOH versetzt. Das Gemisch wird eingedampft und mit 100 ml THF versetzt, 1 h gerührt, filtriert, mit 50 ml Ether versetzt und 10 ml 4N HCl in Dioxan versetzt. Dabei bildet sich ein Öl, der Überstand wird abdekantiert, nochmals mit Ether versetzt und erneut dekantiert. Der ölige Rückstand wird mit 30 ml Isopropanol versetzt. Dabei bildet sich nach 3 Tagen ein Kristallisat, welches abgesaugt, mit Isopropanol gewaschen und getrocknet wird. Ausbeute: 500 mg "A55", Rt. = 1.70 min, ESI 341.

### Beispiel 22

Die Herstellung von N-(2-{3-[3-(3,5-Difluor-phenyl)-6-oxo-6H-pyridazin-1-ylmethyl]-phenyl}-pyrimidin-5-yl)-2-dimethylamino-acetamid ("A85") erfolgt analog nachstehendem Schema 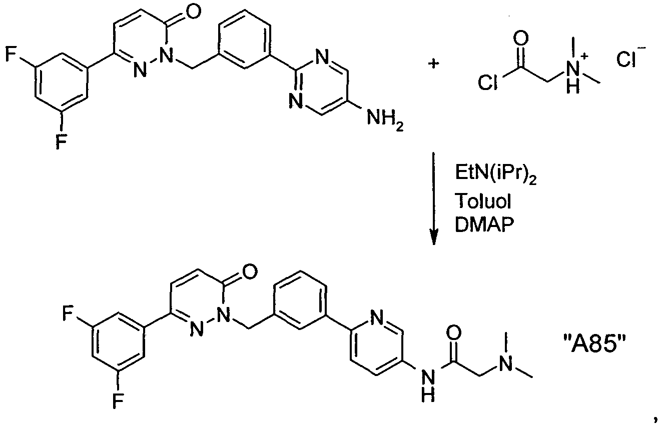 Trifluoracetat, ESI 477.

### Analog erhält man die Verbindungen

N-(2-{3-[3-(3,5-Difluor-phenyl)-6-oxo-6H-pyridazin-1-ylmethyl]-phenyl)-pyrimidin-5-yl)-4-dimethylamino-butyramid Hydrochlorid

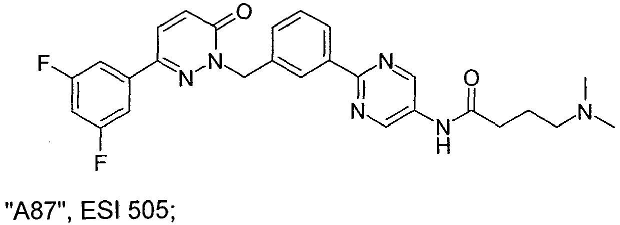

N-(2-{3-[3-(3,5-Difluor-phenyl)-6-oxo-6H-pyridazin-1-ylmethyl]-phenyl)-pyrimidin-5-yl)-3-dimethylamino-propionamid

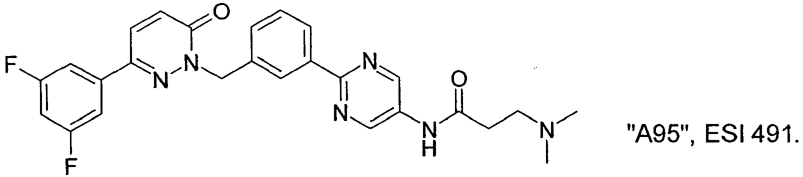

### Beispiel 23

Durch Umsetzung unter Standardbedingungen von 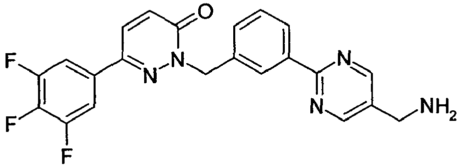 mit Cl-CO-CH₂-N(CH₃)COO-tert.-butyl erhält man nach üblicher Aufarbeitung

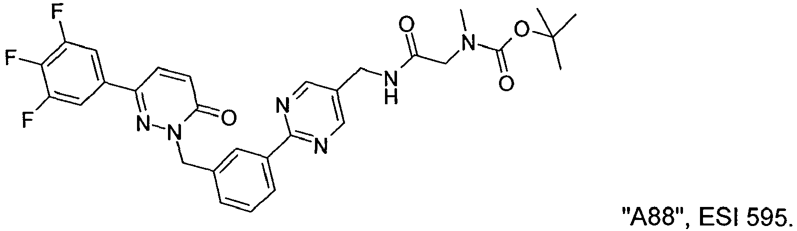

### Analog erhält man die Verbindung

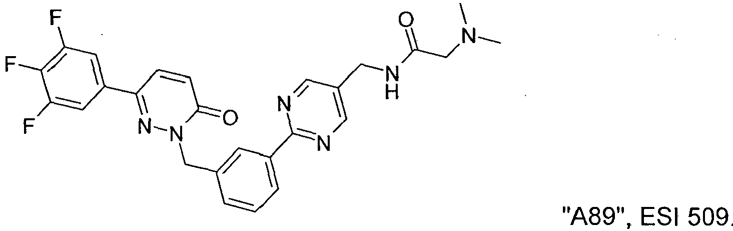

### Beispiel 24

Die Herstellung von 2-{3-[6-Oxo-3-(3,4,5-trifluor-phenyl)-6H-pyridazin-1-ylmethyl]-phenyl}-pyrimidin-5-carbonitril ("A77") und von 2-[3-(5-Aminomethyl-pyrimidin-2-yl)-benzyl]-6-(3,4,5-trifluor-phenyl)-2H-pyridazin-3-on ("A93") erfolgt analog nachstehendem Schema

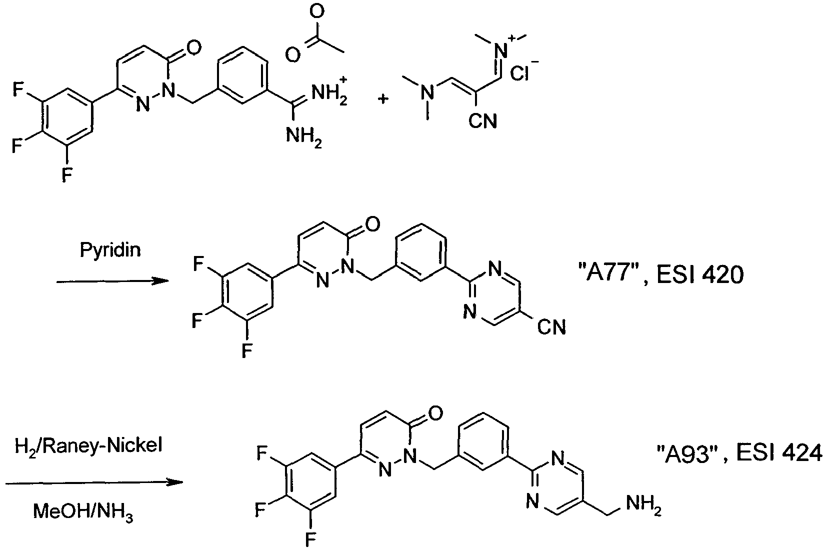

24.1 3.5 g (18.6 mmol) 3-Dimethylamino-2-cyano-2-propen-1-yliden)-dimethylammoniumchlorid (hergestellt analog zu US 3853946) werden in 60 ml Pyridin suspendiert und mit 7,8 g (18.6 mmol) 3-[6-Oxo-3-(3,4,5-trifluor-phenyl)-6H-pyridazin-1-ylmethyl]-benzamidinium Acetat versetzt. Die Suspension wird 4 Stunden bei 100°C gerührt. Nach dem Abkühlen auf Raumtemperatur wird das Gemisch in 300 ml Wasser eingerührt, die Kristalle abgesaugt und mit Wasser gewaschen. Der Feststoff wird im Vakuumtrockenschrank getrocknet;
Ausbeute: 6.14 g "A77" , beigefarbene Kristalle;
HPLC: Rt. = 3.34 min (Methode C); LC-MS: 420 (M+H).

24.2 2.9 g (6.9 mmol) 2-{3-[6-Oxo-3-(3,4,5-trifluor-phenyl)-6H-pyridazin-1-ylmethyl]-phenyl}-pyrimidin-5-carbonitril ("A77") werden in 60 ml THF und 60 ml Methanol gelöst und mit 2 g Raney-Nickel versetzt. Anschließend wird 6 h bei Normaldruck unter Wasserstoffatmosphäre hydriert. Der Katalysator wird abgesaugt, mit THF gewaschen und das Filtrat eingeengt.
Ausbeute: 2.9 g "A93", hellgelber Feststoff; HPLC: 2.53 min (Methode C)
LC-MS: 424 (M+H).

### Beispiel 25

Die Herstellung von 6-(3,5-Difluor-phenyl)-2-[3-(6-oxo-1,6-dihydro-pyrimidin-2-yl)-benzyl]-2H-pyridazin-3-on ("A80"), ESI 393, und von 6-(3,5-Difluor-phenyl)-2-{3-[4-(3-dimethylamino-propoxy)-pyrimidin-2-yl]-benzyl}-2H-pyridazin-3-on ("A81") Hydrochlorid, ESI 478, erfolgt analog nachstehendem Schema

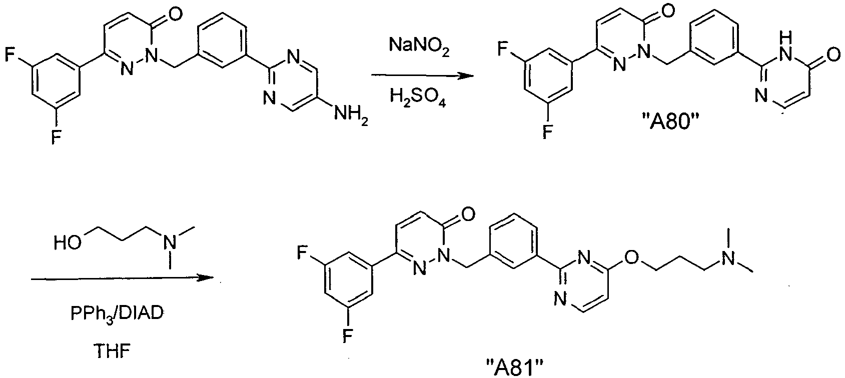

### Beispiel 27

Die Herstellung von 3-(4-Methyl-piperazin-1-yl)-N-(2-{3-[6-oxo-3-(3,4,5-trifluor-phenyl)-6H-pyridazin-1-ylmethyl]-phenyl)-pyrimidin-5-ylmethyl)-propionamid ("A96"), ESI 578, erfolgt analog nachstehendem Schema

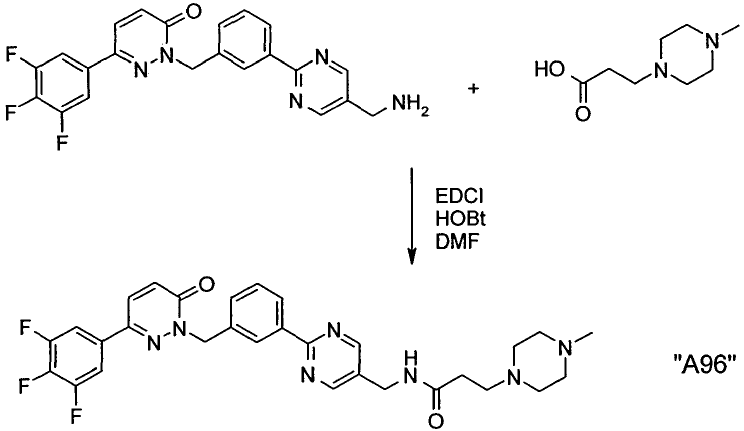

169 mg (0.4 mmol) 2-[3-(5-Aminomethyl-pyrimidin-2-yl)-benzyl]-6-(3,4,5-trifluorphenyl)-2H-pyridazin-3-on und 83 mg (0.48 mmol) 3-(4-Methylpiperazin-1-yl)propansäure werden in 2 ml DMF suspendiert und mit 90 µl (0.8 mmol) *N*-Methylmorpholin, 116 mg (0.60 mmol) EDCI und 72 mg (0.52 mmol) HOBt versetzt und 15 h bei Raumtemperatur gerührt. Das Reaktionsgemisch wird mit 10 ml Wasser versetzt und mit Ethylacetat extrahiert. Das Rohprodukt wird mit Ether kristallisiert.
Ausbeute: 104 mg "A96", beigefarbener Feststoff; HPLC: Rt. = 2.49 min
LC-MS: 578 (M+H).

### Analog werden nachstehende Verbindungen erhalten

2-(4-Methyl-piperazin-1-yl)-N-(2-{3-[6-oxo-3-(3,4,5-trifluor-phenyl)-6H-pyridazin-1-ylmethyl]-phenyl}-pyrimidin-5-ylmethyl)-acetamid, ESI 564, 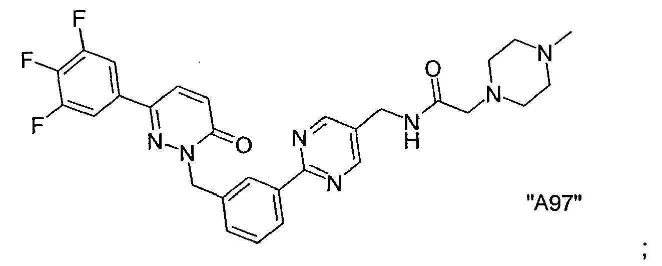 2-Methylamino-N-(2-{3-[6-oxo-3-(3,4,5-trifluor-phenyl)-6H-pyridazin-1-ylmethyl]-phenyl}-pyrimidin-5-ylmethyl)-acetamid, Trifluoracetat, ESI 495 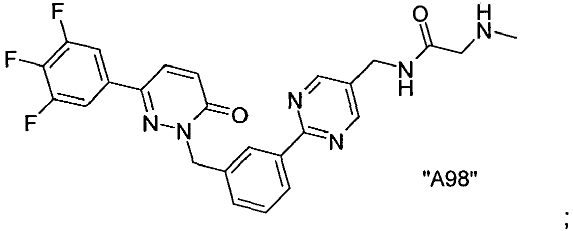 3-Dimethylamino-N-(2-{3-[6-oxo-3-(3,4,5-trifluor-phenyl)-6H-pyridazin-1-ylmethyl]-phenyl}-pyrimidin-5-ylmethyl)-propionamid ("A99") Trifluoracetat, ESI 523,

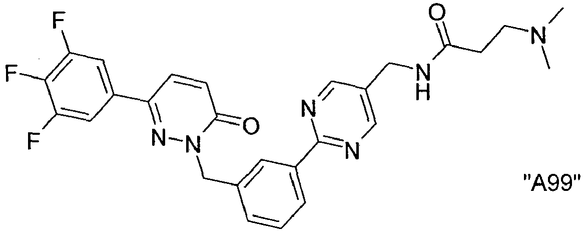

### Beispiel 28

Die Herstellung von
4-(2-{3-[3-(3,5-Difluor-phenyl)-6-oxo-6H-pyridazin-1-ylmethyl]-phenyl-pyrimidin-5-yl)-morpholin-3-on ("A75")
erfolgt analog nachstehendem Schema

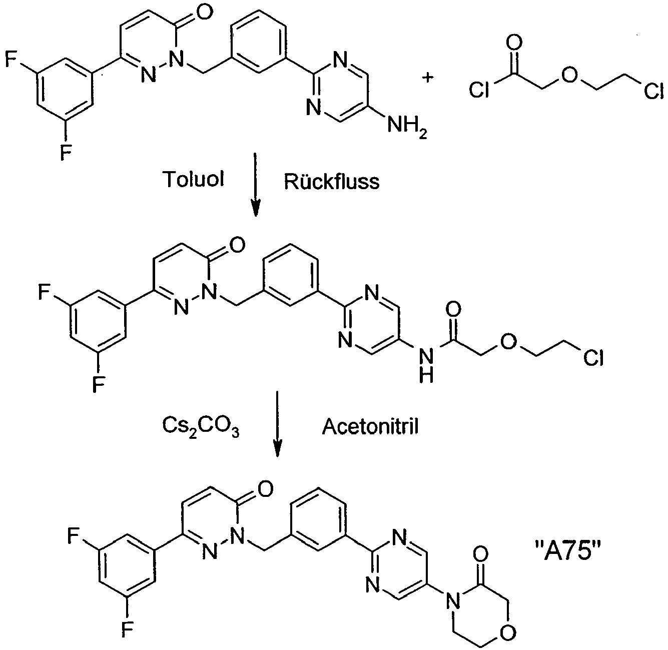

### Beispiel 31

### Analog Beispiel 10 erhält man die nachstehenden Verbindungen

| Verbindung Nr. | Name und/oder Struktur | ESI [M+H]⁺ | HPLC (Rt. in min) Methode |
|---|---|---|---|
| "A114" | 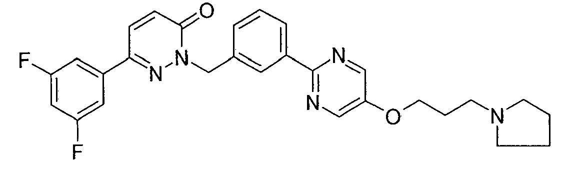 | 504 | |
| "A115" | 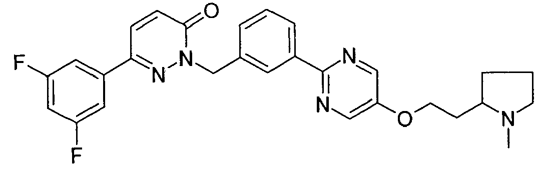 | 504 | |
| "A116" | 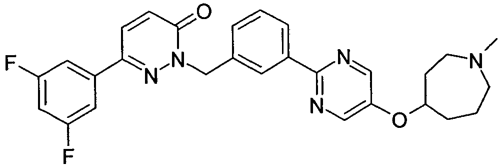 | 504 | |
| "A117" | 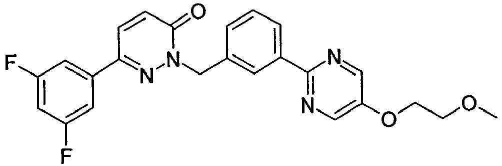 | 451 | |
| "A118" | 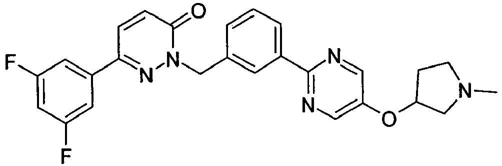 | 476 | |
| | Trifluoracetat | | |
| "A119" | 6-(3,5-Difluor-phenyl)-2-{3-[5-(8-methyl-8-azabicyclo[3.2.1]oct-3-yloxy)-pyrimidin-2-yl]-benzyl}-2H-pyridazin-3-on, Trifluoracetat | 516 | |
| | 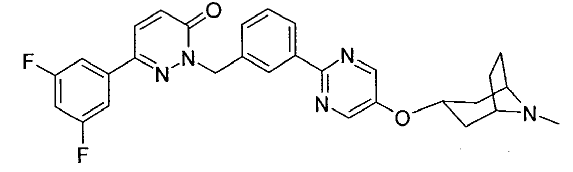 | | |
| "A120" | 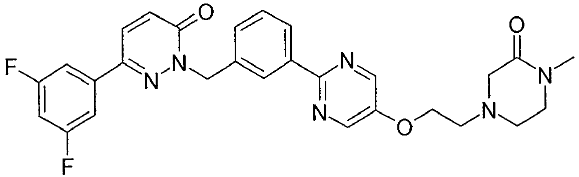 | 533 | |
| "A121" | 6-(3,5-Difluor-phenyl)-2-{3-[5-((S)-1-methyl-pyrrolidin-3-yloxy)-pyrimidin-2-yl]-benzyl)-2H-pyridazin-3-on, Trifluoracetat | 476 | |
| | 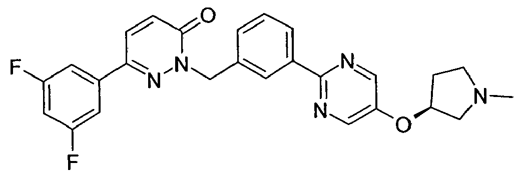 | | |
| "A122" | 6-(3,5-Difluor-phenyl)-2-{3-[5-((R)-1-methyl-pyrrolidin-3-yloxy)-pyrimidin-2-yl]-benzyl}-2H-pyridazin-3-on, Trifluoracetat | 476 | |
| "A123" | 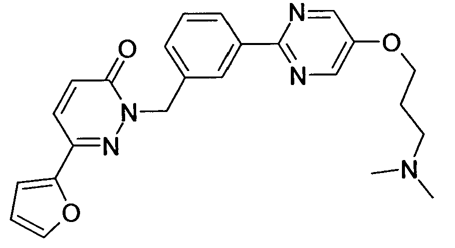 | 432 | |
| "A124" | 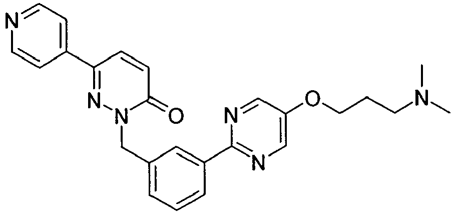 | 443 | |
| "A125" | 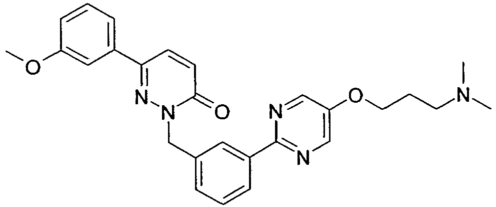 | 472 | |
| | Trifluoracetat | | |
| "A126" | 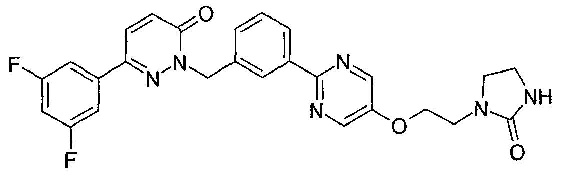 | 505 | |
| "A127" | 6-(3,5-Difluor-phenyl)-2-{3-[5-(2-pyrrolidin-1-yl-ethoxy)-pyrimidin-2 -yl]-benzyl}-2 H-pyridazin-3-on, Hydrochlorid | 490 | |
| "A128" | 6-(3,5-Difluor-phenyl)-2-{3-[5-(3-morpholin-4-yl-propoxy)-pyrimidin-2-yl]-benzyl}-2H-pyridazin-3-on, Trifluoracetat | 520 | |
| "A129" | 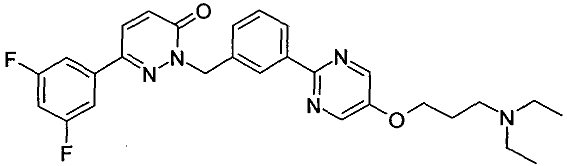 | 506 | |
| | Trifluoracetat | | |
| "A130" | 6-(3,5-Difluor-phenyl)-2-{3-[5-(2-morpholin-4-yl-ethoxy)-pyrimidin-2-yl]-benzyl}-2H-pyridazin-3-on, Hydrochlorid | 506 | |
| "A131" | 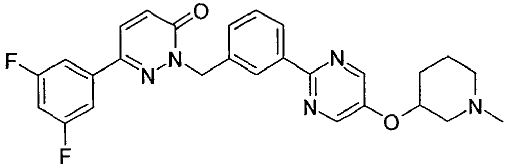 | 490 | |

### Beispiel 32

### Analog Beispiel 10 mit anschließender Boc-Abspaltung erhält man die nachstehenden Verbindungen

| Verbindung Nr. | Name und/oder Struktur | ESI [M+H]⁺ | HPLC (Rt. in min) Methode |
|---|---|---|---|
| "A132" | 6-(3,5-Difluor-phenyl)-2-{3-[5-(4-methylamino-butoxy)-pyrimidin-2-yl]-benzyl}-2H-pyridazin-3-on, Hydrochlorid | 478 | |
| "A133" | 6-(3,5-Difluor-phenyl)-2-{3-[5-(3-methylamino-propoxy)-pyrimidin-2-yl]-benzyl}-2H-pyridazin-3-on, Hydrochlorid | 464 | |
| "A134" | 6-(3,5-Difluor-phenyl)-2-{3-[5-(pyrrolidin-3-ylmethoxy)-pyrimidin-2-yl]-benzyl}-2H-pyridazin-3-on, Hydrochlorid | 476 | |
| "A135" | 6-(3,5-Difluor-phenyl)-2-{3-[5-(3-ethylamino-propoxy)-pyrimidin-2-yl]-benzyl}-2H-pyridazin-3-on, Hydrochlorid | 478 | |
| "A136" | 2-{3-[5-(2-Amino-ethoxy)-pyrimidin-2-yl]-benzyl}-6-(3,5-difluor-phenyl)-2H-pyridazin-3-on, Hydrochlorid | 436 | |
| "A137" | 6-(3,5-Difluor-phenyl)-2-{3-[5-(piperidin-3-yloxy)-pyrimidin-2-yl]-benzyl)-2H-pyridazin-3-on, Hydrochlorid | 476 | |
| "A138" | 6-(3,5-Difluor-phenyl)-2-{3-[5-(piperidin-4-ylmethoxy)-pyrimidin-2-yl]-benzyl}-2H-pyridazin-3-on, Hydrochlorid | 490 | |
| "A139" | 6-(3,5-Difluor-phenyl)-2-{3-[5-(pyrrolidin-3-yloxy)-pyrimidin-2-yl]-benzyl}-2H-pyridazin-3-on, Trifluoracetat | 462 | |
| "A140" | 6-(3,5-Difluor-phenyl)-2-{3-[5-((S)-pyrrolidin-3-yloxy)-pyrimidin-2-yl]-benzyl}-2H-pyridazin-3-on, Trifluoracetat | 462 | |
| "A141" | 6-(3,5-Difluor-phenyl)-2-{3-[5-((R)-pyrrolidin-3-yloxy)-pyrimidin-2-yl]-benzyl}-2H-pyridazin-3-on, Trifluoracetat | 462 | |
| "A142" | 2-{3-[5-(Piperidin-4-yloxy)-pyrimidin-2-yl]-benzyl)-6-pyridin-4-yl-2H-pyridazin-3-on | 441 | |
| | 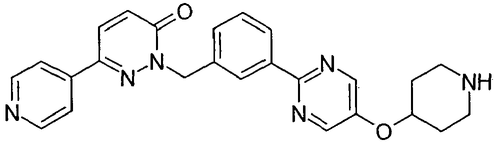 | | |
| "A143" | 4-(6-Oxo-1-{3-[5-(piperidin-4-yloxy)-pyrimidin-2-yl]-benzyl)-1,6-dihydro-pyridazin-3-yl)-benzonitril | 465 | |
| | 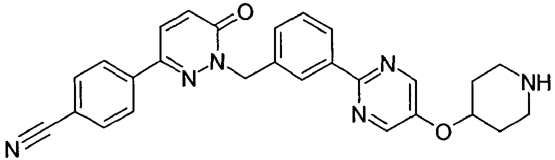 | | |
| "A144" | 3-(6-Oxo-1-{3-[5-(piperidin-4-yloxy)-pyrimidin-2-yl]-benzyl}-1,6-dihydro-pyridazin-3-yl)-benzonitril | 465 | |
| "A145" | 6-(3,5-Difluor-phenyl)-2-{3-[5-(2-piperazin-1-yl-ethoxy)-pyrimidin-2-yl]-benzyl]}-2H-pyridazin-3-on, Trifluoracetat | 505 | |
| "A146" | 6-(3,5-Difluor-phenyl)-2-{3-[5-(piperidin-4-yloxy)-pyrimidin-2-yl]-benzyl}-2H-pyridazin-3-on | | |
| "A147" | 3-(6-Oxo-1-{3-[5-(2-piperazin-1-yl-ethoxy)-pyrimidin-2-yl]-benzyl}-1,6-dihydro-pyridazin-3-yl)-benzonitril | 494 | |
| "A148" | 6-(3-Fluor-phenyl)-2-{3-[5-(piperidin-4-ylmethoxy)-pyrimidin-2-yl]-benzyl}-2H-pyridazin-3-on | 472 | |

### Beispiel 33

### Analog Beispiel 12 mit anschließender Boc-Abspaltung erhält man die nachstehenden Verbindungen

| Verbindung Nr. | Name und/oder Struktur | ESI [M+H]⁺ |
|---|---|---|
| "A149" | 2-{3-[5-(1-Piperidin-4-yl-1H-pyrazol-4-yl)-pyrimidin-2-yl]-benzyl}-6-(3,4,5-trifluor-phenyl)-2H-pyridazin-3-on | 544 |
| | 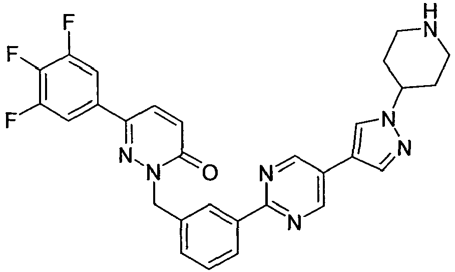 | |
| "A150" | 3-(6-Oxo-1-{3-[5-(1-piperidin-4-yl-1 H-pyrazol-4-yl)-pyrimidin-2-yl]-benzyl}-1,6-dihydro-pyridazin-3-yl)-benzonitril | 515 |
| | 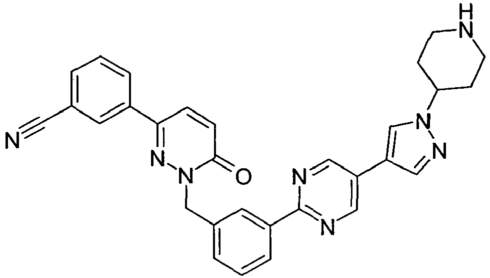 | |
| "A151" | 6-(1-Methyl-1 H-pyrazol-4-yl)-2-{3-[5-(1-piperidin-4-yl-1 H-pyrazol-4-yl)-pyrimidin-2-yl]-benzyl}-2H-pyridazin-3-on, Trifluoracetat | 494 |
| | 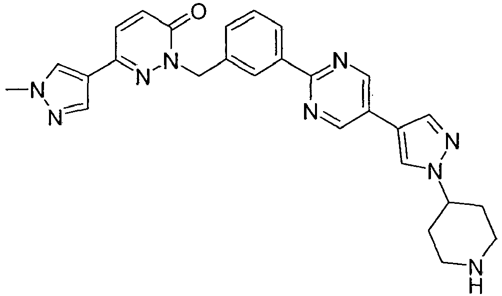 | |
| "A152" | 6-(3-Methoxy-phenyl)-2-{3-[5-(1 -piperidin-4-yl-1 H-pyrazol-4-yl)-pyrimidin-2-yl]-benzyl}-2H-pyridazin-3-on, Trifluoracetat | 520 |
| "A153" | 6-(3-Fluor-phenyl)-2-{3-[5-(1-piperidin-4-yl-1 H-pyrazol-4-yl)-pyrimidin-2-yl]-benzyl}-2H-pyridazin-3-on | 508 |
| "A154" | 6-(3,5-Difluor-phenyl)-2-{3-[5-(1H-pyrazol-4-yl)-pyrimidin-2-yl]-benzyl}-2H-pyridazin-3-on | 443 |
| | 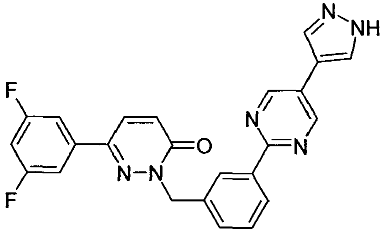 | |
| "A155" | 6-(3,5-Difluor-phenyl)-2-(3-{5-[1-(2-methylamino-ethyl)-1H-pyrazol-4-yl]-pyrimidin-2-yl}-benzyl)-2H-pyridazin-3-on, Hydrochlorid | 500 |
| | 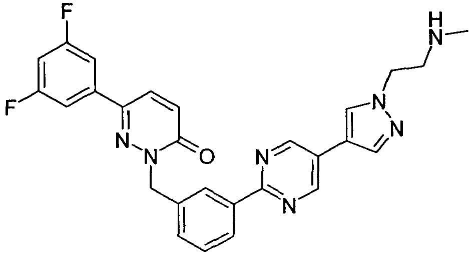 | |
| "A156" | 6-(3-Chlor-phenyl)-2-{3-[5-(1-piperidin-4-yl-1H-pyrazol-4-yl)-pyrimidin-2-yl]-benzyl}2H-pyridazin-3-on, Hydrochlorid | 524 |
| | 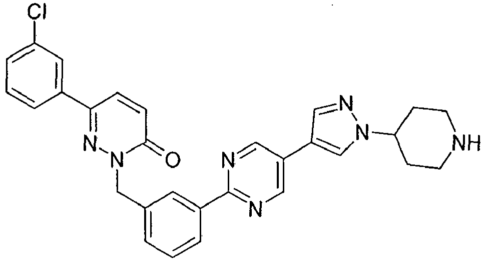 | |
| "A157" | 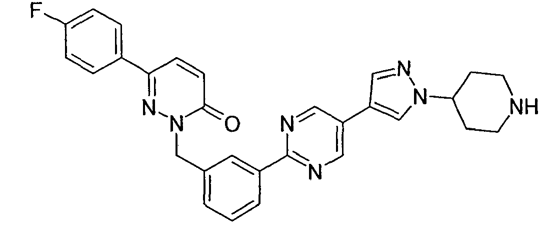 Hydrochlorid | 508 |
| "A158" | 6-(3,5-Difluor-phenyl)-2-(3-{5-[1-(3-methylamino-propyl)-1H-pyrazol-4-yl]-pyrimidin-2-yl}-benzyl)-2H-pyridazin-3-on, Hydrochlorid | 514 |
| | 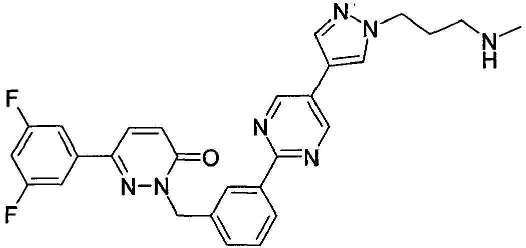 | |
| "A159" | 3-[1-(3-{5-[1-(2-Methylamino-ethyl)-1 H-pyrazol-4-yl]-pyrimidin-2-yl}-benzyl)-6-oxo-1,6-dihydro-pyridazin-3-yl]-benzonitril | 489 |
| | 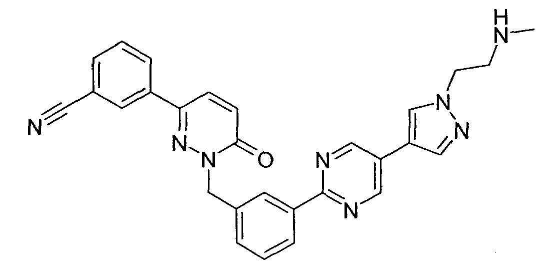 | |
| ¹H-NMR (d₆-DMSO): δ [ppm] = 2.1 (bs, 1H), 2.35 (s, 3H), 2.97 (t, J = 6.2 Hz, 2H), 4,28 (t, J = 6.2 Hz, 2H), 5.53 (s, 2H), 7.23 (d, J = 9.8 Hz, 1 H), 7.58 (m, 2 Hz, 2H), 7.79 (t, J = 8.0 Hz, 1 H), 8.00 (dt, J₁ = 7.5 Hz, J₂ = 1.2 Hz, 1 H), 8.16 (s, 1 H), 8.25 (d, J = 9.8 Hz, 1 H), 8.32 (dt, J₁ = 7.5 Hz, J₂ = 1.2 Hz, 1H), 8.38 (dt, J₁ = 6.5 Hz, J₂ = 1.8 Hz, 1H), 8.45 (t, J = 1.6 Hz, 1H), 8.46 (s, 1H), 8.53 (bs, 1 H), 9.20 (s, 2H) | | |
| "A160" | 6-(3,5-Difluor-phenyl)-2-(3-{5-[1-(2-piperazin-1-yl-ethyl)-1H-pyrazol-4-yl]-pyrimidin-2-yt}-benzyl)-2H-pyridazin-3-on | 555 |
| | 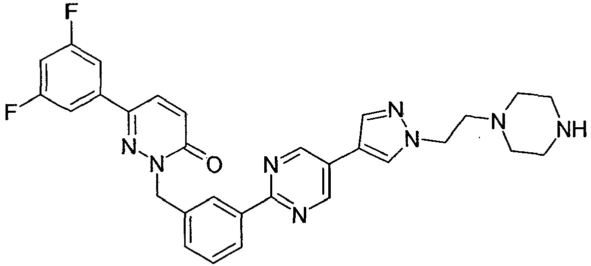 | |
| "A161" | 3-(6-Oxo-1-{3-[5-(1H-pyrazol-4-yl)-pyrimidin-2-yl]-benzyl}-1,6-dihydro-pyridazin-3-yl)-benzonitril | 432 |
| | 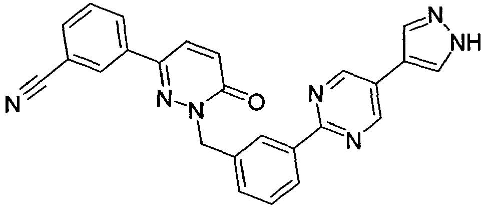 | |
| ¹H-NMR (d₆-DMSO): δ [ppm] = 5.48 (s, 2H), 7.18 (d, J = 10 Hz, 1H), 7.53 (t, J = 7.5 Hz, 1H), 7.55 (m, 1H), 7.73 (t, J = 7.8 Hz, 1H), 7.94 (dt, J₁ = 7.5 Hz, J₂ = 1.2 Hz, 1H), 8.15 (bs, 1H), 8.19 (d, J = 10 Hz, 1H), 8.27 (dt, J₁ = 7.5 Hz, J₂ = 1.2 Hz, 1 H), 8.33 (dt, J₁ = 6.5 Hz, J₂ = 1.8 Hz, 1 H), 8.39 (t, J = 1.6 Hz, 1 H), 8.44 (bs, 1 H), 8.48 (bs, 1 H), 9.18 (s, 2H), 13.2 (bs, 1 H) | | |
| "A162" | 3-[6-Oxo-1-(3-{5-[1-(2-piperazin-1-yl-ethyl)-1H-pyrazol-4-yl]-pyrimidin-2-yl}-benzyl)-1,6-dihydro-pyridazin-3-yl]-benzonitril | 544 |
| | 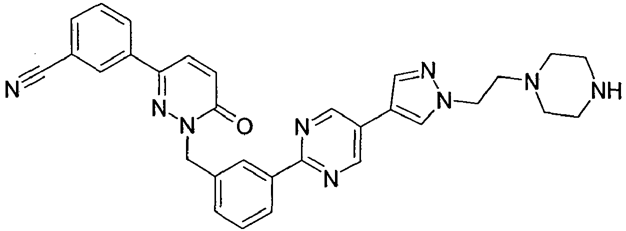 | |

### Beispiel 34

### Analog Beispiel 16 erhält man die nachstehenden Verbindungen

| Verbindung Nr. | Name und/oder Struktur | ESI [M+H]⁺ | HPLC (Rt. in min) Methode |
|---|---|---|---|
| "A163" | 2-[3-(6-Oxo-3-pyridin-4-yl-6H-pyridazin-1-ylmethyl)-phenyl]-pyrimidin-5-carbonsäure-(4-dimethylamino-butyl)-amid, Trifluoracetat | 484 | |
| | 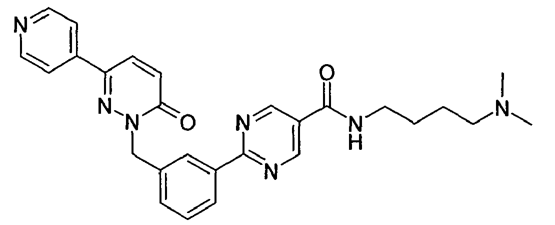 | | |
| "A164" | 2-{3-[3-(4-Cyan-phenyl)-6-oxo-6H-pyridazin-1-ylmethyl]-phenyl}-pyrimidin-5-carbonsäure-(4-dimethylamino-butyl)-amid, Trifluoracetat | 508 | |
| | 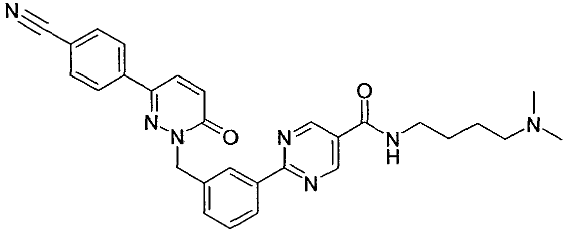 | | |

### Beispiel 35

### Analog Beispiel 27 erhält man die nachstehenden Verbindungen

| Verbindung Nr. | Name und/oder Struktur | ESI [M+H]⁺ | HPLC (Rt. in min) Methode B |
|---|---|---|---|
| "A165" | 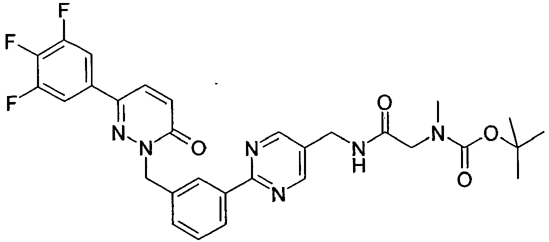 | 595 | 3.18 |
| Durch Boc-Abspaltung erhält man daraus die Verbindung "A98". | | | |
| "A166" | 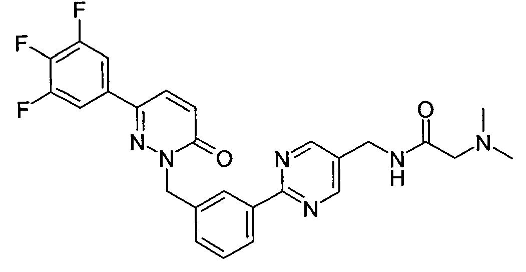 | 509 | 2.59 |
| "A167" | 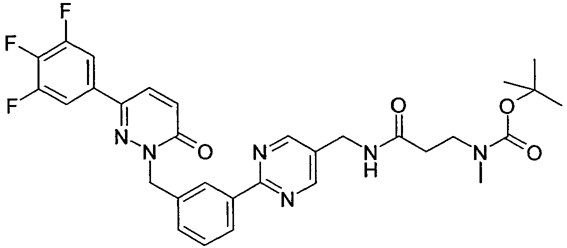 | 631 (M+Na | 3.22 |
| Durch Boc-Abspaltung erhält man daraus die Verbindung "A168", Trifluoracetat, ESI 509 | | | |
| | 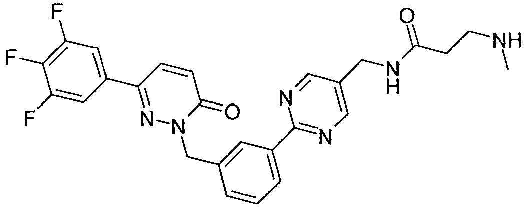 | | |

### Beispiel 36

Die Herstellung der Verbindung 3-[6-Oxo-1-(3-{5-[1-(2-pyrrolidin-1-yl-ethyl)-1H-pyrazol-4-yl]-pyrimidin-2-yl}-benzyl)-1,6-dihydro-pyridazin-3-yl]-benzonitril ("A168") erfolgt analog nachstehendem Schema 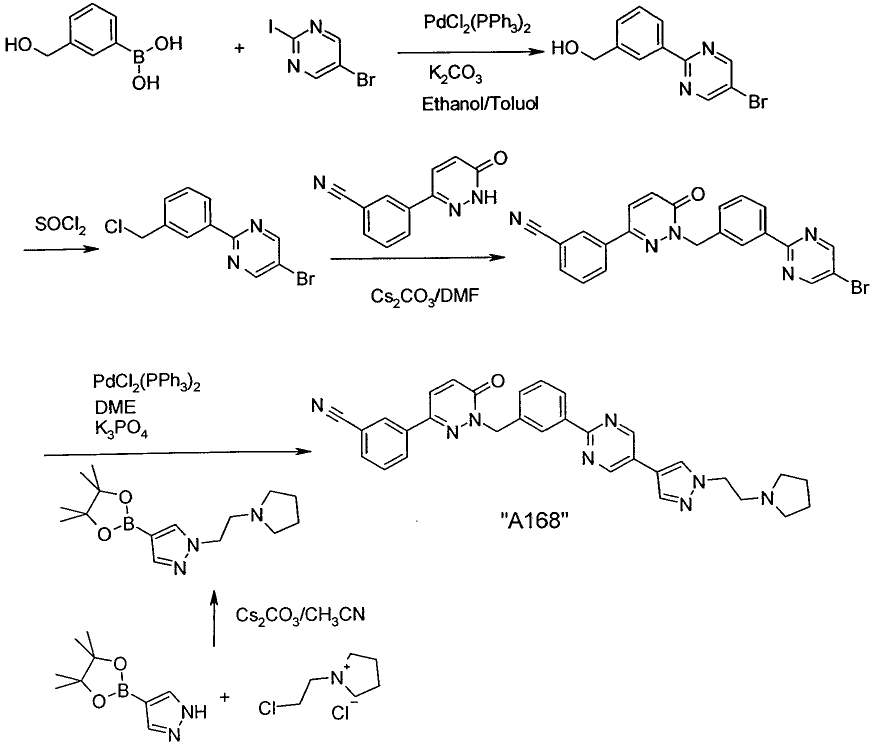
36.1 Eine unter Stickstoff gehaltene Lösung von 95.0 g (332 mmol) 5-Brom-2-iodpyrimidin in 325 ml Toluol wird mit einer Lösung von 70.0 g (660 mmol) Natriumcarbonat in 325 ml Wasser versetzt und das Gemisch auf 80° C erhitzt. Dazu werden 2.3 g (3.3 mmol) Bis(triphenylphosphin)-palladium(II)-chlorid gegeben und anschließend eine Lösung von 50.0 g (329 mmol) 3-(Hydroxymethyl)-benzolboronsäure in 650 ml Ethanol zugetropft. Das Reaktionsgemisch wird 18 Stunden bei 80° C gerührt. Das Reaktionsgemisch wird auf Raumtemperatur abgekühlt und filtriert. Das Filtrat wird mit 1 l Ethylacetat und 1 l Wasser versetzt. Die organische Phase wird abgetrennt, über Natriumsulfat getrocknet und eingedampft. Der Rückstand wird aus 2-Propanol umkristallisiert: [3-(5-Brompyrimidin-2-yl)-phenyl]-methanol als hellgelbe Kristalle; ESI 265,267.
36.2 Zu 159 ml (2.19 mol) auf 30° C gehaltenem Thionylchlorid wird unter Rühren portionsweise 116 g (438 mmol) [3-(5-Brompyrimidin-2-yl)-phenyl]-methanol gegeben. Die Reaktionslösung wird 18 Stunden bei Raumtemperatur gerührt. Das Reaktionsgemisch wird eingedampft. Der Rückstand wird in Toluol aufgenommen und erneut eingedampft. Diese Prozedur wird dreimal wiederholt. Der Rückstand wird aus Toluol umkristallisiert: 5-Brom-2-(3-chlormethyl-phenyl)-pyrimidin als farblose Kristalle; F. 148°C; ESI 283, 285, 286.
36.3 Eine Suspension von 61.1 g (310 mmol) 3-(6-Oxo-1,6-dihydro-pyridazin-3-yl)-benzonitril in 600 ml DMF wird mit 87.9 g (310 mmol) 5-Brom-2-(3-chlormethyl-phenyl)-pyrimidin und 111 g (341 mmol) Caesiumcarbonat versetzt und 24 Stunden bei 40° C gerührt. Das Reaktionsgemisch wird auf 600 ml Wasser gegeben. Der entstandene Niederschlag wird abgesaugt, mit Wasser gewaschen und im Vakuum getrocknet: 3-{1-[3-(5-Brompyrimidin-2-yl)-benzyl]-6-oxo-1,6-dihydro-pyridazin-3-yl}-benzonitril als beige Kristalle, ESI 444, 446.
36.4 Eine Lösung von 10.0 g (50.5 mmol) Pyrazol-4-boronsäure-pinacolester wird in 100 ml Acetonitril gelöst und mit 17.5 g (101 mmol) N-(2-Chlorethyl)-pyrrolidin-hydrochlorid und 49.4 g (152 mmol) Caesiumcarbonat versetzt. Die entstandene Suspension wird 18 Stunden bei Raumtemperatur gerührt. Das Reaktionsgemisch wird abgesaugt und mit Acetonitril gewaschen Das Filtrat wird eingedampft und zwischen Ethylacetat und gesättigter Natriumchlorid-Lösung verteilt. Die organische Phase wird über Natriumsulfat getrocknet und eingedampft: 1-(2-Pyrrolidin-1-yl-ethyl)-4-(4,4,5,5-tetramethyl-[1,3,2]dioxaborolan-2-yl)-1H-pyrazol als helloranges Öl, das allmählich kristallisiert;
   ¹H-NMR (d₆-DMSO): δ [ppm] = 1.25 (s, 12H), 1.65 (m, 4H), 2.44 (m, 4H), 2.79 (t, J = 6.8 Hz, 2H), 4.21 (t, J = 6.8 Hz, 2H), 7.56 (s, 1H), 7.93 (s, 1H).
36.5 Eine Suspension von 2.09 g (4.71 mmol) 3-{1-[3-(5-Brompyrimidin-2-yl)-benzyl]-6-oxo-1,6-dihydro-pyridazin-3-yl}-benzonitril, 1.73 g (5.18 mmol) 1-(2-Pyrrolidin-1-yl-ethyl)-4-(4,4,5,5-tetramethyl-[1,3,2]dioxaborolan-2-yl)-1 H-pyrazol (Gehalt 87%) und 2.00 g (9.42 mmol) Trikaliumphosphat-Trihydrat in 20 ml 1,2-Dimethoxyethan wird unter Stickstoff auf 85° C erhitzt. Dann werden 264 mg (0.377 mmol) Bis(triphenylphosphin)-palladium(II)chlorid und 79 µl (0.57 mmol) Triethylamin zugegeben und 18 Stunden bei 85° C gerührt. Das Reaktionsgemisch wird mit 30 ml Dichlormethan versetzt und über Kieselgur abgesaugt. Das Filtrat wird 100 ml Wasser, 20 ml 2 N NaOH und 50 ml Dichlormethan versetzt. Die organische Phase wird abgetrennt, über Natriumsulfat getrocknet und eingedampft. Der Rückstand wird an einer Kieselgelsäule mit Dichlormethan/Methanol chromatographiert: 3-[6-Oxo-1-(3-{5-[1-(2-pyrrolidin-1-yl-ethyl)-1H-pyrazol-4-yl]-pyrimidin-2-yl}-benzyl)-1,6-dihydro-pyridazin-3-yl]-benzonitril als beige Kristalle; ESI 529;
   ¹H-NMR (d₆-DMSO): δ [ppm] = 1.68 (m, 4H), 2.49 (m, 2H), 2.88 (m, 2H), 3.32 (m, 2H), 4.28 (t, J = 6.8 Hz, 2H), 5.48 (s, 2H), 7.17 (d, J = 10 Hz, 1H), 7.52 (t, J = 7.3 Hz, 1H), 7.55 (m, 1H), 7.73 (t, J = 7.8 Hz, 1H), 7.94 (d, J = 8 Hz, 1H), 8.09 (s, 1H), 8.19 (d, J = 10 Hz, 1H), 8.26 (d, J = 8 Hz, 1H), 8.33 (dt, J₁ = 7.2 Hz, J₂ = 1. 8 Hz, 1 H), 8.39 (t, J = 1.8Hz, 1 H), 8.43 (s, 1 H), 8.48 (bs, 1H),9.14(s, 2H).

### Analog erhält man die nachstehenden Verbindungen

| Verbindung Nr. | Name und/oder Struktur | ESI [M+H]⁺ | |
|---|---|---|---|
| "A169" | 6-(1-Methyl-1H-pyrazol-4-yl)-2-(3-{5-[1-(2-pyrrolidin-1-yl-ethyl)-1 H-pyrazol-4-yl]-pyrimidin-2-yl}-benzyl)-2H-pyridazin-3-on | 508 | |
| | 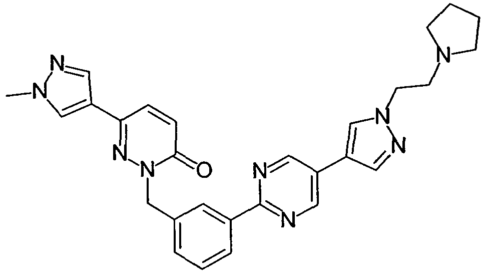 | | |
| ¹H-NMR (d₆-DMSO): δ [ppm] = 1.68 (m, 4H), 2.50 (m, 2H), 2.88 (m, 2H), 3.31 (m, 2H), 3.88 (s, 3H), 4.28 (t, J = 6.8 Hz, 2H), 5.37 (s, 2H), 7.07 (d, J = 9.5 Hz, 1H), 7.50 (m, 2H), 7.83 (d, J = 9.5 Hz, 1H), 7.91 (s, 1H), 8.10 (s, 1H), 8.23 (s, 1 H), 8.32 (d, J = 7.3 Hz, 1 H), 8.38 (bs, 1 H), 8.44 (s, 1 H), 9.14 (s, 2H). | | | |
| "A170" | 6-(3,5-Difluor-phenyl)-2-(3-{5-[1-(2-morpholin-4-yl-ethyl)-1H-pyrazol-4-yl]-pyrimidin-2-yl}-benzyl)-2H-pyridazin-3-on | 556 | |
| | 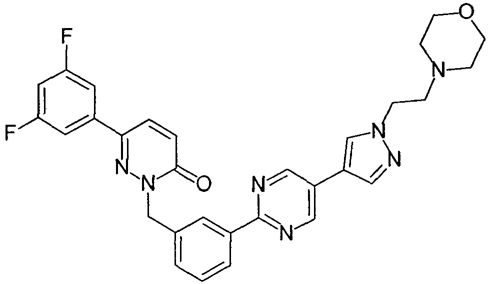 | | |
| "A171" | 6-(3,5-Difluor-phenyl)-2-(3-{5-[1-(2-dimethylamino-ethyl)-1H-pyrazol-4-yl]-pyrimidin-2-yl}-benzyl)-2H-pyridazin-3-on | 514 | |
| | 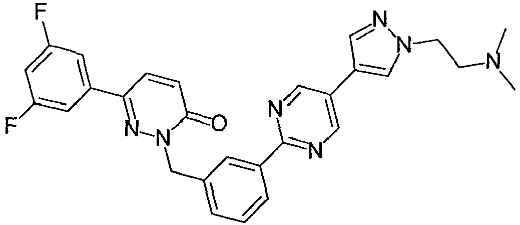 | | |
| "A172" | 6-(3,5-Difluor-phenyl)-2-(3-{5-[1-(3-dimethylamino-propyl)-1H-pyrazol-4-yl]-pyrimidin-2-yl)-benzyl)-2H-pyridazin-3-on | 528 | |
| "A173" | 6-(3,5-Difluor-phenyl)-2-(3-{5-[1-(2-pyrrolidin-1-yl-ethyl)-1H-pyrazol-4-yl]-pyrimidin-2-yl}-benzyl)-2H-pyridazin-3-on | 540 | |
| "A174" | 3-[1-(3-{5-[1-(2-Morpholin-4-yl-ethyl)-1 H-pyrazol-4-yl]-pyrimidin-2-yl}-benzyl)-6-oxo-1,6-dihydro-pyridazin-3-yl]-benzonitril | 545 | |
| | 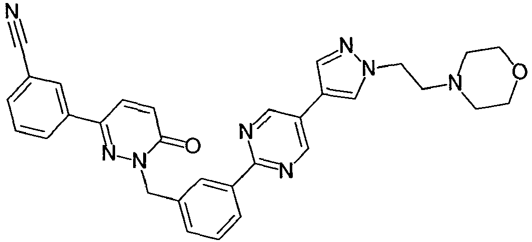 | | |
| ¹H-NMR (d₆-DMSO): δ [ppm] = 2.43 (m, 4H), 2.75 (t, J = 6.5 Hz, 2H), 3.56 (m, 4H), 4.29 (t, J = 6.5 Hz, 2H), 5.47 (s, 2H), 7.17 (d, J = 10 Hz, 1H), 7.53 (m, 2H), 7.72 (t, J = 7.8 Hz, 1 H), 7.93 (dt, J₁ = 7.7, J₂ = 1.3 Hz, 1 H), 8.09 (s, 1H), 8.18 (d, J = 10 Hz, 1H), 8.26 (d, J = 8 Hz, 1H), 8.32 (dt, J₁ = 7 Hz, J₂ = 1.5 Hz, 1 H), 8.38 (t, J = 1.6 Hz, 1 H), 8.42 (s, 1 H), 8.47 (bs, 1 H), 8.13 (s, 2H) | | | |
| "A175" | 2-(3-{5-[1-(2-Morpholin-4-yl-ethyl)-1H-pyrazol-4-yl]-pyrimidin-2-yl}-benzyl)-6-pyridin-3-yl-2H-pyridazin-3-on, Trifluoracetat | 521 | |
| | 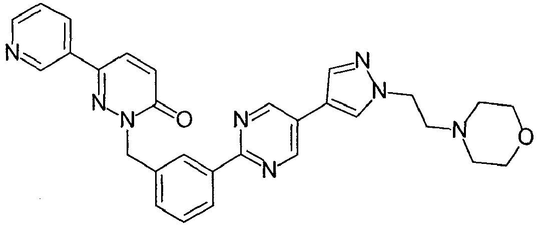 | | |
| "A176" | 6-(1-Methyl-1H-pyrazol-4-yl)-2-(3-{5-[1-(2-morpholin-4-yl-ethyl)-1H-pyrazol-4-yl]-pyrimidin-2-yl}-benzyl)-2H-pyridazin-3-on, Trifluoracetat | 524 | |
| | 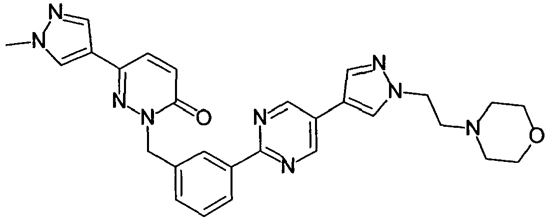 | | |
| "A177" | 2-(3-{5-[1-(2-Morpholin-4-yl-ethyl)-1H-pyrazol-4-yl]-pyrimidin-2-yl}-benzyl)-6-pyridin-4-yl-2H-pyridazin-3-on, Trifluoracetat | 521 | |
| "A178" | 6-(4-Methansulfonyl-phenyl)-2-(3-{5-[1-(2-morpholin-4-yl-ethyl)-1H-pyrazol-4-yl]-pyrimidin-2-yl}-benzyl)-2H-pyridazin-3-on, Hydrochlorid | 598 | |
| "A179" | 6-Pyridin-4-yl-2-(3-{5-[1-(2-pyrrolidin-1-yl-ethyl)-1 H-pyrazol-4-yl]-pyrimidin-2-yl}-benzyl)-2H-pyridazin-3-on | 505 | |
| ¹H-NMR (d₆-DMSO): δ [ppm] = 1.75 (b, 4H), 2.68 (b, 4H), 3.1 (b, 2H), 4.36 (b, 2H), 5.49 (s, 2H), 7.19 (d, J = 9.5 Hz, 1H), 7.54 (m, 2H), 7.91 (d, J = 6.5 Hz, 2H), 8.14 (bs, 1H), 8.18 (d, J =9.5 Hz, 1H), 8.33 (dt, J₁ = 6.5 Hz, J₂ = 1.8 Hz, 1H), 8.46 (m, 2H), 8.72 (d, J = 6.5 Hz, 2H), 9.15 (s, 2H) | | | |
| "A180" | 4-[6-Oxo-1-(3-{5-[1-(2-pyrrolidin-1-yl-ethyl)-1H-pyrazol-4-yl]-pyrimidin-2-yl}-benzyl)-1,6-dihydro-pyridazin-3-yl benzonitril | 529 | |
| | 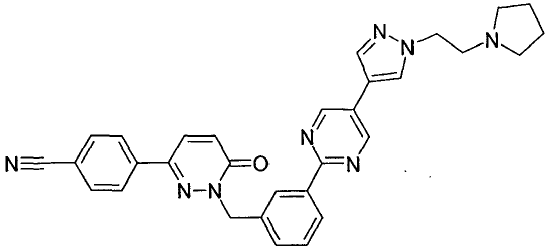 | | |
| ¹H-NMR (d₆-DMSO): δ [ppm] = 1.68 (m, 4H), 2.51 (m, 4H), 2.90 (m, 2H), 4.28 (m, 2H), 5.47 (s, 2H), 7.17 (d, J = 9.5 Hz, 1H), 7.52 (m, 2H), 7.98 (d, J = 9 Hz, 2H), 8.10 (s, 1H), 8.12 (d, J = 9 Hz, 2H), 8.17 (d, J =9.5 Hz, 1H), 8.38 (dt, J₁ = 6.5 Hz, J₂ = 1.8 Hz, 1 H), 8.44 (s, 1 H), 8.45 (bs, 1 H), 9.13 (s, 2H) | | | |
| "A181" | 2-(3-{5-[1-(2-Morpholin-4-yl-ethyl)-1H-pyrazol-4-yl]-pyrimidin-2 -yl}-benzyl)-6-pyridin-4-yl-2H-pyridazin-3-on | 521 | |
| "A183" | 6-(4-Methansulfonyl-phenyl)-2-(3-{5-[1-(2-morpholin-4-yl-ethyl)-1H-pyrazol-4-yl]-pyrimidin-2-yl}-benzyl)-2H-pyridazin-3-on | 598 | |
| "A184" | 6-(5-Methyl-oxazol-2-yl)-2-(3-{5-[1-(2-pyrrolidin-1-yl-ethyl)-1H-pyrazol-4-yl]-pyrimidin-2-yl}-benzyl)-2H-pyridazin-3-on | 509 | |
| | 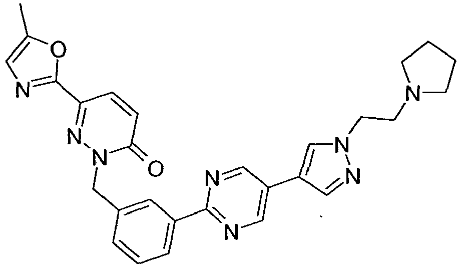 | | |
| "A185" | 6-(3-Fluor-phenyl)-2-(3-{5-[1-(2-pyrrolidin-1-yl-ethyl)-1H-pyrazol-4-yl]-pyrimidin-2-yl}-benzyl)-2H-pyridazin-3-on | 522 | |
| "A186" | 6-(1-Propyl-1H-pyrazol-4-yl)-2-(3-{5-[1-(2-pyrrolidin-1-yl-ethyl)-1H-pyrazol-4-yl]-pyrimidin-2-yl}-benzyl)-2H-pyridazin-3-on | 536 | |
| | 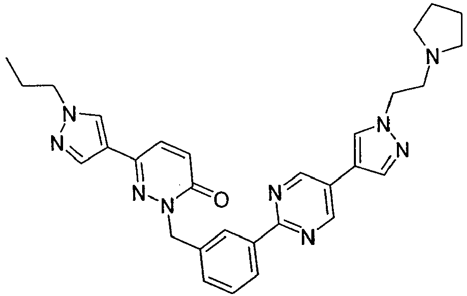 | | |
| "A187" | 2-(3-{5-[1-(2-Pyrrolidin-1-yl-ethyl)-1H-pyrazol-4-yl]-pyrimidin-2-yl}-benzyl)-6-thiophen-3-yl-2H-pyridazin-3-on | 510 | |
| | 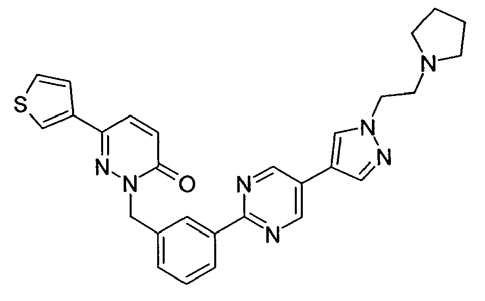 | | |
| "A188" | 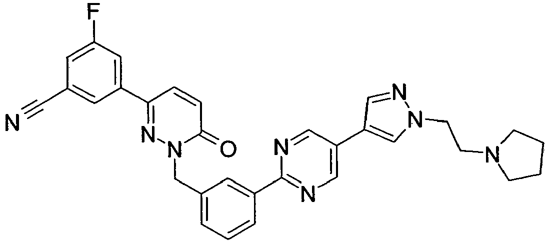 | | |
| "A188a" | 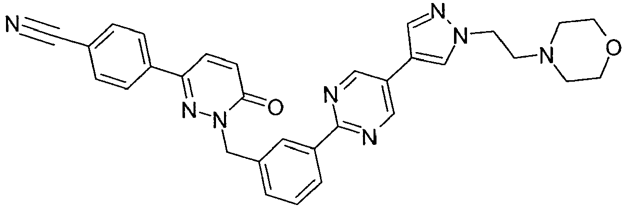 | | |

### Beispiel 37

Die Herstellung der Verbindung 3-(1-{3-[5-(3-Dimethylamino-propoxy)-pyrimidin-2-yl]-benzyl}-6-oxo-1,6-dihydro-pyridazin-3-yl)-benzonitril ("A189") erfolgt analog nachstehendem Schema 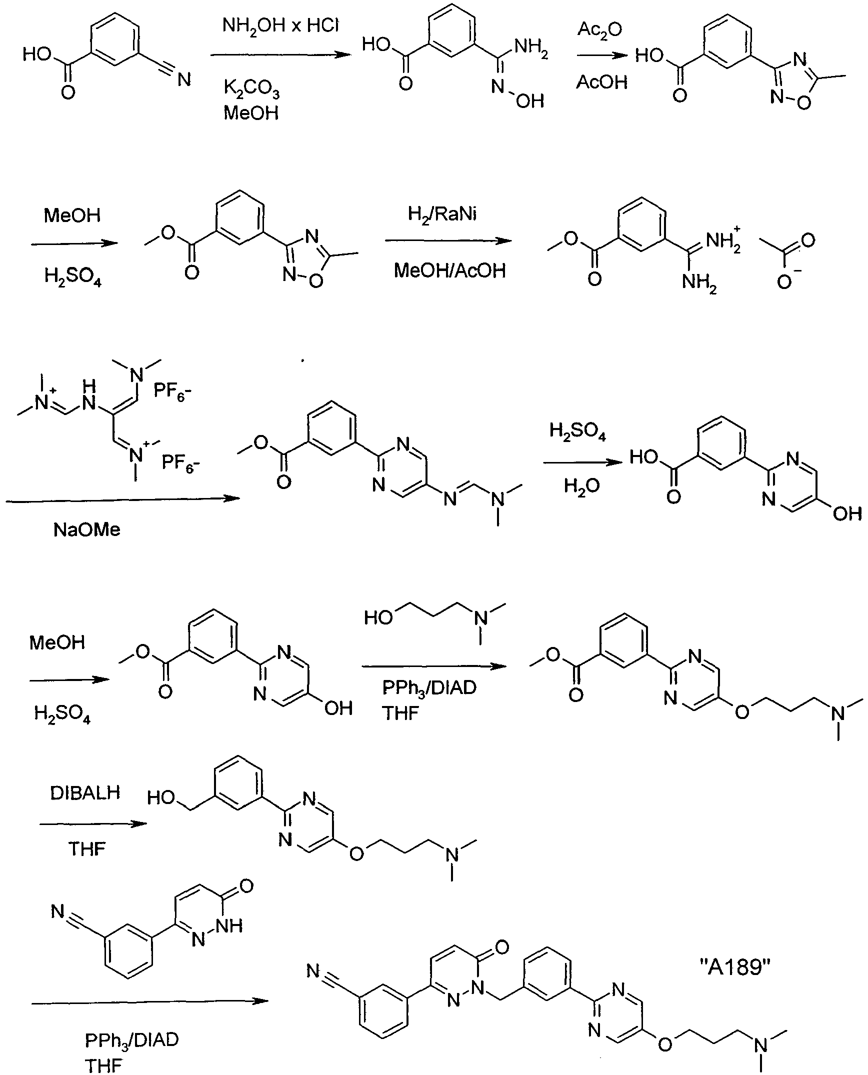
37.1 Zu einer auf 30° C gehaltenen Suspension von 500 g (3.40 mol) 3-Cyan-benzoesäure in 8 I Methanol werden unter Rühren portionsweise 1382 g (10.0 mol) Kaliumcarbonat gegeben. Anschließend werden bei einer Innentemperatur von 40- 45° C 695 g (10.0 mol) Hydroxylammoniumchlorid in kleinen Portionen zugegeben. Dann wird das Reaktionsgemisch 15 Stunden zum Sieden erhitzt. Das Reaktionsgemisch wird im Vakuum eingeengt, der Rückstand in Wasser gelöst und mit 37%iger wässriger Salzsäure angesäuert. Der entstandene Niederschlag wird abgesaugt, mit Wasser gewaschen und im Vakuum getrocknet: 3-(N-Hydroxycarbamimidoyl)-benzoesäure als farblose Kristalle; ESI 181.
37.2 Ein Gemisch von 614 g (3.41 mol) 3-(N-Hydroxycarbamimidoyl)-benzoesäure, 756 ml (8.0 mol) Essigsäureanhydrid und 2 I Essigsäure wird 14 Stunden auf eine Temperatur von 118° C erhitzt. Das Reaktionsgemisch wird auf 6° C gekühlt und abgesaugt. Der Rückstand wird in 2 I Wasser aufgenommen, abgesaugt und gut mit Wasser gewaschen. Der Rückstand wird aus Ethanol/Wasser umkristallisiert: 3-(5-Methyl-[1,2,4]-oxadiazol-3-yl)-benzoesäure als farblose Kristalle; F. 225° C; ESI 205.
37.3 Eine Suspension von 30.0 g (147 mmol) 3-(5-Methyl-[1,2,4]oxa-diazol-3-yl)-benzoesäure in 150 ml Methanol wird mit 7.83 ml (147 mmol) konzentrierter Schwefelsäure versetzt und 18 Stunden zum Sieden erhitzt. Das Reaktionsgemisch wird im Eisbad gekühlt, mit Wasser versetzt, abgesaugt und gut mit Wasser gewaschen: 3-(5-Methyl-[1,2,4]oxadiazol-3-yl)-benzoesäuremethylester als farblose Kristalle; ESI 219.
37.4 Eine Lösung von 327 g (1.47 mol) 3-(5-Methyl-[1,2,4]oxadiazol-3-yl)-benzoesäuremethylester in 3 I Methanol wird mit 150 ml Essigsäure, 150 ml Wasser und 50 g wasserfeuchtem Raney-Nickel versetzt und 18 Stunden bei Raumtemperatur und Normaldruck hydriert. Der Katalysator wird abfiltriert und das Filtrat eingedampft. Der Rückstand wird in tert.-Butylmethylether aufgenommen, zum Sieden erhitzt und abgesaugt. Der Rückstand wird im Vakuum getrocknet: 3-Methoxycarbonylbenzamidinium-Acetat als farblose Kristalle; ESI 179.
37.5 Zu einer Suspension von 259 g (1.09 mol) 3-Methoxycarbonylbenzamidinium-Acetat und 528 g (1.08 mol) ({2-Dimethylamino-1-[dimethylimmoniomethyl]-vinylamino}-methylen)-dimethyl-ammonium-di-hexafluorophosphat (hergestellt gemäß C. B. Dousson et al., Synthesis 2005, 1817) in 1 I Methanol werden unter Rühren 2.2 I einer frisch bereiteten 1.5 M Natriummethanolat-Lösung zugetropft. Dann wird das Reaktionsgemisch innerhalb von 40 min auf 60° C erwärmt und 30 min bei dieser Temperatur gehalten. Dann wird das Reaktionsgemisch auf Raumtemperatur abgekühlt, mit 10 I Dichlormethan verdünnt und dreimal mit je 5 I Wasser gewaschen. Die organische Phase wird über Natriumsulfat getrocknet und eingedampft. Der Rückstand wird aus Ethylacetat umkristallisiert: 3-[5-(Dimethylamino-methylenamino)-pyrimidin-2-yl]-benzoesäuremethylester als beige Kristalle; F. 140° C, ESI 285
37.6 Eine Suspension von 103.5 g (364 mmol) 3-[5-(Dimethylamino-methylenamino)-pyrimidin-2-yl]-benzoesäuremethylester in 1.3 I Wasser wird mit 160 ml (2.88 mol) konzentrierter Schwefelsäure versetzt und 4 Stunden zum Sieden erhitzt. Das Reaktionsgemisch wird auf Raumtemperatur abgekühlt, mit Wasser verdünnt und abgesaugt. Der Rückstand wird mit Wasser gewaschen und im Vakuum getrocknet: 3-(5-Hydroxypyrimidin-2-yl)-benzoesäure als bräunliche Kristalle; ESI 217.
37.7 Eine Suspension von 88.0 g (366 mmol) 3-(5-Hydroxypyrimidin-2-yl)-benzoesäure in 1.4 I Methanol wird mit 32.7 ml (445 mmol) Thionylchlorid versetzt und 2 Stunden auf 80° C erhitzt. Dann werden 20 ml (276 mmol) Thionylchlorid und nach 2 Stunden noch mal 10 ml (138 mmol) Thionylchlorid zugegeben. Nach jeder Zugabe wird das Reaktionsgemisch 2 Stunden bei 80° C gerührt. Das Reaktionsgemisch wird im Vakuum auf ein Volumen von ca. 300 ml eingeengt. Der entstandene Niederschlag wird abfiltriert und im Vakuum getrocknet: 3-(5-Hydroxypyrimidin-2-yl)-benzoesäuremethylester als bräunliche Kristalle; ESI 231.
37.8 Eine unter Stickstoff gehaltene Lösung von 6.1 g (26.5 mmol) 3-(5-Hydroxypyrimidin-2-yl)-benzoesäuremethylester, 10.5 g (39.8 mmol) Triphenylphosphin und 4.76 ml (39.8 mmol) 3-(Dimethylamino)-1-propanol in 200 ml THF wird im Eisbad gekühlt und langsam unter Rühren 8.21 ml (39.8 mmol) Diisopropylazodicarboxylat zugetropft. Nach 2 Stunden Rühren bei Raumtemperatur wird das Reaktionsgemisch im Vakuum eingedampft. Der Rückstand wird zwischen Dichlormethan und gesättigter wässriger Kaliumhydrogensulfat-Lösung verteilt. Die wässrige Phase wird abgetrennt, mit gesättigter wässriger Natronlauge auf einen pH-Wert von 12 gebracht und zweimal mit Dichlormethan extrahiert. Die organische Phase wird über Natriumsulfat getrocknet und eingedampft. Der Rückstand wird an einer Kieselgelsäule mit Dichlormethan/Methanol als Laufmittel chromatographiert: 3-[5-(3-Dimethylamino-propoxy)-pyrimidin-2-yl]-benzoesäuremethylester als farblose Kristalle; ESI 316.
37.9 Zu einer unter Stickstoff gehaltenen Lösung von 12.6 g (40.0 mmol) 3-[5-(3-Dimethylamino-propoxy)-pyrimidin-2-yl]-benzoesäure-methylester in 200 ml THF werden unter Rühren 200 ml einer 1 M Lösung von Diisobutylaluminiumhydrid in THF zugetropft. Nach 1 Stunde Rühren bei Raumtemperatur werden 10 ml einer gesättigten wässrigen Natriumsulfat-Lösung zugetropft. Der entstandene Niederschlag wird abgesaugt und mit Dichlormethan gewaschen. Das Filtrat wird über Natriumsulfat getrocknet und eingedampft. Der Rückstand wird in einem Gemisch von Diethylether und Petrolether aufgenommen. Der entstandene Niederschlag wird abgesaugt, mit Petrolether gewaschen und im Vakuum getrocknet: {3-[5-(3-Dimethylamino-propoxy)-pyrimidin-2-yl]-phenyl}-methanol als farblose Kristalle; F. 95- 97 °C; ESI 288.
37.10 Eine Lösung von 5.06 g (17.6 mmol) {3-[5-(3-Dimethylamino-propoxy)-pyrimidin-2-yl]-phenyl}-methanol in 100 ml THF wird mit 3.16 g (18.0 mmol) 3-(6-Oxo-1,6-dihydro-pyridazin-3-yl)-benzonitril und 6.36 g (24.0 mmol) Triphenylphosphin versetzt. Die entstandene Suspension wird im Eisbad gekühlt und langsam 4.96 ml (24.0 mmol) Diisopropylazodicarboxylat zugetropft. Nach 1 Stunde Rühren bei Raumtemperatur wird tert.-Butylmethylether und 1 N wässrige Salzsäure zugegeben. Die wässrige Phase wird abgetrennt und dreimal mit tert.-Butylmethylether gewaschen. Die wässrige Phase wird mit 2 N Natronlauge auf einen pH-Wert von 14 gebracht und zweimal mit Dichlormethan extrahiert. Die organische Phase wird über Natriumsulfat getrocknet und eingedampft. Der Rückstand an einer Kieselgelsäule mit Dichlormethan/Methanol chromatographiert: 3-(1-{3-[5-(3-Dimethylamino-propoxy)-pyrimidin-2-yl]-benzyl}-6-oxo-1,6-dihydro-pyridazin-3-yl)-benzonitril als farblose Kristalle; F. 128° C; ESI 467;
   ¹H-NMR (d₆-DMSO): δ [ppm] = 1.89 (quintett, J = 6.8 Hz, 2H), 2.15 (s, 6H), 2.37 (t, J = 7 Hz, 2H), 4.21 (t, J = 6.5 Hz, 2H), 5.44 (s, 2H), 7.16 (d, J = 10 Hz, 1 H), 7.48 (m, 2H), 7.72 (t, J = 7.8 Hz, 1 H), 7.92 (dt, J₁ = 7.5 Hz, J₂ = 1.2 Hz, 1H), 8.17 (d, J = 10 Hz, 1H), 8.23 (m, 2H), 8.37 (t, J = 1.6 Hz, 1 H), 8.39 (bs, 1 H), 8.63 (s, 2H).

### Analog erhält man die nachstehenden Verbindungen

"A114", "A24",

| Verbindung Nr. | Name und/oder Struktur | ESI [M+H]⁺ |
|---|---|---|
| "A190" | 2-{3-[5-(3-Dimethylamino-propoxy)-pyrimidin-2-yl]-benzyl}-6-(1-methyl-1H-pyrazol-4-yl)-2H-pyridazin-3-on, Trifluoracetat | 446 |
| | 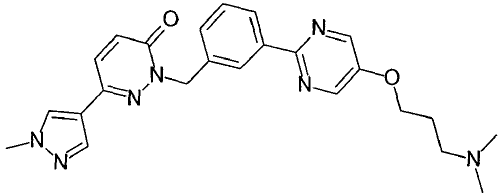 | |
| "A191" | 2-{3-[5-(3-Dimethylamino-popoxy)-pyrimidin-2-yl]-benzyl}-6-(3-fluor-phenyl)-2H-pyridazin-3-on, Hydrochlorid | 460 |
| "A192" | 2-{3-[5-(3-Dimethylamino-propoxy)-pyrimidin-2-yl]-benzyl}-6-thiazol-2-yl-2H-pyridazin-3-on, Hydrochlorid | 449 |
| "A193" | 2-{3-[5-(3-Dimethylamino-propoxy)-pyrimidin-2-yl]-benzyl}-6-phenyl-2H-pyridazin-3-on, Hydrochlorid | 442 |
| "A194" | 4-(1-{3-[5-(3-Dimethylamino-propoxy)-pyrimidin-2-yl]-benzyl}-6-oxo-1,6-dihydro-pyridazin-3-yl)-benzonitril, Hydrochlorid | 467 |
| "A195" | 2-{3-[5-(3-Dimethylamino-propoxy)-pyrimidin-2-yl]-benzyl}-6-p-tolyl-2H-pyridazin-3-on | 456 |
| "A196" | 2-{3-[5-(3-Dimethylamino-propoxy)-pyrimidin-2-yl]-benzyl}-6-(2H-pyrazol-3-yl)-2H-pyridazin-3-on, Trifluoracetat | 432 |
| "A197" | 6-(3,4-Difluor-phenyl)-2-{3-[5-(3-dimethylamino-propoxy)-pyrimidin-2-yl]-benzyl}-2H-pyridazin-3-on, Hydrochlorid | 478 |
| "A198" | 2-{3-[5-(3-Dimethylamino-propoxy)-pyrimidin-2-yl]-benzyl}-6-(4-methanesulfonyl-phenyl)-2H-pyridazin-3-on, Hydrochlorid | 520 |
| "A199" | 2-{3-[5-(3-Dimethylamino-propoxy)-pyrimidin-2-yl]-benzyl}-6-[4-(5-methyl-[1,2,4]oxadiazol-3-yl)-phenyl]-2H-pyridazin-3-on, Hydrochlorid | 524 |
| "A200" | 2-{3-[5-(3-Dimethylamino-propoxy)-pyrimidin-2-yl]-benzyl-6-pyridin-4-yl-2H-pyridazin-3-on, Trifluoracetat | 443 |
| "A201" | 6-(3-Brom-phenyl)-2-{3-[5-(3-dimethylamino-propoxy)-pyrimidin-2-yl]-benzyl}-2H-pyridazin-3-on, Trifluoracetat | 521 |
| "A202" | 2-{3-[5-(3-Dimethylamino-propoxy)-pyrimidin-2-yl]-benzyl}-6-(3,4,5-trifluor-phenyl)-2H-pyridazin-3-on | 496 |
| "A203" | 6-(3,5-Dimethoxy-phenyl)-2-{3-[5-(3-dimethylamino-propoxy)-pyrimidin-2-yl]-benzyl}-2H-pyridazin-3-on, Trifluoracetat | 502 |
| "A204" | 2-{3-[5-(3-Dimethylamino-propoxy)-pyrimidin-2-yl]-benzyl}-6-(3-fluor-4-methoxy-phenyl)-2H-pyridazin-3-on, Hydrochlorid | 490 |
| "A205" | 2-{3-[5-(3-Dimethylamino-propoxy)-pyrimidin-2-yl]-benzyl}-6-(4-methoxy-phenyl)-2H-pyridazin-3-on, Hydrochlorid | 472 |
| "A206" | 2-{3-[5-(3-Dimethylamino-propoxy)-pyrimidin-2-yl]-benzyl}-6-(3-trifluormethyl-phenyl)-2H-pyridazin-3-on, Hydrochlorid | 510 |
| "A207" | 6-(3-Chlor-phenyl)-2-{3-[5-(3-dimethylamino-propoxy)-pyrimidin-2-yl]-benzyl}-2H-pyridazin-3-on | 476 |
| "A208" | 2-{3-[5-(3-Dimethylamino-propoxy)-pyrimidin-2-yl]-benzyl)-6-pyridin-3-yl-2H-pyridazin-3-on | 443 |
| "A209" | 2-{3-[5-(3-Dimethylamino-propoxy)-pyrimidin-2-yl]-benzyl}-6-(1 -methyl-1 H-pyrazol-4-yl)-2H-pyridazin-3-on | 446 |
| "A210" | 6-(3-Chlor-5-fluor-phenyl)-2-{3-[5-(3-dimethylamino-propoxy)-pyrimidin-2-yl]-benzyl}-2H-pyridazin-3-on | 494 |
| ¹H-NMR (d₆-DMSO): δ [ppm] = 1.89 (quintett, J = 6.7 Hz, 2H), 2.16 (s, 6H), 2.38 (t, J = 7 Hz, 2H), 4.21 (t, J = 6.5 Hz, 2H), 5.44 (s, 2H), 7.14 (d, J = 10 Hz, 1 H), 7.48 (m, 2H), 7.54 (dt, J₁ = 8.5 Hz, J₂ = 2 Hz, 1H), 7.77 (dt, J₁ = 10 Hz, J₂ = 1.7 Hz, 1H), 7.85 (t, J = 1.6 Hz, 1H), 8.15 (d, J = 10 Hz, 1H), 8.23 (m, 1 H), 8.37 (bs, 1 H), 8.62 (s, 2H) | | |
| "A211" | 2-{3-[5-(3-Dimethylamino-propoxy)-pyrimidin-2-yl]-benzyl}-6-(4-fluor-3-methoxy-phenyl)-2H-pyridazin-3-on, Hydrochlorid | 490 |
| "A212" | 6-(4-Chlor-phenyl)-2-{3-[5-(3-dimethylamino-propoxy)-pyrimidin-2-yl]-benzyl}-2H-pyridazin-3-on, | 476 |
| | Trifluoracetat | |
| "A213" | 2-{3-[5-(3-Dimethylamino-propoxy)-pyrimidin-2-yl]-benzyl}-6-(4-fluor-phenyl)-2H-pyridazin-3-on, Trifluoracetat | 460 |
| "A214" | 2-{3-[5-(3-Dimethylamino-propoxy)-pyrimidin-2-yl]-benzyl}-6-thiophen-2-yl-2H-pyridazin-3-on, Trifluoracetat | 448 |
| "A215" | N-[4-(1-{3-[5-(3-Dimethylamino-propoxy)-pyrimidin-2-yl]-benzyl}-6-oxo-1,6-dihydro-pyridazin-3-yl)-phenyl]-acetamid, Trifluoracetat | 499 |
| | 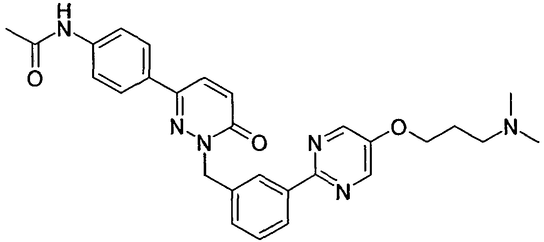 | |
| "A216" | 6-(3,4-Dimethoxy-phenyl)-2-{3-[5-(3-dimethylamino-propoxy)-pyrimidin-2-yl]-benzyl}-2H-pyridazin-3-on, Trifluoracetat | 502 |
| "A217" | 6-Benzo[2,1,3)thiadiazol-5-yl-2-{3-[5-(3-dimethylamino-propoxy)-pyrimidin-2-yl]-benzyl}-2H-pyridazin-3-on | 500 |
| | 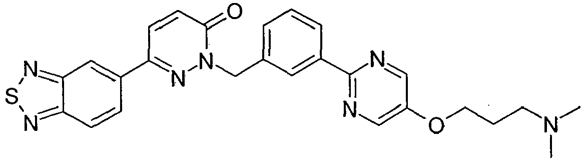 | |
| "A218" | 2-{3-[5-(3-Dimethylamino-propoxy)-pyrimidin-2-yl]-benzyl}-6-furan-3-yl-2H-pyridazin-3-on, Trifluoracetat | 432 |
| "A219" | 2-{3-[5-(3-Dimethylamino-propoxy)-pyrimidin-2-yl]-benzyl}-6-(5-methyl-[1,2,4]oxadiazol-3-yl)-2H-pyridazin-3-on, Hydrochlorid | 448 |
| "A220" | 4-(1-{3-[5-(1-Methyl-piperidin-4-yloxy)-pyrimidin-2-yl]-benzyl}-6-oxo-1,6-dihydro-pyridazin-3-yl)-benzonitril | 479 |
| "A221" | 3-(1-{3-[5-(1-Methyl-piperidin-4-yloxy)-pyrimidin-2-yl]-benzyl}-6-oxo-1,6-dihydro-pyridazin-3-yl)-benzonitril, Hydrochlorid | 479 |
| "A222" | 3-(1-{3-[5-(2-Morpholin-4-yl-ethoxy)-pyrimidin-2-yl]-benzyl}-6-oxo-1,6-dihydro-pyridazin-3-yl)-benzonitril | 495 |
| "A223" | 2-{3-[5-(1-Methyl-piperidin-4-yloxy)-pyrimidin-2-yl]-benzyl}6-pyridin-4-yl-2H-pyridazin-3-on | 455 |
| "A224" | 6-(4-Methansulfonyl-phenyl)-2-{3-[5-(1-methyl-piperidin-4-yloxy)-pyrimidin-2-yl]-benzyl}-2H-pyridazin-3-on, Trifluoracetat | 532 |
| "A225" | 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäure-methylester, Trifluoracetat | 518 |
| | 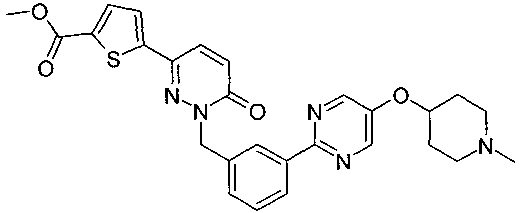 | |
| "A226" | 2-{3-[5-(1-Methyl-piperidin-4-yloxy)-pyrimidin-2-yl]-benzyl-6-(1-methyl-1H-pyrazol-4-yl)-2H-pyridazin-3-on | 458 |
| ¹H-NMR (d₆-DMSO): δ [ppm] = 1.70 (m, 2H), 2.00 (m, 2H), 2.22 (s, 3H), 2.24 (m, 2H), 2.66 (m, 2H), 3.88 (s, 3H), 4.62 (m, 1 H), 5.34 (s, 2H), 7.06 (d, J = 9.5 Hz, 1 H), 7.44 (dt, J₁ = 7.3 Hz, J₂ = 1.5 Hz, 1 H), 7.48 (t, J = 7.5 Hz, 1 H), 7.81 (d, J = 9.5 Hz, 1 H), 7.90 (s, 1 H), 8.22 (m, 2H), 8.25 (s, 1 H), 8.28 (bs, 1 H), 8.65 (s, 2H) | | |
| "A227" | 2-{3-[5-(3-Dimethylamino-propoxy)-pyrimidin-2-yl]-benzyl}-6-(5-oxo-4,5-dihydro-[1,2,4]oxadiazol-3-yl)-2H-pyridazin-3-on, Trifluoracetat | 450 |
| | 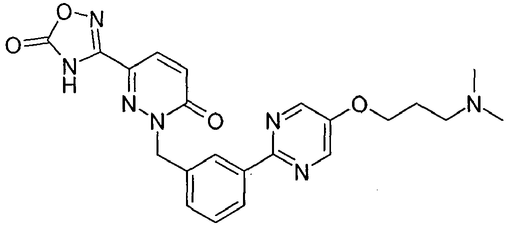 | |
| "A228" | 2-{3-[5-(3-Dimethylamino-propoxy)-pyrimidin-2-yl]-benzyl}-6-piperazin-1-yl-2H-pyridazin-3-on, Trifluoracetat | 450 |
| | 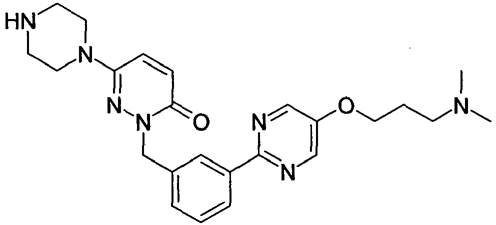 | |
| | [Herstellung verläuft über Boc-geschützte Verbindung und anschließender Abspaltung der Boc-Gruppe] | |

### Beispiel 38

Die Herstellung der Verbindungen
6-(1-Methyl-1H-pyrazol-4-yl)-2-{3-[5-(2-morpholin-4-yl-ethoxy)-pyrimidin-2-yl]-benzyl}-2H-pyridazin-3-on ("A229"),
2-[3-(5-Brorn-pyrimidin-2-yl)-benzyl]-6-(1-methyl-1H-pyrazol-4-yl)-2H-pyridazin-3-on ("A230") und
2-[3-(5-Hydroxy-pyrimidin-2-yl)-benzyl]-6-(1 -methyl-1 H-pyrazol-4-yl)-2H-pyridazin-3-on ("A231")
erfolgt analog nachstehendem Schema 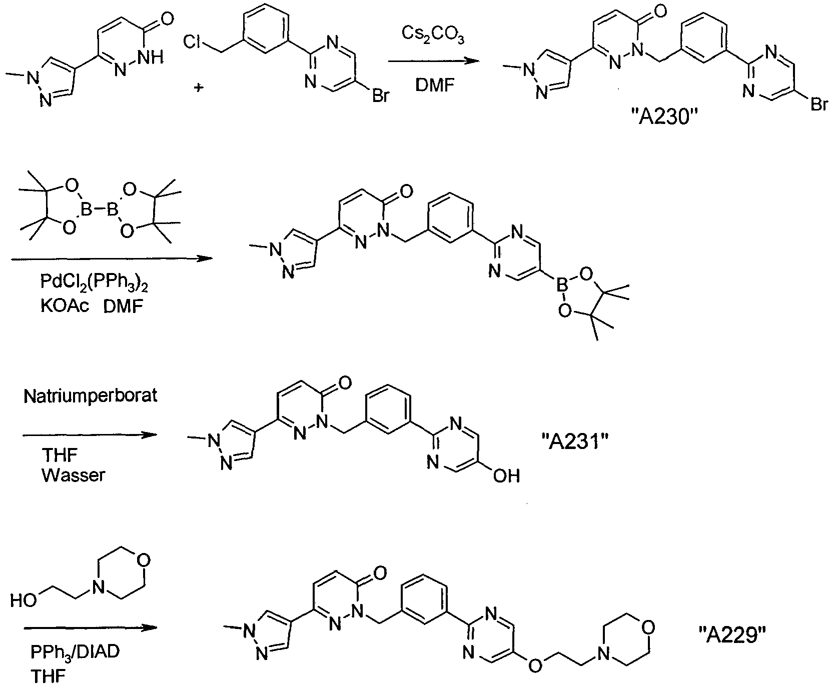
38.1 Eine Suspension von 7.68 g (43.6 mmol) 6-(1-Methyl-1 H-pyrazol-4-yl)-2H-pyridazin-3-on in 90 ml DMF wird mit 12.4 g (43.6 mmol) 5-Brom-2-(3-chlormethyl-phenyl)-pyrimidin und 14.2 g (43.6 mmol) Caesiumcarbonat versetzt und 24 Stunden bei Raumtemperatur gerührt. Das Reaktionsgemisch wird auf 400 ml Wasser gegeben. Der entstandene Niederschlag wird abgesaugt, mit Wasser gewaschen und im Vakuum getrocknet; 2-[3-(5-Brompyrimidin-2-yl)-benzyl]-6-(1-methyl-1H-pyrazol-4-yl)-2H-pyridazin-3-on als gelbbraune Kristalle; F. 184° C; ESI 423, 425.
38.2 Eine Suspension von 14.0 g (33.0 mmol) 2-[3-(5-Brompyrimidin-2-yl)-benzyl]-6-(1-methyl-1H-pyrazol-4-yl)-2H-pyridazin-3-on in 65 ml DMF wird mit 10.9 g (42.9 g) Bis(pinacolato)dibor und 9.72 g (99.0 mmol) Kaliumacetat versetzt und unter Stickstoff auf 70° C erhitzt. Nach 15minütigem Rühren bei dieser Temperatur werden 695 mg (0.99 mmol) Bis(triphenylphosphin)-palladium(II)-chlorid zugegeben und das Reaktionsgemisch 18 Stunden bei 70°C unter Stickstoff gerührt. Man lässt das Reaktionsgemisch auf Raumtemperatur abkühlen, gibt Wasser und Dichlormethan zu, filtriert über Kieselgur und trennt die organische Phase ab. Die organische Phase wird über Natriumsulfat getrocknet, eingedampft und der Rückstand aus 2-Propanol umkristallisiert: 6-(1-Methyl-1 H-pyrazol-4-yl)-2-{3-[5-(4,4,5,5-tetramethyl-[1,3,2]dioxaborolan-2-yl)-pyrimidin-2-yl]-benzyl}-2H-pyridazin-3-on als graue Kristalle; F. 204° C;
   ¹H-NMR (d₆-DMSO): δ [ppm] = 1.34 (s, 12H), 3.87 (s, 3H), 5.35 (s, 2H), 7.05 (d, J = 9.6 Hz, 1 H), 7.52 (m, 2H), 7.80 (d, J = 9.6 Hz, 1 H), 7.89 (s, 1 H), 8.21 (s, 1 H), 8.35 (m, 1 H), 8.45 (bs, 1 H), 9.01 (s, 2H).
38.3 Zu einer Suspension von 13.4 g (28.4 mmol) 6-(1-Methyl-1H-pyrazol-4-yl)-2-{3-[5-(4,4,5,5-tetramethyl-[1,3,2]dioxaborolan-2-yl)-pyrimidin-2-yl]-benzyl}-2H-pyridazin-3-on in 55 ml THF und 55 ml Wasser werden unter Eiskühlung portionsweise 8.50 g (85.1 mmol) Natriumperborat gegeben und 2 Stunden bei Raumtemperatur gerührt. Das Reaktionsgemisch wird über Kieselgur abgesaugt. Das Filtrat wird im Vakuum auf etwa die Hälfte des ursprünglichen Volumens eingeengt und mit 2 N Salzsäure auf einen pH-Wert von 1 gebracht. Der entstandende Niederschlag wird abgesaugt, mit Wasser gewaschen und im Vakuum getrocknet: 2-[3-(5-Hydroxy-pyrimidin-2-yl)-benzyl]-6-(1-methyl-1H-pyrazol-4-yl)-2H-pyridazin-3-on als leicht beige Kristalle; F. 239° C; ESI 361.
38.4 Zu einer Suspension von 360 mg (1.00 mmol) 2-[3-(5-Hydroxy-pyrimidin-2-yl)-benzyl]-6-(1-methyl-1H-pyrazol-4-yl)-2H-pyridazin-3-on in 2 ml THF werden nacheinander 394 mg (1.50 mmol) Triphenylphosphin und 242 µl (2.00 mmol) 4-(2-Hydroxyethyl)morpholin gegeben. Dann werden unter Eiskühlung langsam 294 µl (1.50 mmol) Diisopropylazodicarboxylat zugetropft. Die entstandene Lösung wird 18 Stunden bei Raumtemperatur gerührt. Das Reaktionsgemisch wird im Vakuum eingedampft und der ölige Rückstand in 2-Propanol gelöst. Der nach einiger Zeit entstandene Feststoff wird abgesaugt, mit 2-Propanol und tert.-Butylmethylether gewaschen und im Vakuum getrocknet: 6-(1-Methyl-1H-pyrazol-4-yl)-2-{3-[5-(2-morpholin-4-yl-ethoxy)-pyrimidin-2-yl]-benzyl}-2H-pyridazin-3-on ("A229") als farblose Kristalle; F. 134° C; ESI 474;
   ¹H-NMR (d₆-DMSO): δ [ppm] = 2.48 (m, 4H), 2.73 (t, J = 5.5 Hz, 2H), 3.57 (m, 4H), 3.87 (s, 3H), 4.30 (t, J = 5.5 Hz, 2H), 5.33 (s, 2H), 7.05 (d, J = 9.5 Hz, 1 H), 7.43 (dt, J₁ = 7.3 Hz, J₂ = 1.5 Hz, 1 H), 7.47 (t, J = 7.5 Hz, 1H), 7.80 (d, J = 9.5 Hz, 1H), 7.89 (s, 1H), 8.21 (s, 1H), 8.22 (dt, J₁ = 7.5 Hz, J₂ = 1.5 Hz, 1 H), 8.28 (bs, 1 H), 8.64 (s, 2H).

Durch Salzbildung erhält man aus "A229" das p-Toluolsulfonat und das Phosphat.

### Analog erhält man die nachstehenden Verbindungen

| Verbindung Nr. | Name und/oder Struktur | ESI [M+H]⁺ |
|---|---|---|
| ("A232") | 6-(1-Methyl-1H-pyrazol-4-yl)-2-{3-[5-(2-morpholin-4-yl-ethoxy)-pyrimidin-2-yl]-benzyl}-2H-pyridazin-3-on, Hydrochlorid (von "A229") | 474 |
| ("A233") | 2-{3-[5-(1-Methyl-piperidin-4-ylmethoxy)-pyrimidin-2-yl]-benzyl}-6-(1-methyl-1 H-pyrazol-4-yl)-2H-pyridazin-3-on, Hydrochlorid (von "A237") | 472 |
| ("A234") | 2-{3-[5-(1-Methyl-piperidin-4-ylmethoxy)-pyrimidin-2-yl]-benzyl}-6-(1-methyl-1H-pyrazol-4-yl)-2H-pyridazin-3-on, Trifluoracetat (von "A237") | 472 |
| "A235" | 6-(3-Fluor-phenyl)-2-{3-[5-(2-morpholin-4-yl-ethoxy)-pyrimidin-2-yl]-benzyl)-2H-pyridazin-3-on | 488 |
| "A236" | 6-(1-Methyl-1H-pyrazol-4-yl)-2-{3-[5-(2-morpholin-4-yl-ethoxy)-pyrimidin-2-yl]-benzyl}-2H-pyridazin-3-on, | 474 |
| | Dihydrochlorid (von "A229") | |
| "A237" | 2-{3-[5-(1-Methyl-piperidin-4-ylmethoxy)-pyrimidin-2-yl]-benzyl}-6-(1-methyl-1 H-pyrazol-4-yl)-2H-pyridazin-3-on | 472 |
| "A238" | 2-{3-[5-(1-Methyl-piperidin-4-yloxy)-pyrimidin-2-yl]-benzoyl}-6-(1-methyl-1H-pyrazol-4-yl)-2H-pyridazin-3-on, Formiat | 458 |
| "A240" | 2-{3-[5-(3-Methoxy-propoxy)-pyrimidin-2-yl]-benzyl}-6-(1-methyl-1H-pyrazol-4-yl)-2H-pyridazin-3-on | 433 |
| "A241" | 2-{3-[5-(2-Methoxy-ethoxy)-pyrimidin-2-yl]-benzyl}-6-(1-methyl-1 H-pyrazol-4-yl)-2H-pyridazin-3-on | 419 |
| "A242" | 2-{3-[5-(2-Morpholin-4-yl-ethoxy)-pyrimidin-2-yl]-benzyl}-6-(1-propyl-1 H-pyrazol-4-yl)-2H-pyridazin-3-on | 502 |
| "A243" | 2-(3-{5-[2-(4-Methyl-piperazin-1-yl)-ethoxy]-pyrimid in-2-yl}-benzyl)-6-(1 -methyl-1 H-pyrazol-4-yl)-2H-pyridazin-3-on | 487 |
| "A244" | 2-(3-{5-[2-(4-Methyl-3-oxo-piperazin-1-yl)-ethoxy]-pyrimidin-2-yl}-benzyl)-6-(1-methyl-1H-pyrazol-4-yl)-2H-pyridazin-3-on | 501 |
| | 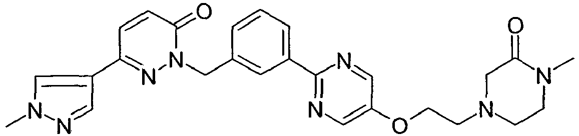 | |
| "A245" | 6-(1-Methyl-1H-pyrazol-4-yl)-2-{3-[5-(3-morpholin-4-yl-propoxy)-pyrimidin-2-yl]-benzyl}-2H-pyridazin-3-on, Trifluoracetat | 488 |
| "A246" | 6-(1-Methyl-1H-pyrazol-4-yl)-2-{3-[5-(2-morpholin-4-yl-propoxy)-pyrimidin-2-yl]-benzyl}2H-pyridazin-3-on, Trifluoracetat | 488 |
| | 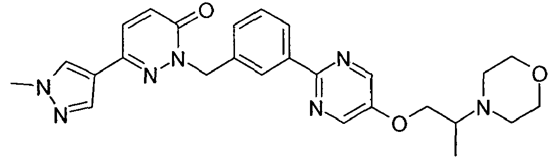 | |
| "A247" | 2-{3-[5-(1-Methyl-2-morpholin-4-yl-ethoxy)-pyrimidin-2-yl]-benzyl}-6-(1-methyl-1H-pyrazol-4-yl)-2H-pyridazin-3-on, Trifluoracetat | 488 |
| | 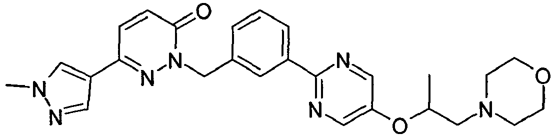 | |
| "A248" | 2-(3-[5-(2-Dimethylamino-ethoxy)-pyrimidin-2-yl]-benzyl}-6-(1-methyl-1H-pyrazol-4-yl)-2H-pyridazin-3-on | 432 |
| "A305" | 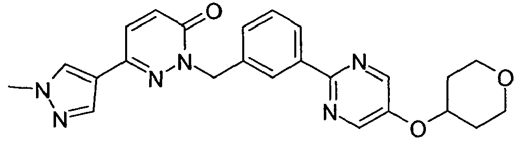 | 445 |
| "A306" | 2-{3-[5-(1-Aza-bicyclo[2.2.2]oct-3-yloxy)-pyrimidin-2-yl]-benzyl-6-(1-methyl-1H-pyrazol-4-yl)-2H-pyridazin-3-one | 470 |
| | 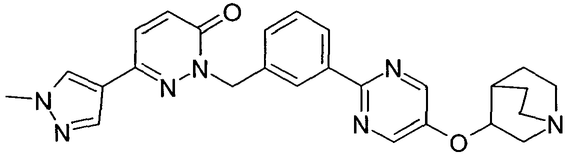 | |
| "A307" | 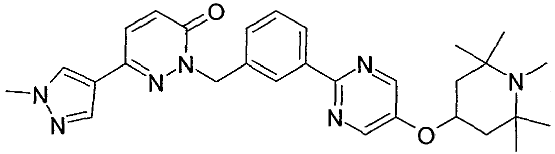 | 514 |
| | Trifluoracetat | |
| "A310" | 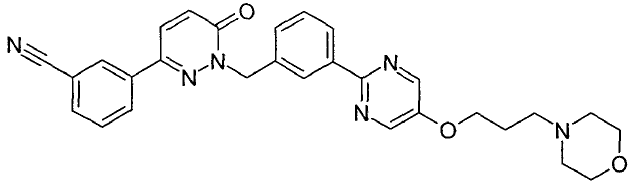 | 509 |
| | Trifluoracetat | |
| "A312" | 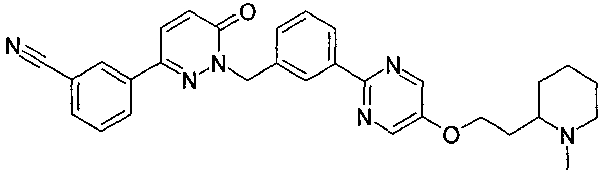 | 507 |
| | Trifluoracetat | |
| "A314" | 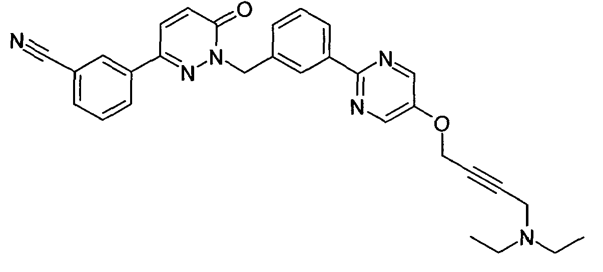 | 505 |
| | Trifluoracetat | |

### Beispiel 39

Alternative Herstellung von "A229"

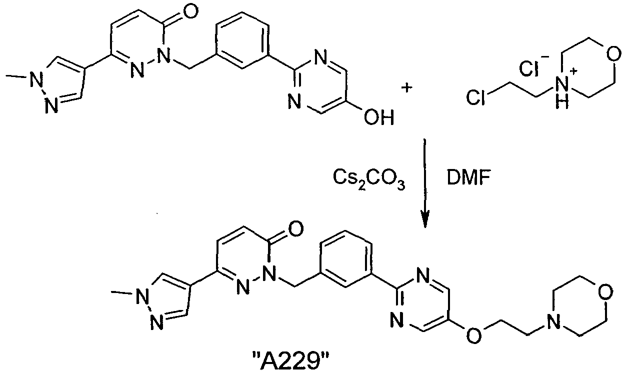

Eine Suspension von 360 mg (1.00 mmol) 2-[3-(5-Hydroxy-pyrimidin-2-yl)-benzyl]-6-(1-methyl-1 H-pyrazol-4-yl)-2H-pyridazin-3-on, 195 mg (1.05 mmol) N-(2-Chlorethyl)-morpholiniumchlorid und 521 mg (1.60 mmol) Caesiumcarbonat in 2 ml DMF wird unter Rühren auf 80° C erhitzt und bei dieser Temperatur 6 Stunden gerührt. Man lässt das Reaktionsgemisch abkühlen und gibt 50 ml Wasser zu. Der entstandene Niederschlag wird abgesaugt, mit Wasser gewaschen und im Vakuum getrocknet: 6-(1-Methyl-1 H-pyrazol-4-yl)-2-{3-[5-(2-morpholin-4-yl-ethoxy)-pyrimidin-2-yl]-benzyl}-2H-pyridazin-3-on als farblose Kristalle.

### Analog erhält man die nachstehenden Verbindungen

| Verbindung Nr. | Name und/oder Struktur | ESI [M+H]⁺ |
|---|---|---|
| "A249" | 2-{3-[5-(2-Methyl-3-morpholin-4-yl-propoxy)-pyrimidin-2-yl]-benzyl}-6-(1-methyl-1 H-pyrazol-4-yl)-2H-pyridazin-3-on | 502 |
| | 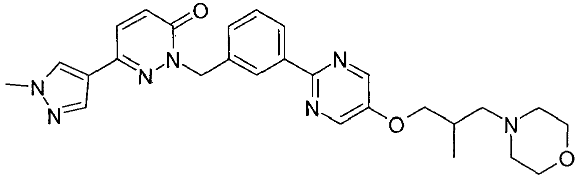 | |
| "A250" | 6-(1-Methyl-1H-pyrazol-4-yl)-2-{3-[5-(2-pyrrolidin-1-yl-ethoxy)-pyrimidin-2-yl]-benzyl}-2H-pyridazin-3-on | 458 |
| "A251" | 2-[3-(5-Ethoxy-pyrimidin-2-yl)-benzyl]-6-(1-methyl-1 H-pyrazol-4-yl)-2H-pyridazin-3-on | 389 |
| "A252" | 6-(1-Methyl-1H-pyrazol-4-yl)-2-{3-[5-(2-morpholin-4-yl-2-oxo-ethoxy)-pyrimidin-2-yl]-benzyl}-2H-pyridazin-3-on | 488 |
| | 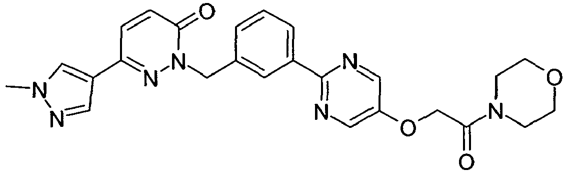 | |
| "A253" | 6-(3-Chlor-phenyl)-2-{3-[5-(2-morpholin-4-yl-ethoxy)-pyrimidin-2-yl]-benzyl}-2H-pyridazin-3-on | 504 |
| "A254" | 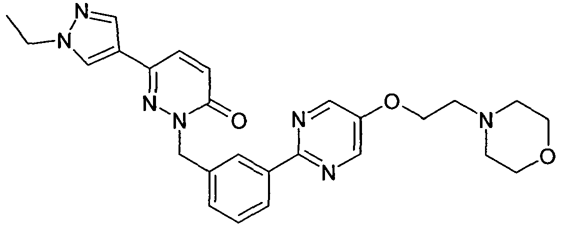 | |
| "A255" | 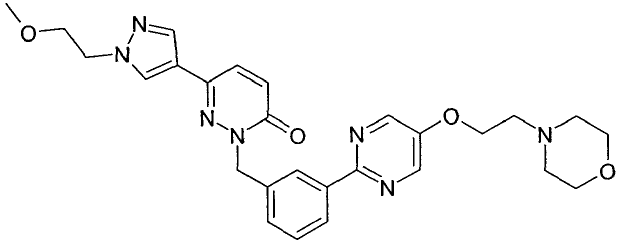 | |
| "A256" | 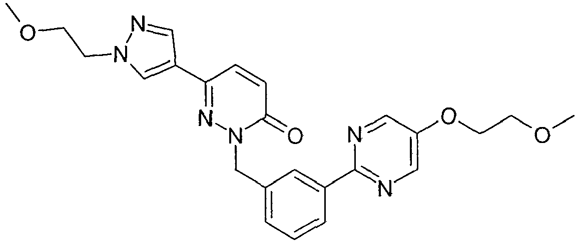 | |
| "A304" | 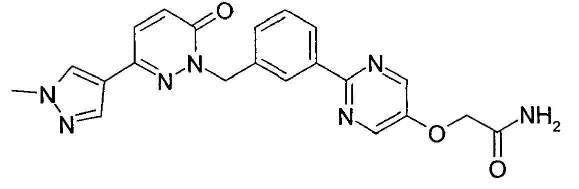 | 418 |

### Beispiel 40

Die Herstellung der Verbindung 3-(1-{3-[5-(1-Methyl-piperidin-4-ylmethoxy)-pyrimidin-2-yl]-benzyl}-6-oxo-1,6-dihydro-pyridazin-3-yl)-benzonitril ("A257") erfolgt analog nachstehendem Schema 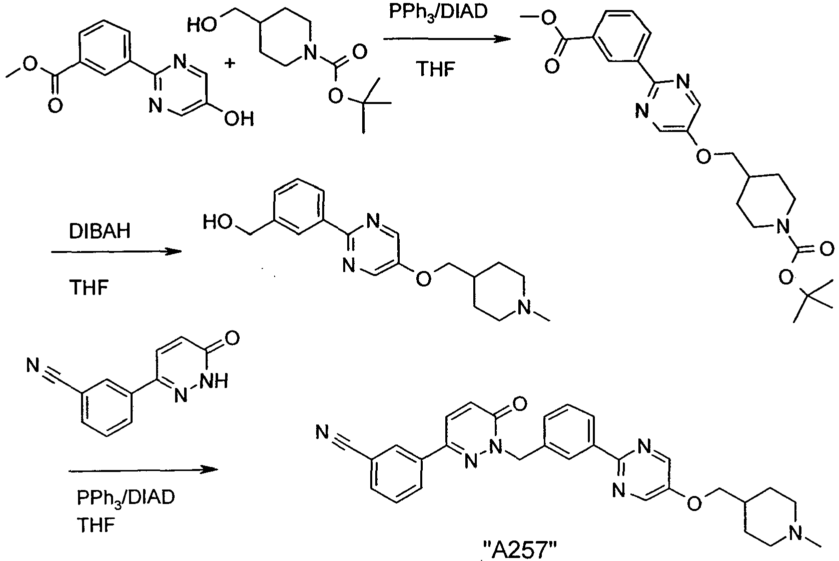
40.1 Zu einer Suspension von 13.0 g (56.5 mmol) 3-(5-Hydroxy-pyrimidin-2-yl)-benzoesäuremethylester und 13.4 g (62.1 mmol) N-Boc-piperidinmethanol in 115 ml THF werden 17.7 g (67.8 mmol) Triphenylphosphin gegeben und auf 5° C gekühlt. Zu der bei dieser Temperatur gehaltenen Suspension werden unter Rühren innerhalb von 45 Minuten 13.3 ml (67.8 mmol) Diisopropylazodicarboxylat zugetropft. Das Reaktionsgemisch wird 1 Stunde bei Raumtemperatur gerührt. Anschließend werden weitere 22.2 g (84.7 mmol) Triphenylphosphin und 16.6 ml (84.7 mmol) Diisopropylazodicarboxylat zugegeben. Das Reaktionsgemisch wird 18 Stunden bei Raumtemperatur gerührt und im Vakuum eingeengt. Der entstandene Feststoff wird abgesaugt, mit Diethylether gewaschen und an einer Kieselgelsäule mit Dichlormethan/Methanol als Laufmittel chromatographiert: 4-[2-(3-Methoxycarbonyl-phenyl)-pyrimidin-5-yloxymethyl]-piperidin-1-carbonsäure-tert.-butylester als zitronengelbe Kristalle;
   F. 166° C; ESI 428.
40.2 Zu einer Suspension von 1.71 g (3.99 mmol) 4-[2-(3-Methoxy-carbonyl-phenyl)-pyrimidin-5-yloxymethyl]-piperidin-1-carbonsäure-tert.-butylester in 20 ml THF werden unter Stickstoff 25 ml (25 mmol) einer 1 M Lösung von Diisobutylaluminiumhydrid in THF zugetropft. Das Reaktionsgemisch wird 1 Stunde bei Raumtemperatur gerührt und mit 1 ml einer gesättigten Natriumsulfat-Lösung versetzt. Der entstandene Niederschlag wird abgesaugt und mit THF und heißem 2-Propanol gewaschen. Das Filtrat wird eingedampft und aus tert.-Butylmethylether umkristallisiert: {3-[5-(1-Methyl-piperidin-4-ylmethoxy)-pyrimidin-2-yl]-phenyl}-methanol als beige Kristalle; F. 175° C; ESI 314.
40.3 Zu einer Lösung von 313 mg (1.00 mmol) {3-[5-(1-Methyl-piperidin-4-ylmethoxy)-pyrimidin-2-yl]-phenyl}-methanol in 2 ml THF werden nacheinander 264 mg (1.30 mmol) 3-(6-Oxo-1,6-dihydro-pyridazin-3-yl)-benzonitril und 397 mg (1.5 mmol) Triphenylphosphin gegeben. Das Reaktionsgemisch wird im Eisbad gekühlt und unter Rühren 294 µl (1.5 mmol) Diisopropylazodicarboxylat zugetropft. Das Reaktionsgemisch wird 18 Stunden bei Raumtemperatur gerührt und eingedampft. Der Rückstand wird an einer Kieselgelsäule mit Dichlormethan / Methanol chromatographiert. Die Produkt enthaltenden Fraktionen werden vereinigt, eingedampft, der Rückstand mit tert.-Butylmethylether digeriert, abgesaugt und im Vakuum getrocknet: 3-(1-{3-[5-(1-Methyl-piperidin-4-ylmethoxy)-pyrimidin-2-yl]-benzyl}-6-oxo-1,6-dihydro-pyridazin-3-yl)-benzonitril als farblose Kristalle; F. 177 °C; ESI 493;
   ¹H-NMR (d₆-DMSO): δ [ppm] = 1.33 (m, 2H), 1.75 (m, 3H), 1.89 (m, 2H), 2.17 (s, 3H), 2.80 (m, 2H), 4.05 (d, J = 6.1 Hz, 2H), 5.45 (s, 2H), 7.16 (d, J = 10 Hz, 1H), 7.49 (m, 2H), 7.73 (t, J = 7.8 Hz, 1H), 7.93 (d, J = 7.8 Hz, 1H), 8.17 (d, J = 10 Hz, 1H), 8.24 (m, 2H), 8.38 (m, 2H), 8.64 (s, 2H).

Durch Salzbildung erhält man aus "A257" das Hemisulfat, Citrat, Tartrat, Sulfat, Succinat und Hydrochlorid.

### Beispiel 41

Die Herstellung der Verbindungen
2-[3-(5-Brom-pyridin-2-yl)-benzyl]-6-(3,5-difluor-phenyl)-2H-pyridazin-3-on ("A258") und
6-(3,5-Difluor-phenyl)-2-{3-[5-(1-piperidin-4-yl-1H-pyrazol-4-yl)-pyridin-2-yl]-benzyl}-2H-pyridazin-3-on ("A259")
erfolgt analog nachstehendem Schema 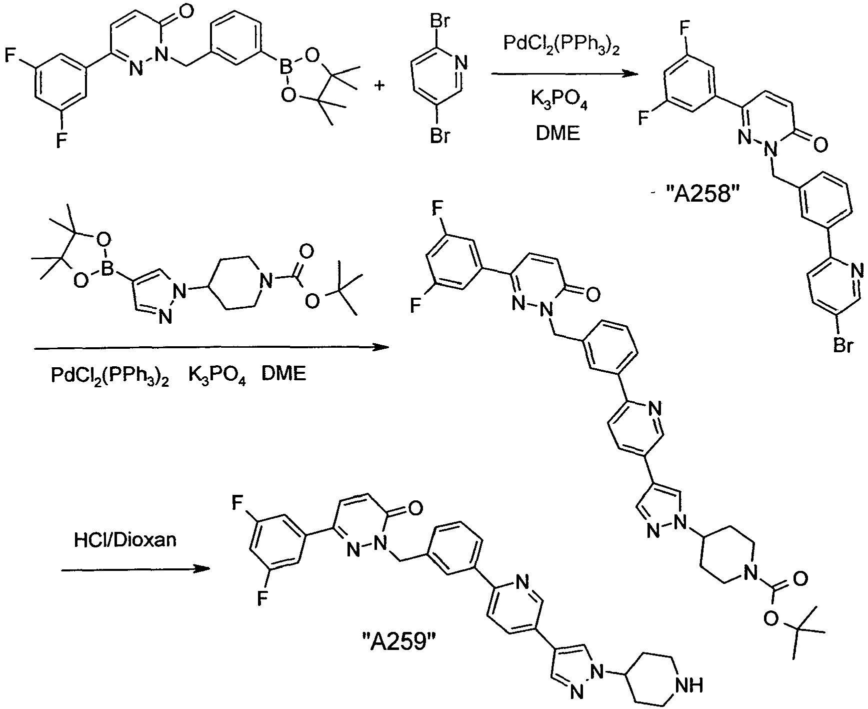
41.1 Eine Suspension von 695 mg (1.64 mmol) 6-(3,5-Difluorphenyl)-2-[3-(4,4,5,5-tetramethyl-[1,3,2]dioxaborolan-2-yl)-benzyl]-2H-pyridazin-3-on (Herstellung siehe Beispiel 11), 427 mg (1.80 mmol) 2,5-Dibrompyridin und 695 mg (3.28 mmol) tri-Kaliumphosphat-Trihydrat in 10 ml 1,2-Dimethoxyethan wird unter Stickstoff auf 80° C erhitzt. Dann werden 92 mg (0.13 mmol) Bis(triphenylphosphin)-palladium(II)-chlorid zugegeben und das Reaktionsgemisch 18 Stunden bei 80° C gerührt. Man lässt das Reaktionsgemisch abkühlen und gibt Wasser zu. Der entstandende Niederschlag wird abgesaugt, mit Wasser gewaschen und getrocknet: 2-[3-(5-Brompyridin-2-yl)-benzyl]-6-(3,5-difluor-phenyl)-2H-pyridazin-3-on als gelbliche Kristalle; ESI 453, 455.
41.2 Eine Suspension von 333 mg (0.732 mmol) 2-[3-(5-Brompyridin-2-yl)-benzyl]-6-(3,5-difluor-phenyl)-2H-pyridazin-3-on, 304 mg (0.805 mmol) 4-[4-(4,4,5,5-Tetramethyl-[1,3,2]dioxaborolan-2-yl)-pyrazol-1-yl]-piperidin-1-carbonsäure-tert.-butylester und 311 mg (1.46 mmol) tri-Kaliumphosphat-Trihydrat in 2 ml 1,2-Dimethoxyethan wird unter Stickstoff auf 80° C erhitzt. Dann werden 43 mg (0.06 mmol) Bis(triphenylphosphin)-palladium(II)-chlorid zugegeben und das Reaktionsgemisch 2 Stunden bei 80° C gerührt. Man lässt das Reaktionsgemisch abkühlen und gibt Wasser zu. Der entstandende Niederschlag wird abgesaugt und mit Wasser gewaschen. Der Rückstand wird aus 2-Propanol umkristallisiert: 4-[4-(6-{3-[3-(3,5-Difluorphenyl)-6-oxo-6H-pyridazin-1-ylmethyl]-phenyl}-pyridin-3-yl)-pyrazol-1-yl]-piperidin-1-carbonsäure-tert.-butylester als graue Kristalle; ESI 625.
41.3 347 mg (0.556 mmol) 4-[4-(6-{3-[3-(3,5-Difluorphenyl)-6-oxo-6H-pyridazin-1-ylmethyl]-phenyl}-pyridin-3-yl)-pyrazol-1-yl]-piperidin-1-carbonsäure-tert.-butylester werden mit 5 ml 4 N HCl in Dioxan versetzt. Der entstandene Niederschlag wird abfiltriert und in einem Gemisch aus 2 N Natronlauge und Dichlormethan gelöst. Die organische Phase wird abgetrennt, über Natriumsulfat getrocknet und eingedampft. Der Rückstand wird aus 2-Propanol umkristallisiert: 6-(3,5-Difluorphenyl)-2-{3-[5-(1-piperidin-4-yl-1H-pyrazol-4-yl)-pyridin-2-yl]-benzyl}-2H-pyridazin-3-on als hellgelbe Kristalle; ESI 525;
   ¹H-NMR (d₆-DMSO): δ [ppm] = 1.82 (m, 2H), 2.00 (m, 2H), 2.07 (bs, 1H), 2.61 (m, 2H), 3.06 (m, 2H), 4.22 (m, 1H), 5.45 (s, 2H), 7.15 (d, J = 9.5 Hz, 1 H), 7.35 (m, 1 H), 7.42 (d, J = 7.5 Hz, 1 H), 7.48 (t, J = 7.8 Hz, 1H), 7.67 (m, 2H), 7.93 (d, J = 8 Hz, 1H), 8.02 (m, 2H), 8.06 (d, J = 8 Hz, 1H), 8.15 (d, J = 9.5 Hz, 1H), 8.19 (bs, 1H), 8.39 (s, 1H), 8.93 (bs, 1H).

### Beispiel 42

Die Herstellung der Verbindungen
3-(1-{3-[5-(3-Dimethylamino-propoxy)-pyrimidin-2-yl]-benzyl}-6-oxo-1,6-dihydro-pyridazin-3-yl)-benzamid ("A260") und von "A261"
erfolgt analog nachstehendem Schema

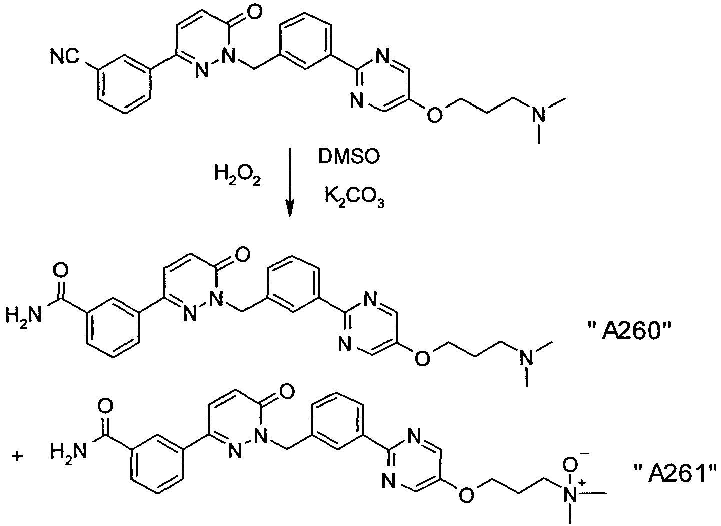

### Beispiel 43

Die Herstellung der Verbindungen
3-{1-[3-(5-Brom-pyrimidin-2-yl)-benzyl]-6-oxo-1,6-dihydro-pyridazin-3-yl}-benzonitril ("A262"),
3-{1-[3-(5-Hydroxy-pyrimidin-2-yl)-benzyl]-6-oxo-1,6-dihydro-pyridazin-3-yl}-benzonitril ("A263"),
3-(6-Oxo-1-{3-[5-(piperidin-4-ylmethoxy)-pyrimidin-2-yl]-benzyl}-1,6-dihydro-pyridazin-3-yl)-benzonitril ("A264"),
4-(2-{3-[3-(3-Cyan-phenyl)-6-oxo-6H-pyridazin-1-ylmethyl]-phenyl}-pyrimidin-5-yloxymethyl)-piperidin-1-carbonsäure-tert.-butylester ("A265") und
die alternative Synthese von "A257"
erfolgt analog nachstehendem Schema 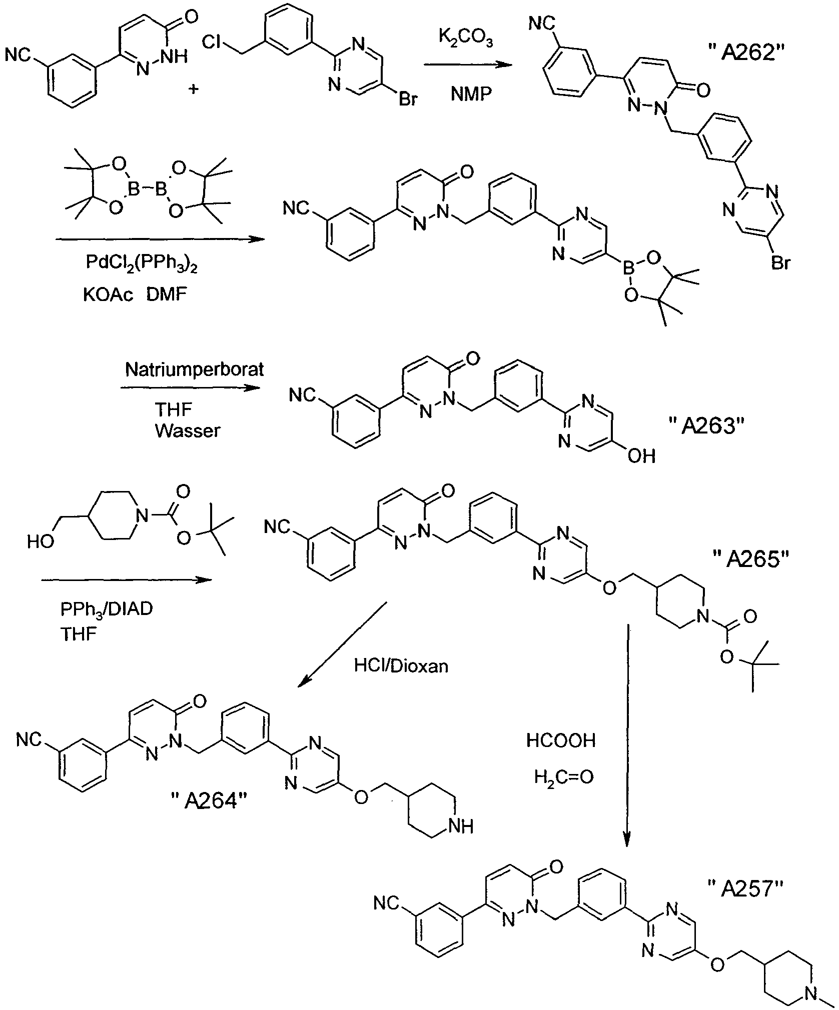
43.1 Eine Suspension von 4.15 g (20 mmol) 3-(6-Oxo-1,6-dihydropyridazin-3-yl)-benzonitril in 40 ml 1-Methyl-2-pyrrolidon wird mit 6.00 g (21 mmol) 5-Brom-2-(3-chlormethyl-phenyl)-pyrimidin und 2.76 g (341 mmol) Kaliumcarbonat versetzt und 18 Stunden bei 80° C gerührt. Das Reaktionsgemisch wird auf 200 ml Wasser gegeben. Der entstandene Niederschlag wird abgesaugt, mit Wasser gewaschen und im Vakuum getrocknet: 3-{1-[3-(5-Brompyrimidin-2-yl)-benzyl]-6-oxo-1,6-dihydropyridazin-3-yl}-benzonitril ("A262") als beige Kristalle, ESI 444, 446.
43.2 Eine Lösung von 18.0 g (41.0 mmol) 3-{1-[3-(5-Brompyrimidin-2-yl)-benzyl]-6-oxo-1,6-dihydro-pyridazin-3-yl}-benzonitril in 85 ml DMF wird mit 11.8 g (47 mmol) Bis(pinacolato)dibor und 11.9 g (122 mmol) Kaliumacetat versetzt und unter Stickstoff auf 80° C erhitzt. Nach 15minütigem Rühren bei dieser Temperatur werden 273 mg (1.22 mmol) Palladium(II)-acetat zugegeben und das Reaktionsgemisch 2 Stunden bei 80°C unter Stickstoff gerührt. Man lässt das Reaktionsgemisch auf Raumtemperatur abkühlen, gibt Wasser und Dichlormethan zu, filtriert über Kieselgur und trennt die organische Phase ab. Die organische Phase wird über Natriumsulfat getrocknet und eingedampft: 3-(6-Oxo-1-{3-[5-(4,4,5,5-tetramethyl-[1,3,2]dioxaborolan-2-yl)-pyrimidin-2-yl]-benzyl}-1,6-dihydro-pyridazin-3-yl)-benzonitril als grauer Feststoff, der ohne weitere Reinigung in die folgende Reaktion eingesetzt wird.
43.3 Zu einer Suspension von 5.33 g (10.9 mmol) 3-(6-Oxo-1-{3-[5-(4,4,5,5-tetramethyl-[1,3,2]dioxaborolan-2-yl)-pyrimidin-2-yl]-benzyl}-1,6-dihydro-pyridazin-3-yl)-benzonitril in 35 ml THF und 35 ml Wasser werden unter Eiskühlung portionsweise 4.93 g (49.4 mmol) Natriumperborat gegeben und 2 Stunden bei Raumtemperatur gerührt. Das Reaktionsgemisch wird mit 300 ml Dichlormethan und 100 ml gesättigter Ammoniumchlorid-Lösung versetzt. Die organische Phase wird abgetrennt, über Natriumsulfat getrocknet und eingedampft. Der Rückstand wird aus Methanol umkristallisiert: 3-{1-[3-(5-Hydroxy-pyrimidin-2-yl)-benzyl]-6-oxo-1,6-dihydro-pyridazin-3-yl}-benzonitril ("A263") als bräunlicher Feststoff; F. 248° C; ESI 382.
43.4 Zu einer Suspension von 25 g (65.6 mmol) 3-{1-[3-(5-Hydroxypyrimidin-2-yl)-benzyl]-6-oxo-1,6-dihydro-pyridazin-3-yl}-benzonitril in 250 ml THF werden nacheinander 15.6 g (68.8 mmol) N-Boc-4-piperidin-methanol und 19.1 g (72.1 mmol) Triphenylphosphin gegeben. Dann werden unter Eiskühlung langsam 14.9 ml (72.1 mmol) Diisopropylazodicarboxylat zugetropft. Die entstandene Lösung wird noch 2 Stunden bei Raumtemperatur gerührt. Das Reaktionsgemisch wird mit 750 ml 2-Propanol und 13.1 ml einer 0.5 M Lösung von Kaliumhydroxid in Ethanol versetzt. Der entstandene Niederschlag wird abgesaugt, mit Diethylether gewaschen und im Vakuum getrocknet: 4-(2-{3-[3-(3-Cyan-phenyl)-6-oxo-6H-pyridazin-1-ylmethyl]-phenyl}-pyrimidin-5-yloxymethyl)-piperidin-1-carbonsäure-tert.-butylester ("A265") als farblose Kristalle; F. 178° C; ESI 579.
43.5 Eine Lösung von 1.22 g (2.10 mmol) 4-(2-{3-[3-(3-Cyan-phenyl)-6-oxo-6H-pyridazin-1-ylmethyl]-phenyl}-pyrimidin-5-yloxymethyl)-piperidin-1-carbonsäure-tert.-butylester in 12 ml einer 4N Lösung von Chlorwasserstoff in Dioxan wird 16 h bei Raumtemperatur gerührt, wobei sich ein unlöslicher Niederschlag bildet. Die überstehende Lösung wird abdekantiert. Der Rückstand wird mit Dichlormethan und einer gesättigten Natriumhydrogencarbonat-Lösung versetzt. Die organische Phase wird abgetrennt, über Natriumsulfat getrocknet und im Vakuum eingedampft. Der Rückstand wird an einer Kieselgelsäule mit Dichlormethan/Methanol chromatographiert: 3-(6-Oxo-1-{3-[5-(piperidin-4-ylmethoxy)-pyrimidin-2-yl]-benzyl}-1,6-dihydro-pyridazin-3-yl)-benzonitril ("A264") als farblose Kristalle; ESI 479.
43.6 Eine Lösung von 16.0 g (28.0 mmol) 4-(2-{3-[3-(3-Cyan-phenyl)-6-oxo-6H-pyridazin-1-ylmethyl]-phenyl}-pyrimidin-5-yloxymethyl)-piperidin-1-carbonsäure-tert.-butylester in 80 ml Ameisensäure wird mit 6.60 ml 35%iger wässriger Formaldehyd-Lösung versetzt und 2 Stunden bei einer Temperatur von 110° C gerührt. Das Reaktionsgemisch wird mit 300 ml Wasser versetzt und im Vakuum auf ein Volumen von 150 ml eingeengt. Es wird mit 200 ml Dichlormethan extrahiert. Die organische Phase wird mit NatriumhydrogencarbonatLösung gewaschen, über Natriumsulfat getrocknet und eingedampft. Der Rückstand wird aus 2-Propanol umkristallisiert: 3-(1-{3-[5-(1-Methyl-piperidin-4-ylmethoxy)-pyrimidin-2-yl]-benzyl}-6-oxo-1,6-dihydro-pyridazin-3-yl)-benzonitril ("A257") als farblose Kristalle; F. 177°C, ESI 493.

### Analog erhält man die nachstehenden Verbindungen

| Verbindung Nr. | Name und/oder Struktur | ESI [M+H]⁺ |
|---|---|---|
| "A266" | 6-(1-Methyl-1H-pyrazol-4-yl)-2-{3-[5-(piperidin-4-yloxy)-pyrimidin-2-yl]-benzyl}-2H-pyridazin-3-on | 444 |
| "A267" | 6-(1-Methyl-1 H-pyrazol-4-yl)-2-{3-[5-(piperidin-4-ylmethoxy)-pyrimidin-2-yl]-benzyl}-2H-pyridazin-3-on | 458 |
| "A268" | 3-(1-{3-[5-(3-Methylamino-propoxy)-pyrimidin-2-yl]-benzyl}-6-oxo-1,6-dihydro-pyridazin-3-yl)-benzonitril, Hydrochlorid | 453 |
| | 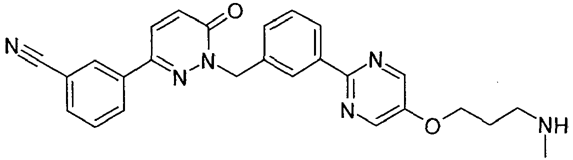 | |
| "A269" | 3-[1-(3-{5-[2-(4-Methyl-3-oxo-piperazin-1-yl)-ethoxy]-pyrimidin-2-yl}-benzyl)-6-oxo-1,6-dihydro-pyridazin-3-yl]-benzonitril | 522 |
| | 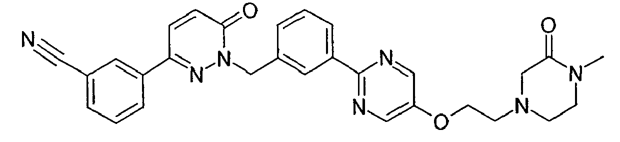 | |
| "A270" | 3-[1-(3-{5-[2-(4-Methyl-piperazin-1-yl)-ethoxy]-pyrimidin-2-yl}-benzyl)-6-oxo-1,6-dihydro-pyridazin-3-yl]-benzonitril | 508 |
| "A271" | 3-(1-{3-[5-(2-Methoxy-ethoxy)-pyrimidin-2-yl]-benzyl}-6-oxo-1,6-dihydro-pyridazin-3-yl)-benzonitril | 440 |
| "A272" | 3-(1-{3-[5-(3-Methoxy-propoxy)-pyrimidin-2-yl]-benzyl}-6-oxo-1,6-dihydro-pyridazin-3-yl)-benzonitril | 454 |
| "A273" | 6-(3-Fluor-phenyl)-2-{3-[5-(1-methyl-piperidin-4-ylmethoxy)-pyrimidin-2-yl]-benzyl}-2H-pyridazin-3-on | 486 |
| "A274" | 2-{3-[5-(1-Methyl-piperidin-4-yloxy)-pyrimidin-2-yl]-benzyl}-6-(1-propyl-1H-pyrazol-4-yl)-2H-pyridazin-3-on | 486 |
| ¹H-NMR (d₆-DMSO): δ [ppm] = 0.83 (t, J = 7.4 Hz, 3H), 1.69 (m, 2H), 1.80 (sextett, J = 7.2 Hz, 2H), 1.98 (m, 2H), 2.20 (s, 3H), 2.22 (m, 2H), 2.63 (m, 2H), 4.09 (t, J = 6.8 Hz, 2H), 4.60 (m, 1H), 5.34 (s, 2H), 7.05 (d, J = 9.5 Hz, 1H), 7.43 (dt, J₁ = 7.3 Hz, J₂ = 1.5 Hz, 1 H), 7.47 (t, J = 7.5 Hz, 1 H), 7.82 (d, J = 9.5 Hz, 1 H), 7.90 (s, 1 H), 8.21 (dt, J₁ = 7.5 Hz, J₂ = 1.5 Hz, 1 H), 8.25 (s, 1 H), 8.28 (bs, 1 H), 8.64 (s, 2H) | | |
| "A275" | 6-(3-Chlor-phenyl)-2-{3-[5-(1-methyl-piperidin-4-ylmethoxy)-pyrimidin-2-yl]-benzyl}-2H-pyridazin-3-on | 503 |
| "A276" | 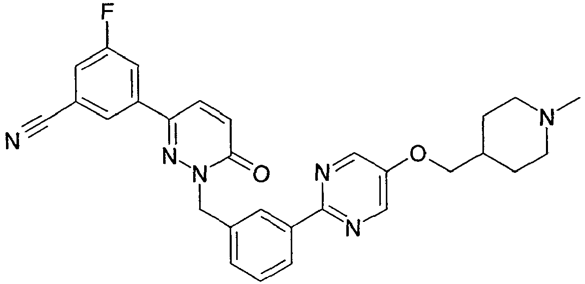 | |
| "A276a" | 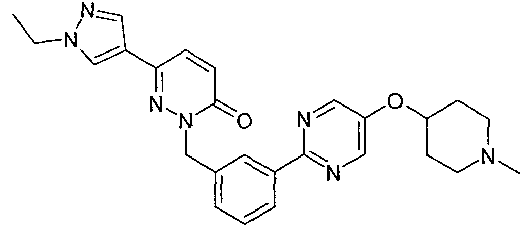 | |

### Beispiel 44

44.1 Herstellung von 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäure ("A277")
   2 g (3.85 mmol) 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäuremethylester ("A225") werden in 50 ml THF und 5 ml Wasser gelöst und mit 283 mg (11.6 mmol) Lithiumhydroxid versetzt. Die Lösung wird 15 h bei Raumtemperatur gerührt. Das Reaktionsgemisch wird eingeengt, der Rückstand in 200 ml Wasser gelöst und mit 200 ml Ethylacetat ausgeschüttelt. Die wässrige Phase wird mit 2 x 200 ml Ethylacetat gewaschen. Die organische Phase wird verworfen, die wässrige Phase wird mit 1 N HCl auf pH 7-8 gebracht und mit 2 x 300 ml Ethylacetat extrahiert. Die organische Phase wird über Natriumsulfat getrocknet und zum Rückstand eingedampft; Ausbeute: 1.2 g "A277"; HPLC: Rt. = 2.27 min; LC-MS: 504 (M+H).
44.2 Herstellung von 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäureamid ("A278")
   150 mg (0.30 mmol) 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäure ("A277") werden in 2 ml DMF suspendiert und mit 1 ml (5.9 mmol) 10% Ammoniaklösung in THF, 67 µl (0.60 mmol) *N*-Methylmorpholin, 115 mg (0.60 mmol) EDCI und 41 mg (0.30 mmol) HOBt versetzt und 15 h bei Raumtemperatur gerührt. Das Reaktionsgemisch wird eingeengt und mittels präparativer HPLC aufgereinigt; Ausbeute: 10 mg "A278" Trifluoracetat, weißer Feststoff; HPLC: Rt. = 2.15 min; LC-MS: 503 (M+H).
44.3 Herstellung von 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäure-methylamid ("A279")
   150 mg (0.30 mmol) 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäure ("A277") werden in 2 ml DMF suspendiert und mit 205 mg (2.98 mmol) Methylamin Hydrochlorid, 67 µl (0.60 mmol) *N*-Methylmorpholin, 115 mg (0.60 mmol) EDCI, 41 mg (0.30 mmol) HOBt und 1.01 ml (5.96 mmol) N-Ethyldiisopropylamin versetzt und 15 h bei Raumtemperatur gerührt. Es werden nochmals 205 mg (2.98 mmol) Methylamin Hydrochlorid, 67 µl (0.60 mmol) *N*-Methylmorpholin, 115 mg (0.60 mmol) EDCI, 41 mg (0.30 mmol) HOBt und 1.01 ml (5.96 mmol) *N*-Ethyldiisopropylamin zugegeben und 15 h bei Raumtemperatur gerührt. Das Reaktionsgemisch wird eingeengt und mittels präparativer HPLC aufgereinigt; Ausbeute: 99 mg "A279" Trifluoracetat, weißer Feststoff; HPLC: Rt. = 2.22 min; LC-MS: 517 (M+H)

### Beispiel 45

Herstellung von 2-{3-[5-(3-Dimethylamino-propoxy)-pyrimidin-2-yl]-benzyl}-6-(5-oxo-4,5-dihydro-[1,2,4]oxadiazol-3-yl)-2H-pyridazin-3-on ("A227")

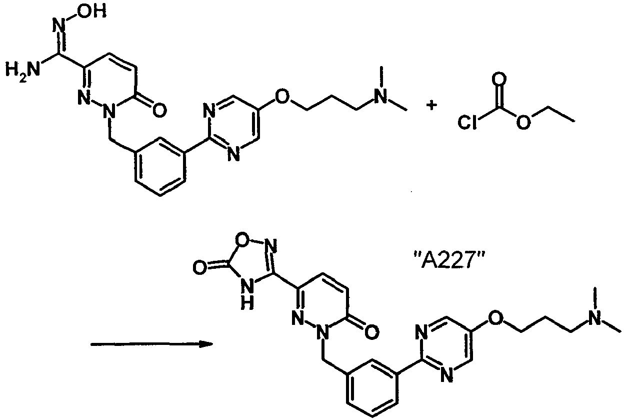

500 mg (1.18 mmol) 1-{3-[5-(3-Dimethylamino-propoxy)-pyrimidin-2-yl]-benzyl}-N-hydroxy-6-oxo-1,6-dihydro-pyridazin-3-carboxamidin werden in 15 ml DMF gelöst und mit 286 µl (3.54 mmol) Pyridin versetzt. Anschließend werden unter Rühren 124 µl (1.30 mmol) Ethylchlorformiat zugegeben und die Lösung 15 bei 80° C und anschließend 72 h bei 100°C gerührt. Das Reaktionsgemisch wird eingeengt und mittels präparativer HPLC aufgereinigt; Ausbeute: 21.2 mg "A227" Trifluoracetat; HPLC: Rt. = 2.07 min; LC-MS: 450 (M+H).

### Beispiel 46

Herstellung von 2-[3-(5-Amino-pyrazin-2-yl)-benzyl]-6-(3,5-difluor-phenyl)-2H-pyridazin-3-on ("A280")

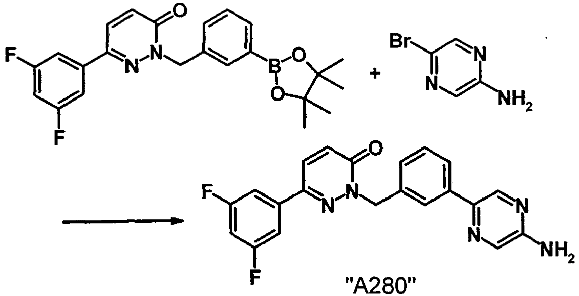

150 mg (0.35 mmol) 6-(3,5-Difluor-phenyl)-2-[3-(4,4,5,5-tetramethyl-[1,3,2]dioxaborolan-2-yl)-benzyl]-2H-pyridazin-3-on, 63 mg (0.35 mmol) 5-Brom-pyrazin-2-ylamin und 167 mg (1.99 mmol) Natriumhydrogencarbonat werden mit 5 ml Wasser und 5 ml Acetonitril versetzt und mehrfach entgast. Unter Argonatmosphäre werden 20 mg (0.017 mmol) Tetrakis-(triphenylphosphin)-palladium(0) zugeben und anschließend unter Rühren 15 h bei 80°C erhitzt. Anschließend werden nochmals 20 mg (0.017 mmol) Tetrakis(triphenylphosphin)-palladium(0) zugegeben und weitere 24 h bei 80°C gerührt. Die heiße Suspension wird filtriert. Das Filtrat wird zur Hälfte eingeengt. Nach dem Abkühlen auf Raumtemperatur wird der entstandene Niederschlag abgesaugt und mit wenig Wasser gewaschen. Der Rückstand wird mittels präparativer HPLC aufgereinigt; Ausbeute: 21 mg "A280"; HPLC: Rt. = 2.68 min (Methode C); LC-MS: 392 (M+H).

### Analog erhält man die nachstehende Verbindung

| Verbindung Nr. | Name und/oder Struktur | ESI [M+H]⁺ |
|---|---|---|
| "A282" | 2-[3-(6-Amino-pyridazin-3-yl)-benzyl]-6-(3,5-difluorphenyl)-2H-pyridazin-3-on | 392 |

### Beispiel 47

Herstellung von (E)-3-(2-{3-[6-Oxo-3-(3,4,5-trifluor-phenyl)-6H-pyridazin-1-ylmethyl]-phenyl}-pyrimidin-5-yl)-acrylsäure-methylester ("A283") 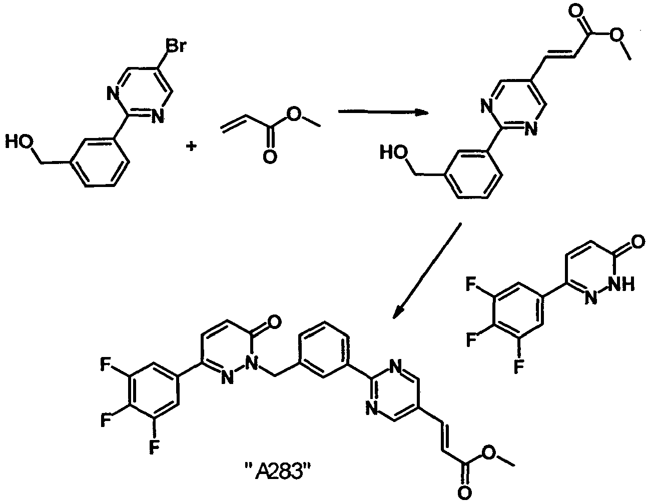
47.1 100 mg (0.38 mmol) [3-(5-Brom-pyrimidin-2-yl)-phenyl]-methanol und 51 µl (0.56 mmol) Methylacrylat werden in 2 ml DMF suspendiert und mit 20 mg (0.075 mmol) Triphenylphosphin, 222 mg (2.26 mmol) Kaliumacetat und 157 mg (0.57 mmol) Tetra-*n*-butylammoniumchlorid versetzt. Das Reaktionsgemisch wird entgast, mit Argon gespült und unter Argonatmosphäre mit 17 mg (0.075 mmol) Palladium(II)-acetat versetzt. Es wird 2 h auf 80°C erhitzt. Nach dem Abkühlen wird mit Wasser versetzt, wobei sich ein hellgrauer Niederschlag bildet. Dieser wird abgesaugt, mit Wasser gewaschen und im Vakuum getrocknet. Das Produkt wird ohne weitere Aufreinigung weiter umgesetzt; Ausbeute: 111 mg: HPLC: Rt. = 2,42 min (Methode C); LC-MS: 271 (M+H).
47.2 90 mg (0.4 mmol) 6-(3,4,5-Trifluor-phenyl)-2H-pyridazin-3-on und 111 mg (0.41 mmol) (E)-3-[2-(3-Hydroxymethyl-phenyl)-pyrimidin-5-yl]-acrylsäuremethylester werden mit 200 mg (0.6 mmol) polymergebundenem Triphenylphosphin (ca. 3 mmol Triphenylphosphin pro g) in 3 ml THF suspendiert und 30 min bei Raumtemperatur geschüttelt. Es wird auf 0°C gekühlt und 95 µl (0.6 mmol) Diethylazodicarboxylat werden zugegeben. Das Reaktionsgemisch wird 24 h bei Raumtemperatur geschüttelt. Das Reaktionsgemisch wird mittels präparativer HPLC aufgereinigt; Ausbeute: 7 mg "A283"; HPLC: Rt. = 3.41 min (Methode C); LC-MS: 479 (M+H).

### Beispiel 48

Herstellung von 2-{3-[5-((E)-3-Amino-propenyl)-pyrimidin-2-yl]-benzyl}-6-(3,4,5-trifluor-phenyl)-2H-pyridazin-3-on ("A284") 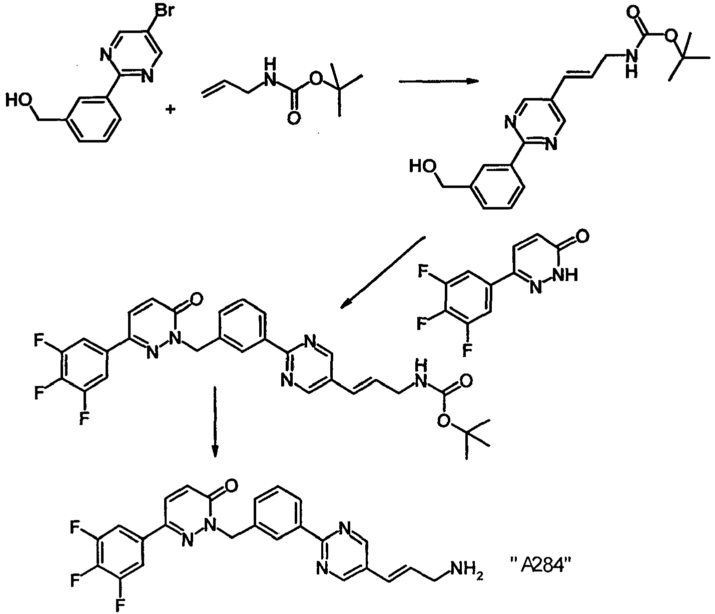
48.1 812 mg (3.06 mmol) [3-(5-Brom-pyrimidin-2-yl)-phenyl]-methanol und 722 mg (4.59 mmol) *tert*.-Butyl-*N*-allylcarbamat werden in 16 ml DMF suspendiert und mit 160 mg (0.61 mmol) Triphenylphosphin, 1.8 g (4.6 mmol) Kaliumacetat und 1.28 g (4.59 mmol) Tetra-*n*-butylammonium-chlorid versetzt. Das Reaktionsgemisch wird entgast und mit Argon gespült und unter Argonatmosphäre mit 137 mg (0.0.61 mmol) Palladium(II)-acetat versetzt. Es wird 2 h auf 80°C erhitzt. Nach dem Abkühlen wird über Kieselgur abgesaugt und das Filtrat in Wasser gegeben und mit 2 x 100 ml Ethylacetat extrahiert, über Natriumsulfat getrocknet und eingedampft. Das Produkt wurde ohne weitere Aufreinigung weiter umgesetzt; Ausbeute: 380 mg; HPLC: Rt. = 2,66 min (Methode C); LC-MS: 342 (M+H).
48.2 66 mg (0.29 mmol) 6-(3,4,5-Trifluor-phenyl)-2H-pyridazin-3-on und 142 mg (0.29 mmol) {(E)-3-[2-(3-Hydroxymethyl-phenyl)-pyrimidin-5-yl]-allyl}-carbamsäure-*tert.*-butylester werden mit 145 mg (0.44 mmol) polymergebundenem Triphenylphosphin (ca. 3 mmol Triphenylphosphin pro g) in 3 ml THF suspendiert und 30 min bei Raumtemperatur geschüttelt. Es wird auf 0°C gekühlt und 69 µl (0.44 mmol) Diethylazodicarboxylat werden zugegeben. Das Reaktionsgemisch wird 24 h bei Raumtemperatur geschüttelt. Das Reaktionsgemisch wird mittels präparativer HPLC aufgereinigt: Ausbeute: 28 mg; HPLC: Rt. = 3.50 min (Methode C); LC-MS: 550 (M+H).
48.3 28 mg (0.051 mmol) [(Z)-3-(2-{3-[6-Oxo-3-(3,4,5-trifluor-phenyl)-6H-pyridazin-1-ylmethyl]-phenyl}-pyrimidin-5-yl)-allyl]-carbaminsäure-*tert.-*butylester werden in 4 ml Dichlormethan gelöst und mit 79 µl (1.02 mmol) Trifluoressigsäure versetzt. Das Reaktionsgemisch wird 15 h bei Raumtemperatur gerührt und eingeengt. Der Rückstand wird mittels präparativer HPLC aufgereinigt; Ausbeute: 11 mg "A284" Trifluoracetat; HPLC: Rt. = 2.64 min (Methode C); LC-MS: 450 (M+H).

### Beispiel 49

Herstellung von 2-{3-[5-(3-Amino-propyl)-pyrimidin-2-yl]-benzyl}-6-(3,4,5-trifluor-phenyl)-2H-pyridazin-3-on ("A285") 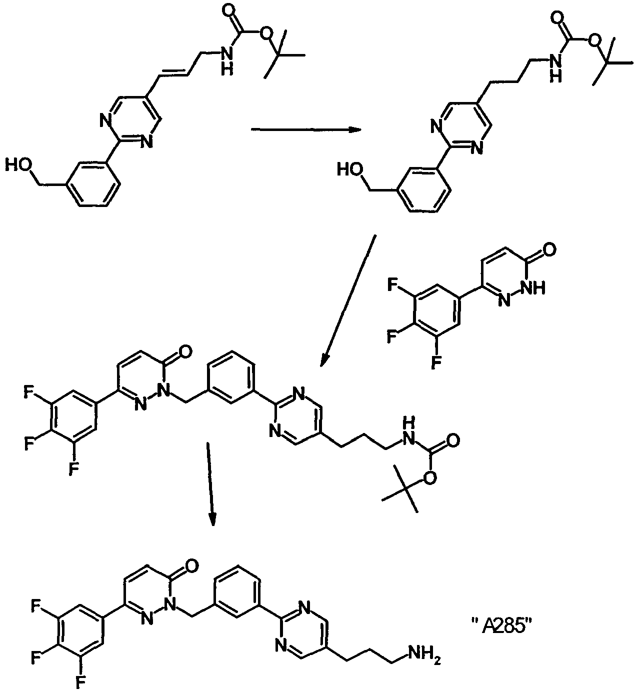
49.1 280 mg (0.82 mmol) {(E)-3-[2-(3-Hydroxymethyl-phenyl)-pyrimidin-5-yl]-allyl}-carbamsäure-*tert.*-butylester werden in 10 ml THF gelöst, mit 300 mg Platin auf Aktivkohle (5%, enthält 56 % Wasser) unter Wasserstoffatmosphäre 17 h bei Raumtemperatur geschüttelt. Der Katalysator wird abgesaugt und das Filtrat zum Rückstand eingedampft; Ausbeute: 289 mg; HPLC: Rt. = 2.60 min (Methode C) LC-MS: 344 (M+H).
49.2 195 mg (0.86 mmol) 6-(3,4,5-Trifluor-phenyl)-2H-pyridazin-3-on und 369 mg (0.86 mmol) {3-[2-(3-Hydroxymethyl-phenyl)-pyrimidin-5-yl]-propyl}-carbaminsäure-*tert.*-butylester werden mit 430 mg (1.29 mmol) polymergebundenem Triphenylphosphin (ca. 3 mmol Triphenylphosphin pro g) in 10 ml THF suspendiert und 30 min bei Raumtemperatur geschüttelt. Es wird auf 0°C gekühlt und 297 mg (0.1.29 mmol) Di-*tert.-*butylazodicarboxylat werden zugegeben. Das Reaktionsgemisch wird 24 h bei Raumtemperatur geschüttelt. Es werden weitere 430 mg (1.29 mmol) polymergebundenes Triphenylphosphin (ca. 3 mmol Triphenylphosphin pro g) und 297 mg (0.1.29 mmol) Di-*tert.*-butylazodicarboxylat zugegeben und das Reaktionsgemisch 24 h bei Raumtemperatur geschüttelt. Das Reaktionsgemisch wird filtriert, der Rückstand eingedampft und der Rückstand mittels präparativer HPLC aufgereinigt; Ausbeute: 333 mg; HPLC: Rt. = 3.45 min; LC-MS: 552 (M+H).
49.3 70 mg (0.127 mmol) [3-(2-{3-[6-Oxo-3-(3,4,5-trifluor-phenyl)-6H-pyridazin-1-ylmethyl]-phenyl}-pyrimidin-5-yl)-propyl]-carbaminsaure-*tert.-*butylester werden in 3 ml Dichlormethan gelöst und mit 195 µl (2.54 mmol) Trifluoressigsäure versetzt. Das Reaktionsgemisch wird 15 h bei Raumtemperatur gerührt und eingeengt. Der Rückstand wird mit Diethylether digeriert und im Vakuum getrocknet; Ausbeute: 74 mg "A285"; HPLC: Rt. = 2.63 min (Methode C); LC-MS: 452 (M+H).

### Beispiel 50

Herstellung von 2-{3-[5-(4-Methyl-piperazin-1-yl)-pyrimidin-2-yl]-benzyl}-6-(1-methyl-1 H-pyrazol-4-yl)-2H-pyridazin-3-on ("A286")
50.1 Herstellung von 3-(5-Amino-pyrimidin-2-yl)-benzoesäure-methylester 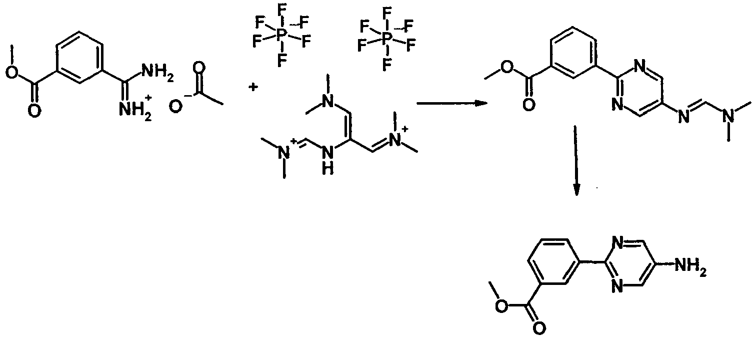
   65.4 g (274 mmol) 3-Carbamimidoyl-benzoesäuremethylester wird in 800 ml Methanol suspendiert und mit 134 g (274 mmol) ({2-Dimethylamino-1-[dimethylimmoniomethyl]-vinylamino}-methylen)-dimethyl-ammonium-di-hexafluorophosphat versetzt. Zu dieser Suspension wird 102 ml (548 mmol) 30%ige Natriummethanolatlösung in Methanol zugetropft. Es entsteht eine Lösung. Diese wird 1 Stunde bei 60°C Innentemperatur gerührt. Nach dem Abkühlen auf Raumtemperatur werden weitere 20 ml 30%ige Natriummethanolatlösung in Methanol zugetropft und 1 Stunde bei 60°C gerührt. Nach dem Abkühlen auf Raumtemperatur wird der entstandene Niederschlag abgesaugt, der Rückstand in 1 I Wasser suspendiert, 30 min bei Raumtemperatur gerührt. Der Niederschlag wird abgesaugt und im Vakuumtrockenschrank bei 80°C getrocknet; Ausbeute: 68.5 g; HPLC: Rt. = 2.03 min (Methode C); LC-MS: 285 (M+H).
   10.2 g (35.9 mmol) 3-[5-(Dimethylamino-methylenamino)-pyrimidin-2-yl]-benzoesäuremethylester werden in 1 I Methanol suspendiert. Unter leichter Kühlung (ca. 5-10°C) werden 5.3 ml (107.3 mmol) rauchende Schwefelsäure tropfenweise zugetropft (Achtung, stark exotherme Reaktion). Nach beendeter Zugabe wird zunächst 30 min bei RT und anschließend bei 88° Ölbadtemperatur gerührt. Die Reaktion wird mittels HPLC verfolgt. Nach 20 h wird die klare, dunkelgelbe Lösung zum Rückstand abgezogen. Der Rückstand wird in 600 ml Ethylacetat gelöst und mit 2 x 150 ml 1 N NaOH und 2 x 1 N HCl gewaschen, über Natriumsulfat getrocknet und eingedampft; Ausbeute: 3g; HPLC: Rt. = 2.17 min (Methode C); LC-MS: 300 (M+H).
50.2 Herstellung von {3-[5-(4-Methyl-piperazin-1-yl)-pyrimidin-2-yl]-phenyl}-methanol

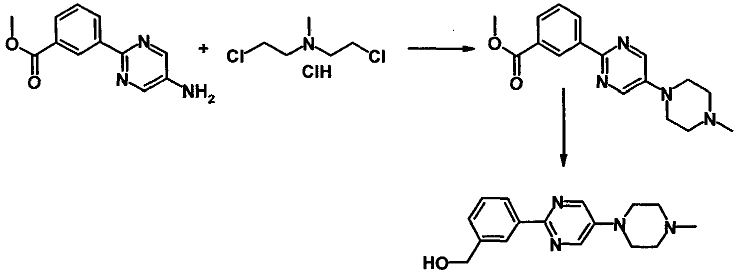

2.5 g (10.9 mmol) 3-(5-Amino-pyrimidin-2-yl)-benzoesäuremethylester werden in 10 ml NMP gelöst, mit 2.59 g (18.5 mmol) Kaliumcarbonat und 3.6 g (18.5 mmol) Bis-(2-chlor-ethyl)-ethyl-amin Hydrochlorid versetzt. Die Suspension wird unter Argonatmosphäre 15 h bei 120°C gerührt. Anschließend wird weitere 12 h bei 140°C gerührt. Nach dem Abkühlen auf Raumtemperatur wird das Reaktionsgemisch in 150 ml Wasser eingerührt. Der entstandene Niederschlag wird über Kieselgur abgesaugt und verworfen. Das Filtrat wird mit 32%-iger NaOH auf pH=14 eingestellt. Die leicht getrübte Lösung wird mit 2 x 200 ml Ethylacetat extrahiert. Die vereinigten organischen Phasen werden mit gesättigter Natriumchloridlösung gewaschen, über Natriumsulfat getrocknet und zum Rückstand eingeengt und im Vakuum getrocknet. Das Produkt wird ohne weitere Aufreinigung weiter umgesetzt; Ausbeute: 860 mg; HPLC: Rt. = 2.11 min (Methode C); LC-MS: 313 (M+H).

860 mg (2.75 mmol) 3-[5-(4-Methyl-piperazin-1-yl)-pyrimidin-2-yl]-benzoesäuremethylester werden in 16 ml THF gelöst und bei Raumtemperatur werden 13.8 ml (13.8 mmol) 1 M Diisobutylaluminiumhydrid in THF zugetropft und das Reaktionsgemisch wird 1 h bei Raumtemperatur gerührt. Es werden weitere 13.8 ml (13.8 mmol) 1 M Diisobutylaluminiumhydrid in THF zugetropft und das Reaktionsgemisch wird 1 h bei Raumtemperatur gerührt. Das Reaktionsgemisch wird unter Eiskühlung mit 3 ml gesättigter Natriumsulfatlösung versetzt. Das gelartige Gemisch wird mit Dichlormethan versetzt, 30 min gerührt und filtriert. Das Filtrat wird über Natriumsulfat getrocknet und eingedampft.
Ausbeute: 300 mg, gelber Feststoff. Das Produkt wird ohne weitere Aufreinigung weiter umgesetzt; HPLC: 1.68 min (Methode C); LC-MS: 285 (M+H).

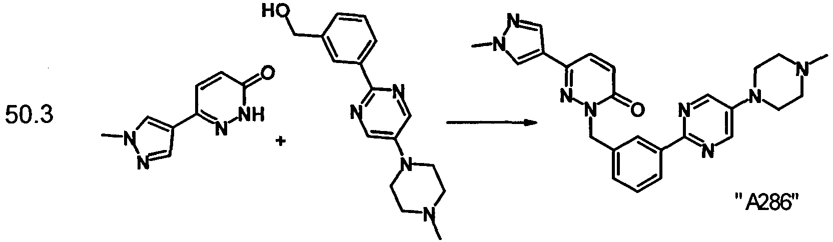

71 mg (0.40 mmol) 6-(1-Methyl-1H-pyrazol-4-yl)-2H-pyridazin-3-on und 163 mg (0.40 mmol) {3-[5-(4-Methyl-piperazin-1-yl)-pyrimidin-2-yl]-phenyl}-methanol werden mit 200 mg (0.60 mmol) polymergebundenem Triphenylphosphin (ca. 3 mmol Triphenylphosphin pro g) in 3 ml THF und 1 ml DMF suspendiert und 30 min bei Raumtemperatur geschüttelt. Es werden 139 mg (0.60 mmol) Di-*tert.*-butylazodicarboxylat zugegeben. Das Reaktionsgemisch wird 1 h bei Raumtemperatur geschüttelt. Es werden weitere 200 mg (0.6 mmol) polymergebundenes Triphenylphosphin (ca. 3 mmol Triphenylphosphin pro g) und 139 mg (0.60 mmol) Di-*tert.-*butylazodicarboxylat zugegeben und das Reaktionsgemisch 2 h bei Raumtemperatur geschüttelt. Das Reaktionsgemisch wird filtriert, der Rückstand eingedampft und der Rückstand mittels präparativer HPLC aufgereinigt; Ausbeute: 18 mg "A286"; HPLC: Rt. = 2.08 min (Methode C); LC-MS: 443 (M+H).

### Beispiel 51

Herstellung von 3-(1-{3-[5-(4-Methyl-piperazin-1-yl)-pyrimidin-2-yl]-benzyl}-6-oxo-1,6-dihydro-pyridazin-3-yl)-benzonitril ("A287")

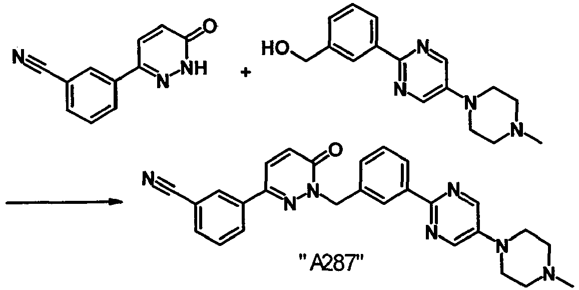

149 mg (0.76 mmol) 3-(6-Oxo-1,6-dihydro-pyridazin-3-yl)-benzonitril und 256 mg (0.76 mmol) {3-[5-(4-Methyl-piperazin-1-yl)-pyrimidin-2-yl]-phenyl}-methanol werden mit 378 mg (1.13 mmol) polymergebundenem Triphenylphosphin (ca. 3 mmol Triphenylphosphin pro g) in 5 ml DMF suspendiert und 30 min bei Raumtemperatur geschüttelt. Es werden 266 mg (1.134 mmol) Di-*tert.*-butylazodicarboxylat zugegeben. Das Reaktionsgemisch wird 2 h bei Raumtemperatur geschüttelt. Es werden weitere 378 mg (1.13 mmol) polymergebundenes Triphenylphosphin (ca. 3 mmol Triphenylphosphin pro g) und 266 mg (1.134 mmol) Di-*tert.*-butylazodicarboxylat zugegeben und das Reaktionsgemisch 2 h bei Raumtemperatur geschüttelt. Das Reaktionsgemisch wird filtriert, das Filtrat eingedampft und der Rückstand mittels Säulenchromatographie an Kieselgel aufgereinigt; Ausbeute: 59 mg "A287"; HPLC: Rt. = 2.38 min (Methode C); LC-MS: 464 (M+H).

### Beispiel 52

Herstellung von 3-{6-Oxo-1-[3-(5-piperazin-1-yl-pyrimidin-2-yl)-benzyl]-1,6-dihydro-pyridazin-3-yl}-benzonitril ("A288")
52.1 Herstellung von 4-[2-(3-Hydroxymethyl-phenyl)-pyrimidin-5-yl]-piperazin-1-carbonsäure-*tert.*-butylester 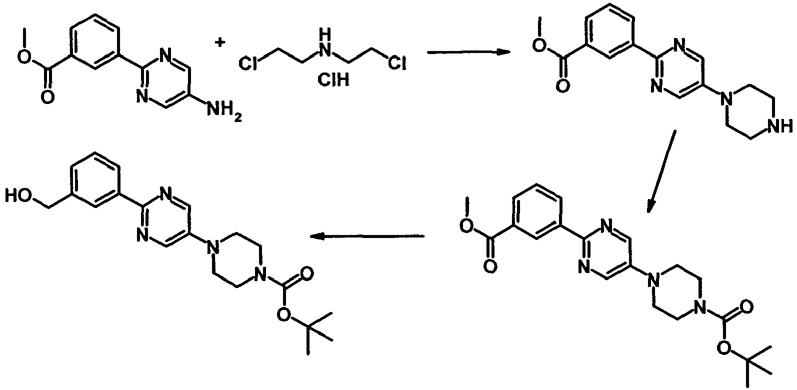
   3.2 g (13.95 mmol) 3-(5-Amino-pyrimidin-2-yl)-benzoesäuremethylester werden in 80 ml NMP gelöst, mit 4.73 g (25.96 mmol) Bis(2-chlorethyl)-ammoniumchlorid und 3.13 g (23.73 mmol) Kaliumcarbonat versetzt. Die Suspension wird unter Argonatmosphäre 7 Tage bei 130°C gerührt. Das Reaktionsgemisch wird filtriert, das Filtrat wird in 1 l Diethylether eingerührt. Dabei ölt ein Rückstand aus. Die organische Phase wird abgetrennt und verworfen. Der Rückstand wird mit 500 ml Ethylacetat und 200 ml gesättigter Natriumhydrogencarbonatlösung versetzt, die organische Phase abgetrennt und die wässrige Phase nochmals mit 500 ml Ethylacetat extrahiert. Die organischen Phasen werden vereinigt, über Natriumsulfat getrocknet und eingedampft. Der Rückstand wird ohne weitere Aufarbeitung weiter umgesetzt; Ausbeute: 2.4 g; HPLC: Rt. = 2.07 min (Methode C); LC-MS: 299 (M+H).
   2.4 g (5.4 mmol) 3-(5-Piperazin-1-yl-pyrimidin-2-yl)-benzoesäuremethylester wird in 15 ml DMF gelöst, mit 2.98 g (21.6 mmol) Kaliumcarbonat und 1.5 ml (7.0 mmol) Di-*tert.*-butyldicarbonat versetzt und 30 min bei Raumtemperatur gerührt. Das Reaktionsgemisch wird filtriert und das Filtrat eingeengt. Der Rückstand wird in 200 ml Ethylacetat und 50 ml gesättigter Natriumhydrogencarbonatlösung aufgenommen. Die organische Phase wird abgetrennt und mit 50 ml 1 N HCl gewaschen, über Natriumsulfat getrocknet und eingeengt. Das Produkt wird ohne weitere Aufreinigung weiter umgesetzt; Ausbeute: 1.1 g; HPLC: 3.18 min (Methode C); LC-MS: 399 (M+H).
   862 mg (2.16 mmol) 4-[2-(3-Methoxycarbonyl-phenyl)-pyrimidin-5-yl]-piperazine-1-carbonsäure-*tert.*-butylester werden in 15 ml THF gelöst und bei Raumtemperatur mit 10.8 ml (10.8 mmol) 1 M Diisobutylaluminiumhydrid in THF versetzt. Das Reaktionsgemisch wird 1 h bei Raumtemperatur gerührt. Das Reaktionsgemisch wird unter Eiskühlung mit 3 ml ges. Natriumsulfatlösung versetzt. Das gelartige Gemisch wird mit 30 ml Dichlormethan und 5 ml Methanol versetzt, 10 min gerührt und über Kieselgur abgesaugt. Das Filtrat wird über Natriumsulfat getrocknet und eingedampft. Der Rückstand wird in Dichlormethan gelöst, filtriert und das Filtrat eingedampft. Das Produkt wird ohne weitere Aufreinigung weiter umgesetzt; Ausbeute: 677 mg; HPLC: 2.66 min (Methode C); LC-MS: 371 (M+H).
52.2

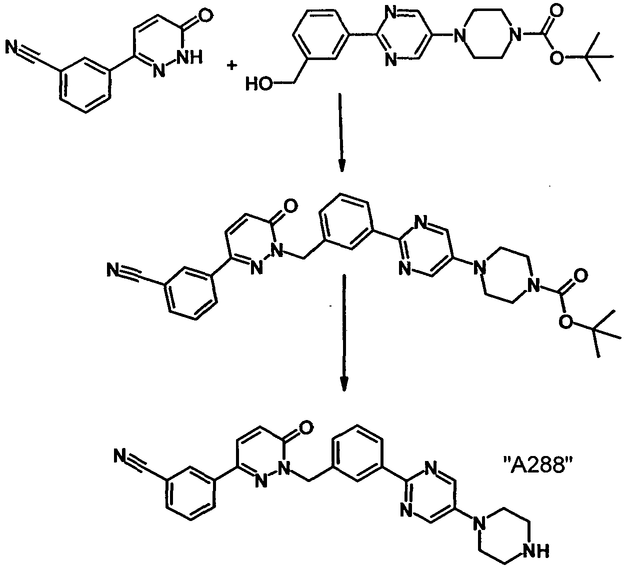

94 mg (0.48 mmol) 3-(6-Oxo-1,6-dihydro-pyridazin-3-yl)-benzonitril und 177 mg (0.48 mmol) 4-[2-(3-Hydroxymethyl-phenyl)-pyrimidin-5-yl]-piperazin-1-carbonsäure-*tert.*-butylester werden mit 240 mg (0.72 mmol) polymergebundenem Triphenylphosphin (ca. 3 mmol Triphenylphosphin pro g) in 4 ml THF und 1 ml DMF suspendiert und 30 min bei Raumtemperatur geschüttelt. Es werden 168 mg (0.72 mmol) Di-*tert.*-butylazodicarboxylat zugegeben. Das Reaktionsgemisch wird filtriert, der Rückstand eingedampft und der Rückstand mittels Säulenchromatographie an Kieselgel aufgereinigt; Ausbeute: 143 mg; HPLC: Rt. = 3.24 min (Methode C); LC-MS: 550 (M+H).

143 mg (0.26 mmol4-(2-{3-[3-(3-Cyan-phenyl)-6-oxo-6H-pyridazin-1-ylmethyl]-phenyl}-pyrimidin-5-yl)-piperazine-1-carbaminsaure-*tert.-*butylester werden in 6 ml Acetonitril gelöst und mit 6 ml 4 M HCl in Dioxan versetzt. Das Reaktionsgemisch wird 1 h bei Raumtemperatur gerührt und eingeengt. Der Rückstand wird in Wasser und Ethylacetat aufgenommen, die Wasserphase mit NaOH auf pH 12 gebracht und mit Ethylacetat und Dichlormethan extrahiert. Die organischen Phasen werden vereinigt, über Natriumsulfat getrocknet und mittels Säulenchromatographie aufgereinigt. Ausbeute: 117 mg "A288" HPLC: Rt. = 2.36 min (Methode C); LC-MS: 450 (M+H).

### Herstellung einer Vorstufe für die Herstellung von "A289" und "A290"

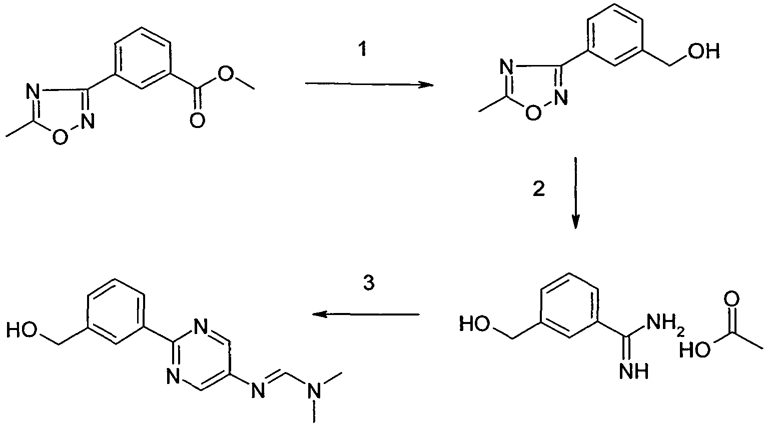

### 1. Herstellung von [3-(5-Methyl-[1,2,4]oxadiazol-3-yl)-phenyl]-methanol

In einem 250 ml Dreihalskolben werden 3,46 g 3-(5-Methyl-[1,2,4]oxadiazol-3-yl)-benzoesäuremethylester (15,86 mmol) in 50 ml abs. THF gelöst und anschließend unter Stickstoffatmosphäre bei 0°C unter Rühren portionsweise 0,691 g LiBH4 (31,71 mmol) eingetragen und 20 h ohne Kühlung nachgerührt. Zur Aufarbeitung wird unter Rühren das Reaktionsgemisch durch langsames Zutropfen von 1 N HCl auf pH7 eingestellt, mit 100 ml Wasser verdünnt und 3x mit 50 ml Dichlormethan extrahiert. Die vereinigten organischen Phasen werden 1x 100 ml Wasser gewaschen, über Natriumsulfat getrocknet und am Rotationsverdampfer zur Trockne eingeengt. Die Aufreinigung erfolgt chromatographisch (50 g Kieselgel / DCM + 0-1 % MeOH). Das Produkt wird aus Diethylether/Petrolether kristallisiert; F. 57-58 °C.

### 2. Herstellung von 3-Hydroxymethyl-benzamidiniumacetat

124,84 g [3-(5-Methyl-[1,2,4]oxadiazol-3-yl)-phenyl]-methanol (569,39 mmol) werden in einer Mischung aus 1300 ml Methanol, 100 ml Eisessig und 100 ml Wasser mit 40 g RaNi (wassernass) versetzt und bei Raumtemperatur und Normaldruck bis zu einer Wasserstoffaufnahme von 14,7 I (45 h) hydriert. Zur Aufarbeitung wird der Katalysator abfiltiert und die verbleibende Lösung zum Rückstand eingeengt und der Rückstand in Methyl-tert.-butylether aufgekocht und abfiltiert. Das Kristallisat wirde über Nacht im Vakuum getrocknet.

### 3. Herstellung von N'-[2-(3-Hydroxymethyl-phenyl)-pyrimidin-5-yl]-N,N-dimethyl-formamidin

In einem N₂-gespülten 100 ml Dreihalskolben wurden unter CaCl₂-Schutz 716 mg 3-Hydroxymethyl-benzamidiniumacetat (3,41 mmol) und 1,66 g ({2-Dimethylamino-1-[dimethylimmoniomethyl]-vinylamino}methylene)-dimethyl-ammonium-dihexafluorophosphat (Aminoreducton Precursor) (3,41 mmol) in 15 ml abs. Methanol sususpendiert und unter Rühren eine frisch hergestellte Lösung von 0,235 g Na in 5 ml abs. Methanol zugetropft. Das Reaktionsgemisch rührt 30 min bei 60 °C, wobei eine klare Lösung entsteht. Zur Aufarbeitung wird der Reaktionsansatz mit 50 ml Dichlormethan verdünnt, 2x mit 20 ml Wasser gewaschen, zum Rückstand abgezogen und chromatographisch aufgereinigt (Kieselgel DCM + 0-5 % MeOH); F. 105-6 °C.

### Beispiel 53

Herstellung von 6-(4-Methansulfonyl-phenyl)-2-[3-(5-piperazin-1-yl-pyrimidin-2-yl)-benzyl]-2H-pyridazin-3-on ("A289") 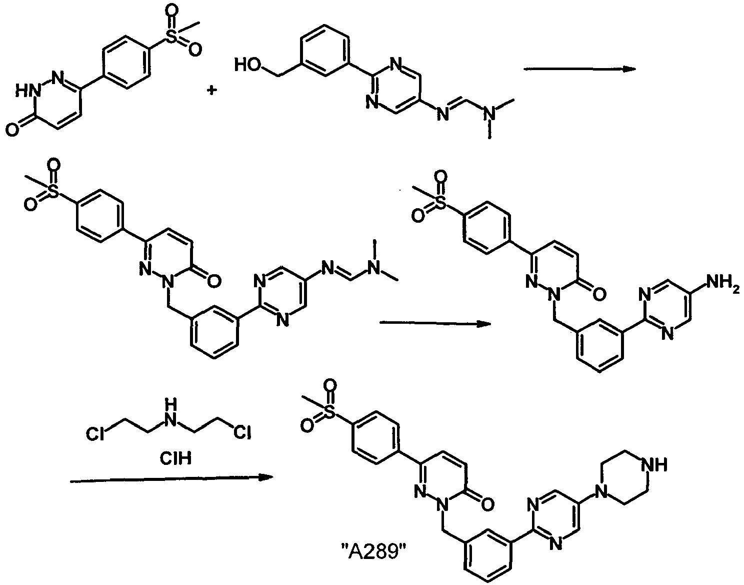
53.1 1.95 g (7.8 mmol) 6-(4-Methansulfonyl-phenyl)-2H-pyridazin-3-on und 2 g (7.8 mmol) *N*'-[2-(3-Hydroxymethyl-phenyl)-pyrimidin-5-yl]-*N*,*N-*dimethyl-formamidin werden mit 3.9 g (11.7 mmol) polymergebundenem Triphenylphosphin (ca. 3 mmol Triphenylphosphin pro g) in 50 ml THF und 15 ml DMF suspendiert und 30 min bei Raumtemperatur geschüttelt. Es werden 2.75 g (11.7 mmol) Di-*tert.*-butylazodicarboxylat zugegeben. Das Reaktionsgemisch wird 15 h bei Raumtemperatur geschüttelt. Es werden weitere 2.6 g (7.8 mmol) polymergebundenem Triphenylphosphin (ca. 3 mmol Triphenylphosphin pro g) und 1.80 g (7.8 mmol) Di-*tert.-*butylazodicarboxylat zugegeben. Das Reaktionsgemisch wird 15 h bei Raumtemperatur geschüttelt. Das Reaktionsgemisch wird filtriert und das Filtrat eingeengt. Der ölige Rückstand wird mit 1 N HCl (100 ml) versetzt und mit Ethylacetat (100 ml) extrahiert. Die saure Wasserphase wird nochmals mit Ethylacetat gewaschen und dann mit festem Natriumhydrogencarbonat auf pH7 gebracht. Es wird 2 x mit Ethylacetat extrahiert. Die organische Phase wird eingedampft und der Rückstand im Vakuum getrocknet; Ausbeute: 1 g; HPLC: Rt. = 2.19 min (Methode C); LC-MS: 489 (M+H).
53.2 1.7 g (3.48 mmol) *N*-(2-{3-[3-(4-Methansulfonyl-phenyl)-6-oxo-6H-pyridazin-1-ylmethyl]-phenyl}-pyrimidin-5-yl)-*N*,*N*-dimethyl-formamidin werden in 30 ml Dioxan und 30 ml Wasser gelöst und mit 1.68 g (12.2 mmol) Kaliumcarbonat versetzt. Das Reaktionsgemisch wird 15 h refluxiert. Nach dem Abkühlen auf Raumtemperatur wird das Reaktionsgemisch auf ca. 30 ml eingeengt, der entstandene Niederschlag abgesaugt, mit Wasser gewaschen und im Vakuum getrocknet. Ausbeute: 1.5 g HPLC: 2.30 min (Methode C); LC-MS: 434 (M+H).
53.3 1.4 g (3.23 mmol) 2-[3-(5-Amino-pyrimidin-2-yl)-benzyl]-6-(4-methanesulfonyl-phenyl)-2H-pyridazin-3-on werden in 30 ml NMP gelöst, mit 1.59 g (8.72 mmol) Bis-(2-chlor-ethyl)-ethyl-amin Hydrochlorid und 1.22 g (8.72 mmol) Kaliumcarbonat versetzt. Die Suspension wird unter Argonatmosphäre 5 Tage bei 130°C gerührt. Das Reaktionsgemisch wird filtriert, das Filtrat wird in 200 ml Diethylether eingerührt. Dabei ölt ein Rückstand aus. Der Rückstand wird mittels Säulenchromatographie an Kieselgel aufgereinigt. Das so erhaltene Produkt wird mittels präparativer HPLC aufgereinigt; Ausbeute: 41 mg "A289" Trifluoracetat; HPLC: Rt. = 2.19 min (Methode C); LC-MS: 503 (M+H).

### Beispiel 54

Herstellung von 4-{1-[3-(5-Amino-pyrimidin-2-yl)-benzyl]-6-oxo-1,6-dihydro-pyridazin-3-yl}-benzonitril ("A290") 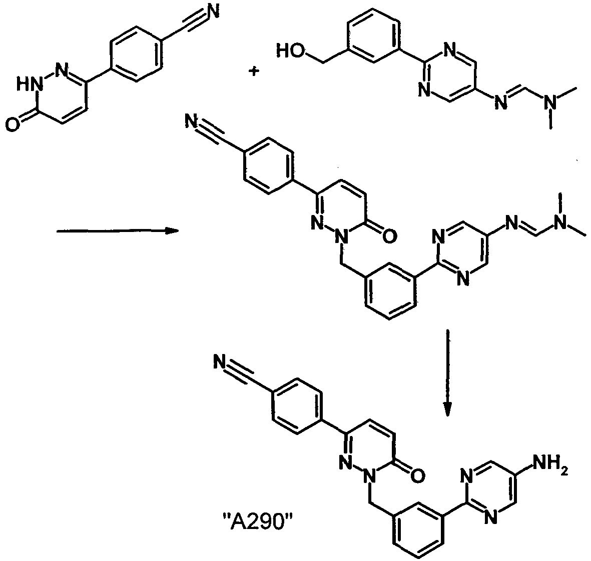
54.1 1.5 g (7.8 mmol) 6-(4-Cyanphenyl)-2H-pyridazin-3-on und 2 g (7.8 mmol) *N*'-[2-(3-Hydroxymethyl-phenyl)-pyrimidin-5-yl]-*N*,*N*-dimethyl-formamidin werden mit 3.9 g (11.7 mmol) polymergebundenem Triphenylphosphin (ca. 3 mmol Triphenylphosphin pro g) in 50 ml THF und 15 ml DMF suspendiert und 30 min bei Raumtemperatur geschüttelt. Es werden 2.75 g (11.7 mmol) Di-*tert.*-butylazodicarboxylat zugegeben. Das Reaktionsgemisch wird 15 h bei Raumtemperatur geschüttelt. Es werden weitere 2.6 g (7.8 mmol) polymergebundenem Triphenylphosphin (ca. 3 mmol Triphenylphosphin pro g) und 1.80 g (7.8 mmol) Di-*tert.*-butylazodicarboxylat zugegeben. Das Reaktionsgemisch wird 15 h bei Raumtemperatur geschüttelt. Das Reaktionsgemisch wird filtriert und das Filtrat eingeengt. Der ölige Rückstand wird mit 1 N HCl (100 ml) versetzt und mit Ethylacetat (100 ml) extrahiert. Die saure Wasserphase wird nochmals mit Ethylacetat gewaschen und dann mit festem Natriumhydrogencarbonat auf pH7 gebracht. Es wird 2 x mit Ethylacetat extrahiert. Die organische Phase wird eingedampft und der Rückstand im Vakuum getrocknet; Ausbeute: 1.2 g; HPLC: Rt. = 1.59 min (Methode C); LC-MS: 436 (M+H).
54.2 1.2 g (3.48 mmol) *N*'-(2-{3-[3-(4-Cyanphenyl)-6-oxo-6H-pyridazin-1-ylmethyl]-phenyl}-pyrimidin-5-yl)-*N*,*N*-dimethyl-formamidin werden in 50 ml Dioxan und 50 ml Wasser gelöst und mit 1.2 g (8.7 mmol) Kaliumcarbonat versetzt. Das Reaktionsgemisch wird 15 h refluxiert. Nach dem Abkühlen auf Raumtemperatur wird das Reaktionsgemisch auf ca. 30 ml eingeengt, der entstandene Niederschlag abgesaugt, mit Wasser gewaschen und im Vakuum getrocknet. Der Rückstand wird mittels Säulenchromatographie an Kieselgel aufgereinigt; Ausbeute: 145 mg "A290"; HPLC: 2.49 min (Methode C); LC-MS: 381 (M+H).

Analog erhält man die Verbindung 3-{1-[3-(5-Amino-pyrimidin-2-yl)-benzyl]-6-oxo-1,6-dihydro-pyridazin-3-yl}-benzonitril ("A291"); ESI 381.

### Beispiel 55

Herstellung von
6-(1-Methyl-1H-pyrazol-4-yl)-2-[3-(5-piperazin-1-yl-pyrimidin-2-yl)-benzyl]-2H-pyridazin-3-on ("A292") und
2-[3-(5-Amino-pyrimidin-2-yl)-benzyl]-6-(1-methyl-1H-pyrazol-4-yl)-2H-pyridazin-3-on ("A293")
55.1 Herstellung von *N*,*N*-Dimethyl-*N'*-(2-{3-[3-(1-methyl-1H-pyrazol-4-yl)-6-oxo-6H-pyridazin-1-ylmethyl]-phenyl}-pyrimidin-5-yl)-formamidin 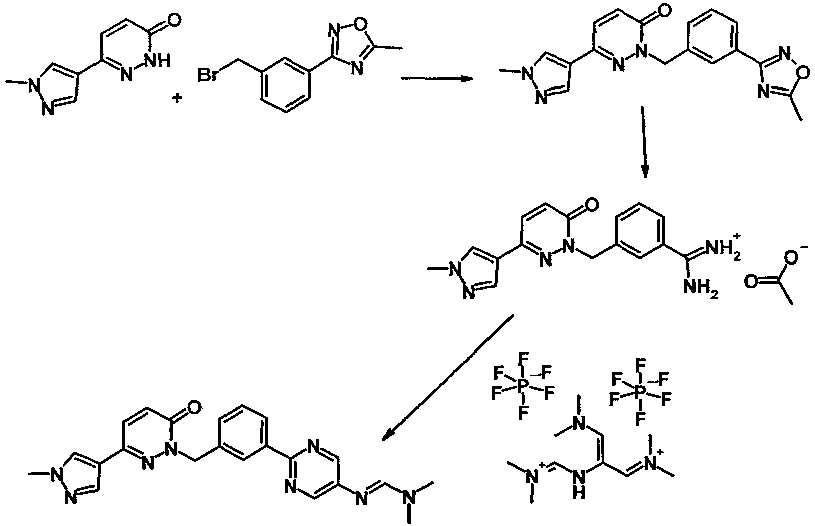
   Eine Lösung von 1.7 g (4.8 mmol) 6-(1-Methyl-1H-pyrazol-4-yl)-2H-pyridazin-3-on und 1.22 g (4.8 mmol) 3-(3-Brommethyl-phenyl)-5-methyl-[1,2,4]oxadiazol (hergestellt nach W. W. K. R. Mederski et al, Tetrahedron 55, 1999, 12757-12770) in 50 ml DMF wird mit 3.33 g (24.1 mmol) Kaliumcarbonat versetzt und die entstandene Suspension 5 Tage bei Raumtemperatur gerührt. Das Reaktionsgemisch wird mit Wasser versetzt und mit Ethylacetat extrahiert. Die organische Phase wird mit Wasser gewaschen, über Natriumsulfat getrocknet und eingeengt. Der Rückstand wird mit Isopropanol versetzt und 15 min gerührt, filtriert, der Rückstand mit Iospropanol und Diethylether nachgewaschen und im Vakuum getrocknet. Ausbeute: 740 mg; HPLC: Rt. = 2.42 min (Methode C); LC-MS: 349 (M+H).
   Eine Lösung aus 6.77 g (19.4 mmol) 6-(1-Methyl-1H-pyrazol-4-yl)-2-[3-(5-methyl-[1,2,4]oxadiazol-3-yl)-benzyl]-2H-pyridazin-3-on in 300 ml Methanol wird mit 2 ml Essigsäure, 2 ml Wasser und 6 g Raney-Nickel versetzt und 2 Tage bei Raumtemperatur und unter Wasserstoffatmosphäre hydriert. Das Reaktionsgemisch wird filtriert und das Filtrat eingedampft und im Vakuum getrocknet. Das Produkt wurde ohne weitere Aufreinigung weiter umgesetzt; Ausbeute: 6 g; HPLC: 1.74 min (Methode C); LC-MS: 309 (M+H).
   Eine Suspension von 7.5 g (20.4 mmol) 3-[6-Oxo-3-(1-Methyl-1H-pyrazol-4-yl)-6H-pyridazin-1-ylmethyl]-benzamidinium-Acetat und 9.94 g (20.4 mmol) ({2-Dimethylamino-1-[dimethylimmoniomethyl]-vinylamino}-methylen)-dimethyl-ammonium-di-hexafluorophosphat werden in 70 ml Methanol gelöst und 7.6 ml (40.7 mmol) 30%ige Natriummethanolatlösung in Methanol werden zugetropft. Das Reaktionsgemisch wird langsam auf 60° C erwärmt und 60 Minuten bei dieser Temperatur gerührt. Nach dem Abkühlen auf Raumtemperatur werden weitere 5.6 ml (30.0 mmol) 30%iges Natriummethanolatlösung in Methanol zugetropft und 2 h bei 60°C gerührt. Nach dem Abkühlen wird das Lösungsmittel abdestilliert und der Rückstand mit Wasser versetzt. Die wässrige Phase wird abdekantiert, der Rückstand mit Ethylacetat versetzt und 15 min bei Raumtemperatur gerührt. Der Niederschlag wird abgesaugt, mit Ethylacetat gewaschen und im Vakuum getrocknet; Ausbeute: 6.8 g beigefarbener Feststoff; HPLC: 2.05 min (Methode C); LC-MS: 415 (M+H).
55.2

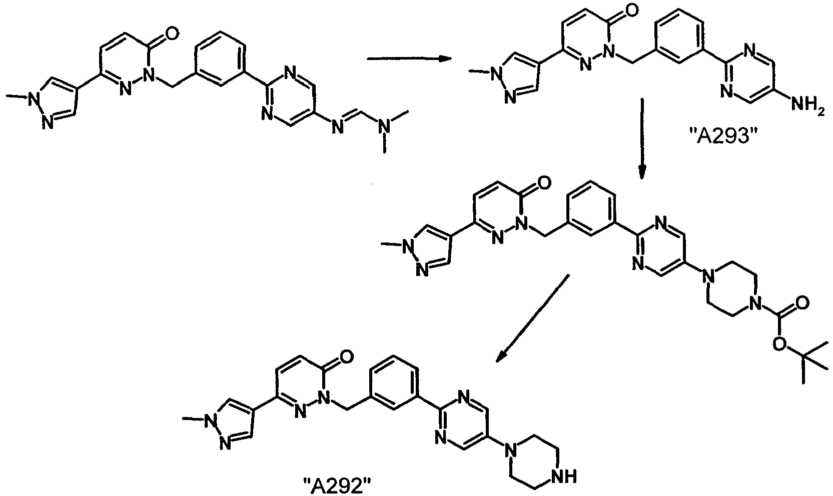

Zu einer Lösung von 5.5 g (40 mmol) Kaliumcarbonat in 130 ml Wasser werden 130 ml Dioxan und 5 g (11.4 mmol) *N*,*N*-Dimethyl-*N'*-(2-{3-[3-(1-methyl-1H-pyrazol-4-yl)-6-oxo-6H-pyridazin-1-ylmethyl]-phenyl}-pyrimidin-5-yl)-formamidin gegeben. Das Reaktionsgemisch wird 15 h zum Sieden erhitzt und anschließend auf Raumtemperatur abgekühlt. Das Dioxan wird abdestilliert, der entstandene Niederschlag abgesaugt, mit Wasser gewaschen und im Vakuum getrocknet; Ausbeute: 3.6 g "A293"; HPLC: 2.11 min (Methode C); LC-MS: 360 (M+H).

Eine unter Stickstoff gehaltene Lösung von 1 g (2.78 mmol) 2-[3-(5-Aminopyrimidin-2-yl)-benzyl]-6-(1-methyl-1 H-pyrazol-4-yl)-2H-pyridazin-3-on ("A293") in 25 ml 1-Methylpyrrolidon wird mit 1.4 g (7.5 mmol) Bis-(2-chlorethyl)-methyl-ammoniumchlorid versetzt und das Reaktionsgemisch 5 Tage auf 130° C erhitzt. Das Reaktionsgemisch wird abgekühlt, filtriert und das Filtrat in 200 ml Diethylether gegeben. Es fällt ein öliger Rückstand aus, der mit 100 ml gesättigter Natriumhydrogencarbonatlösung versetzt wird und mit 3 x 150 ml Dichlormethan extrahiert wird. Die organischen Phasen werden über Natriumsulfat getrocknet und eingedampft. 300 mg dieses Rückstands werden in 5 ml DMF gelöst und mit 387 mg Kaluimcarbonat und 195 µl (0.91 mmol) Di-*tert.*-butyldicarbonat versetzt und 1 h bei Raumtemperatur gerührt. Das Reaktionsgemisch wird filtriert und das Filtrat wird eingeengt. Der Rückstand wird in Dichlormethan suspendiert und mit gesättigter Natriumhydrogencarbonat gewaschen. Die organische Phase wird über Natriumsulfat getrocknet und anschließend eingeengt. Der Rückstand wird mittels Säulenchromatographie an Kieselgel aufgereinigt; Ausbeute: 36 mg; HPLC: 2.89 min (Methode C); LC-MS: 529 (M+H).

90 mg (0.17 mmol) 4-(2-{3-[3-(1-Methyl-1H-pyrazol-4-yl)-6-oxo-6H-pyridazin-1-ylmethyl]-phenyl}-pyrimidin-5-yl)-piperazine-1-carbonsäure-*tert.*-butylester werden in 10 ml Dioxan gelöst und mit 1 ml 4 N HCl in Dioxan versetzt. Das Reaktionsgemisch wird 15 h bei Raumtemperatur gerührt und anschließend zum Rückstand eingeengt; Ausbeute: 80 mg "A292" Hydrochlorid; HPLC: 2.05 min (Methode C); LC-MS: 429 (M+H).

### Beispiel 57

Herstellung von 2-{3-[5-(2-Hydroxy-ethoxy)-pyrimidin-2-yl]-benzyl}-6-(1-methyl-1 H-pyrazol-4-yl)-2H-pyridazin-3-on ("A294")

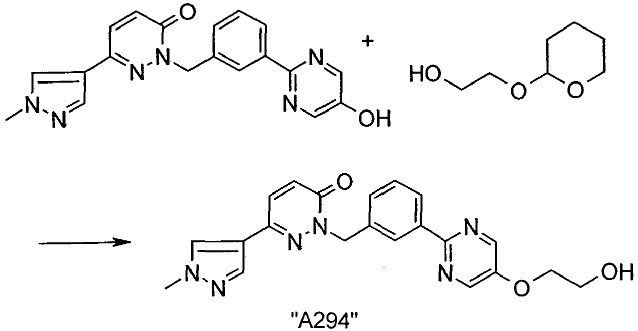

In einem 25 ml Dreihalskolben werden unter Schutzgasatmosphäre 252 mg 2-[3-(5-Hydroxy-pyrimidin-2-yl)-benzyl]-6-(1-methyl-1H-pyrazol-4-yl)-2H-pyridazin-3-on (0,7 mmol) in abs. THF suspendiert, 0,19 ml 2-(Tetrahydro-pyran-2-yloxy)-ethanol (1,4 mmol) und 367 mg Triphenylphosphin (1,4 mmol) zugegeben und 30 min bei RT gerührt. Anschließend werden 275 µl Diisopropylazodicarboxylat (1,4 mmol) zugetropft und das Reaktionsgemisch wird 2 h bei RT nachgerührt. Zur Aufarbeitung wird das Reaktionsgemisch mit 20 ml Dichlormethan verdünnt, mit 10 ml Wasser gewaschen, über Natriumsulfat getrocknet, zum Rückstand abgezogen und chromatographisch aufgereinigt. (Kieselgel: MtB-Ether -> DCM -> DCM : 30 % MeOH). Das THP-geschützte Produkt wird in 5 ml 4 N HCl in Dioxan 20 h bei RT gerührt. Die Reaktionslösung wird zum Rückstand evapuriert und aus Methanol/Diethylether kristallisiert. Man erhält "A294"; ESI 405; F. 182-3 °C.

### Analog werden die nachstehenden Verbindungen erhalten

| Verbindung Nr. | Name und/oder Struktur | ESI [M+H]⁺ |
|---|---|---|
| "A295" | 3-(1-{3-[5-(3-Hydroxy-propoxy)-pyrimidin-2-yl]-benzyl}-6-oxo-1,6-dihydro-pyridazin-3-yl)-benzonitril | 440 |
| "A296" | 3-(1-{3-[5-(2-Hydroxy-ethoxy)-pyrimidin-2-yl]-benzyl}-6-oxo-1,6-dihydro-pyridazin-3-yl)-benzonitril | 426 |
| "A297" | 2-{3-[5-(3-Hydroxy-propoxy)-pyrimidin-2-yl]-benzyl}-6-(1-methyl-1H-pyrazol-4-yl)-2H-pyridazin-3-on | 419 |

### Beispiel 58

Herstellung von 1-{3-[5-(3-Dimethylamino-propoxy)-pyrimidin-2-yl]-phenyl}-ethanol (Vorstufe zu "A298")

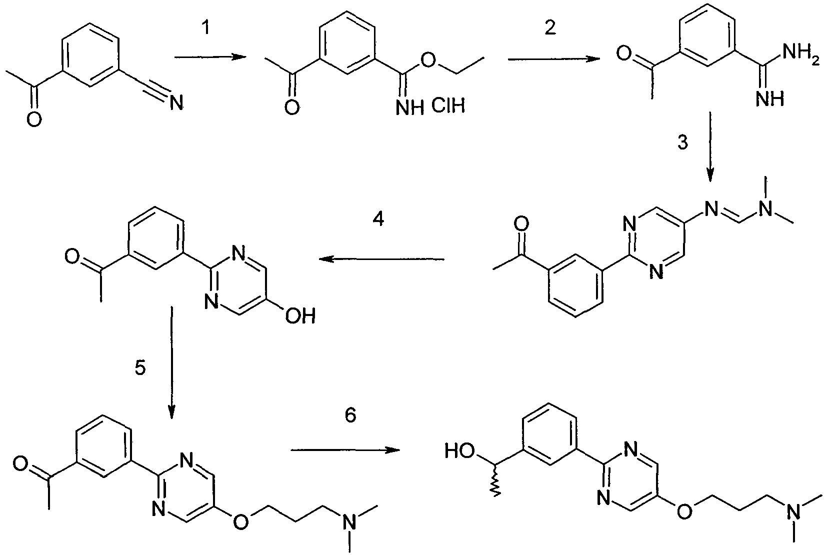

### 1. Herstellung von 3-Acetyl-benzimidinsäureethylester

In einem 500 ml Einhalskolben werden 30 g 3-Cyanacetophenon (207 mmol) in 170 ml einer 10 % igen Lösung von HCl in Diethylether suspendiert, auf 0 °C abgekühlt und 18,68 ml abs. Ethanol zugegeben. Das Reaktionsgemisch rührt 14 Tage bei RT. Zur Aufarbeitung wird das Reaktionsgemisch mit 500 ml Diethylether verdünnt, der Niederschlag abgesaugt, mit reichlich Diethylether nachgewaschen und der Rückstand im Vakuumtrockenschrank bei 50 °C getrocknet; F. 122-4 °C.

### 2. Herstellung von 3-Acetylbenzamidin

In einem 1000 ml Einhalskolben werden 17,453 g 3-Acetyl-benzimidinsäureethylester in 190 ml abs. Ethanol suspendiert und anschließend 190 ml einer 10 % igen Lösung von Ammoniak in Ethanol zugegeben und der Reaktionsansatz 3 h refluxiert. Der Reaktionsansatz wird zur Trockne einrotiert und roh in die nächste Stufe eingesetzt; LC-MS: 0,886 min / M+H⁺: 163,2 g/mol.

### 3. Herstellung von N'-[2-(3-Acetyl-phenyl)-pyrimidin-5-yl]-N,N-dimethyl-formamidin

In einem N₂-gespülten 1000 ml Dreihalskolben werden unter CaCl₂-Schutz 16,18 g 3-Acetylbenzamidin (Gehalt 77 %) (76,62 mmol) und 37,41 g ({2-Dimethylamino-1-[dimethylimmoniomethyl]-vinylamino}methylene)-dimethyl-ammonium-dihexafluorophosphat (Aminoreducton Precursor) (76,62 mmol) in 200 ml abs. Methanol suspendiert und unter Rühren eine frisch hergestellte 1,5 M Natriummethanolat-Lösung in Methanol zugetropft. Das Reaktionsgemisch rührt 30 min bei 60 °C, wobei eine klare Lösung entsteht. Zur Aufarbeitung werden ca. 90 % des Methanols am Roationsverdampfer entfernt, der verbleibende Rückstand mit 300 ml Dichlormethan verdünnt, 2x mit 100 ml Wasser gewaschen, über Natriumsulfat getrocknet und zum Rückstand abgezogen. Die Aufreinigung erfolgt chromatographisch (Kieselgel DCM + 1-5 % MeOH). Die Produktfraktionen weurden vereinigt, zum Rückstand abgezogen und mit i-PrOH verrührt; F. 146-8 °C.

### 4. Herstellung von 1-[3-(5-Hydroxy-pyrimidin-2-yl)-phenyl]-ethanon

In einem 250 ml Einhalskolben, versehen mit Magnetrührer und Kühler, werden 5,10 g N'-[2-(3-Acetyl-phenyl)-pyrimidin-5-yl]-N,N-dimethyl-formamidin (19 mmol) in 65 ml Wasser suspendiert, 8,44 ml Schwefelsäure 95-97% (152 mmol) zugegeben und 2 h bei 130°C Badtemperatur gerührt. Es wird mit Eiswasser verdünnt, wobei sich ein dunkelbraunes Harz abscheidet. Die wässrige Lösung wird abdekantiert und mit Dichlormethan extrahiert. Die vereinigten Dichlormethanphasen werden getrocknet, filtriert, zum Rückstand abgezogen, der Rückstand mit Ether verrieben, abgesaugt und getrocknet (=K1). Das abgeschiedene dunkelbraune Harz wird mit Tetrahydrofuran ausgerührt, abgesaugt, das Kristallisat verworfen und die Mutterlauge zum Rückstand abgezogen (=R1). Die wässrige Phase aus der Dichlormethanextraktin wird zum Rückstand abgezogen, der Rückstand 2 x mit Tetrahydrofuran ausgerührt, die vereinigten abdekantierten Lösungen mit Dichlormethan verdünnt, getrocknet, filtriert und zum Rückstand abgezogen (=R2). R1 und R2 werden vereinigt, auf Kieselgel aufgezogen und chromatograhpisch gereinigt (Kieselgel / Dichlormethan + 0-5% Methanol). Der Chromatographierückstand wird mit Ether verrieben, abgesaugt, mit Ether gewaschen und getrocknet (=K2). K1 und K2 werden vereinigt; F.199-200 °C.

### 5. Herstellung von 1-{3-[5-(3-Dimethylamino-propoxy)-pyrimidin-2-yl]-phenyl}-ethanon

In einer N₂-gespülten Apparatur werden unter CaCl₂-Schutz 2,4 g 1-[3-(5-Hydroxy-pyrimidin-2-yl)-phenyl]-ethanon (11,2 mmol) in 40 ml abs. THF suspendiert, 1,576 ml 3-(Dimethylamino)-1-propanol (13,44 mmol) und 5,602 g polymergebundenes Triphenylphosphin (16,81 mmol) zugegeben und 30 min bei RT gerührt. Unter Eis- / H₂O-Kühlung und Rühren werden 3,87 g Di-tert.-butylazodicarboxylat (16,81 mmol) zugegeben und 2 h bei RT nachgerührt. Zur Aufarbeitung wird das Polymer durch Filtration entfernt, mit reichlich Dichlormethan nachgewaschen und das Filtrat 1 x mit Wasser und 2 x mit wässr. 1 N HCl extrahiert. Die vereinigten HCl Extrakte werden mit NaOH alkalisch gestellt und 3 x mit 50 ml Dichlormethan extrahiert. Die Dichlormethanextrakte werden vereinigt, über Natriumsulfat getrocknet, zum Rückstand abgezogen und aus Petrolether 40-60 kristallisiert; F. 61-2 °C.

### 6. Herstellung von 1-{3-[5-(3-Dimethylamino-propoxy)-pyrimidin-2-yl]-phenyl}-ethanol

In einem 100 ml Einhalskolben werden 3,114 g 1-{3-[5-(3-Dimethylaminopropoxy)-pyrimidin-2-yl]-phenyl}-ethanon (10,4 mmol) in 30 ml abs. Ethanol gelöst und anschließend unter Eis-/Wasser-Kühlung und Rühren portionsweise 0,394 g Natriumborhydrid (10,4 mmol) zugegeben und der Reaktionsansatz 20 h bei RT nachgerührt. Zur Aufarbeitung wird das Reaktionsgemisch mit 50 ml Dichlormethan verdünnt, 2x gegen Wasser geschüttelt, die Dichlormethanphase zum Rückstand abgezogen und chromatographisch aufgereinigt (Kieselgel / DCM/MeOH 9:1);
HPLC : RT: 2,40 Min;
LC-MS: 1,330 min / M+H⁺: 302,2 g/mol.

### Beispiel 59

Herstellung von 3-[1-(1-{3-[5-(3-Dimethylamino-propoxy)-pyrimidin-2-yl]-phenyl}-ethyl)-6-oxo-1,6-dihydro-pyridazin-3-yl]-benzonitril ("A298")

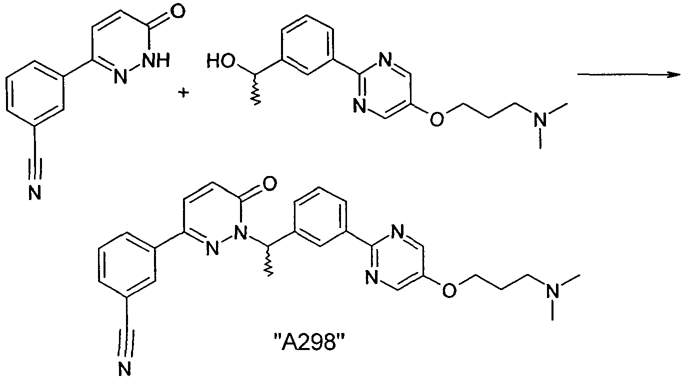

In einer N₂-gespülten Apparatur wird unter CaCl₂-Schutz 197 mg 3-Cyan-phenylpyridazinon (1,00 mmol) in einer Mischung aus 5 ml abs. THF und 1ml abs. DMF suspendiert, 301 mg 1-{3-[5-(3-Dimethylamino-propoxy)-pyrimidin-2-yl]-phenyl}-ethanol (1,00 mmol) und 500 mg polymergebundenes Triphenylphosphin (1,5 mmol) zugegeben, 30 min bei RT gerührt und anschließend unter Eis- / H₂O-Kühlung und Rühren 345 mg Di-tert.-butylazodicarboxylat (1,5 mmol) zugegeben und 2 h bei RT nachgerührt. Zur Aufarbeitung wird das Reaktionsgemisch mit 10 ml Methanol verdünnt und das Polymer durch Filtration entfernt. Der Rückstand wirde mit Dichlormethan gewaschen und das vereinigte Filtrat am Rotationsverdampfer zum Rückstand abgezogen und chromatographisch gereinigt (Kieselgel: DCM + 0-30 % MeOHF.
Man erhält "A298", F. 105-7 °C.

### Analog werden die nachstehenden Verbindungen erhalten

| Verbindung Nr. | Name und/oder Struktur | ESI [M+H]⁺ |
|---|---|---|
| "A299" | 6-(3,5-Difluor-phenyl)-2-(1-{3-[5-(3-dimethylamino-propoxy)-pyrimidin-2-yl]-phenyl}-ethyl)-2H-pyridazin-3-on | 492 |
| "A300" | 6-(3,5-Difluor-phenyl)-2-((R)-1-{3-[5-(3-dimethylamino-propoxy)-pyrimidin-2-yl]-phenyl}-ethyl)-2H-pyridazin-3-on, Hydrochlorid | 492 |
| "A301" | 6-(3,5-Difluor-phenyl)-2-((S)-1-{3-[5-(3-dimethylamino-propoxy)-pyrimidin-2-yl]-phenyl}-ethyl)-2H-pyridazin-3-on, Hydrochlorid | 492 |

### Beispiel 60

Herstellung von 3-(1-{3-[5-(1-Methyl-1-oxy-piperidin-4-ylmethoxy)-pyrimidin-2-yl]-benzyl}-6-oxo-1,6-dihydro-pyridazin-3-yl)-benzonitril ("A302")

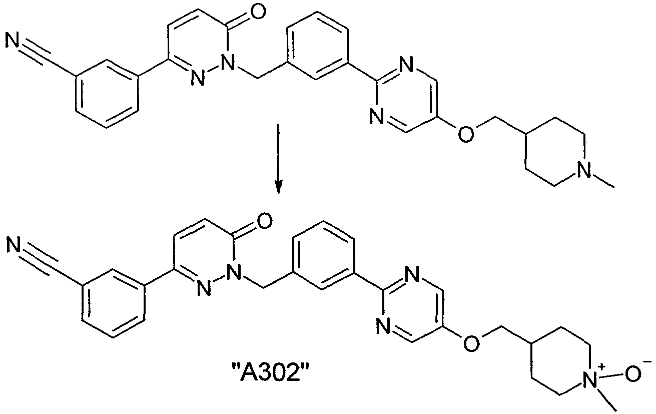

In einem Reaktionsgläschen, versehen mit einem Magnetrührer, werden 100 mg 3-(1-{3-[5-(1-Methyl-piperidin-4-ylmethoxy)-pyrimidin-2-yl]-benzyl}-6-oxo-1,6-dihydro-pyridazin-3-yl)-benzonitril (0,203 mmol) in 5 ml Wasser und 5 ml Acetonitril suspendiert, 100 µl Perhydrol (0,979 mmol) zugegeben und 24 h bei RT gerührt. Dann wird in Wasser gegossen, mit Dichlormethan extrahiert, die vereinigten Dichlormethanphasen getrocknet, filtriert und zum Rückstand abgezogen. Der Rückstand wird auf Kieselgel aufgezogen und chromatographiert (Dichlormethan + 0-50% Methanol). Der Chromatographierückstand wird gefriergetrocknet; ESI 509; F. 85 °C (Zersetzung).

### Beispiel 61

Herstellung von 3-(1-{3-[5-(1-Formyl-piperidin-4-ylmethoxy)-pyrimidin-2-yl]-benzyl}-6-oxo-1,6-dihydro-pyridazin-3-yl)-benzonitril, ESI 409:

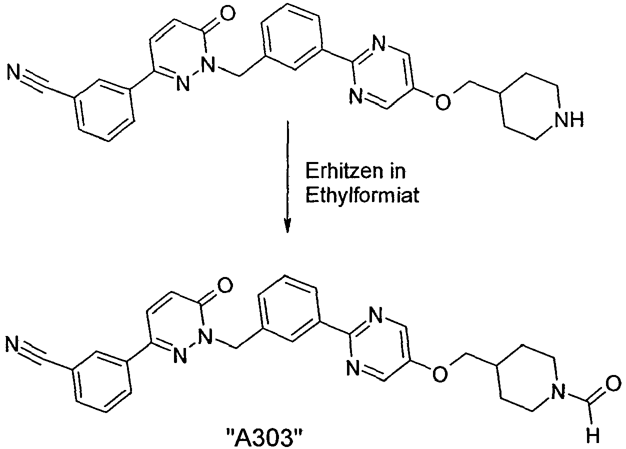

### Beispiel 62

Durch Umsetzung von 3-(6-Oxo-1-{3-[5-(piperidin-4-ylmethoxy)-pyrimidin-2-yl]-benzyl}-1,6-dihydro-pyridazin-3-yl)-benzonitril mit Dimethylaminoethylchlorid-Hydrochlorid und Caesiumcarbonat in DMF und nachfolgender chromatographischer Trennung erhält man 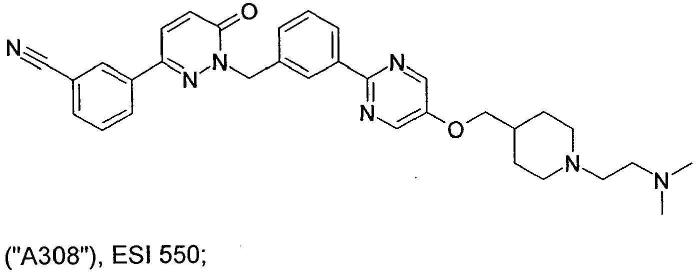 und 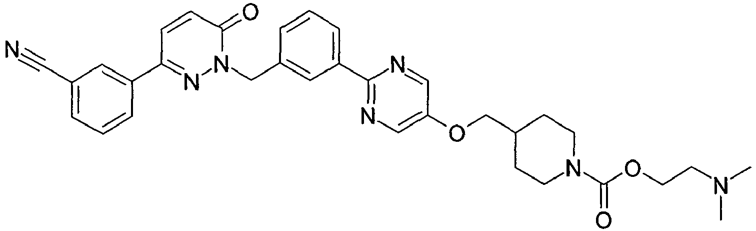 Trifluoracetat ("A 309"), ESI 594.

### Beispiel 63

Durch Umsetzung von 3-(6-Oxo-1-{3-[5-(piperidin-4-ylmethoxy)-pyrimidin-2-yl]-benzyl}-1,6-dihydro-pyridazin-3-yl)-benzonitril mit beta-Bromethylmethylether und Caesiumcarbonat in DMF erhält man die Verbindung 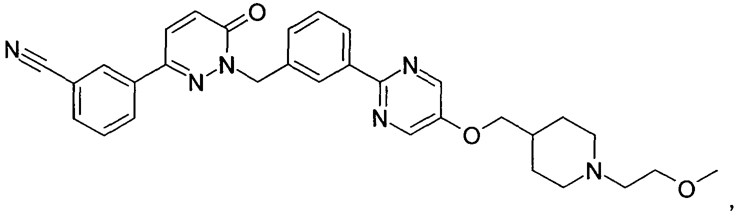 Trifluoracetat ("A311"), ESI 537.

### Beispiel 64

Durch Umsetzung von 3-(6-Oxo-1-{3-[5-(piperidin-4-ylmethoxy)-pyrimidin-2-yl]-benzyl}-1,6-dihydro-pyridazin-3-yl)-benzonitril mit Bromethan und Caesiumcarbonat in DMF erhält man die Verbindung 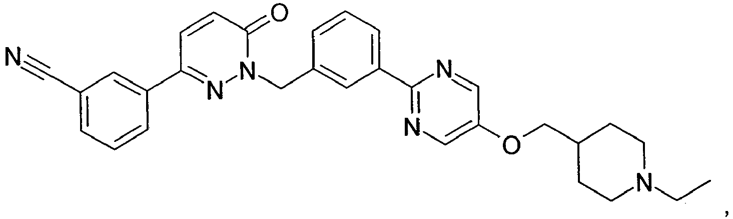 Trifluoracetat ("A313"), ESI 507.

### Pharmakologische Daten

**Tabelle 2 Met-Kinase-Inhibierung einiger repräsentativer Verbindungen der Formel I**

| Verbindung Nr. | IC₅₀ (Zell Assay) | IC₅₀ (Enzym Assay) |
|---|---|---|
| "A13" | B | |
| "A14" | A | |
| "A15" | A | |
| "A16" | A | |
| "A17" | A | |
| "A18" | A | |
| "A19" | A | |
| "A20" | A | |
| "A22" | A | |
| "A23" | A | |
| "A26" | A | |
| "A35" | A | |
| "A57" | A | |
| "A63" | A | |
| "A64" | A | |
| "A66" | A | |
| "A102" | A | |
| "A168" | A | |
| "A169" | A | |
| "A189" | A | |
| "A209" | A | |
| "A226" | A | |
| "A229" | A | |
| "A237" | A | |
| "A257" | A | |
| "A287" | A | |
| "A288" | A | |

| | | |
|---|---|---|
| IC₅₀: 1 nM - 1 µM = A 1 µM - 10 µM = B > 10 µM = C | | |

Die in Tabelle 2 aufgeführten Verbindungen sind die besonders bevorzugten erfindungsgemäßen Verbindungen.

Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Arzneimittel, enthaltend mindestens eine Verbindung der Formel I 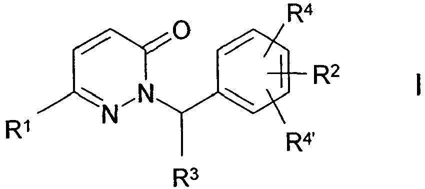 worin
R¹ Ar oder Het,
R² einen ungesättigten, gesättigten oder aromatischen 6-gliedrigen Heterocyclus mit 1 bis 4 N-, O- und/oder S-Atomen, der ein-, zwei- oder dreifach durch N=CR³N(R³)₂, SR³, NO₂, CN, COOR³, CON(R³)₂, NR³COA, NR³SO₂A, SO₂N(R³)₂, S(O)ₘA, [C(R³)₂]ₙN(R³)₂, [C(R³)₂]ₙHet, O[C(R³)₂]ₚOR³, O[C(R³)₂]ₙN(R³)₂, O[C(R³)₂]ₙC=C[C(R³)₂]ₙN(R³)₂, O[C(R³)₂]ₙN⁺O⁻(R³)₂, O[C(R³)₂]ₙHet, S[C(R3)₂]ₙN(R³)₂, S[C(R³)₂]ₙHet, NR³[C(R³)₂]ₙN(R³)₂, NR³[C(R³)₂]ₙHet, NHCON(R³)₂, NHCONH[C(R³)₂]ₙN(R³)₂, NHCONH[C(R³)₂]ₙHet, [C(R³)₂]ₙNHCO[C(R³)₂]ₙN(R³)₂, [C(R³)₂]ₙNHCO[C(R³)2]ₙHet, CON(R³)₂, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, COA, CH=CH-COOR³ und/oder CH=CH-N(R³)₂ substituiert ist,
R³ H oder A,
R⁴, R^{4'} jeweils unabhängig voneinander H, Hal, A, OR³, CN, COOR³, CON(R³)₂, NR³COA, NR³SO₂A, SO₂N(R³)₂ oder S(O)ₘA,
Ar unsubstituiertes oder ein-, zwei- oder dreifach durch Hal, A, [C(R³)₂]ₙOR³, [C(R³)₂]ₙN(R³)₂, SR³, NO₂, CN, COOR³, CON(R³)₂, NR³COA, NR³SO₂A, SO₂N(R³)₂, S(O)ₘA, CO-Het, Het, O[C(R³)₂]ₙN(R³)₂, O[C(R³)₂]ₙHet, NHCOOA, NHCON(R³)₂, NHCOO[C(R³)₂]ₙN(R³)₂, NHCOO[C(R³)₂]ₙHet, NHCONH[C(R³)₂]ₙN(R³)₂, NHCONH[C(R³)₂]ₙHet, OCONH[C(R³)₂]ₙN(R³)₂, OCONH[C(R³)₂]ₙHet, CONR³[C(R³)₂]ₙN(R³)₂, CONR³[C(R³)₂]ₙHet und/oder COA substituiertes Phenyl, Naphthyl oder Biphenyl,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei-, drei-, vier- oder fünffach durch Hal, A, [C(R³)₂]ₙOR³, [C(R³)₂]ₙN(R³)₂, SR³, NO₂, CN, COOR³, CON(R³)₂, NR³COA, NR³SO₂A, SO₂N(R³)₂, S(O)ₘA, CO-Het¹, [C(R³)₂]ₙHet¹, O[C(R³)₂]ₙN(R³)₂, O[C(R³)₂]ₙHet¹, NHCOOA, NHCON(R³)₂, NHCOO[C(R³)₂]ₙN(R³)₂, NHCOO[C(R³)₂]ₙHet¹, NHCONH[C(R³)₂]ₙN(R³)₂, NHCONH[C(R³)₂]ₙHet¹, OCONH[C(R³)₂]ₙN(R³)₂, OCONH[C(R³)₂]ₙHet¹, CO-Het¹, CHO, COA, =S, =NH, =NA und/oder =O (Carbonylsauerstoff) substituiert sein kann, und wobei ein Ringstickstoff oxidiert sein kann,
Het¹ einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
A unverzweigtes oder verzweigtes Alkyl mit 1-10 C-Atomen, worin 1-7 H-Atome durch F ersetzt sein können und/oder worin eine oder zwei nicht-benachbarte CH₂-Gruppen durch O, NH, S, SO, SO₂ und/oder durch CH=CH-Gruppen ersetzt sein können, oder cyclisches Alkyl mit 3-7 C-Atomen,
Hal F, Cl, Br oder I,
m 0, 1 oder 2,
n 0, 1, 2, 3 oder 4,
p 1, 2, 3 oder 4
bedeuten,
und/oder ihre pharmazeutisch verwendbaren Solvate, Salze,
Tautomere und Stereoisomere, einschließlich deren Mischungen in allen Verhältnissen, sowie gegebenenfalls Träger- und/oder Hilfsstoffe, wobei 0,5 mg bis 1 g einer Verbindung der Formel I enthalten ist.

2. Arzneimittel nach Anspruch 1, worin
R² einen ungesättigten, gesättigten oder aromatischen 6-gliedrigen Heterocyclus mit 1 bis 4 N- und/oder O-Atomen, der ein-, zwei- oder dreifach durch N=CR³N(R³)₂, CN, COOR³, [C(R³)₂]ₙN(R³)₂, [C(R³)₂]ₙHet, O[C(R³)₂]ₚOR³, O[C(R³)₂]ₙN(R³)₂, O[C(R³)₂]ₙC≡C[C(R³)₂]ₙN(R³)₂, O[C(R³)₂]ₙN⁺O⁻(R³)₂, O[C(R³)₂]ₙHet, NR³[C(R³)₂]ₙN(R³)₂, NR³[C(R³)₂]ₙHet, [C(R³)₂]ₙNHCO[C(R³)₂]ₙN(R³)₂, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ und/oder CH=CH-N(R³)₂ substituiert ist,
bedeutet.

3. Arzneimittel nach Anspruch 1 oder 2, worin
Ar unsubstituiertes oder ein-, zwei- oder dreifach durch A, Hal, CN, S(O)ₘA, NR³COA, CON(R³)₂, O(C(R³)₂]ₙ-N(R³)₂, [C(R³)₂]ₙOR³, CONR³[C(R³)₂]ₙN(R³)₂ und/oder CONR³[C(R³)₂]ₙHet substituiertes Phenyl, Naphthyl oder Biphenyl,
bedeutet,
sowie ihre pharmazeutisch verwendbaren Derivate, Solvate, Salze,
Tautomere und Stereoisomere, einschließlich deren Mischungen in allen Verhältnissen.

4. Arzneimittel nach einem oder mehreren der Ansprüche 1-3, worin
R⁴, R^{4'} H bedeuten.

5. Arzneimittel nach einem oder mehreren der Ansprüche 1-4, worin
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei-, drei-, vier- oder fünffach durch A, CHO, COOR³, CON(R³)₂, [C(R³)₂]ₙHet¹, [C(R³)₂]ₙOR³, [C(R³)₂]ₙN(R³)₂, O[C(R³)₂]ₙHet¹ und/oder =O (Carbonylsauerstoff) substituiert sein kann, und wobei ein Ringstickstoff oxidiert sein kann,
bedeutet.

6. Arzneimittel nach einem oder mehreren der Ansprüche 1-5, worin
Het¹ einen einkernigen gesättigten Heterocyclus mit 1 bis 2 N und/oder O-Atomen, der ein- oder zweifach durch A und/oder =O (Carbonylsauerstoff) substituiert sein kann
bedeutet.

7. Arzneimittel nach einem oder mehreren der Ansprüche 1-6, worin
A unverzweigtes oder verzweigtes Alkyl mit 1-8 C-Atomen, worin 1-7 H-Atome durch F ersetzt sein können,
bedeutet.

8. Arzneimittel nach einem oder mehreren der Ansprüche 1-7, worin
R¹ Ar oder Benzo[2,1,3]thiadiazolyl bedeutet.

9. Arzneimittel nach einem oder mehreren der Ansprüche 1-8, worin
R³ H, Methyl, Ethyl oder Propyl
bedeutet.

10. Arzneimittel nach einem oder mehreren der Ansprüche 1-9, worin
R² ein-, zwei- oder dreifach durch N=CR³N(R³)₂, CN, COOR³, [C(R³)₂]ₙN(R³)₂, [C(R³)₂]ₙHet, O[C(R³)₂]ₚOR³, O[C(R³)₂]ₙN(R³)₂, O[C(R³)₂]ₙC≡C[C(R³)₂]ₙN(R³)₂, O[C(R³)₂]ₙN⁺O⁻(R³)₂, O[C(R³)₂]ₙHet, NR³[C(R³)₂]ₙN(R³)₂, NR³[C(R³)₂]ₙHet, [C(R³)₂]ₙNHCO[C(R³)₂]ₙN(R³)₂, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ und/oder CH=CH-N(R³)₂ substituiertes Pyrimidinyl, Pyridazinyl, Pyridinyl, [1,3]Oxazinanyl, Morpholinyl, Piperidinyl oder Piperazinyl
bedeutet.

11. Arzneimittel nach einem oder mehreren der Ansprüche 1-10, worin
Het unsubstituiertes oder ein-, zwei-, drei-, vier- oder fünffach durch A, CHO, COOR³, CON(R³)₂, [C(R³)₂]ₙHet¹, [C(R³)₂]ₙOR³, [C(R³)₂]ₙN(R³)₂, O[C(R³)₂]ₙHet¹ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl, Aza-bicyclo[3.2.1]octyl, Aza-bicyclo[2.2.2]octyl, Imidazolidinyl, Azepanyl oder Benzo[2,1,3]thiadiazolyl, und wobei ein Ringstickstoff oxidiert sein kann,
bedeutet.

12. Arzneimittel nach einem oder mehreren der Ansprüche 1-10, worin
Het¹ unsubstituiertes oder ein- oder zweifach durch A und/oder =O (Carbonylsauerstoff) substituiertes Pyrrolidinyl, Piperidinyl, Piperazinyl oder Morpholinyl
bedeutet.

13. Arzneimittel nach einem oder mehreren der Ansprüche 1-12, worin
R¹ Ar oder Het,
R² ein-, zwei- oder dreifach durch N=CR³N(R³)₂, CN, COOR³, [C(R³)₂]ₙN(R³)₂, [C(R³)_{2]n}Het, O[C(R³)₂]ₚOR³, O[C(R³)₂]ₙN(R³)₂, O[C(R³)₂]ₙC≡C[C(R³)₂]ₙN(R³)₂, O[C(R³)₂]ₙN⁺O⁻(R³)₂, O[C(R³)₂]ₙHet, NR³[C(R³)₂]ₙN(R³)₂, NR³[C(R³)₂]ₙHet, [C(R³)₂]ₙNHCO[C(R³)₂]ₙN(R³)₂, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ und/oder CH=CH-N(R³)₂ substituiertes Pyrimidinyl, Pyridazinyl, Pyridinyl, [1,3]Oxazinanyl, Morpholinyl, Piperidinyl oder Piperazinyl,
R³ H, Methyl, Ethyl oder Propyl,
R⁴, R^{4'} H,
Ar unsubstituiertes oder ein-, zwei- oder dreifach durch A, Hal, CN, S(O)ₘA, NR³COA, CON(R³)₂, O[C(R³)₂]ₙ-N(R³)₂, [C(R³)₂]ₙOR³, CONR³[C(R³)₂]ₙN(R³)₂ und/oder CONR³[C(R³)₂]ₙHet substituiertes Phenyl, Naphthyl oder Biphenyl,
Het unsubstituiertes oder ein-, zwei-, drei-, vier- oder fünffach durch A, CHO, COOR³, CON(R³)₂, [C(R³)₂]ₙHet¹, [C(R³)₂]ₙOR³, [C(R³)₂]ₙN(R³)₂, O[C(R³)₂]ₙHet¹ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl, Aza-bicyclo[3.2.1]octyl, Aza-bicyclo[2.2.2]octyl, Imidazolidinyl, Azepanyl oder Benzo[2,1,3]thiadiazolyl, und wobei ein Ringstickstoff oxidiert sein kann,
Het¹ unsubstituiertes oder ein- oder zweifach durch A und/oder =O (Carbonylsauerstoff) substituiertes Pyrrolidin, Piperidin, Piperazin oder Morpholin,
A unverzweigtes oder verzweigtes Alkyl mit 1-8 C-Atomen, worin 1-7 H-Atome durch F ersetzt sein können,
Hal F, Cl, Br oder I,
m 0, 1 oder 2,
n 0, 1, 2, 3 oder 4,
p 1, 2, 3 oder 4
bedeuten.

14. Arzneimittel nach Anspruch 1, enthaltend mindestens eine Verbindung der Formel I ausgewählt aus der Gruppe
| Nr. | Name und/oder Struktur |
|---|---|
| "A11" | 2-[3-(5-Aminopyridin-2-yl)-benzyl]-6-(3,5-difluorphenyl)-2H-pyridazin-3-on |
| "A12" | 6-(3,5-Difluor-phenyl)-2-{3-[5-(4-methyl-piperazin-1-yl)-pyridin-2-yl]-benzyl}-2H-pyridazin-3-on |
| "A13" | 6-(3,5-Difluorphenyl)-2-[3-(4-piperazin-1-yl-pyrimidin-2-yl)-benzyl]-2H-pyridazin-3-on |
| "A14" | 6-(3,5-Difluorphenyl)-2-{3-[5-(4-methylpiperazin-1-ylmethyl)-pyrimidin-2-yl]-benzyl}-2H-pyridazin-3-on |
| "A16" | N'-(2-{3-[3-(3,5-Difluorphenyl)-6-oxo-6H-pyridazin-1-ylmethyl]-phenyl}-pyrimidin-5-yl)-N,N-dimethyl-formamidin |
| "A17" | 2-[3-(5-Aminopyrimidin-2-yl)-benzyl]-6-(3,5-difluorphenyl)-2H-pyridazin-3-on |
| "A18" | 6-(3,5-Difluorphenyl)-2-{3-[5-(4-methylpiperazin-1-yl)-pyrimidin-2-yl]-benzyl}-2H-pyridazin-3-on |
| "A19" | 6-(3,5-Difluor-phenyl)-2-[3-(5-piperazin-1-yl-pyrimidin-2-yl)-benzyl]-2H-pyridazin-3-on |
| "A20" | 2-{3-[5-(4-Methyl-piperazin-1-yl)-pyrimidin-2-yl]-benzyl}-6-(3,4,5-trifluor-phenyl)-2H-pyridazin-3-on |
| "A22" | 6-(3,5-Difluorphenyl)-2-{3-[5-(3-dimethylamino-propoxy)-pyrimidin-2-yl]-benzyl}-2H-pyridazin-3-on |
| "A23" | 6-(3,5-Difluor-phenyl)-2-{3-[5-(1-methyl-piperidin-4-yloxy)-pyrimidin-2-yl]-benzyl}-2H-pyridazin-3-on |
| | 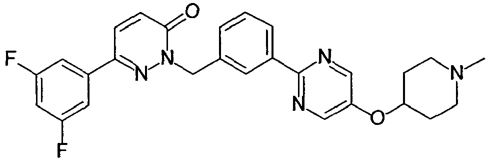 |
| "A24" | 6-(3,5-Difluor-phenyl)-2-{3-[5-(3-dimethylamino-propoxy)-pyridin-2-yl]-benzyl}-2H-pyridazin-3-on |
| "A26" | 6-(3,5-Difluorphenyl)-2-{3-[5-(1-methyl-1H-pyrazol-4-yl)-pyrimidin-2-yl]-benzyl}-2H-pyridazin-3-on |
| "A27" | 6-(3,5-Difluor-phenyl)-2-{3-[6-(4-methyl-piperazin-1-yl)-pyridazin-3-yl]-benzyl}-2H-pyridazin-3-on |
| "A28" | 6-(3,5-Diftuor-phenyl)-2-{3-[6-(3-dimethylamino-propoxy)-pyridazin-3-yl]-benzyl}-2H-pyridazin-3-on |
| "A29" | 2-{3-[6-Oxo-3-(3,4,5-trifluor-phenyl)-6H-pyridazin-1-ylmethyl]-phenyl}-pyrimidin-5-carbonsäureethylester |
| "A30" | 2-{3-[3-(3,5-Difluor-phenyl)-6-oxo-6H-pyridazin-1-ylmethyl]-phenyl}-pyrimidin-5-carbonsäure-ethylester |
| | 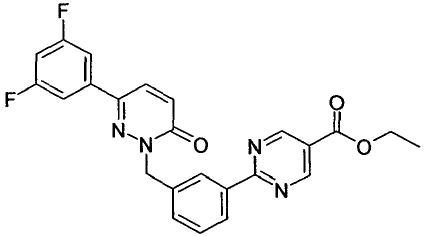 |
| "A31" | 2-{3-[6-Oxo-3-(3,4,5-trifluor-phenyl)-6H-pyridazin-1-ylmethyl]-phenyl}-pyrimidin-5-carbonsäure |
| "A32" | 2-{3-[3-(3,5-Difluor-phenyl)-6-oxo-6H-pyridazin-1-ylmethyl]-phenyl}-pyrimidin-5-carbonsäure |
| "A33" | 2-{3-[6-Oxo-3-(3,4,5-trifluor-phenyl)-6H-pyridazin-1-ylmethyl]-phenyl}-pyrimidin-5-carbonsäure(2-dimethylamino-ethyl)-amid |
| "A34" | 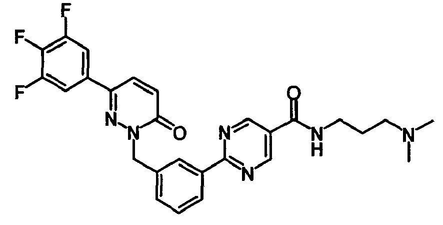 |
| "A35" | 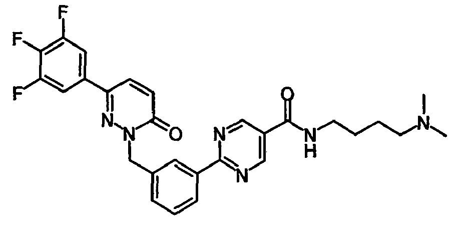 |
| "A36" | 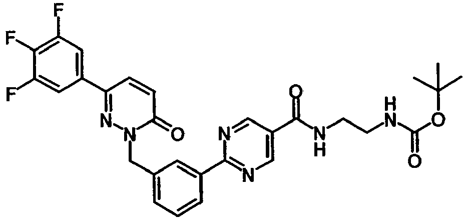 |
| "A37" | 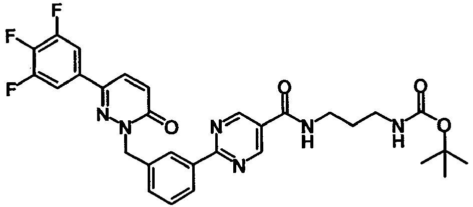 |
| "A38" | 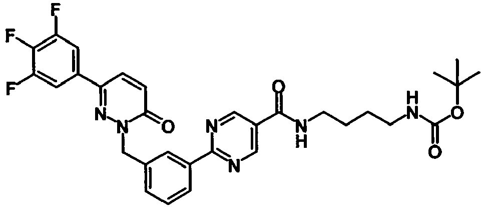 |
| "A39" | 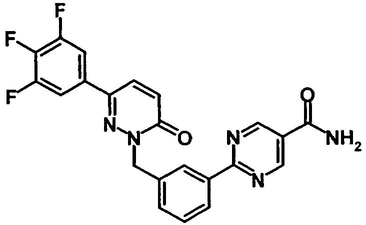 |
| "A40" | 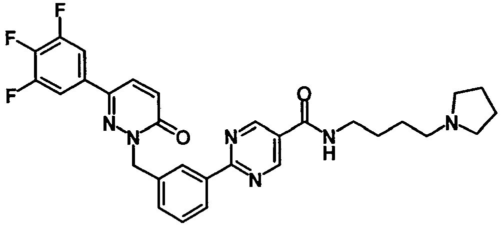 |
| "A41 " | 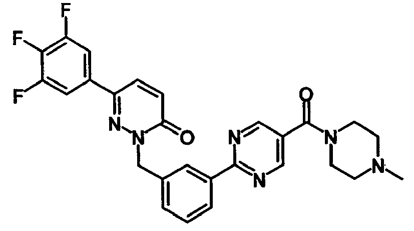 |
| "A42" | 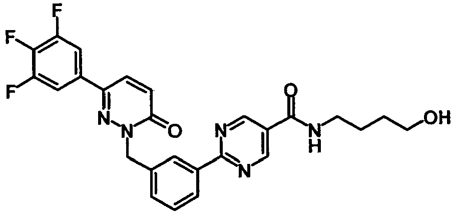 |
| "A43" | 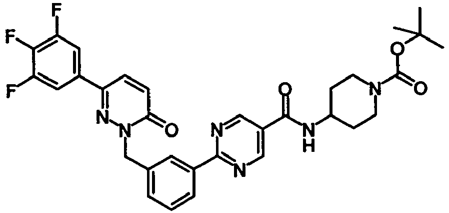 |
| "A44" | 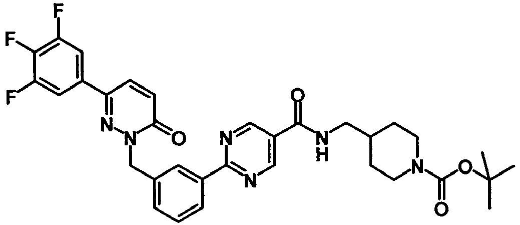 |
| "A44a" | 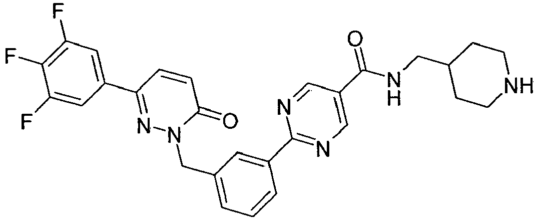 |
| "A45" | 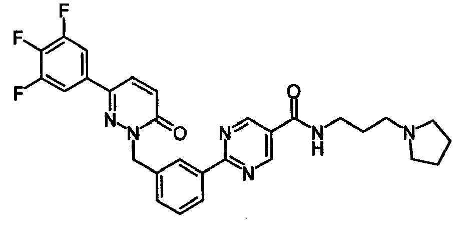 |
| "A46" | 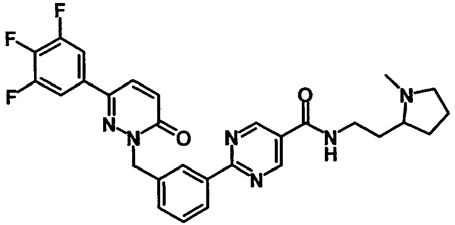 |
| "A47" | 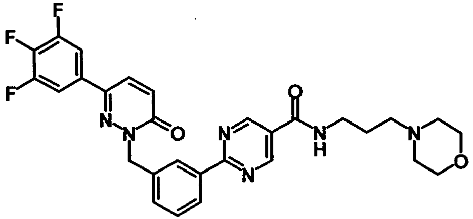 |
| "A48" | 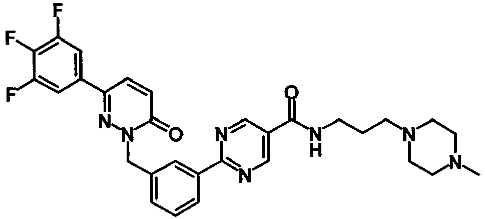 |
| "A49" | 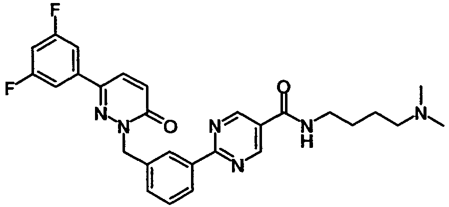 |
| "A50" | 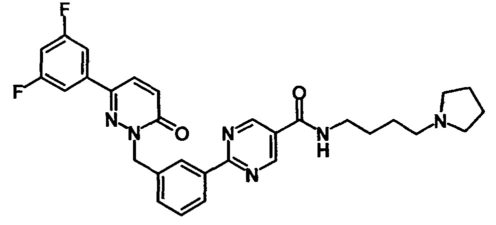 |
| "A51" | 2-{3-[6-Oxo-3-(3,4,5-trifluor-phenyl)-6H-pyridazin-1-ylmethyl]-phenyl}-pyrimidin-5-carbonsäure-[2-(1H-imidazol-4-yl)-ethyl]-amid |
| "A52" | 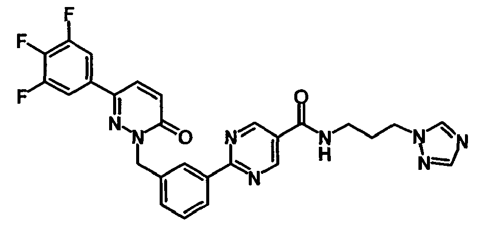 |
| "A53" | 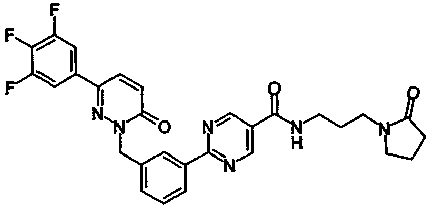 |
| "A54" | 2-[3-(5-Chlor-pyrimidin-2-yl)-benzyl]-6-(3,4,5-trifluor-phenyl)-2H-pyridazin-3-on |
| "A55" | 4-{1-[3-(5-Methyl-pyrimidin-2-yl)-benzyl]-6-oxo-1,6-dihydro-pyridazin-3-yl}-N-(3-piperidin-1-yl-propyl)-benzamid |
| "A56" | 6-(3,5-Difluor-phenyl)-2-{3-[5-(3-pyrrolidin-1-yl-propoxy)-pyrimidin-2-yl]-benzyl}-2H-pyridazin-3-on |
| "A57" | 6-(3,5-Difluor-phenyl)-2-(3-{5-[2-(4-methyl-piperazin-1-yl)-ethoxy]-pyrimidin-2-yl}-benzyl)-2H-pyridazin-3-on |
| "A58" | 6-(3,5-Difluor-phenyl)-2-[3-(5-dimethylaminomethyl-pyrimidin-2-yl)-benzyl]-2H-pyridazin-3-on |
| "A59" | 6-(3,5-Difluorphenyl)-2-{3-[4-(methyl-piperidin-4-yl-amino)-pyrimidin-2-yl]-benzyl}-2H-pyridazin-3-on |
| | 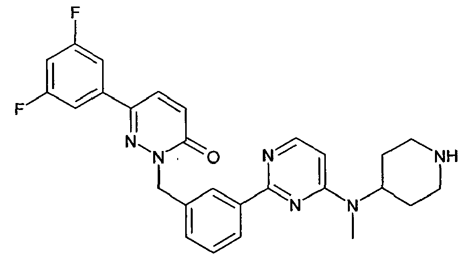 |
| "A60" | 2-{3-[5-(3-Dimethylamino-propoxy)-pyrimidin-2-yl]-benzyl}-6-(3,4,5-trifluor-phenyl)-2H-pyridazin-3-on |
| | |
| | |
| "A63" | 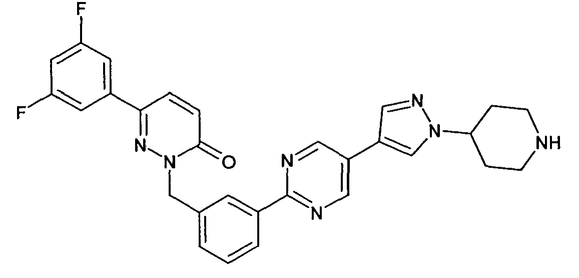 |
| "A64" | 2-{3-[5-(2-Dimethylamino-ethoxy)-pyrimidin-2-yl]-benzyl}-6-(3,5-difluor-phenyl)-2H-pyridazin-3-on |
| "A65" | 2-{3-[5-(Piperazin-1-yl)-pyrimidin-2-yl]-benzyl}-6-(3,4,5-trifluor-phenyl)-2H-pyridazin-3-on |
| "A66" | 6-(3,5-Difluor-phenyl)-2-{3-[5-(1-methyl-piperidin-4-ylmethoxy)-pyrimidin-2-yl]-benzyl}-2H-pyridazin-3-on |
| "A67" | 6-(3,5-Difluor-phenyl)-2-{3-[6-(3-dimethylamino-propylamino)-pyridazin-3-yl]-benzyl}-pyridazin-3-on |
| "A68" | 6-(3,5-Difluor-phenyl)-2-{3-[6-(2-dimethylamino-ethylamino)-pyridazin-3-yl]-benzyl}-pyridazin-3-on |
| "A69" | 6-(3,5-Difluor-phenyl)-2-{3-[6-(4-dimethylamino-butylamino)-pyridazin-3-yl]-benzyl}-pyridazin-3-on |
| "A70" | 6-(3,5-Difluor-phenyl)-2-{3-[6-(1-methyl-piperidin-4-ylamino)-pyridazin-3-yl]-benzyl}-pyridazin-3-on |
| "A71" | 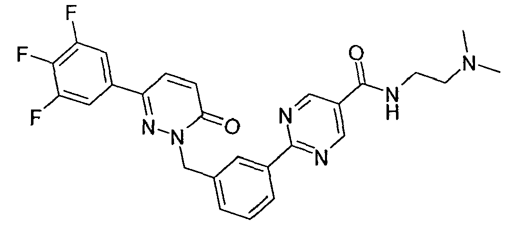 |
| "A72" | 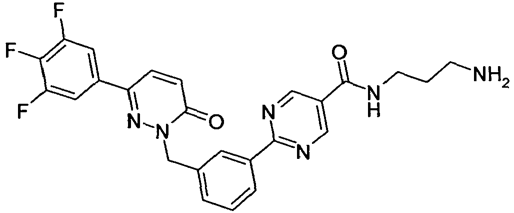 |
| "A73" | 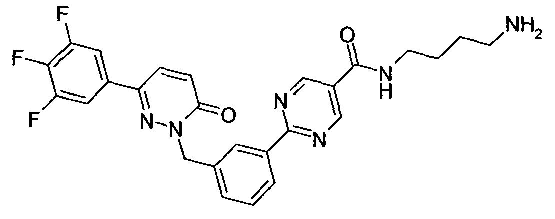 |
| "A74" | 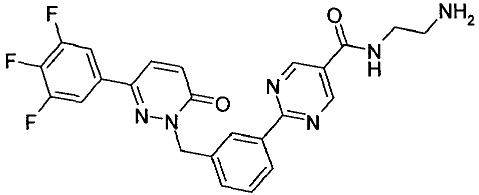 |
| "A75" | 4-(2-{3-[3-(3,5-Difluor-phenyl)-6-oxo-6H-pyridazin-1-ylmethyl]-phenyl}-pyrimidin-5-yl)-morpholin-3-on |
| | 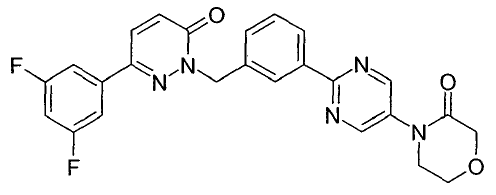 |
| "A76" | N'-(2-{3-[3-(3,4,5-Trifluorphenyl)-6-oxo-6H-pyridazin-1-ylmethyl]-phenyl}-pyrimidin-5-yl)-N,N-dimethyl-formamidin |
| "A77" | 2-{3-[6-Oxo-3-(3,4,5-trifluor-phenyl)-6H-pyridazin-1-ylmethyl]-phenyl}-pyrimidin-5-carbonitril |
| | 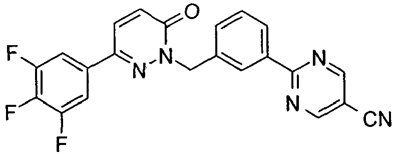 |
| "A81" | 6-(3,5-Difluor-phenyl)-2-{3-[4-(3-dimethylamino-propoxy)-pyrimidin-2-yl]-benzyl}-2H-pyridazin-3-on |
| "A82" | 2-[3-(5-Aminopyrimidin-2-yl)-benzyl]-6-(3,4,5-trifluorphenyl)-2H-pyridazin-3-on |
| "A84" | 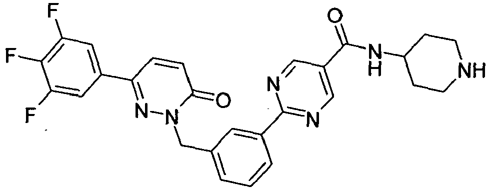 |
| "A85" | N-(2-{3-[3-(3,5-Difluor-phenyl)-6-oxo-6H-pyridazin-1-ylmethyl]-phenyl}-pyrimidin-5-yl)-2-dimethylamino-acetamid |
| | 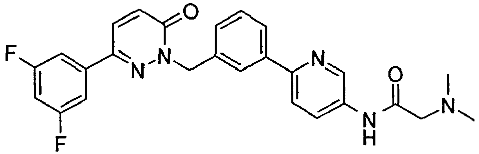 |
| "A87" | N-(2-{3-[3-(3,5-Difluor-phenyl)-6-oxo-6H-pyridazin-1-ylmethyl]-phenyl}pyrimidin-5-yl)-4-dimethylamino-butyramid |
| | 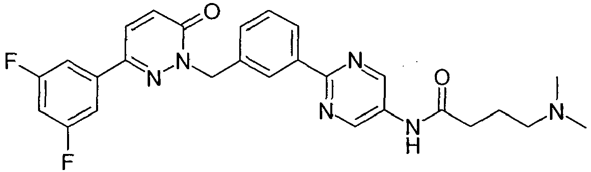 |
| "A88" | 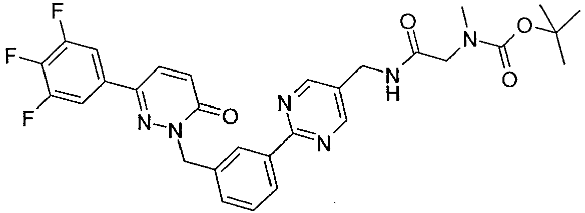 |
| "A89" | 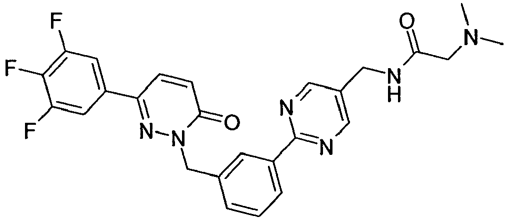 |
| "A90" | 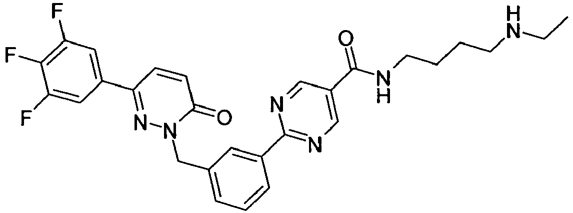 |
| | |
| "A92" | 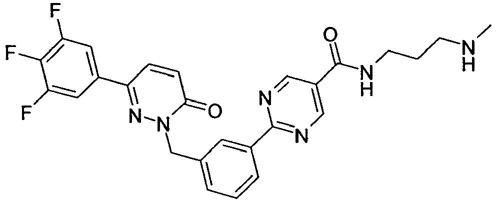 |
| "A93" | 2-[3-(5-Aminomethyl-pyrimidin-2-yl)-benzyl]-6-(3,4,5-trifluorphenyl)-2H-pyridazin-3-on |
| "A95" | N-(2-{3-[3-(3,5-Difluor-phenyl)-6-oxo-6H-pyridazin-1-ylmethyl]-phenyl}-pyrimidin-5-yl)-3-dimethylamino-propionamid |
| | 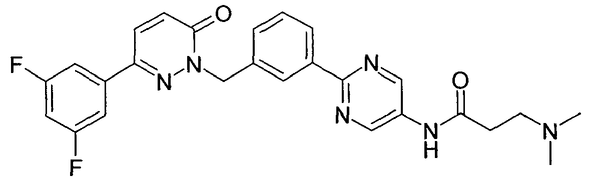 |
| "A96" | 3-(4-Methyl-piperazin-1-yl)-N-(2-{3-[6-oxo-3-(3,4,5-trifluor-phenyl)-6H-pyridazin-1-ylmethyl]-phenyl}-pyrimidin-5-ylmethyl)-propionamid |
| | 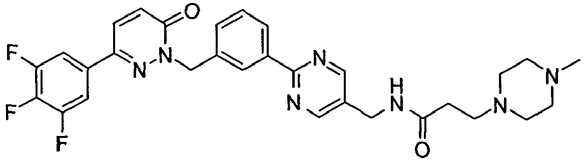 |
| "A97" | 2-(4-Methyl-piperazin-1-yl)-N-(2-{3-[6-oxo-3-(3,4,5-trifluor-phenyl)-6H-pyridazin-1-ylmethyl]-phenyl}-pyrimidin-5-ylmethyl)-acetamid |
| | 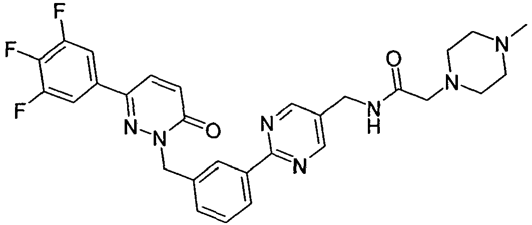 |
| "A98" | 2-Methylamino-N-(2-{3-[6-oxo-3-(3,4,5-trifluor-phenyl)-6H-pyridazin-1-ylmethyl]-phenyl}-pyrimidin-5-ylmethyl)-acetamid |
| | 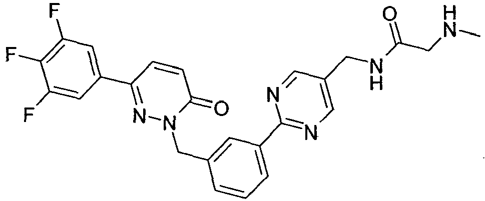 |
| "A99" | 3-Dimethylamino-N-(2-{3-[6-oxo-3-(3,4,5-trifluor-phenyl)-6H-pyridazin-1-ylmethyl]-phenyl}-pyrimidin-5-ylmethyl)-propionamid |
| | 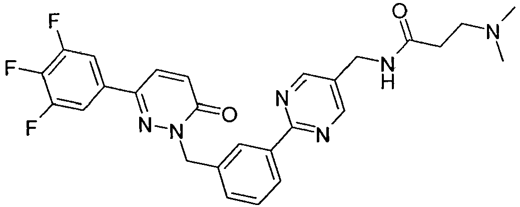 |
| "A101" | 6-(3,5-Difluor-phenyl)-2-[3-(5-hydroxymethyl-pyrimidin-2-yl)-benzyl]-2H-pyridazin-3-on |
| "A102" | 6-(3,5-Difluor-phenyl)-2-{3-[5-(piperidin-4-yloxy)-pyrimidin-2-yl]-benzyl}-2H-pyridazin-3-on |
| "A103" | 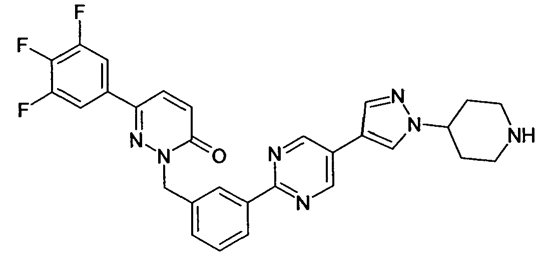 |
| "A104" | 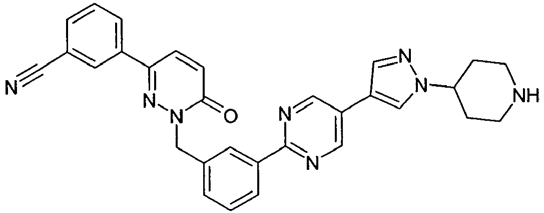 |
| "A105" | 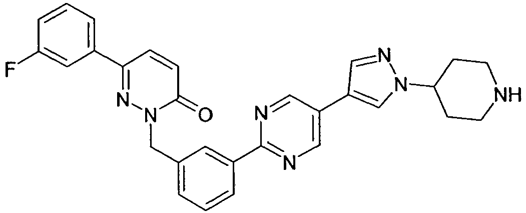 |
| "A106" | 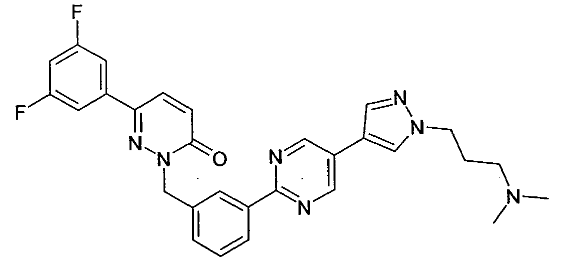 |
| "A107" | 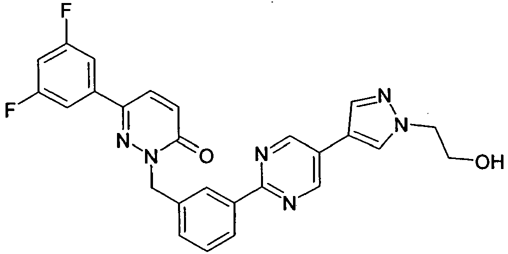 |
| "A108" | 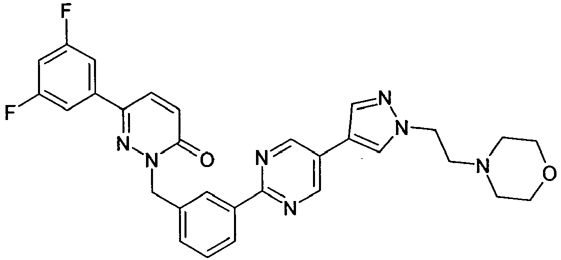 |
| "A109" | 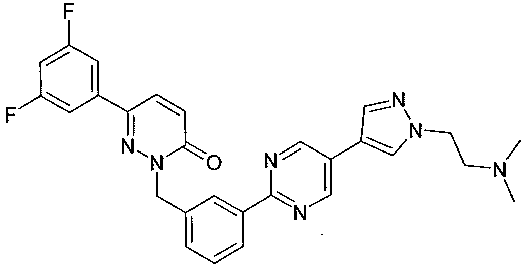 |
| "A110" | 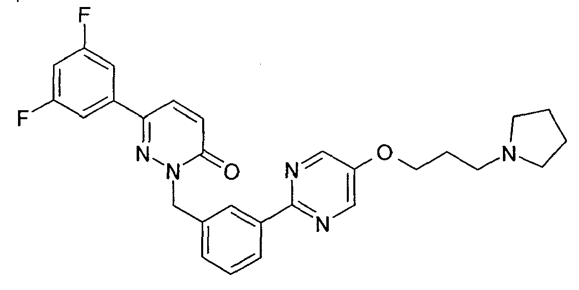 |
| "A111" | 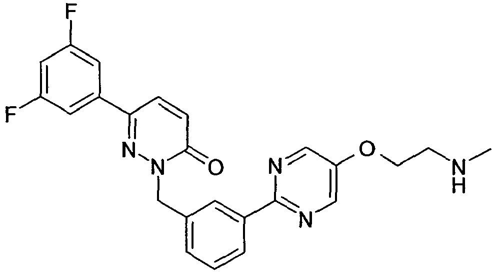 |
| "A114" | 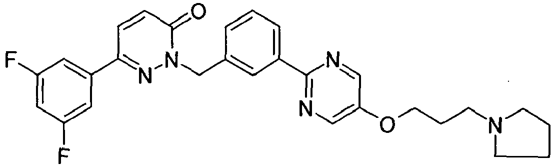 |
| "A115" | 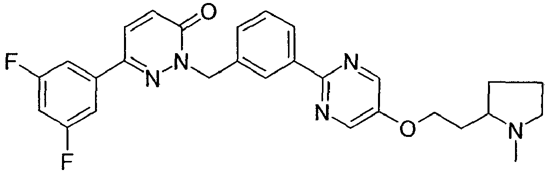 |
| "A116" | 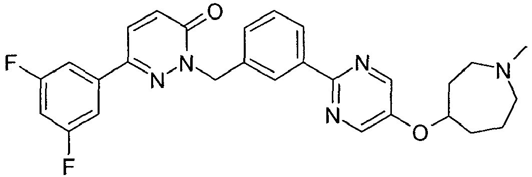 |
| "A117" | 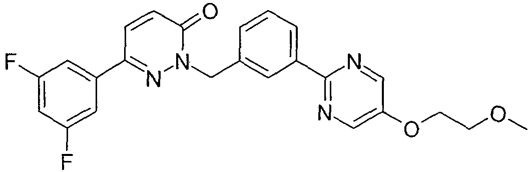 |
| "A118" | 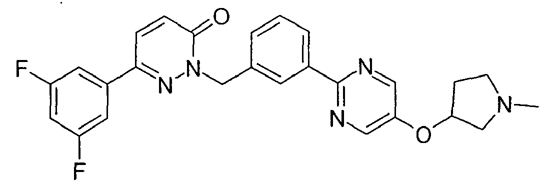 |
| "A119" | 6-(3,5-Difluor-phenyl)-2-{3-[5-(8-methyl-8-aza-bicyclo[3.2.1]oct-3-yloxy)-pyrimidin-2-yl]-benzyl}-2H-pyridazin-3-on |
| | 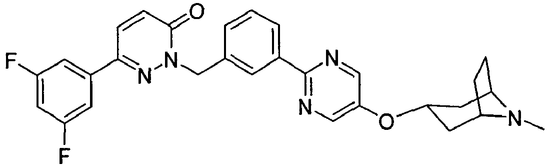 |
| "A120" | 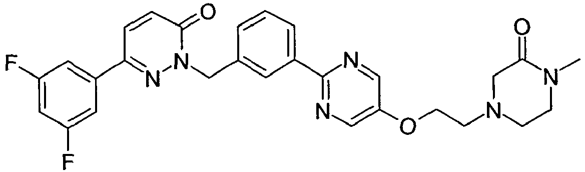 |
| "A121" | 6-(3,5-Difluor-phenyl)-2-{3-[5-((S)-1-methyl-pyrrolidin-3-yloxy)-pyrimidin-2-yl]-benzyl}-2H-pyridazin-3-on, |
| | 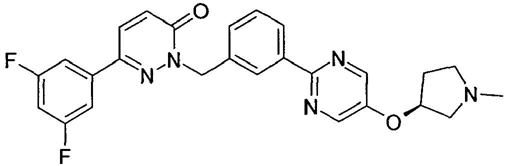 |
| "A122" | 6-(3,5-Difluor-phenyl)-2-{3-[5-((R)-1-methyl-pyrrolidin-3-yloxy)-pyrimidin-2-yl]-benzyl}-2H-pyridazin-3-on, |
| "A123" | 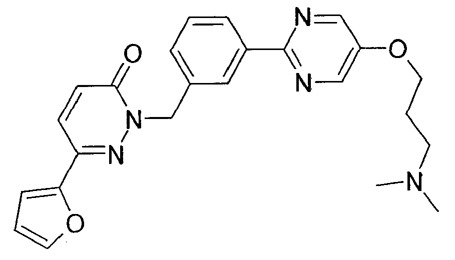 |
| "A124" | 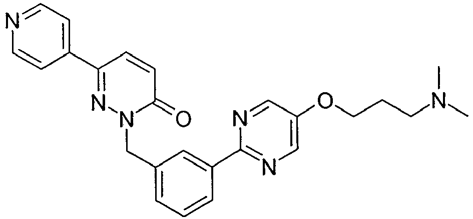 |
| "A125" | 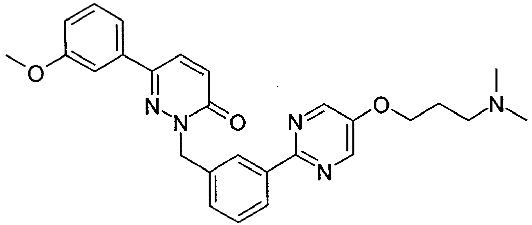 |
| "A126" | 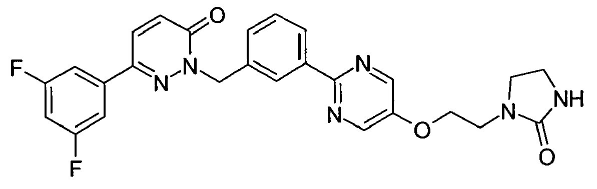 |
| "A127" | 6-(3,5-Difluor-phenyl)-2-{3-[5-(2-pyrrolidin-1-yl-ethoxy)-pyrimidin-2-yl]-benzyl}-2H-pyridazin-3-on |
| "A128" | 6-(3,5-Difluor-phenyl)-2-{3-[5-(3-morpholin-4-yl-propoxy)-pyrimidin-2-yl]-benzyl}-2H-pyridazin-3-on |
| "A129" | 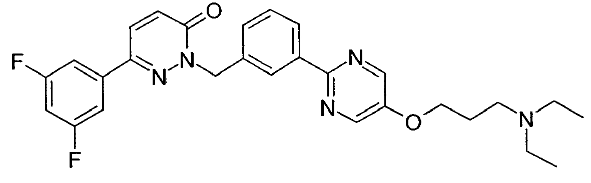 |
| "A130" | 6-(3,5-Difluor-phenyl)-2-{3-[5-(2-morpholin-4-yl-ethoxy)-pyrimidin-2-yl]-benzyl}-2H-pyridazin-3-on, Hydrochlorid |
| "A131" | 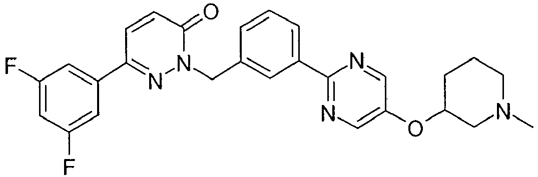 |
| "A132" | 6-(3,5-Difluor-phenyl)-2-{3-[5-(4-methylamino-butoxy)-pyrimidin-2-yl]-benzyl}-2H-pyridazin-3-on |
| "A133" | 6-(3,5-Difluor-phenyl)-2-{3-[5-(3-methylamino-propoxy)-pyrimidin-2-yl]-benzyl}-2H-pyridazin-3-on |
| "A134" | 6-(3,5-Difluor-phenyl)-2-{3-[5-(pyrrolidin-3-ylmethoxy)-pyrimidin-2-yl]-benzyl}-2H-pyridazin-3-on |
| "A135" | 6-(3,5-Difluor-phenyl)-2-{3-[5-(3-ethylamino-propoxy)-pyrimidin-2-yl]-benzyl}-2H-pyridazin-3-on |
| "A136" | 2-{3-[5-(2-Amino-ethoxy)-pyrimidin-2-yl]-benzyl}-6-(3,5-difluor-phenyl)-2H-pyridazin-3-on |
| "A137" | 6-(3,5-Difluor-phenyl)-2-{3-[5-(piperidin-3-yloxy)-pyrimidin-2-yl]-benzyl}-2H-pyridazin-3-on |
| "A138" | 6-(3,5-Difluor-phenyl)-2-{3-[5-(piperidin-4-ylmethoxy)-pyrimidin-2-yl]-benzyl}-2H-pyridazin-3-on |
| "A139" | 6-(3,5-Difluor-phenyl)-2-{3-[5-(pyrrolidin-3-yloxy)-pyrimidin-2-yl]-benzyl}-2H-pyridazin-3-on |
| "A140" | 6-(3,5-Difluor-phenyl)-2-{3-[5-((S)-pyrrolidin-3-yloxy)-pyrimidin-2-yl]-benzyl}-2H-pyridazin-3-on |
| "A141" | 6-(3,5-Difluor-phenyl)-2-{3-[5-((R)-pyrrolidin-3-yloxy)-pyrimidin-2-yl]-benzyl}-2H-pyridazin-3-on |
| "A142" | 2-{3-[5-(Piperidin-4-yloxy)-pyrimidin-2-yl]-benzyl}-6-pyridin-4-yl-2H-pyridazin-3-on |
| | 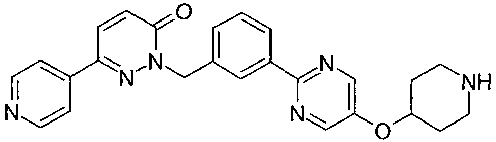 |
| "A143" | 4-(6-Oxo-1-{3-[5-(piperidin-4-yloxy)-pyrimidin-2-yl]-benzyl)-1,6-dihydro-pyridazin-3-yl)-benzonitril |
| | 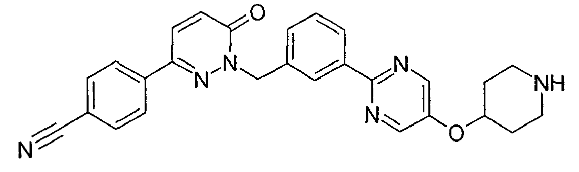 |
| "A144" | 3-(6-Oxo-1-{3-[5-(piperidin-4-yloxy)-pyrimidin-2-yl]-benzyl)-1,6-dihydro-pyridazin-3-yl)-benzonitril |
| "A145" | 6-(3,5-Difluor-phenyl)-2-{3-[5-(2-piperazin-1-yl-ethoxy)-pyrimidin-2-yl]-benzyl}-2H-pyridazin-3-on |
| "A146" | 6-(3,5-Difluor-phenyl)-2-{3-[5-(piperidin-4-yloxy)-pyrimidin-2-yl]-benzyl}-2H-pyridazin-3-on |
| "A147" | 3-(6-Oxo-1-{3-[5-(2-piperazin-1-yl-ethoxy)-pyrimidin-2-yl]-benzyl}-1,6-dihydro-pyridazin-3-yl)-benzonitril |
| "A148" | 6-(3-Fluor-phenyl)-2-{3-[5-(piperidin-4-ylmethoxy)-pyrimidin-2-yl]-benzyl}-2H-pyridazin-3-on |
| "A149" | 2-{3-[5-(1-Piperidin-4-yl-1H-pyrazol-4-yl)-pyrimidin-2-yl]-benzyl}-6-(3,4,5-trifluor-phenyl)-2H-pyridazin-3-on |
| | 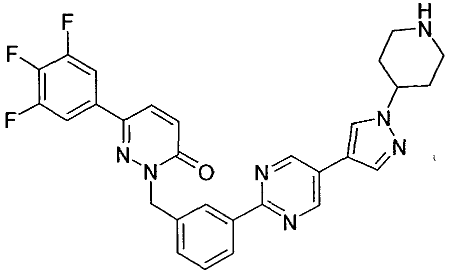 |
| "A150" | 3-(6-Oxo-1-{3-[5-(1-piperidin-4-yl-1H-pyrazol-4-yl)-pyrimidin-2-yl]-benzyl}-1,6-dihydro-pyridazin-3-yl)-benzonitril |
| | 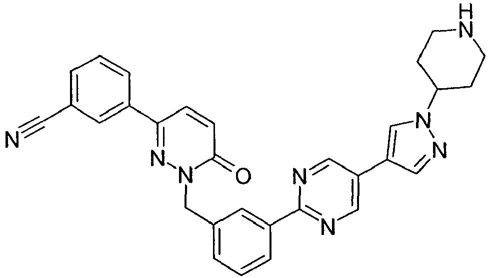 |
| "A151" | 6-(1-Methyl-1H-pyrazol-4-yl)-2-{3-[5-(1-piperidin-4-yl-1H-pyrazol-4-yl)-pyrimidin-2-yl]-benzyl}-2H-pyridazin-3-on |
| | 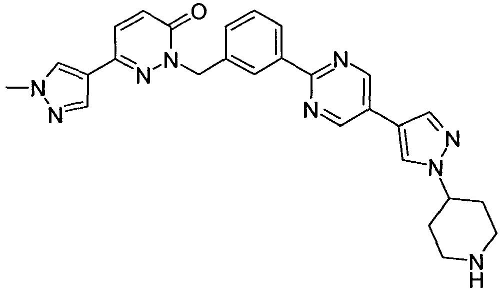 |
| "A152" | 6-(3-Methoxy-phenyl)-2-{3-[5-(1-piperidin-4-yl-1H-pyrazol-4-yl)-pyrimidin-2-yl]-benzyl}-2H-pyridazin-3-on |
| "A153" | 6-(3-Fluor-phenyl)-2-{3-[5-(1-piperidin-4-yl-1H-pyrazol-4-yl)-pyrimidin-2-yl]-benzyl}-2H-pyridazin-3-on |
| "A154" | 6-(3,5-Difluor-phenyl)-2-{3-[5-(1H-pyrazol-4-yl)-pyrimidin-2-yl]-benzyl}-2H-pyridazin-3-on |
| | 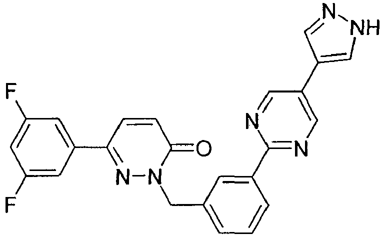 |
| "A155" | 6-(3,5-Difluor-phenyl)-2-(3-{5-[1-(2-methylamino-ethyl)-1H-pyrazol-4-yl]-pyrimidin-2-yl}-benzyl)-2H-pyridazin-3-on, |
| | 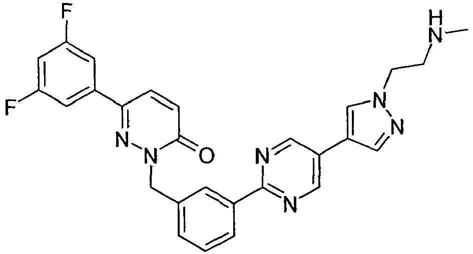 |
| "A156" | 6-(3-Chlor-phenyl)-2-{3-[5-(1-piperidin-4-yl-1H-pyrazol-4-yl)-pyrimidin-2-yl]-benzyl}-2H-pyridazin-3-on |
| | 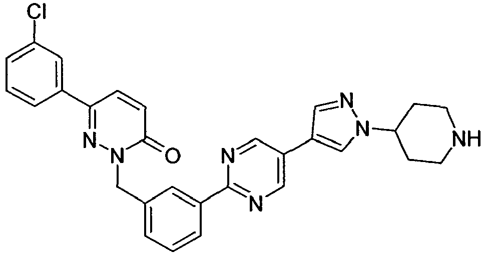 |
| "A157" | 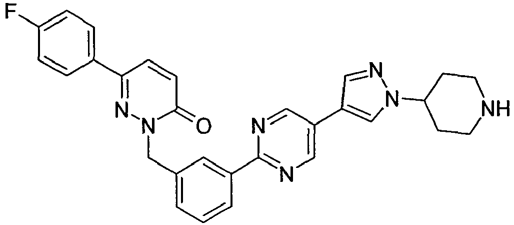 |
| "A158" | 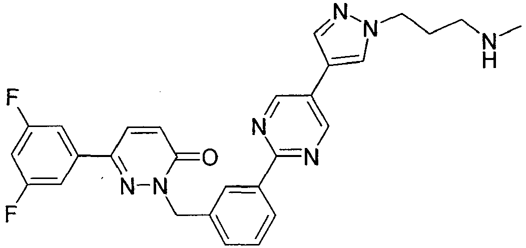 6-(3,5-Difluor-phenyl)-2-(3-{5-[1-(3-methylamino-propyl)-1H-pyrazol-4-yl]-pyrimidin-2-yl}-benzyl)-2H-pyridazin-3-on |
| "A159" | 3-[1-(3-{5-[1-(2-Methylamino-ethyl)-1H-pyrazol-4-yl]-pyrimidin-2-yl}-benzyl)-6-oxo-1,6-dihydro-pyridazin-3-yl]-benzonitril |
| | 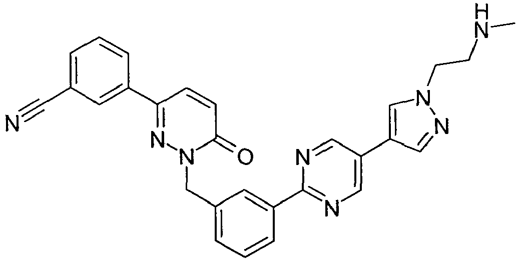 |
| "A160" | 6-(3,5-Difluor-phenyl)-2-(3-{5-[1-(2-piperazin-1-yl-ethyl)-1H-pyrazol-4-yl]-pyrimidin-2-yl}-benzyl)-2H-pyridazin-3-on |
| | 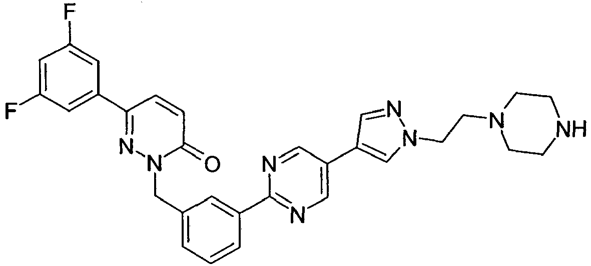 |
| "A161" | 3-(6-Oxo-1-{3-[5-(1H-pyrazol-4-yl)-pyrimidin-2-yl]-benzyl)-1,6-dihydro-pyridazin-3-yl)-benzonitril |
| | 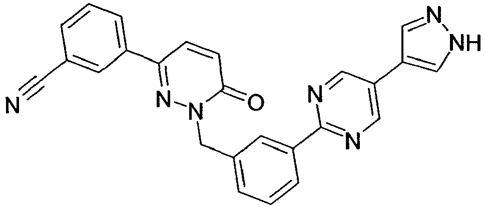 |
| "A162" | 3-[6-Oxo-1-(3-{5-[1-(2-piperazin-1-yl-ethyl)-1H-pyrazol-4-yl]-pyrimidin-2-yl}-benzyl)-1,6-dihydro-pyridazin-3-yl]-benzonitril |
| | 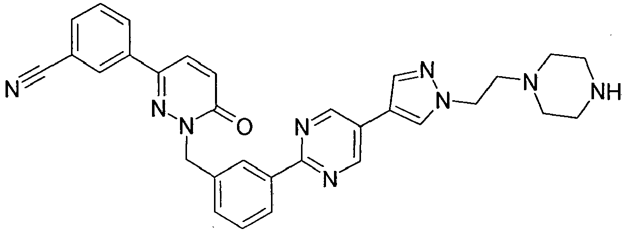 |
| "A163" | 2-[3-(6-Oxo-3-pyridin-4-yl-6H-pyridazin-1-ylmethyl)-phenyl]-pyrimidin-5-carbonsäure-(4-dimethylamino-butyl)-amid |
| | 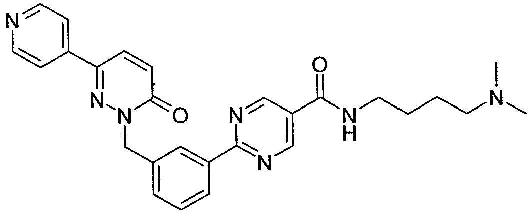 |
| "A164" | 2-{3-[3-(4-Cyan-phenyl)-6-oxo-6H-pyridazin-1-ylmethyl]-phenyl}-pyrimidin-5-carbonsäure-(4-dimethylamino-butyl)-amid |
| | 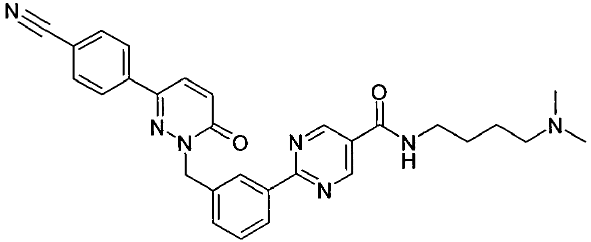 |
| "A165" | 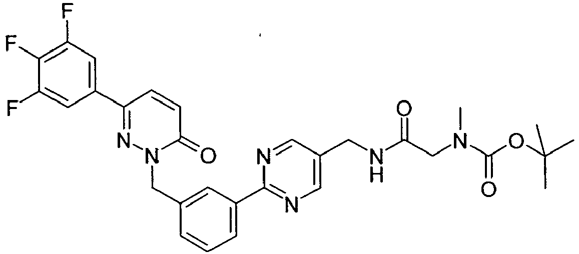 |
| "A166" | 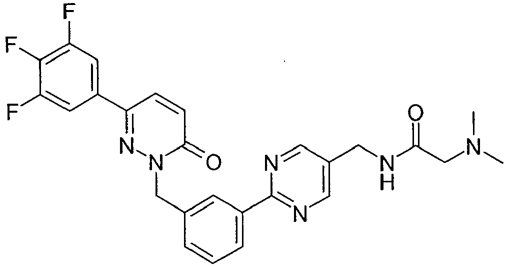 |
| "A167" | 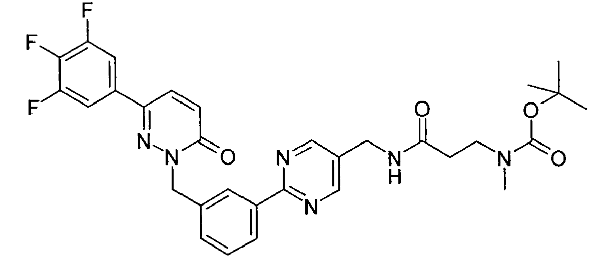 |
| "A168" | 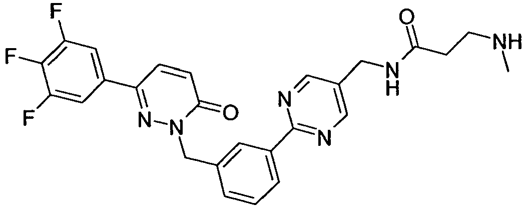 |
| "A169" | 6-(1-Methyl-1H-pyrazol-4-yl)-2-(3-{5-[1-(2-pyrrolidin-1-yl-ethyl)-1H-pyrazol-4-yl]-pyrimidin-2-yl}-benzyl)-2H-pyridazin-3-on |
| | 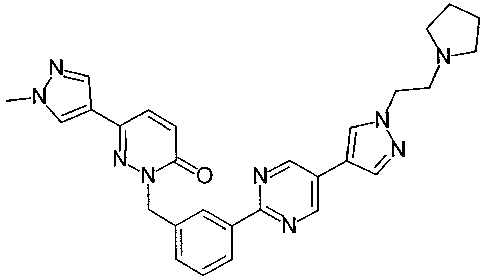 |
| "A170" | 6-(3,5-Difluor-phenyl)-2-(3-{5-[1-(2-morpholin-4-yl-ethyl)-1H-pyrazol-4-yl]-pyrimidin-2-yl}-benzyl)-2H-pyridazin-3-on |
| | 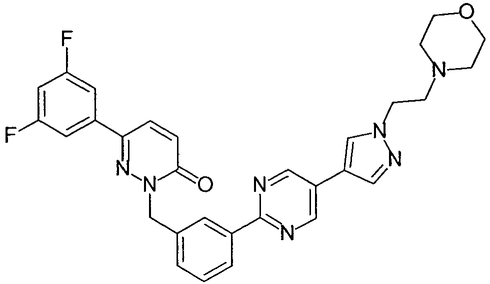 |
| "A171" | 6-(3,5-Difluor-phenyl)-2-(3-{5-[1-(2-dimethylamino-ethyl)-1H-pyrazol-4-yl]-pyrimidin-2-yl}-benzyl)-2H-pyridazin-3-on |
| | 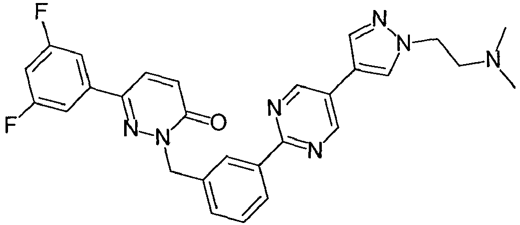 |
| "A172" | 6-(3,5-Difluor-phenyl)-2-(3-{5-[1-(3-dimethylamino-propyl)-1H-pyrazol-4-yl]-pyrimidin-2-yl}-benzyl)-2H-pyridazin-3-on |
| "A173" | 6-(3,5-Difluor-phenyl)-2-(3-{5-[1-(2-pyrrolidin-1-yl-ethyl)-1 H-pyrazol-4-yl]-pyrimidin-2-yl}-benzyl)-2H-pyridazin-3-on |
| "A174" | 3-[1-(3-{5-[1-(2-Morpholin-4-yl-ethyl)-1H-pyrazol-4-yl]-pyrimidin-2-yl}-benzyl)-6-oxo-1,6-dihydro-pyridazin-3-yl]-benzonitril |
| | 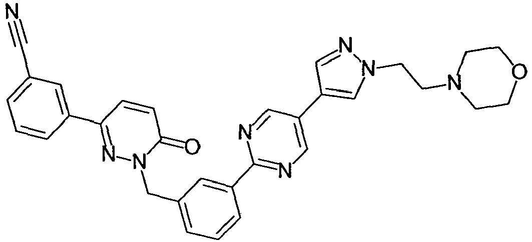 |
| "A175" | 2-(3-{5-[1-(2-Morpholin-4-yl-ethyl)-1H-pyrazol-4-yl]-pyrimidin-2-yl}-benzyl)-6-pyridin-3-yl-2H-pyridazin-3-on |
| | 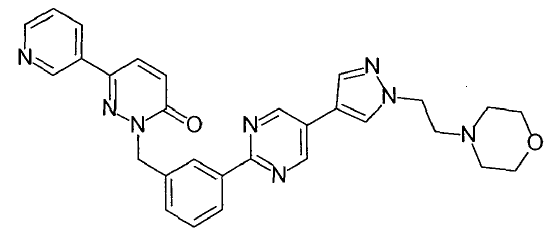 |
| "A176" | 6-(1-Methyl-1H-pyrazol-4-yl)-2-(3-{5-[1-(2-morpholin-4-yl-ethyl)-1H-pyrazol-4-yl]-pyrimidin-2-yl}-benzyl)-2H-pyridazin-3-on |
| | 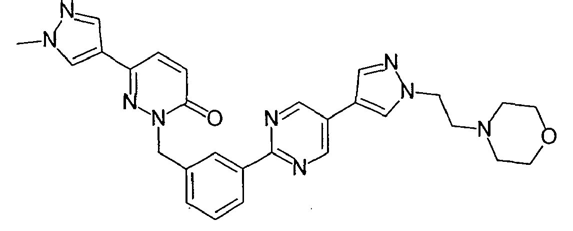 |
| "A177" | 2-(3-{5-[1-(2-Morpholin-4-yl-ethyl)-1H-pyrazol-4-yl]-pyrimidin-2-yl}-benzyl)-6-pyridin-4-yl-2H-pyridazin-3-on |
| "A178" | 6-(4-Methansulfonyl-phenyl)-2-(3-{5-[1-(2-morpholin-4-yl-ethyl)-1H-pyrazol-4-yl]-pyrimidin-2-yl}-benzyl)-2H-pyridazin-3-on |
| "A179" | 6-Pyridin-4-yl-2-(3-{5-[1-(2-pyrrolidin-1-yl-ethyl)-1H-pyrazol-4-yl]-pyrimidin-2-yl}-benzyl)-2H-pyridazin-3-on |
| "A180" | 4-[6-Oxo-1-(3-{5-[1-(2-pyrrolidin-1-yl-ethyl)-1H-pyrazol-4-yl]-pyrimidin-2-yl}-benzyl)-1,6-dihydro-pyridazin-3-yl]-benzonitril |
| | 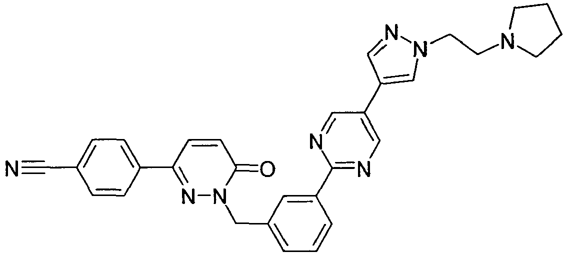 |
| "A181" | 2-(3-{5-[1-(2-Morpholin-4-yl-ethyl)-1H-pyrazol-4-yl]-pyrimidin-2-yl}-benzyl)-6-pyridin-4-yl-2H-pyridazin-3-on |
| "A183" | 6-(4-Methansulfonyl-phenyl)-2-(3-{5-[1-(2-morpholin-4-yl-ethyl)-1H-pyrazol-4-yl]-pyrimidin-2-yl}-benzyl)-2H-pyridazin-3-on |
| "A184" | 6-(5-Methyl-oxazol-2-yl)-2-(3-{5-[1-(2-pyrrolidin-1-yl-ethyl)-1H-pyrazol-4-yl]-pyrimidin-2-yl}-benzyl)-2H-pyridazin-3-on |
| | 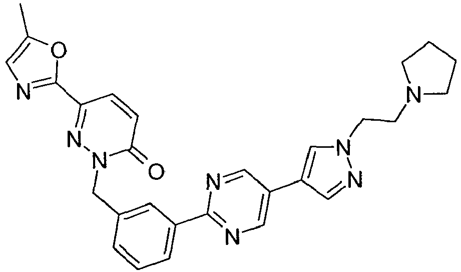 |
| "A185" | 6-(3-Fluor-phenyl)-2-(3-{5-[1-(2-pyrrolidin-1-yl-ethyl)-1H-pyrazol-4-yl]-pyrimidin-2-yl}-benzyl)-2H-pyridazin-3-on |
| "A186" | 6-(1-Propyl-1H-pyrazol-4-yl)-2-(3-{5-[1-(2-pyrrolidin-1-yl-ethyl)-1H-pyrazol-4-yl]-pyrimidin-2-yl}-benzyl)-2H-pyridazin-3-on |
| | 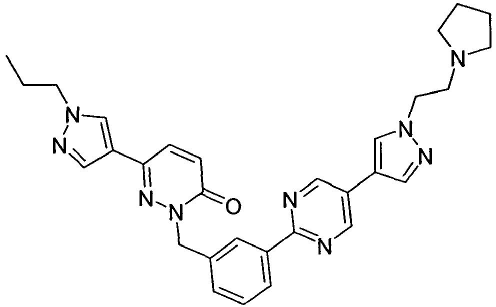 |
| "A187" | 2-(3-{5-[1-(2-Pyrrolidin-1-yl-ethyl)-1H-pyrazol-4-yl]-pyrimidin-2-yl}-benzyl)-6-thiophen-3-yl-2H-pyridazin-3-on |
| | 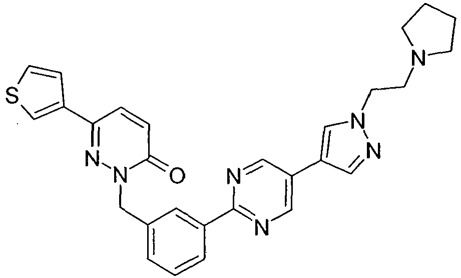 |
| "A188" | 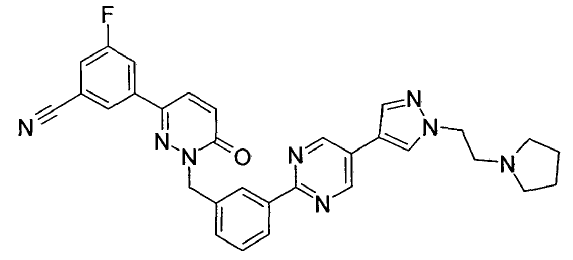 |
| "A188a" | 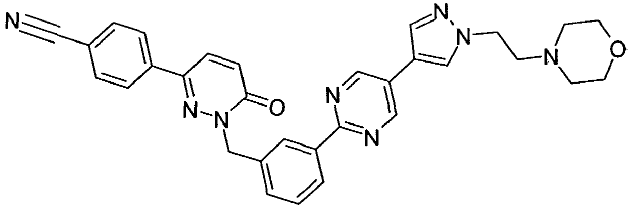 |
| "A189" | 3-(1-{3-[5-(3-Dimethylamino-propoxy)-pyrimidin-2-yl]-benzyl}-6-oxo-1,6-dihydro-pyridazin-3-yl)-benzonitril |
| "A190" | 2-{3-[5-(3-Dimethylamino-propoxy)-pyrimidin-2-yl]-benzyl}-6-(1-methyl-1 H-pyrazol-4-yl)-2H-pyridazin-3-on |
| | 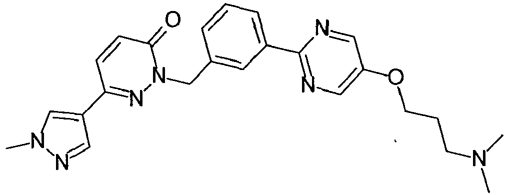 |
| "A191" | 2-{3-[5-(3-Dimethylamino-propoxy)-pyrimidin-2-yl]-benzyl}-6-(3-fluor-phenyl)-2H-pyridazin-3-on |
| "A192" | 2-{3-[5-(3-Dimethylamino-propoxy)-pyrimidin-2-yl]-benzyl}-6-thiazol-2-yl-2H-pyridazin-3-on |
| "A193" | 2-{3-[5-(3-Dimethylamino-propoxy)-pyrimidin-2-yl]-benzyl}-6-phenyl-2H-pyridazin-3-on |
| "A194" | 4-(1-{3-[5-(3-Dimethylamino-propoxy)-pyrimidin-2-yl]-benzyl}-6-oxo-1,6-dihydro-pyridazin-3-yl)-benzonitril |
| "A195" | 2-{3-[5-(3-Dimethylamino-propoxy)-pyrimidin-2-yl]-benzyl}-6-p-tolyl-2H-pyridazin-3-on |
| "A196" | 2-{3-[5-(3-Dimethylamino-propoxy)-pyrimidin-2-yl]-benzyl}-6-(2H-pyrazol-3-yl)-2H-pyridazin-3-on |
| "A197" | 6-(3,4-Difluor-phenyl)-2-{3-[5-(3-dimethylamino-propoxy)-pyrimidin-2-yl]-benzyl}-2H-pyridazin-3-on |
| "A198" | 2-{3-[5-(3-Dimethylamino-propoxy)-pyrimidin-2-yl]-benzyl}-6-(4-methanesulfonyl-phenyl)-2H-pyridazin-3-on |
| "A199" | 2-{3-[5-(3-Dimethylamino-propoxy)-pyrimidin-2-yl]-benzyl}-6-[4-(5-methyl-[1,2,4]oxadiazol-3-yl)-phenyl]-2H-pyridazin-3-on |
| "A200" | 2-{3-[5-(3-Dimethylamino-propoxy)-pyrimidin-2-yl]-benzyl}-6-pyridin-4-yl-2H-pyridazin-3-on |
| "A201" | 6-(3-Brom-phenyl)-2-{3-[5-(3-dimethylamino-propoxy)-pyrimidin-2-yl]-benzyl}-2H-pyridazin-3-on |
| "A202" | 2-{3-[5-(3-Dimethylamino-propoxy)-pyrimidin-2-yl]-benzyl}-6-(3,4,5-trifluor-phenyl)-2H-pyridazin-3-on |
| "A203" | 6-(3,5-Dimethoxy-phenyl)-2-{3-[5-(3-dimethylamino-propoxy)-pyrimidin-2-yl]-benzyl}-2H-pyridazin-3-on |
| "A204" | 2-{3-[5-(3-Dimethylamino-propoxy)-pyrimidin-2-yl]-benzyl}-6-(3-fluor-4-methoxy-phenyl)-2H-pyridazin-3-on |
| "A205" | 2-{3-[5-(3-Dimethylamino-propoxy)-pyrimidin-2-yl]-benzyl}-6-(4-methoxy-phenyl)-2H-pyridazin-3-on |
| "A206" | 2-{3-[5-(3-Dimethylamino-propoxy)-pyrimidin-2-yl]-benzyl}-6-(3-trifluormethyl-phenyl)-2H-pyridazin-3-on, |
| "A207" | 6-(3-Chlor-phenyl)-2-{3-[5-(3-dimethylamino-propoxy)-pyrimidin-2-yl]-benzyl}-2H-pyridazin-3-on |
| "A208" | 2-{3-[5-(3-Dimethylamino-propoxy)-pyrimidin-2-yl]-benzyl}-6-pyridin-3-yl-2H-pyridazin-3-on |
| "A209" | 2-{3-[5-(3-Dimethylamino-propoxy)-pyrimidin-2-yl]-benzyl}-6-(1-methyl-1 H-pyrazol-4-yl)-2H-pyridazin-3-on |
| "A210" | 6-(3-Chlor-5-fluor-phenyl)-2-{3-[5-(3-dimethylamino-propoxy)-pyrimidin-2-yl]-benzyl}-2H-pyridazin-3-on |
| "A211" | 2-{3-[5-(3-Dimethylamino-propoxy)-pyrimidin-2-yl]-benzyl}-6-(4-fluor-3-methoxy-phenyl)-2H-pyridazin-3-on |
| "A212" | 6-(4-Chlor-phenyl)-2-{3-[5-(3-dimethylamino-propoxy)-pyrimidin-2-yl]-benzyl}-2H-pyridazin-3-on |
| "A213" | 2-{3-[5-(3-Dimethylamino-propoxy)-pyrimidin-2-yl]-benzyl}-6-(4-fluor-phenyl)-2H-pyridazin-3-on |
| "A214" | 2-{3-[5-(3-Dimethylamino-propoxy)-pyrimidin-2-yl]-benzyl}-6-thiophen-2-yl-2H-pyridazin-3-on |
| "A215" | N-[4-(1-{3-[5-(3-Dimethylamino-propoxy)-pyrimidin-2-yl]-benzyl}-6-oxo-1,6-dihydro-pyridazin-3-yl)-phenyl]-acetamid |
| | 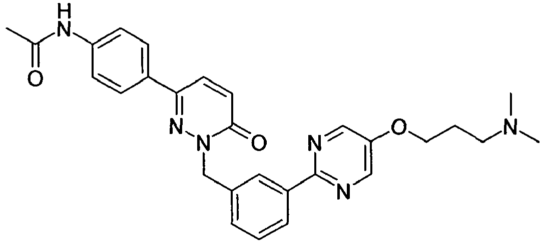 |
| "A216" | 6-(3,4-Dimethoxy-phenyl)-2-{3-[5-(3-dimethylamino-propoxy)-pyrimidin-2-yl]-benzyl}-2H-pyridazin-3-on |
| "A217" | 6-Benzo[1,2,5]thiadiazol-5-yl-2-{3-[5-(3-dimethylamino-propoxy)-pyrimidin-2-yl]-benzyl}-2H-pyridazin-3-on |
| | 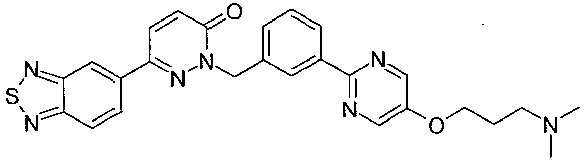 |
| "A218" | 2-{3-[5-(3-Dimethylamino-propoxy)-pyrimidin-2-yl]-benzyl}-6-furan-3-yl-2H-pyridazin-3-on |
| "A219" | 2-{3-[5-(3-Dimethylamino-propoxy)-pyrimidin-2-yl]-benzyl}-6-(5-methyl-[1,2,4]oxadiazol-3-yl)-2H-pyridazin-3-on |
| "A220" | 4-(1-{3-[5-(1-Methyl-piperidin-4-yloxy)-pyrimidin-2-yl]-benzyl}-6-oxo-1,6-dihydro-pyridazin-3-yl)-benzonitril |
| "A221" | 3-(1-{3-[5-(1-Methyl-piperidin-4-yloxy)-pyrimidin-2-yl]-benzyl}-6-oxo-1,6-dihydro-pyridazin-3-yl)-benzonitril |
| "A222" | 3-(1-{3-[5-(2-Morpholin-4-yl-ethoxy)-pyrimidin-2-yl]-benzyl)-6-oxo-1,6-dihydro-pyridazin-3-yl)-benzonitril |
| "A223" | 2-{3-[5-(1-Methyl-piperidin-4-yloxy)-pyrimidin-2-yl]-benzyl}-6-pyridin-4-yl-2H-pyridazin-3-on |
| "A224" | 6-(4-Methansulfonyl-phenyl)-2-{3-[5-(1-methyl-piperidin-4-yloxy)-pyrimidin-2-yl]-benzyl}-2H-pyridazin-3-on |
| "A225" | 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäure-methylester |
| | 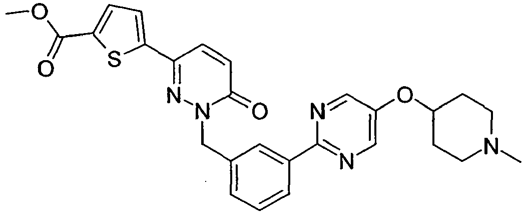 |
| "A226" | 2-{3-[5-(1-Methyl-piperid)n-4-yloxy)-pyrimidin-2-yl]-benzyl}-6-(1-methyl-1 H-pyrazol-4-yl)-2H-pyridazin-3-on |
| "A227" | 2-{3-[5-(3-Dimethylamino-propoxy)-pyrimidin-2-yl]-benzyl}-6-(5-oxo-4,5-dihydro-[1,2,4]oxadiazol-3-yl)-2H-pyridazin-3-on |
| | 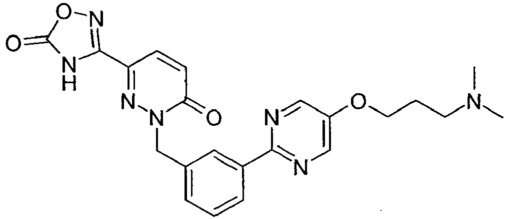 |
| "A228" | 2-{3-[5-(3-Dimethylamino-propoxy)-pyrimidin-2-yl]-benzyl}-6-piperazin-1-yl-2H-pyridazin-3-on |
| | 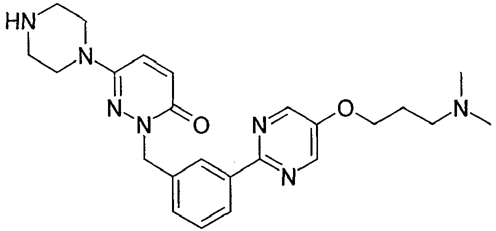 |
| "A229" | 6-(1-Methyl-1H-pyrazol-4-yl)-2-{3-[5-(2-morpholin-4-yl-ethoxy)-pyrimidin-2-yl]-benzyl}-2H-pyridazin-3-on |
| "A235" | 6-(3-Fluor-phenyl)-2-{3-[5-(2-morpholin-4-yl-ethoxy)-pyrimidin-2-yl]-benzyl}-2H-pyridazin-3-on |
| "A237" | 2-{3-[5-(1-Methyl-piperidin-4-ylmethoxy)-pyrimidin-2-yl]-benzyl}-6-(1-methyl-1H-pyrazol-4-yl)-2H-pyridazin-3-on |
| "A238" | 2-{3-[5-(1-Methyl-piperidin-4-yloxy)-pyrimidin-2-yl]-benzyl}-6-(1-methyl-1 H-pyrazol-4-yl)-2H-pyridazin-3-on |
| "A240" | 2-{3-[5-(3-Methoxy-propoxy)-pyrimidin-2-yl]-benzyl}-6-(1-methyl-1 H-pyrazol-4-yl)-2H-pyridazin-3-on |
| "A241" | 2-{3-[5-(2-Methoxy-ethoxy)-pyrimidin-2-yl]-benzyl}-6-(1-methyl-1 H-pyrazol-4-yl)-2H-pyridazin-3-on |
| "A242" | 2-{3-[5-(2-Morpholin-4-yl-ethoxy)-pyrimidin-2-yl]-benzyl}-6-(1-propyl-1H-pyrazol-4-yl)-2H-pyridazin-3-on |
| "A243" | 2-(3-{5-[2-(4-Methyl-piperazin-1-yl)-ethoxy]-pyrimidin-2-yl}-benzyl)-6-(1-methyl-1H-pyrazol-4-yl)-2H-pyridazin-3-on |
| "A244" | 2-(3-{5-[2-(4-Methyl-3-oxo-piperazin-1-yl)-ethoxy]-pyrimidin-2-yl}-benzyl)-6-(-methyl-1H-pyrazol-4-yl)-2H-pyridazin-3-on |
| | 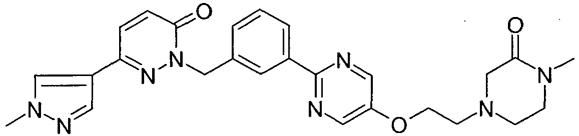 |
| "A245" | 6-(1-Methyl-1H-pyrazol-4-yl)-2-{3-[5-(3-morpholin-4-yl-propoxy)-pyrimidin-2-yl]-benzyl}-2H-pyridazin-3-on |
| "A246" | 6-(1-Methyl-1H-pyrazol-4-yl)-2-{3-[5-(2-morpholin-4-yl-propoxy)-pyrimidin-2-yl]-benzyl}-2H-pyridazin-3-on |
| | 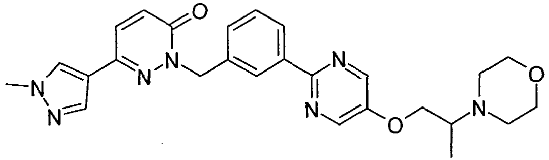 |
| "A247" | 2-{3-[5-(1-Methyl-2-morpholin-4-yl-ethoxy)-pyrimidin-2-yl]-benzyl}-6-(1-methyl-1H-pyrazol-4-yl)-2H-pyridazin-3-on |
| | 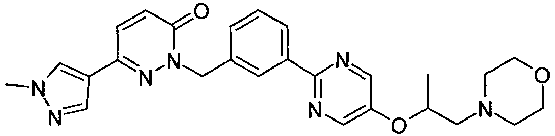 |
| "A248" | 2-{3-[5-(2-Dimethylamino-ethoxy)-pyrimidin-2-yl]-benzyl}-6-(1-methyl-1 H-pyrazol-4-yl)-2H-pyridazin-3-on |
| "A249" | 2-{3-[5-(2-Methyl-3-morpholin-4-yl-propoxy)-pyrimidin-2-yl]-benzyl}-6-(1-methyl-1H-pyrazol-4-yl)-2H-pyridazin-3-on |
| | 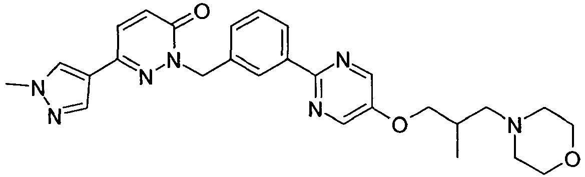 |
| "A250" | 6-(1-Methyl-1H-pyrazol-4-yl)-2-{3-[5-(2-pyrrolidin-1-yl-ethoxy)-pyrimidin-2-yl]-benzyl}-2H-pyridazin-3-on |
| "A251" | 2-[3-(5-Ethoxy-pyrimidin-2-yl)-benzyl]-6-(1-methyl-1H-pyrazol-4-yl)-2H-pyridazin-3-on |
| "A252" | 6-(1-Methyl-1H-pyrazol-4-yl)-2-{3-[5-(2-morpholin-4-yl-2-oxo-ethoxy)-pyrimidin-2-yl]-benzyl}-2H-pyridazin-3-on |
| | 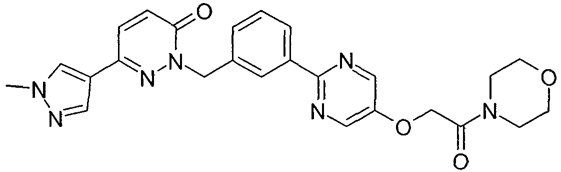 |
| "A253" | 6-(3-Chlor-phenyl)-2-{3-[5-(2-morpholin-4-yl-ethoxy)-pyrimidin-2-yl]-benzyl}-2H-pyridazin-3-on |
| "A254" | 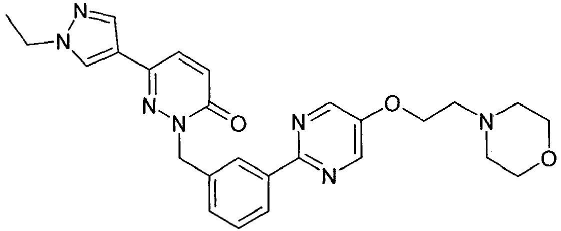 |
| "A255" | 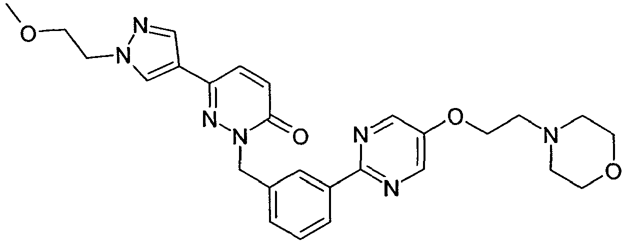 |
| "A256" | 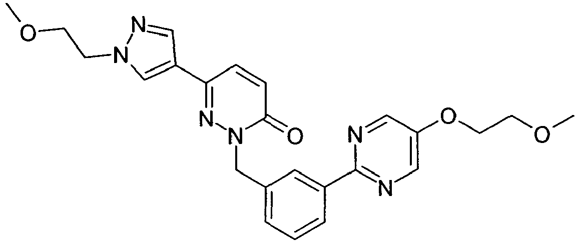 |
| "A257" | 3-(1-{3-[5-(1-Methyl-piperidin-4-ylmethoxy)-pyrimidin-2-yl]-benzyl}-6-oxo-1,6-dihydro-pyridazin-3-yl)-benzonitril |
| "A259" | 6-(3,5-Difluor-phenyl)-2-{3-[5-(1-piperidin-4-yl-1H-pyrazol-4-yl)-pyridin-2-yl]-benzyl}-2H-pyridazin-3-on |
| "A260" | 3-(1-{3-[5-(3-Dimethylamino-propoxy)-pyrimidin-2-yl]-benzyl}-6-oxo-1,6-dihydro-pyridazin-3-yl)-benzamid |
| "A261" | 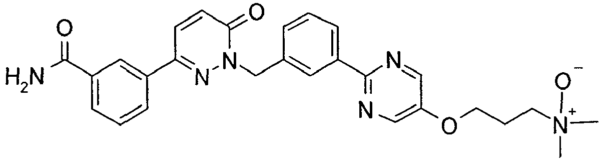 |
| "A264" | 3-(6-Oxo-1-{3-[5-(piperidin-4-ylmethoxy)-pyrimidin-2-yl]-benzyl}-1,6-dihydro-pyridazin-3-yl)-benzonitril |
| "A265" | 4-(2-{3-[3-(3-Cyan-phenyl)-6-oxo-6H-pyridazin-1-ylmethyl]-phenyl}-pyrimidin-5-yloxymethyl)-piperidin-1-carbonsäure-tert.-butylester |
| "A266" | 6-(1-Methyl-1H-pyrazol-4-yl)-2-{3-[5-(piperidin-4-yloxy)-pyrimidin-2-yl]-benzyl}-2H-pyridazin-3-on |
| "A267" | 6-(1-Methyl-1H-pyrazol-4-yl)-2-{3-[5-(piperidin-4-ylmethoxy)-pyrimidin-2-yl]-benzyl}-2H-pyridazin-3-on |
| "A268" | 3-(1-{3-[5-(3-Methylamino-propoxy)-pyrimidin-2-yl]-benzyl)-6-oxo-1,6-dihydro-pyridazin-3-yl)-benzonitril |
| | 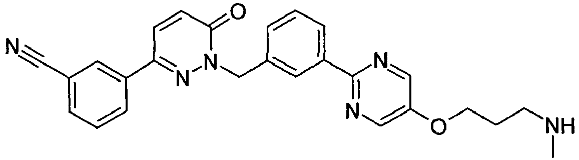 |
| "A269" | 3-[1-(3-{5-[2-(4-Methyl-3-oxo-piperazin-1-yl)-ethoxy]-pyrimidin-2-yl}-benzyl)-6-oxo-1,6-dihydro-pyridazin-3-yl]-benzonitril |
| | 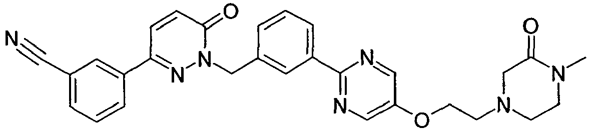 |
| "A270" | 3-[1-(3-{5-[2-(4-Methyl-piperazin-1-yl)-ethoxy]-pyrimidin-2-yl}-benzyl)-6-oxo-1,6-dihydro-pyridazin-3-yl]-benzonitril |
| "A271" | 3-(1-{3-[5-(2-Methoxy-ethoxy)-pyrimidin-2-yl]-benzyl}-6-oxo-1,6-dihydro-pyridazin-3-yl)-benzonitril |
| "A272" | 3-(1-{3-[5-(3-Methoxy-propoxy)-pyrimidin-2-yl]-benzyl}-6-oxo-1,6-dihydro-pyridazin-3-yl)-benzonitril |
| "A273" | 6-(3-Fluor-phenyl)-2-{3-[5-(1-methyl-piperidin-4-ylmethoxy)-pyrimidin-2-yl]-benzyl}-2H-pyridazin-3-on |
| "A274" | 2-{3-[5-(1-Methyl-piperidin-4-yloxy)-pyrimidin-2-yl]-benzyl}-6-(1-propyl-1H-pyrazol-4-yl)-2H-pyridazin-3-on |
| "A275" | 6-(3-Chlor-phenyl)-2-{3-[5-(1-methyl-piperidin-4-ylmethoxy)-pyrimidin-2-yl]-benzyl}-2H-pyridazin-3-on |
| "A276" | 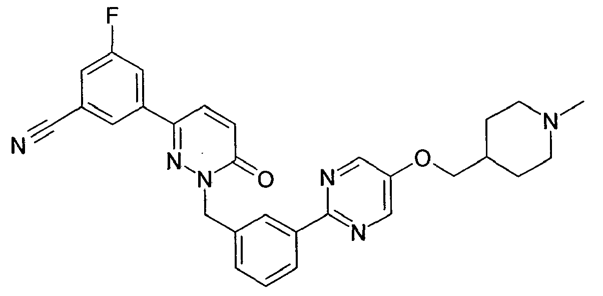 |
| "A276a" | 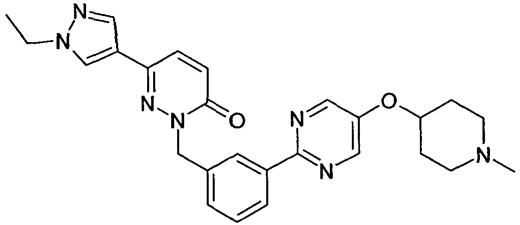 |
| "A277" | 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäure |
| "A278" | 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäureamid |
| "A279" | 5-(1-{3-[5-(1-Methyl-piperidin-4-yloxy)-pyrimidin-2-yl]-benzyl}-6-oxo-1,6-dihydro-pyridazin-3-yl)-thiophen-2-carbonsäure-methylamid |
| "A280" | 2-[3-(5-Amino-pyrazin-2-yl)-benzyl]-6-(3,5-difluor-phenyl)-2H-pyridazin-3-on |
| "A282" | 2-[3-(6-Amino-pyridazin-3-yl)-benzyl]-6-(3,5-difluor-phenyl)-2H-pyridazin-3-on |
| "A283" | (E)-3-(2-{3-[6-Oxo-3-(3,4,5-trifluor-phenyl)-6H-pyridazin-1-ylmethyl]-phenyl}-pyrimidin-5-yl)-acrylsäure-methylester |
| "A284" | 2-{3-[5-((E)-3-Amino-propenyl)-pyrimidin-2-yl]-benzyl}-6-(3,4,5-trifluor-phenyl)-2H-pyridazin-3-on |
| "A285" | 2-{3-[5-(3-Amino-propyl)-pyrimidin-2-yl]-benzyl}-6-(3,4,5-trifluor-phenyl)-2H-pyridazin-3-on |
| "A286" | 2-{3-[5-(4-Methyl-piperazin-1-yl)-pyrimidin-2-yl]-benzyl}-6-(1-methyl-1H-pyrazol-4-yl)-2H-pyridazin-3-on |
| "A287" | 3-(1-{3-[5-(4-Methyl-piperazin-1-yl)-pyrimidin-2-yl]-benzyl}-6-oxo-1,6-dihydro-pyridazin-3-yl)-benzonitril |
| "A288" | 3-{6-Oxo-1-[3-(5-piperazin-1-yl-pyrimidin-2-yl)-benzyl]-1,6-dihydro-pyridazin-3-yl}-benzonitril |
| "A289" | 6-(4-Methansulfonyl-phenyl)-2-[3-(5-piperazin-1-yl-pyrimidin-2-yl)-benzyl]-2H-pyridazin-3-on |
| "A290" | 4-{1-[3-(5-Amino-pyrimidin-2-yl)-benzyl]-6-oxo-1,6-dihydro-pyridazin-3-yl)-benzonitril |
| "A291" | 3-{1-[3-(5-Amino-pyrimidin-2-yl)-benzyl]-6-oxo-1,6-dihydro-pyridazin-3-yl}-benzonitril |
| "A292" | 6-(1-Methyl-1H-pyrazol-4-yl)-2-[3-(5-piperazin-1-yl-pyrimidin-2-yl)-benzyl]-2H-pyridazin-3-on |
| "A293" | 2-[3-(5-Amino-pyrimidin-2-yl)-benzyl]-6-(1-methyl-1H-pyrazol-4-yl)-2H-pyridazin-3-on |
| "A294" | 2-{3-[5-(2-Hydroxy-ethoxy)-pyrimidin-2-yl]-benzyl}-6-(1-methyl-1 H-pyrazol-4-yl)-2H-pyridazin-3-on |
| "A295" | 3-(1-{3-[5-(3-Hydroxy-propoxy)-pyrimidin-2-yl]-benzyl}-6-oxo-1,6-dihydro-pyridazin-3-yl)-benzonitril |
| "A296" | 3-(1-{3-[5-(2-Hydroxy-ethoxy)-pyrimidin-2-yl]-benzyl}-6-oxo-1,6-dihydro-pyridazin-3-yl)-benzonitril |
| "A297" | 2-{3-[5-(3-Hydroxy-propoxy)-pyrimidin-2-yl]-benzyl}-6-(1-methyl-1 H-pyrazol-4-yl)-2H-pyridazin-3-on |
| "A298" | 3-[1-(1-{3-[5-(3-Dimethylamino-propoxy)-pyrimidin-2-yl]-phenyl}-ethyl)-6-oxo-1,6-dihydro-pyridazin-3-yl]-benzonitril |
| "A299" | 6-(3,5-Difluor-phenyl)-2-(1-{3-[5-(3-dimethylamino-propoxy)-pyrimidin-2-yl]-phenyl}-ethyl)-2H-pyridazin-3-on |
| "A300" | 6-(3,5-Difluor-phenyl)-2-((R)-1-{3-[5-(3-dimethylamino-propoxy)-pyrimidin-2-yl]-phenyl}-ethyl)-2H-pyridazin-3-on |
| "A301" | 6-(3,5-Difluor-phenyl)-2-((S)-1-{3-[5-(3-dimethylamino-propoxy)-pyrimidin-2-yl]-phenyl}-ethyl)-2H-pyridazin-3-on |
| "A302" | 3-(1-{3-[5-(1-Methyl-1-oxy-piperidin-4-ylmethoxy)-pyrimidin-2-yl]-benzyl}-6-oxo-1,6-dihydro-pyridazin-3-yl)-benzonitril |
| "A303" | 3-(1-{3-[5-(1-Formyl-piperidin-4-ylmethoxy)-pyrimidin-2-yl]-benzyl}-6-oxo-1,6-dihydro-pyridazin-3-yl)-benzonitril |
| "A304" | 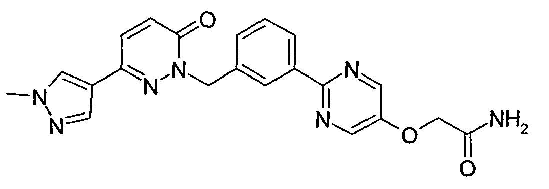 |
| "A305" | 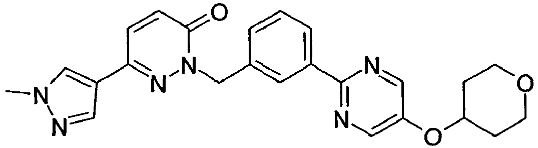 |
| "A306" | 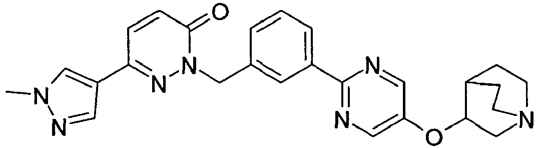 |
| "A307" | 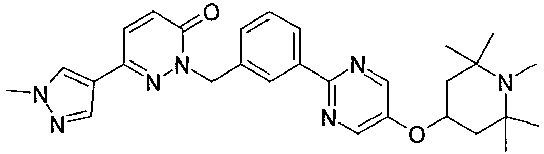 |
| "A308" | 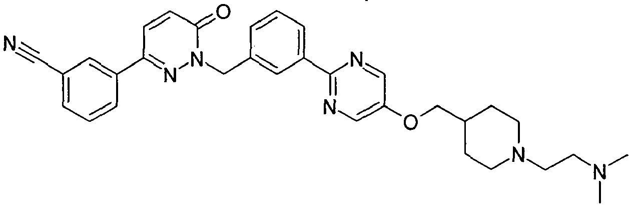 |
| "A309" | 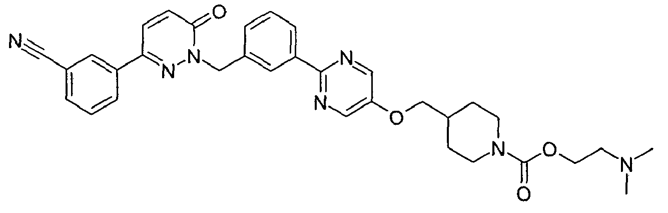 |
| "A310" | 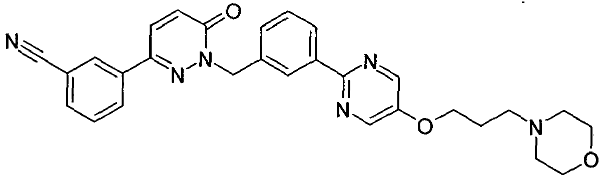 |
| "A311" | 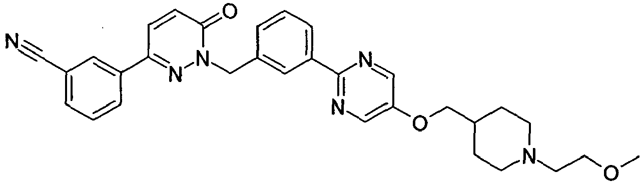 |
| "A312" | 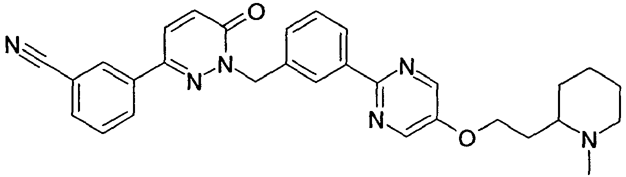 |
| "A313" | 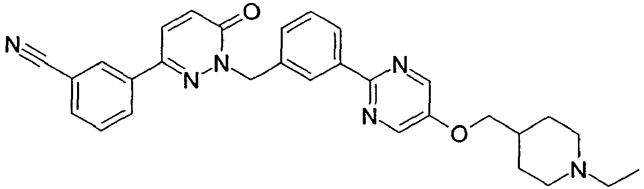 |
| "A314" | 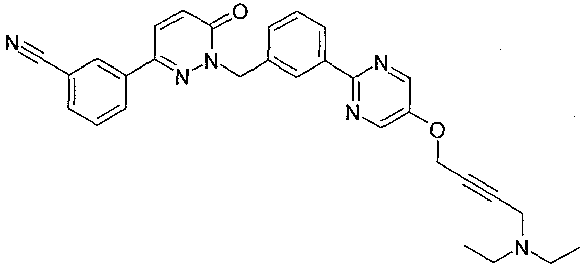 |

15. Arzneimittel nach einem oder mehreren der Ansprüche 1-14, wobei 1 mg bis 700 mg einer Verbindung der Formel I enthalten ist.

16. Arzneimittel nach Anspruch 15, wobei 5 mg bis 100 mg einer Verbindung der Formel I enthalten ist.

## Claims

1. Medicament comprising at least one compound of the formula I 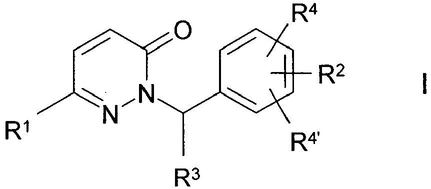 in which
R¹ denotes Ar or Het,
R² denotes an unsaturated, saturated or aromatic 6-membered heterocycle having 1 to 4 N, O and/or S atoms, which is mono-, di- or trisubstituted by N=CR³N(R³)₂, SR³, NO₂, CN, COOR³, CON(R³)₂, NR³COA, NR³SO₂A, SO₂N(R³)₂, S(O)ₘA, [C(R³)₂]ₙN(R³)₂, [C(R³)₂]ₙHet, O[C(R³)₂]ₚOR³, O[C(R³)₂]ₙN(R³)₂, O[C(R³)₂]ₙC≡C[C(R³)₂]ₙN(R³)₂, O[C(R³)₂]ₙN⁺O⁻(R³)₂, O[C(R³)₂]ₙHet, S[C(R³)₂]ₙN(R³)₂, S[C(R³)₂]ₙHet, NR³[C(R³)₂]ₙN(R³)₂, NR³[C(R³)₂]ₙHet, NHCON(R³)₂, NHCONH[C(R³)₂]ₙN(R³)₂, NHCONH[C(R³)₂]ₙHet, [C(R³)₂]ₙNHCON[C(R³)₂]ₙ(R³)₂, [C(R³)₂]ₙNHCON[C(R³)₂]ₙHet, CON(R³)₂, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, COA, CH=CH-COOR³ and/or CH=CH-N(R³)₂,
R³ denotes H or A,
R⁴, R^{4'} each, independently of one another, denote H, Hal, A, OR³, CN, COOR³, CON(R³)₂, NR³COA, NR³SO₂A, SO₂N(R³)₂ or S(O)ₘA,
Ar denotes phenyl, naphthyl or biphenyl, each of which is unsubstituted or mono-, di- or trisubstituted by Hal, A, [C(R³)₂]ₙOR³, [C(R³)₂]ₙN(R³)₂, SR³, NO₂, CN, COOR³, CON(R³)₂, NR³COA, NR³SO₂A, SO₂N(R³)₂, S(O)ₘA, CO-Het, Het, O[C(R³)₂]ₙN(R³)₂, O[C(R³)₂]ₙHet, NHCOOA, NHCON(R³)₂, NHCOO[C(R³)₂]ₙN(R³)₂, NHCOO[C(R³)₂]ₙHet, NHCONH[C(R³)₂]ₙN(R³)₂, NHCONH[C(R³)₂]ₙHet, OCONH[C(R³)₂]ₙN(R³)₂, OCONH[C(R³)₂]ₙHet, CONR³[C(R³)₂]ₙN(R³)₂, CONR³[C(R³)₂]ₙHet and/or COA,
Het denotes a mono-, bi- or tricyclic saturated, unsaturated or aromatic heterocycle having 1 to 4 N, O and/or S atoms, which may be unsubstituted or mono-, di-, tri-, tetra- or pentasubstituted by Hal, A, [C(R³)₂]ₙOR³, [C(R³)₂]ₙN(R³)₂, SR³, NO₂, CN, COOR³, CON(R³)₂, NR³COA, NR³SO₂A, SO₂N(R³)₂, S(O)ₘA, CO-Het¹, [C(R³)₂]ₙHet¹, O[C(R³)₂]ₙN(R³)₂, O[C(R³)₂]ₙHet¹, NHCOOA, NHCON(R³)₂, NHCOO[C(R³)₂]ₙN(R³)₂, NHCOO[C(R³)₂]ₙHet¹, NHCONH[C(R³)₂]ₙN(R³)₂, NHCONH[C(R³)₂]ₙHet¹, OCONH[C(R³)₂]ₙN(R³)₂, OCONH[C(R³)₂]ₙHet¹, CO-Het¹, CHO, COA, =S, =NH, =NA and/or =O (carbonyl oxygen), and where a ring nitrogen may be oxidised,
Het¹ denotes a monocyclic saturated heterocycle having 1 to 2 N and/or O atoms, which may be mono- or disubstituted by A, OA, OH, Hal and/or =O (carbonyl oxygen),
A denotes unbranched or branched alkyl having 1-10 C atoms, in which 1-7 H atoms may be replaced by F and/or in which one or two non-adjacent CH₂ groups may be replaced by O, NH, S, SO, SO₂ and/or by CH=CH groups, or cyclic alkyl having 3-7 C atoms,
Hal denotes F, Cl, Br or I,
m denotes 0, 1 or 2,
n denotes 0, 1, 2, 3 or 4,
p denotes 1, 2, 3 or 4,
and/or pharmaceutically usable solvates, salts, tautomers and stereoisomers thereof, including mixtures thereof in all ratios, and optionally excipients and/or adjuvants, where 0.5 mg to 1 g of a compound of the formula I is present.

2. Medicament according to Claim 1 in which
R² denotes an unsaturated, saturated or aromatic 6-membered heterocycle having 1 to 4 N and/or O atoms, which is mono-, di- or trisubstituted by N=CR³N(R³)₂, CN, COOR³, [C(R³)₂]ₙN(R³)₂, [C(R³)₂]ₙHet, O[C(R³)₂]ₚOR³, O[C(R³)₂]ₙN(R³)₂, O[C(R³)₂]ₙC≡C[C(R³)₂]ₙN(R³)₂, O[C(R³)₂]ₙN⁺O⁻(R³)₂, O[C(R³)₂]ₙHet, NR³[C(R³)₂]ₙN(R³)₂, NR³[C(R³)₂]ₙHet, [C(R³)₂]ₙNHCO[C(R³)₂]ₙN(R³)₂, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ and/or CH=CH-N(R³)₂.

3. Medicament according to Claim 1 or 2 in which
Ar denotes phenyl, naphthyl or biphenyl, each of which is unsubstituted or mono-, di- or trisubstituted by A, Hal, CN, S(O)ₘA, NR³COA, CON(R³)₂, O[C(R³)₂]ₙN(R³)₂, [C(R³)₂]ₙOR³, CONR³[C(R³)₂]ₙN(R³)₂ and/or CONR³[C(R³)₂]ₙHet,
and pharmaceutically usable derivatives, solvates, salts, tautomers
and stereoisomers thereof, including mixtures thereof in all ratios.

4. Medicament according to one or more of Claims 1-3 in which
R⁴, R^{4'} denote H.

5. Medicament according to one or more of Claims 1-4 in which
Het denotes a mono-, bi- or tricyclic saturated, unsaturated or aromatic heterocycle having 1 to 4 N, O and/or S atoms, which may be unsubstituted or mono-, di-, tri-, tetra- or pentasubstituted by A, CHO, COOR³, CON(R³)₂, [C(R³)₂]ₙHet¹, [C(R³)₂]ₙOR³, [C(R³)₂]ₙN(R³)₂, O[C(R³)₂]ₙHet¹ and/or =O (carbonyl oxygen), and where a ring nitrogen may be oxidised.

6. Medicament according to one or more of Claims 1-5 in which
Het¹ denotes a monocyclic saturated heterocycle having 1 to 2 N and/or O atoms, which may be mono- or disubstituted by A and/or =O (carbonyl oxygen).

7. Medicament according to one or more of Claims 1-6 in which
A denotes unbranched or branched alkyl having 1-8 C atoms, in which 1-7 H atoms may be replaced by F.

8. Medicament according to one or more of Claims 1-7 in which
R¹ denotes Ar or benzo-2,1,3-thiadiazolyl.

9. Medicament according to one or more of Claims 1-8 in which
R³ denotes H, methyl, ethyl or propyl.

10. Medicament according to one or more of Claims 1-9 in which
R² denotes pyrimidinyl, pyridazinyl, pyridinyl, 1,3-oxazinanyl, morpholinyl, piperidinyl or piperazinyl, each of which is mono-, di- or trisubstituted by N=CR³N(R³)₂, CN, COOR³, [C(R³)₂]ₙN(R³)₂, [C(R³)₂]ₙHet, O[C(R³)₂]ₚOR³, O(C(R³)₂]ₙN(R³)₂,
O[C(R³)₂]ₙC≡C[C(R³)₂]ₙN(R³)₂, O[C(R³)₂]ₙN⁺O⁻(R³)₂, O[C(R³)₂]ₙHet, NR³[C(R³)₂]ₙN(R³)₂, NR³[C(R³)₂]ₙHet, [C(R³)₂]ₙNHCO[C(R³)₂]ₙN(R³)₂, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ and/or CH=CH-N(R³)₂.

11. Medicament according to one or more of Claims 1-10 in which
Het denotes piperidinyl, piperazinyl, pyrrolidinyl, morpholinyl, furyl, thienyl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazolyl, pyridazinyl, pyrazinyl, benzimidazolyl, benzotriazolyl, indolyl, benzo1,3-dioxolyl, indazolyl, azabicyclo[3.2.1]octyl, azabicyclo[2.2.2]octyl, imidazolidinyl, azepanyl or benzo2,1,3-thiadiazolyl, each of which is unsubstituted or mono-, di-, tri-, tetra- or pentasubstituted by A, CHO, COOR³, CON(R³)₂, [C(R³)₂]ₙHet¹, [C(R³)₂]ₙOR³, [C(R³)₂]ₙN(R³)₂, O[C(R³)₂]ₙHet¹ and/or =O (carbonyl oxygen), and where a ring nitrogen may be oxidised.

12. Medicament according to one or more of Claims 1-11 in which
Het¹ denotes pyrrolidinyl, piperidinyl, piperazinyl or morpholinyl, each of which is unsubstituted or mono- or disubstituted by A and/or =O (carbonyl oxygen).

13. Medicament according to one or more of Claims 1-12 in which
R¹ denotes Ar or Het,
R² denotes pyrimidinyl, pyridazinyl, pyridinyl, 1,3-oxazinanyl, morpholinyl, piperidinyl or piperazinyl, each of which is mono-, di- or trisubstituted by N=CR³N(R³)₂, CN, COOR³, [C(R³)₂]ₙN(R³)₂, [C(R³)₂]ₙHet, O[C(R³)₂]ₚOR³, O[C(R³)₂]ₙN(R³)₂, O[C(R³)₂]ₙC≡C[C(R³)₂]ₙN(R³)₂, O[C(R³)₂]ₙN⁺O⁻(R³)₂, O[C(R³)₂]ₙHet, NR³[C(R³)₂]ₙN(R³)₂, NR³[C(R³)₂]ₙHet, [C(R³)₂]ₙNHCO[C(R³)₂]ₙN(R³)₂, [C(R³)₂]ₙNHCO[C(R³)₂]ₙHet, CONR³[C(R³)₂]ₙN(R³)₂, CONR³[C(R³)₂]ₙNR³COOA, CONR³[C(R³)₂]ₙOR³, CONR³[C(R³)₂]ₙHet, COHet, CH=CH-COOR³ and/or CH=CH-N(R³)₂,
R³ denotes H, methyl, ethyl or propyl,
R⁴, R^{4'} denote H,
Ar denotes phenyl, naphthyl or biphenyl, each of which is unsubstituted or mono-, di- or trisubstituted by A, Hal, CN, S(O)ₘA, NR³COA, CON(R³)₂, O[C(R³)₂]ₙN(R³)₂, [C(R³)₂]ₙOR³, CONR³[C(R³)₂]ₙN(R³)₂ and/or CONR³[C(R³)₂]ₙHet,
Het denotes piperidinyl, piperazinyl, pyrrolidinyl, morpholinyl, furyl, thienyl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazolyl, pyridazinyl, pyrazinyl, benzimidazolyl, benzotriazolyl, indolyl, benzo1,3-dioxolyl, indazolyl, azabicyclo[3.2.1]octyl, azabicyclo[2.2.2]octyl, imidazolidinyl, azepanyl or benzo2,1,3-thiadiazolyl, each of which is unsubstituted or mono-, di-, tri-, tetra- or pentasubstituted by A, CHO, COOR³, CON(R³)₂, [C(R³)₂]ₙHet¹, [C(R³)₂]ₙOR³, [C(R³)₂]ₙN(R³)₂, O[C(R³)₂]ₙHet¹ and/or =O (carbonyl oxygen), and where a ring nitrogen may be oxidised,
Het¹ denotes pyrrolidine, piperidine, piperazine or morpholine, each of which is unsubstituted or mono- or disubstituted by A and/or =O (carbonyl oxygen),
A denotes unbranched or branched alkyl having 1-8 C atoms, in which 1-7 H atoms may be replaced by F,
Hal denotes F, Cl, Br or I,
m denotes 0, 1 or 2,
n denotes 0, 1, 2, 3 or 4,
p denotes 1, 2, 3 or 4.

14. Medicament according to Claim 1, comprising at least one compound of the formula I selected from the group
| No. | Name and/or structure |
|---|---|
| "A11" | 2-[3-(5-aminopyridin-2-yl)benzyl]-6-(3,5-difluorophenyl)-2H-pyridazin-3-one |
| "A12" | 6-(3,5-difluorophenyl)-2-{3-[5-(4-methylpiperazin-1-yl)-pyridin-2-yl]benzyl}-2H-pyridazin-3-one |
| "A13" | 6-(3,5-difluorophenyl)-2-[3-(4-piperazin-1-ylpyrimidin-2-yl)-benzyl]-2H-pyridazin-3-one |
| "A14" | 6-(3,5-difluorophenyl)-2-{3-[5-(4-methylpiperazin-1-yl-methyl)pyrimidin-2-yl]benzyl}-2H-pyridazin-3-one |
| "A16" | N'-(2-{3-[3-(3,5-difluorophenyl)-6-oxo-6H-pyridazin-1-yl-methyl]phenyl}pyrimidin-5-yl)-N,N-dimethylformamidine |
| "A17" | 2-[3-(5-aminopyrimidin-2-yl)benzyl]-6-(3,5-difluorophenyl)-2H-pyridazin-3-one |
| "A18" | 6-(3,5-difluorophenyl)-2-{3-[5-(4-methylpiperazin-1-yl)-pyrimidin-2-yl]benzyl}-2H-pyridazin-3-one |
| "A 19" | 6-(3,5-difluorophenyl)-2-[3-(5-piperazin-1-ylpyrimidin-2-yl)-benzyl]-2H-pyridazin-3-one |
| "A20" | 2-{3-[5-(4-methylpiperazin-1-yl)pyrimidin-2-yl]benzyl}-6-(3,4,5-trifluorophenyl)-2H-pyridazin-3-one |
| "A22" | 6-(3,5-difluorophenyl)-2-{3-[5-(3-dimethylaminopropoxy)-pyrimidin-2-yl]benzyl}-2H-pyridazin-3-one |
| "A23" | 6-(3,5-difluorophenyl)-2-{3-[5-(1-methylpiperidin-4-yloxy)-pyrimidin-2-yl]benzyl}-2H-pyridazin-3-one |
| | 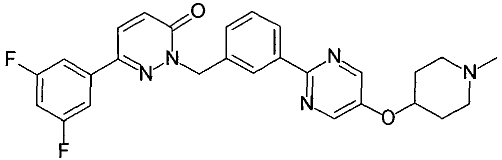 |
| "A24" | 6-(3,5-difluorophenyl)-2-{3-[5-(3-dimethylaminopropoxy)-pyridin-2-yl]benzyl}-2H-pyridazin-3-one |
| "A26" | 6-(3,5-difluorophenyl)-2-{3-[5-(1-methyl-1H-pyrazol-4-yl)-pyrimidin-2-yl]benzyl}-2H-pyridazin-3-one |
| "A27" | 6-(3,5-difluorophenyl)-2-{3-[6-(4-methylpiperazin-1-yl)-pyridazin-3-yl]benzyl}-2H-pyridazin-3-one |
| "A28" | 6-(3,5-difluorophenyl)-2-{3-[6-(3-dimethylaminopropoxy)-pyridazin-3-yl]benzyl}-2H-pyridazin-3-one |
| "A29" | ethyl 2-{3-[6-oxo-3-(3,4,5-trifluorophenyl)-6H-pyridazin-1-ylmethyl]phenyl}pyrimidine-5-carboxylate |
| "A30" | ethyl 2-{3-[3-(3,5-difluorophenyl)-6-oxo-6H-pyridazin-1-yl-methyl]phenyl}pyrimidine-5-carboxylate |
| | 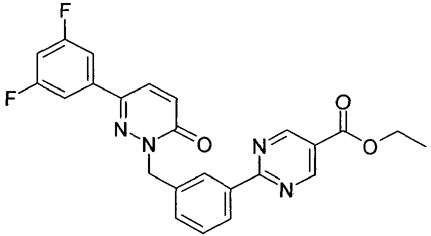 |
| "A31" | 2-{3-[6-oxo-3-(3,4,5-trifluorophenyl)-6H-pyridazin-1-yl-methyl]phenyl}pyrimidine-5-carboxylic acid |
| "A32" | 2-{3-[3-(3,5-difluorophenyl)-6-oxo-6H-pyridazin-1-ylmethyl]-phenyl}pyrimidine-5-carboxylic acid |
| "A33" | N-(2-dimethylaminoethyl)-2-{3-[6-oxo-3-(3,4,5-trifluoro-phenyl)-6H-pyridazin-1-ylmethyl]phenyl}pyrimidine-5-carboxamide |
| "A34" | 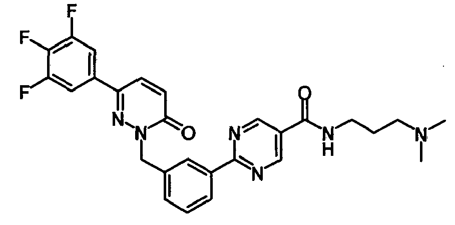 |
| "A35" | 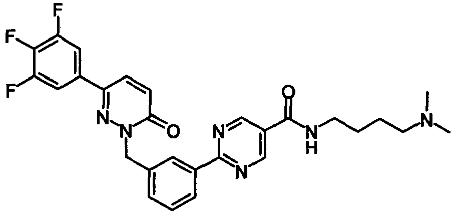 |
| "A36" | 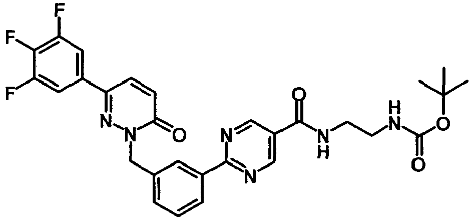 |
| "A37" | 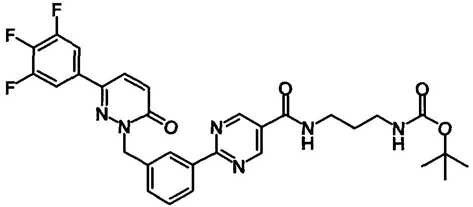 |
| "A38" | 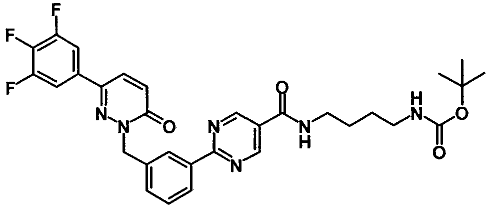 |
| "A39" | 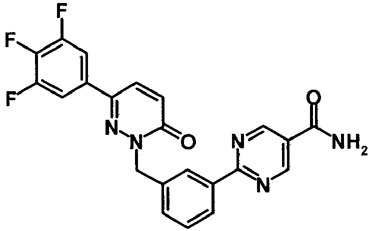 |
| "A40" | 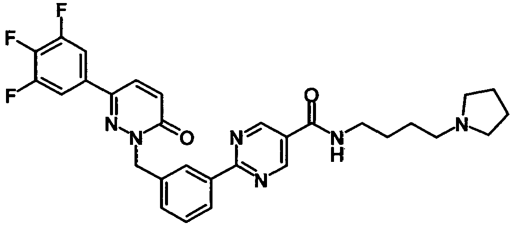 |
| "A41" | 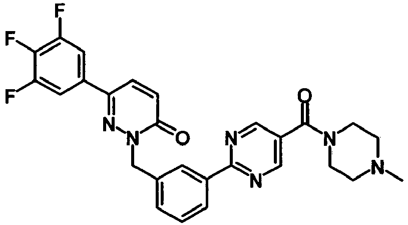 |
| "A42" | 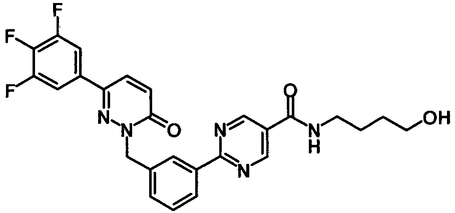 |
| "A43" | 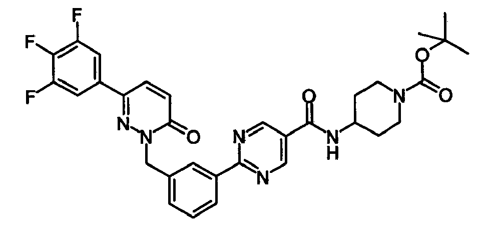 |
| "A44" | 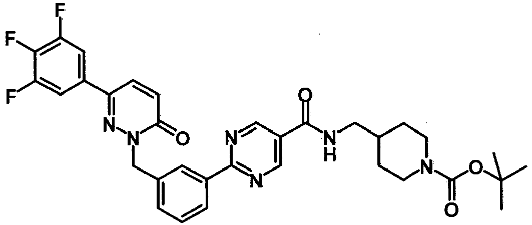 |
| "A44a" | 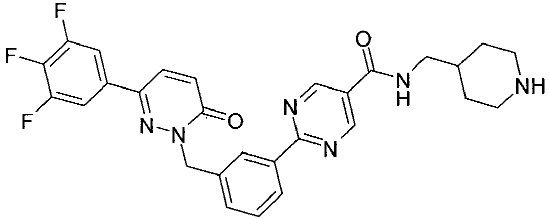 |
| "A45" | 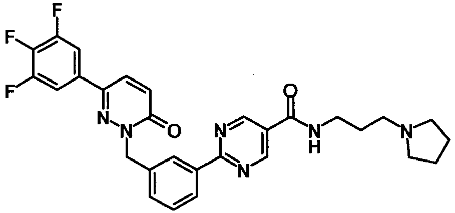 |
| "A46" | 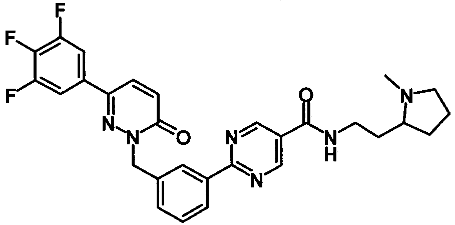 |
| "A47" | 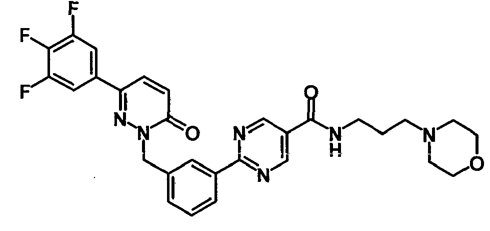 |
| "A48" | 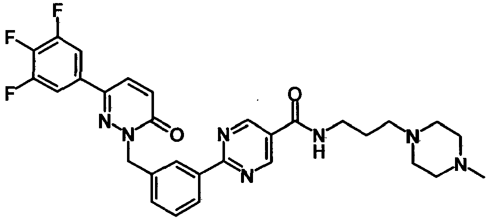 |
| "A49" | 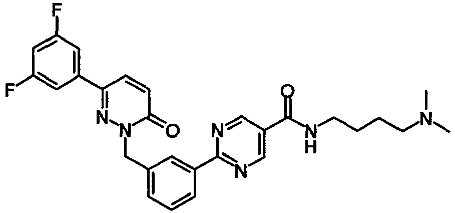 |
| "A50" | 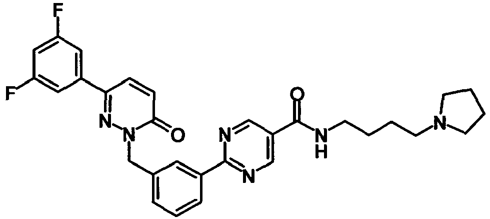 |
| "A51" | N-[2-(1H-imidazol-4-yl)ethyl]-2-{3-[6-oxo-3-(3,4,5-trifluoro-phenyl)-6H-pyridazin-1-ylmethyl]phenyl}pyrimidine-5-carboxamide |
| "A52" | 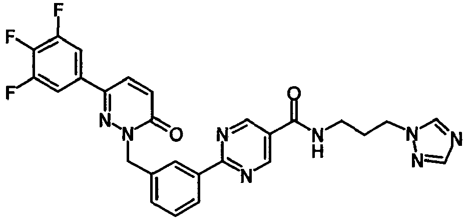 |
| "A53" | 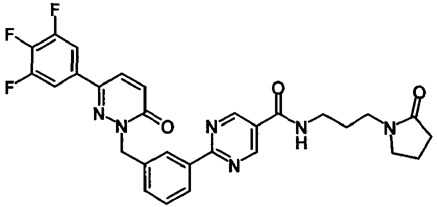 |
| "A54" | 2-[3-(5-chloropyrimidin-2-yl)benzyl]-6-(3,4,5-trifluorophenyl)-2H-pyridazin-3-one |
| "A55" | 4-{1-[3-(5-methylpyrimidin-2-yl)benzyl]-6-oxo-1,6-dihydro-pyridazin-3-yl}-N-(3-piperidin-1-ylpropyl)benzamide |
| "A56" | 6-(3,5-difluorophenyl)-2-{3-[5-(3-pyrrolidin-1-ylpropoxy)-pyrimidin-2-yl]benzyl}-2H-pyridazin-3-one |
| "A57" | 6-(3,5-difluorophenyl)-2-(3-{5-[2-(4-methylpiperazin-1-yl)-ethoxy]pyrimidin-2-yl}benzyl)-2H-pyridazin-3-one |
| "A58" | 6-(3,5-difluorophenyl)-2-[3-(5-dimethylaminomethyl-pyrimidin-2-yl)benzyl]-2H-pyridazin-3-one |
| "A59" | 6-(3,5-difluorophenyl)-2-{3-[4-(methylpiperidin-4-yl-amino)pyrimidin-2-yl]benzyl}-2H-pyridazin-3-one |
| | 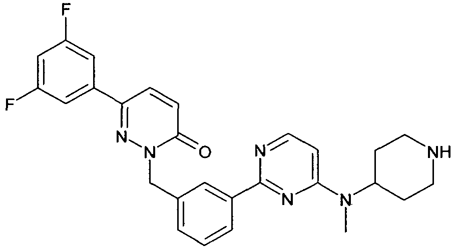 |
| "A60" | 2-{3-[5-(3-dimethylaminopropoxy)pyrimidin-2-yl]benzyl}-6-(3,4,5-trifluorophenyl)-2H-pyridazin-3-one |
| "A63" | 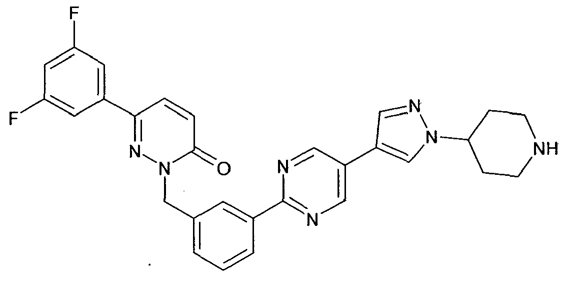 |
| "A64" | 2-{3-[5-(2-dimethylaminoethoxy)pyrimidin-2-yl]benzyl}-6-(3,5-difluorophenyl)-2H-pyridazin-3-one |
| "A65" | 2-{3-[5-(piperazin-1-yl)pyrimidin-2-yl]benzyl}-6-(3,4,5-trifluorophenyl)-2H-pyridazin-3-one |
| "A66" | 6-(3,5-difluorophenyl)-2-{3-[5-(1-methylpiperidin-4-yl-methoxy)pyrimidin-2-yl]benzyl}-2H-pyridazin-3-one |
| "A67" | 6-(3,5-difluorophenyl)-2-{3-[6-(3-dimethylaminopropyl-amino)pyridazin-3-yl]benzyl}pyridazin-3-one |
| "A68" | 6-(3,5-difluorophenyl)-2-{3-[6-(2-dimethylaminoethylamino)-pyridazin-3-yl]benzyl}pyridazin-3-one |
| "A69" | 6-(3,5-difluorophenyl)-2-{3-[6-(4-dimethylaminobutylamino)-pyridazin-3-yl]benzyl}pyridazin-3-one |
| "A70" | 6-(3,5-difluorophenyl)-2-{3-[6-(1-methylpiperidin-4-ylamino)-pyridazin-3-yl]benzyl}pyridazin-3-one |
| "A71" | 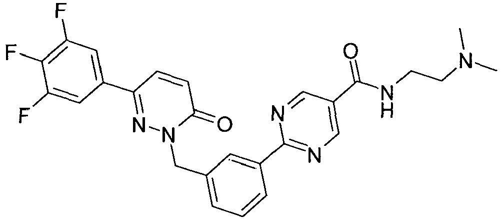 |
| "A72" | 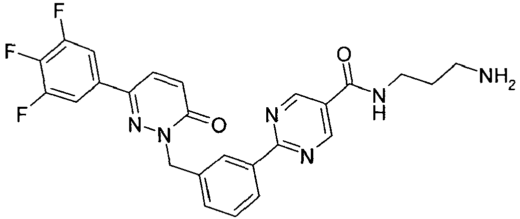 |
| "A73" | 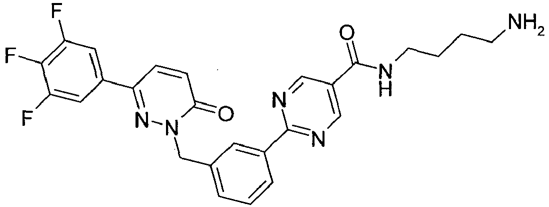 |
| "A74" | 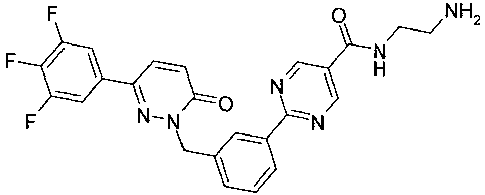 |
| "A75" | 4-(2-{3-[3-(3,5-difluorophenyl)-6-oxo-6H-pyridazin-1-yl-methyl]phenyl}pyrimidin-5-yl)morpholin-3-one |
| | 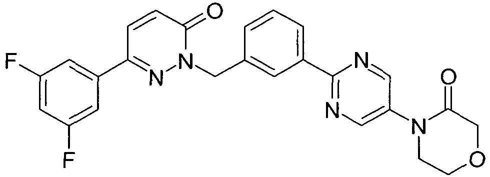 |
| "A76" | N'-(2-{3-[3-(3,4,5-trifluorophenyl)-6-oxo-6H-pyridazin-1-yl-methyl]phenyl}pyrimidin-5-yl)-N,N-dimethylformamidine |
| "A77" | 2-{3-[6-oxo-3-(3,4,5-trifluorophenyl)-6H-pyridazin-1-yl-methyl]phenyl}pyrimidine-5-carbonitrile |
| | 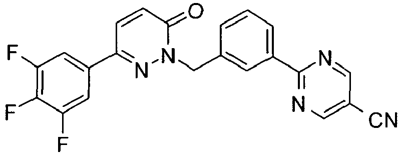 |
| "A81" | 6-(3,5-difluorophenyl)-2-{3-[4-(3-dimethylaminopropoxy)-pyrimidin-2-yl]benzyl}-2H-pyridazin-3-one |
| "A82" | 2-[3-(5-aminopyrimidin-2-yl)benzyl]-6-(3,4,5-trifluorophenyl)-2H-pyridazin-3-one |
| "A84" | 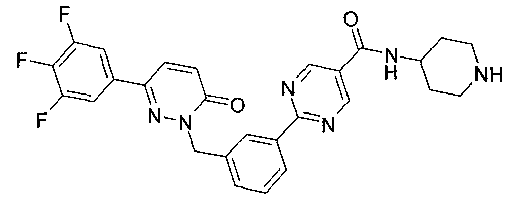 |
| "A85" | N-(2-{3-[3-(3,5-difluorophenyl)-6-oxo-6H-pyridazin-1-yl-methyl]phenyl}pyrimidin-5-yl)-2-dimethylaminoacetamide |
| | 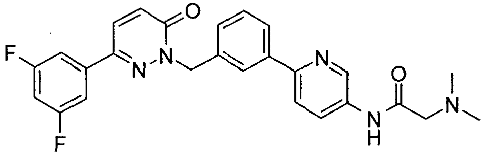 |
| "A87" | N-(2-{3-[3-(3,5-difluorophenyl)-6-oxo-6H-pyridazin-1-yl-methyl]phenyl}pyrimidin-5-yl)-4-dimethylaminobutyramide |
| | 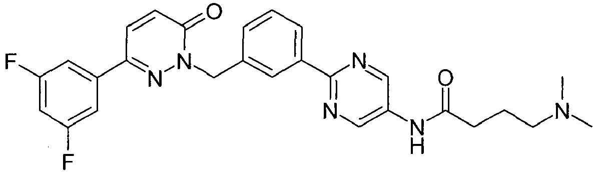 |
| "A88" | 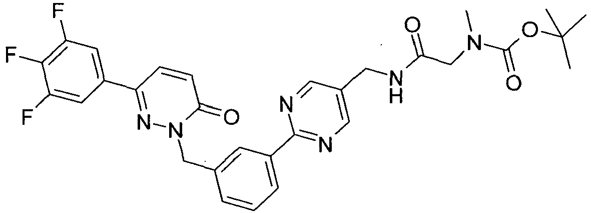 |
| "A89" | 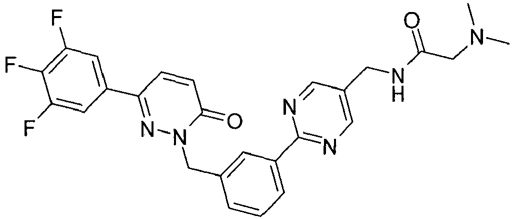 |
| "A90" | 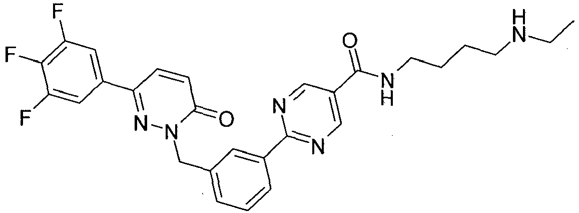 |
| | |
| "A92" | 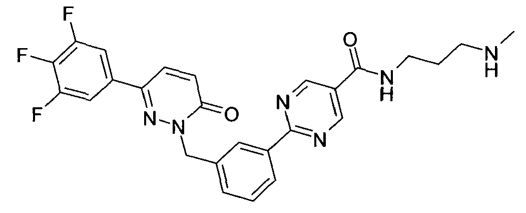 |
| "A93" | 2-[3-(5-aminomethylpyrimidin-2-yl)benzyl]-6-(3,4,5-trifluoro-phenyl)-2H-pyridazin-3-one |
| "A95" | N-(2-{3-[3-(3,5-difluorophenyl)-6-oxo-6H-pyridazin-1-yl-methyl]phenyl}pyrimidin-5-yl)-3-dimethylaminopropion-amide |
| | 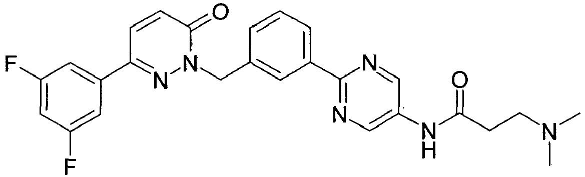 |
| "A96" | 3-(4-methylpiperazin-1-yl)-N-(2-{3-[6-oxo-3-(3,4,5-trifluoro-phenyl)-6H-pyridazin-1-ylmethyl]phenyl}pyrimidin-5-yl-methyl)propionamide |
| | 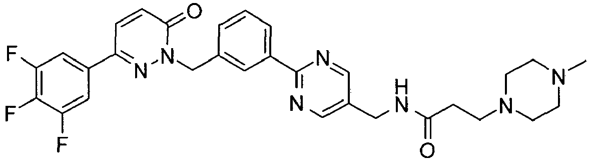 |
| "A97" | 2-(4-methylpiperazin-1-yl)-N-(2-{3-[6-oxo-3-(3,4,5-trifluoro-phenyl)-6H-pyridazin-1-ylmethyl]phenyl}pyrimidin-5-yl-methyl)acetamide |
| | 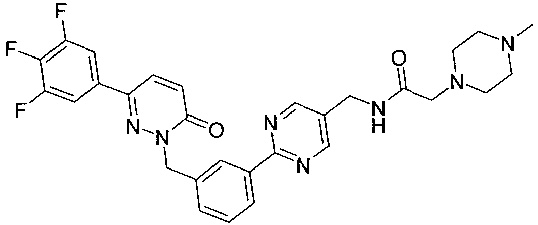 |
| "A98" | 2-methylamino-N-(2-{3-[6-oxo-3-(3,4,5-trifluorophenyl)-6H-pyridazin-1-ylmethyl]phenyl}pyrimidin-5-ylmethyl)acetamide |
| | 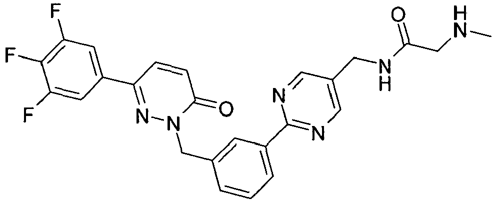 |
| "A99" | 3-dimethylamino-N-(2-{3-[6-oxo-3-(3,4,5-trifluorophenyl)-6H-pyridazin-1-ylmethyl]phenyl}pyrimidin-5-ylmethyl)propion-amide |
| | 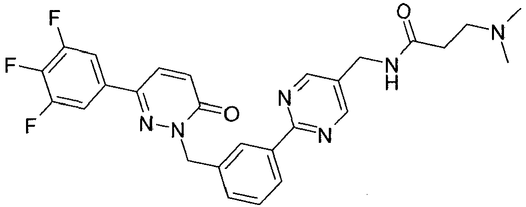 |
| "A101" | 6-(3,5-difluorophenyl)-2-[3-(5-hydroxymethylpyrimidin-2-yl)-benzyl]-2H-pyridazin-3-one |
| "A102" | 6-(3,5-difluorophenyl)-2-{3-[5-(piperidin-4-yloxy)pyrimidin-2-yl]benzyl}-2H-pyridazin-3-one |
| "A103" | 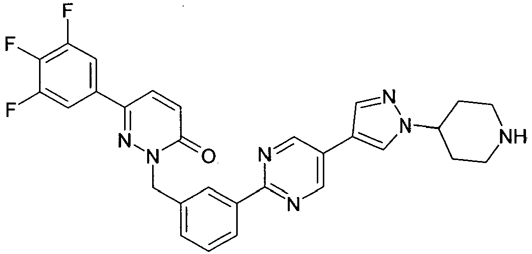 |
| "A104" | 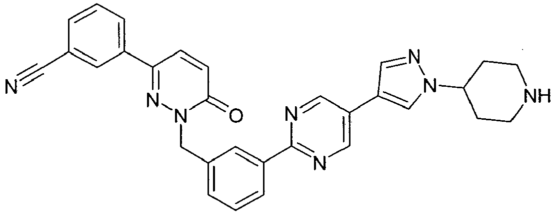 |
| "A105" | 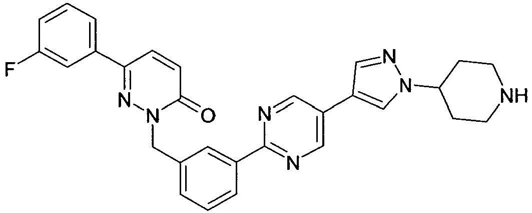 |
| "A106" | 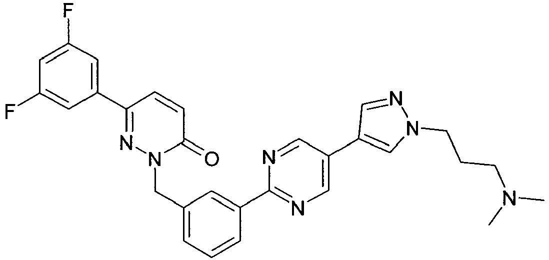 |
| "A107" | 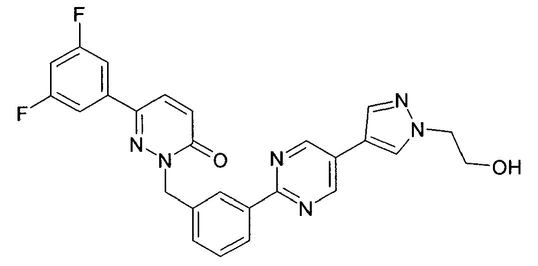 |
| "A108" | 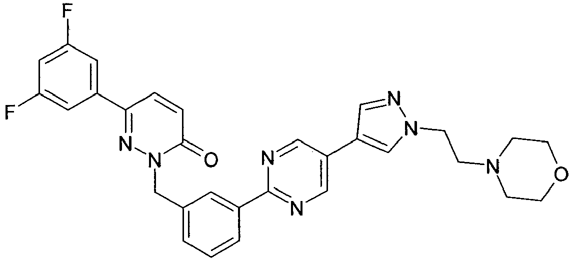 |
| "A109" | 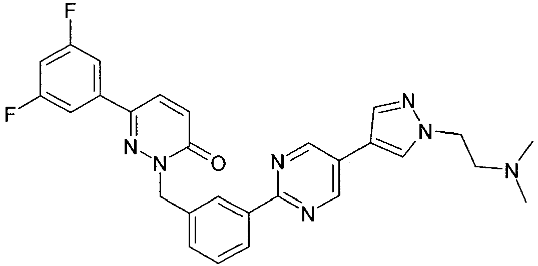 |
| "A110" | 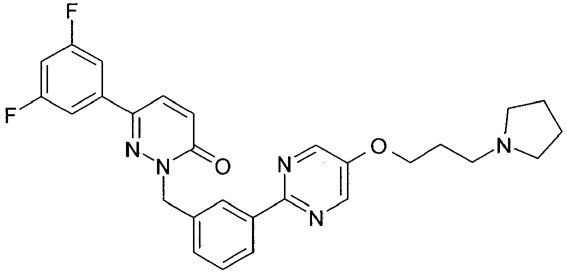 |
| "A111" | 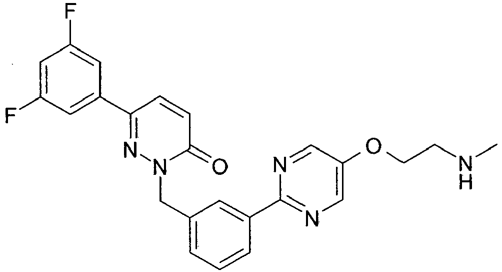 |
| "A114" | 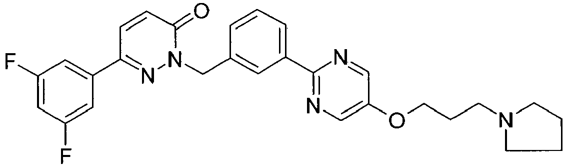 |
| "A115" | 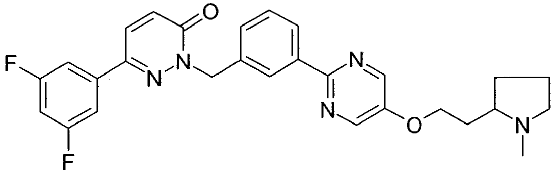 |
| "A116" | 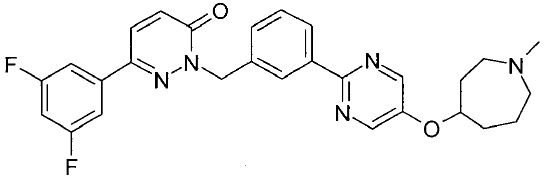 |
| "A117" | 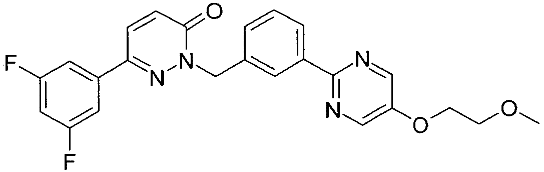 |
| "A118" | 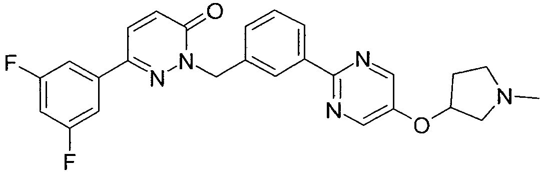 |
| "A119" | 6-(3,5-difluorophenyl)-2-{3-[5-(8-methyl-8-azabicyclo-[3.2.1]oct-3-yloxy)pyrimidin-2-yl]benzyl}-2H-pyridazin-3-one |
| | 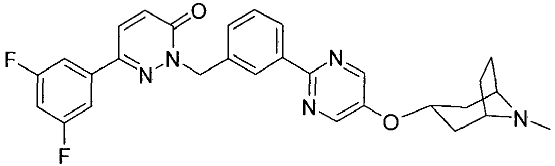 |
| "A120" | 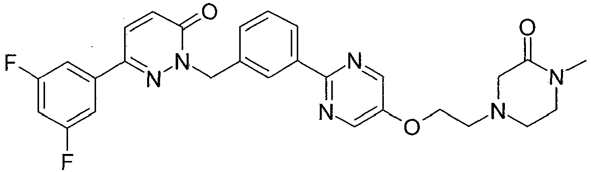 |
| "A121" | 6-(3,5-difluorophenyl)-2-{3-[5-((S)-1-methylpyrrolidin-3-yl-oxy)pyrimidin-2-yl]benzyl}-2H-pyridazin-3-one |
| | 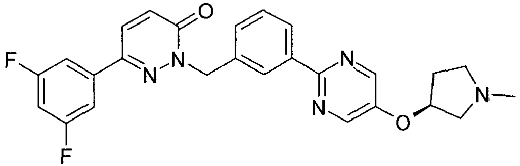 |
| "A122" | 6-(3,5-difluorophenyl)-2-{3-[5-((R)-1-methylpyrrolidin-3-yl-oxy)pyrimidin-2-yl]benzyl}-2H-pyridazin-3-one |
| "A123" | 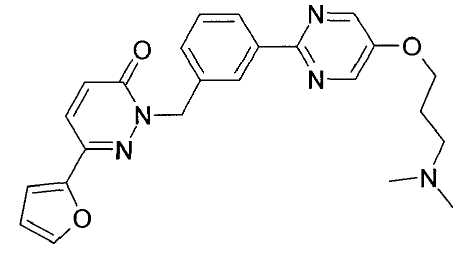 |
| "A124" | 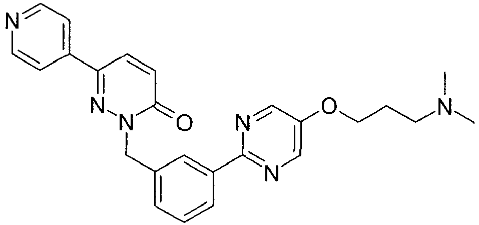 |
| "A125" | 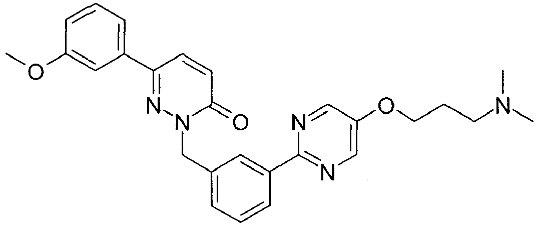 |
| "A126" | 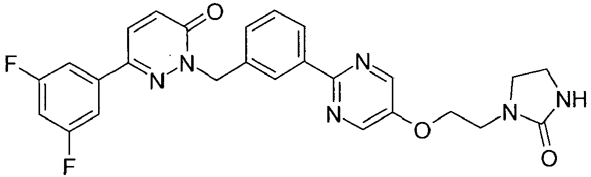 |
| "A127" | 6-(3,5-difluorophenyl)-2-{3-[5-(2-pyrrolidin-1-ylethoxy)-pyrimidin-2-yl]benzyl}-2H-pyridazin-3-one |
| "A128" | 6-(3,5-difluorophenyl)-2-{3-[5-(3-morpholin-4-ylpropoxy)-pyrimidin-2-yl]benzyl}-2H-pyridazin-3-one |
| "A129" | 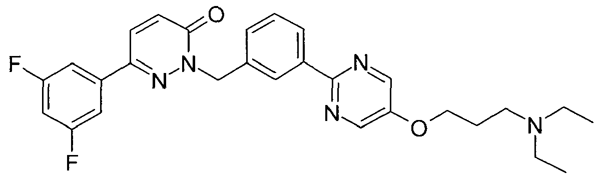 |
| "A130" | 6-(3,5-difluorophenyl)-2-{3-[5-(2-morpholin-4-ylethoxy)-pyrimidin-2-yl]benzyl}-2H-pyridazin-3-one, hydrochloride |
| "A131" | 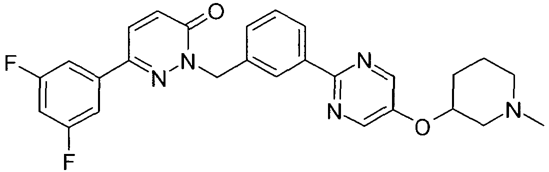 |
| "A132" | 6-(3,5-difluorophenyl)-2-{3-[5-(4-methylaminobutoxy)-pyrimidin-2-yl]benzyl}-2H-pyridazin-3-one |
| "A133" | 6-(3,5-difluorophenyl)-2-{3-[5-(3-methylaminopropoxy)-pyrimidin-2-yl]benzyl}-2H-pyridazin-3-one |
| "A134" | 6-(3,5-difluorophenyl)-2-{3-[5-(pyrrolidin-3-ylmethoxy)-pyrimidin-2-yl]benzyl}-2H-pyridazin-3-one |
| "A135" | 6-(3,5-difluorophenyl)-2-{3-[5-(3-ethylaminopropoxy)-pyrimidin-2-yl]benzyl}-2H-pyridazin-3-one |
| "A136" | 2-{3-[5-(2-aminoethoxy)pyrimidin-2-yl]benzyl}-6-(3,5-difluoro-phenyl)-2H-pyridazin-3-one |
| "A137" | 6-(3,5-difluorophenyl)-2-{3-[5-(piperidin-3-yloxy)pyrimidin-2-yl]benzyl}-2H-pyridazin-3-one |
| "A138" | 6-(3,5-difluorophenyl)-2-{3-[5-(piperidin-4-ylmethoxy)-pyrimidin-2-yl]benzyl}-2H-pyridazin-3-one |
| "A139" | 6-(3,5-difluorophenyl)-2-{3-[5-(pyrrolidin-3-yloxy)pyrimidin-2-yl]benzyl}-2H-pyridazin-3-one |
| "A140" | 6-(3,5-difluorophenyl)-2-{3-[5-((S)-pyrrolidin-3-yloxy)-pyrimidin-2-yl]benzyl}-2H-pyridazin-3-one |
| "A141" | 6-(3,5-difluorophenyl)-2-{3-[5-((R)-pyrrolidin-3-yloxy)-pyrimidin-2-yl]benzyl}-2H-pyridazin-3-one |
| "A142" | 2-{3-[5-(piperidin-4-yloxy)pyrimidin-2-yl]benzyl}-6-pyridin-4-yl-2H-pyridazin-3-one |
| | 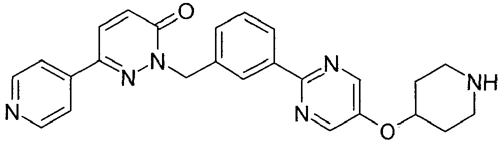 |
| "A143" | 4-(6-oxo-1-{3-[5-(piperidin-4-yloxy)pyrimidin-2-yl]benzyl}-1,6-dihydropyridazin-3-yl)benzonitrile |
| | 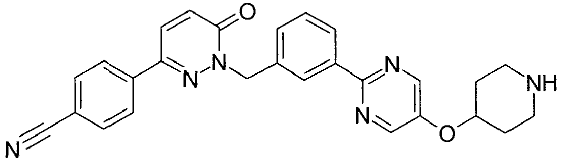 |
| "A144" | 3-(6-oxo-1-{3-[5-(piperidin-4-yloxy)pyrimidin-2-yl]benzyl}-1,6-dihydropyridazin-3-yl)benzonitrile |
| "A145" | 6-(3,5-difluorophenyl)-2-{3-[5-(2-piperazin-1-ylethoxy)-pyrimidin-2-yl]benzyl}-2H-pyridazin-3-one |
| "A146" | 6-(3,5-difluorophenyl)-2-{3-[5-(piperidin-4-yloxy)pyrimidin-2-yl]benzyl}-2H-pyridazin-3-one |
| "A147" | 3-(6-oxo-1-{3-[5-(2-piperazin-1-ylethoxy)pyrimidin-2-yl]-benzyl}-1,6-dihydropyridazin-3-yl)benzonitrile |
| "A148" | 6-(3-fluorophenyl)-2-{3-[5-(piperidin-4-ylmethoxy)pyrimidin-2-yl]benzyl}-2H-pyridazin-3-one |
| "A149" | 2-{3-[5-(1-piperidin-4-yl-1H-pyrazol-4-yl)pyrimidin-2-yl]-benzyl}-6-(3,4,5-trifluorophenyl)-2H-pyridazin-3-one |
| | 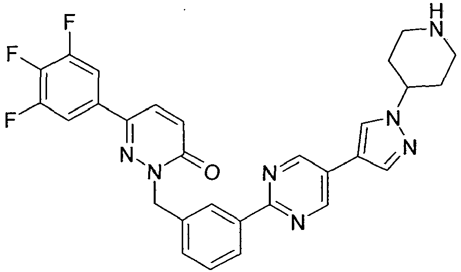 |
| "A150" | 3-(6-oxo-1-{3-[5-(1-piperidin-4-yl-1H-pyrazol-4-yl)pyrimidin-2-yl]benzyl}-1,6-dihydropyridazin-3-yl)benzonitrile |
| | 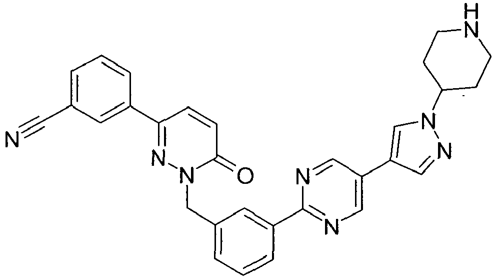 |
| "A151" | 6-(1-methyl-1H-pyrazol-4-yl)-2-{3-[5-(1-piperidin-4-yl-1H-pyrazol-4-yl)pyrimidin-2-yl]benzyl}-2H-pyridazin-3-one |
| | 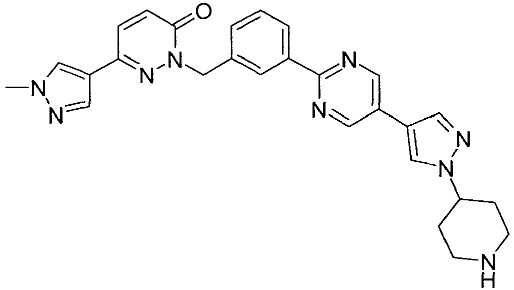 |
| "A152" | 6-(3-methoxyphenyl)-2-{3-[5-(1-piperidin-4-yl-1H-pyrazol-4-yl)pyrimidin-2-yl]benzyl}-2H-pyridazin-3-one |
| "A153" | 6-(3-fluorophenyl)-2-{3-[5-(1-piperidin-4-yl-1H-pyrazol-4-yl)-pyrimidin-2-yl]benzyl}-2H-pyridazin-3-one |
| "A 154" | 6-(3,5-difluorophenyl)-2-{3-[5-(1H-pyrazol-4-yl)pyrimidin-2-yl]benzyl}-2H-pyridazin-3-one |
| | 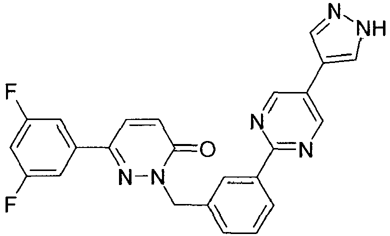 |
| "A155" | 6-(3,5-difluorophenyl)-2-(3-{5-[1-(2-methylaminoethyl)-1H-pyrazol-4-yl]pyrimidin-2-yl}benzyl)-2H-pyridazin-3-one |
| | 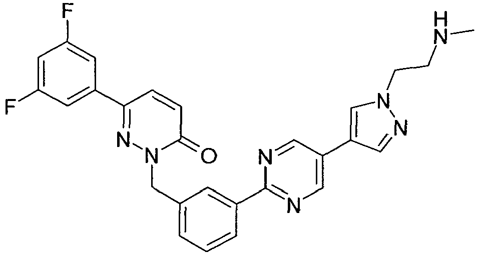 |
| "A156" | 6-(3-chlorophenyl)-2-{3-[5-(1-piperidin-4-yl-1H-pyrazol-4-yl)-pyrimidin-2-yl]benzyl}-2H-pyridazin-3-one |
| | 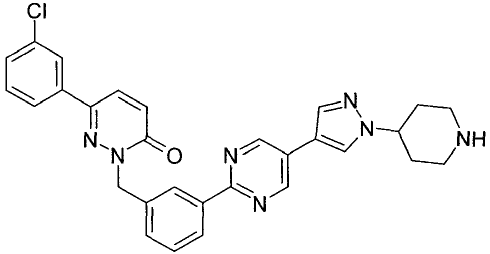 |
| "A157" | 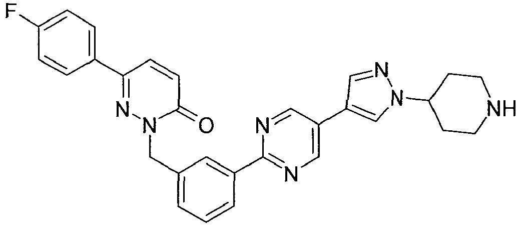 |
| "A158" | 6-(3,5-difluorophenyl)-2-(3-{5-[1-(3-methylaminopropyl)-1H-pyrazol-4-yl]pyrimidin-2-yl}benzyl)-2H-pyridazin-3-one |
| | 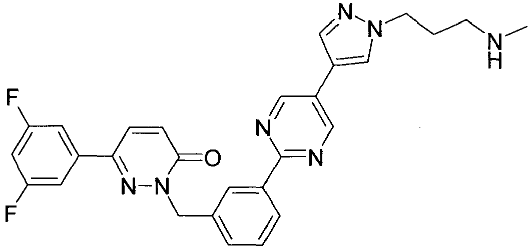 |
| "A159" | 3-[1-(3-{5-[1-(2-methylaminoethyl)-1H-pyrazol-4-yl]pyrimidin-2-yl}benzyl)-6-oxo-1,6-dihydropyridazin-3-yl]benzonitrile |
| | 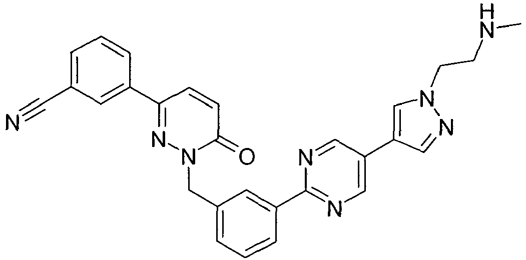 |
| "A160" | 6-(3,5-difluorophenyl)-2-(3-{5-[1-(2-piperazin-1-ylethyl)-1H-pyrazol-4-yl]pyrimidin-2-yl}benzyl)-2H-pyridazin-3-one |
| | 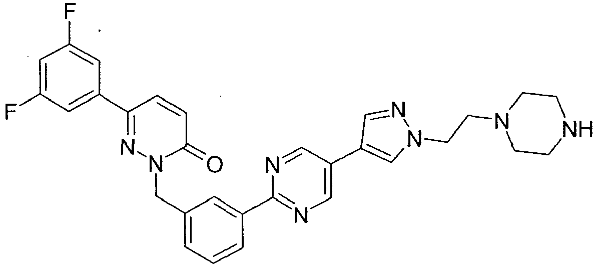 |
| "A161" | 3-(6-oxo-1-{3-[5-(1H-pyrazol-4-yl)pyrimidin-2-yl]benzyl}-1,6-dihydropyridazin-3-yl)benzonitrile |
| | 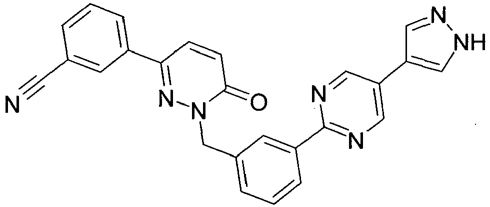 |
| "A162" | 3-[6-oxo-1-(3-{5-[1-(2-piperazin-1-ylethyl)-1H-pyrazol-4-yl]-pyrimidin-2-yl}benzyl)-1,6-dihydropyridazin-3-yl]benzonitrile |
| | 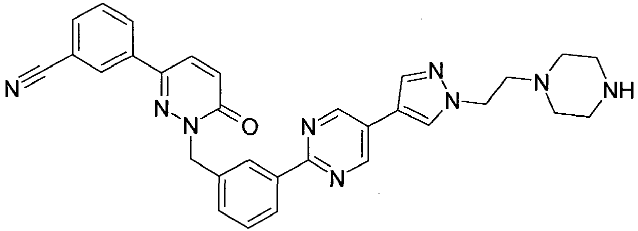 |
| "A163" | N-(4-dimethylaminobutyl)-2-[3-(6-oxo-3-pyridin-4-yl-6H-pyridazin-1-ylmethyl)phenyl]pyrimidine-5-carboxamide |
| | 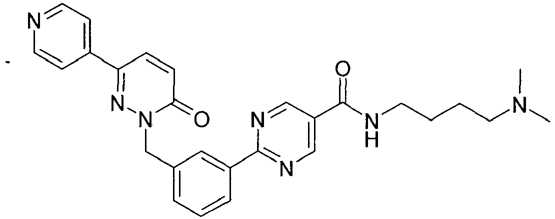 |
| "A164" | N-(4-dimethylaminobutyl)-2-{3-[3-(4-cyanophenyl)-6-oxo-6H-pyridazin-1-ylmethyl]phenyl}pyrimidine-5-carboxamide |
| | 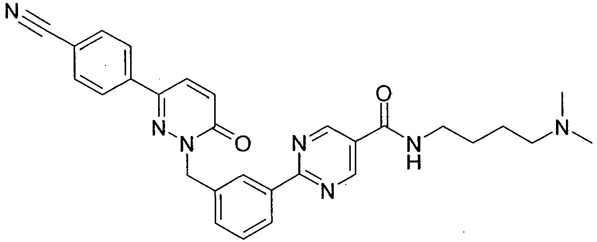 |
| "A165" | 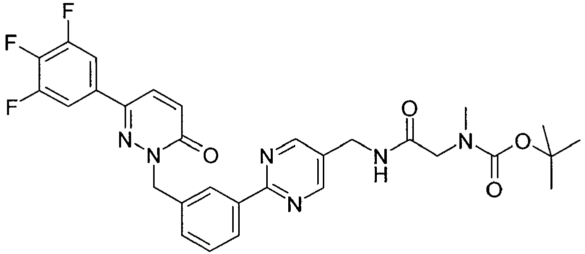 |
| "A166" | 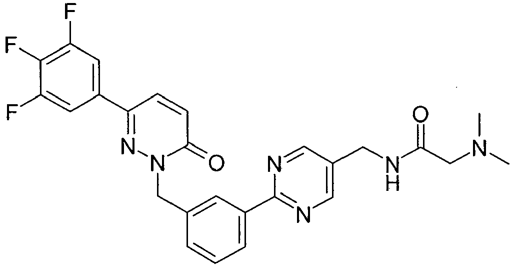 |
| "A167" | 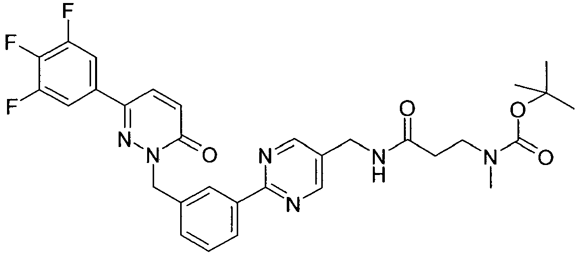 |
| "A168" | 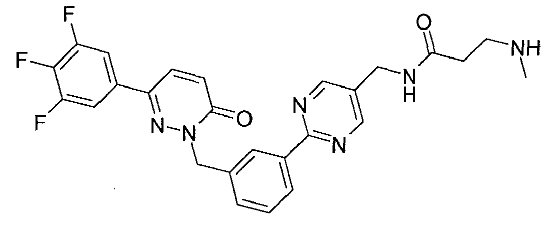 |
| "A169" | 6-(1-methyl-1H-pyrazol-4-yl)-2-(3-{5-[1-(2-pyrrolidin-1-yl-ethyl)-1H-pyrazol-4-yl]pyrimidin-2-yl}benzyl)-2H-pyridazin-3-one |
| | 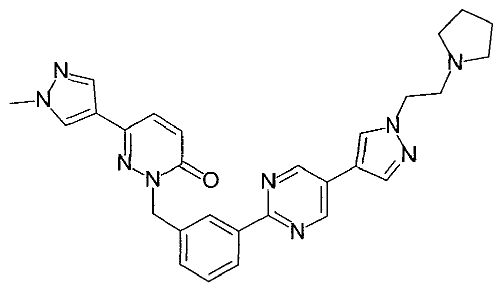 |
| "A170" | 6-(3,5-difluorophenyl)-2-(3-{5-[1-(2-morpholin-4-ylethyl)-1H-pyrazol-4-yl]pyrimidin-2-yl}benzyl)-2H-pyridazin-3-one |
| | 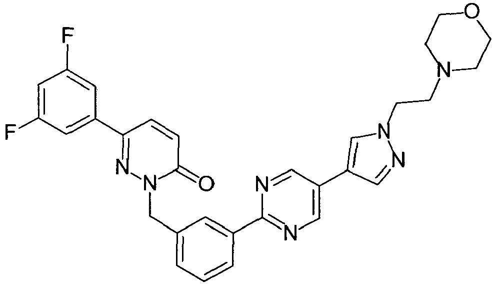 |
| "A171" | 6-(3,5-difluorophenyl)-2-(3-{5-[1-(2-dimethylaminoethyl)-1H-pyrazol-4-yl]pyrimidin-2-yl}benzyl)-2H-pyridazin-3-one |
| | 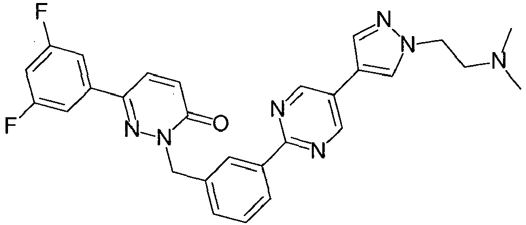 |
| "A172" | 6-(3,5-difluorophenyl)-2-(3-{5-[1-(3-dimethylaminopropyl)-1H-pyrazol-4-yl]pyrimidin-2-yl}benzyl)-2H-pyridazin-3-one |
| "A173" | 6-(3,5-difluorophenyl)-2-(3-{5-[1-(2-pyrrolidin-1-ylethyl)-1H-pyrazol-4-yl]pyrimidin-2-yl}benzyl)-2H-pyridazin-3-one |
| "A174" | 3-[1-(3-{5-[1-(2-morpholin-4-ylethyl)-1H-pyrazol-4-yl]-pyrimidin-2-yl}benzyl)-6-oxo-1,6-dihydropyridazin-3-yl]-benzonitrile |
| | 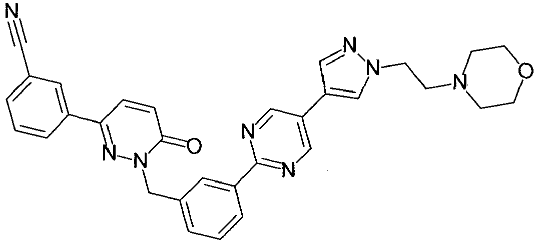 |
| "A175" | 2-(3-{5-[1-(2-morpholin-4-ylethyl)-1H-pyrazol-4-yl]pyrimidin-2-yl}benzyl)-6-pyridin-3-yl-2H-pyridazin-3-one |
| | 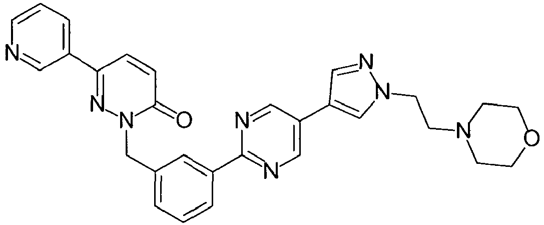 |
| "A 176" | 6-(1-methyl-1H-pyrazol-4-yl)-2-(3-{5-[1-(2-morpholin-4-yl-ethyl)-1H-pyrazol-4-yl]pyrimidin-2-yl}benzyl)-2H-pyridazin-3-one |
| | 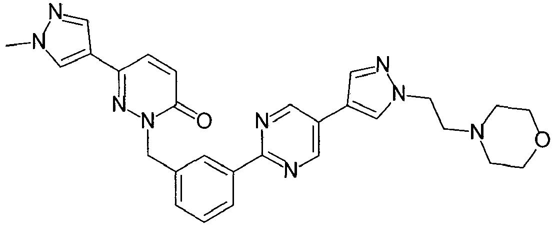 |
| "A177" | 2-(3-{5-[1-(2-morpholin-4-ylethyl)-1H-pyrazol-4-yl]pyrimidin-2-yl}benzyl)-6-pyridin-4-yl-2H-pyridazin-3-one |
| "A178" | 6-(4-methanesulfonylphenyl)-2-(3-{5-[1-(2-morpholin-4-yl-ethyl)-1H-pyrazol-4-yl]pyrimidin-2-yl}benzyl)-2H-pyridazin-3-one |
| "A179" | 6-pyridin-4-yl-2-(3-{5-[1-(2-pyrrolidin-1-ylethyl)-1H-pyrazol-4-yl]pyrimidin-2-yl}benzyl)-2H-pyridazin-3-one |
| "A180" | 4-[6-oxo-1-(3-{5-[1-(2-pyrrolidin-1-ylethyl)-1H-pyrazol-4-yl]-pyrimidin-2-yl}benzyl)-1,6-dihydropyridazin-3-yl]benzonitrile |
| | 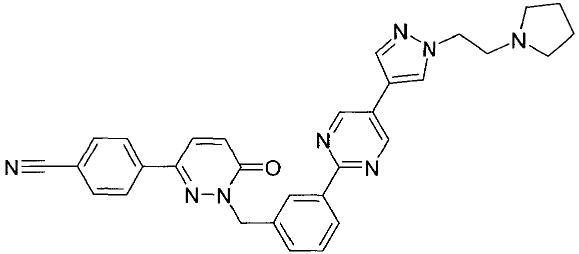 |
| "A181" | 2-(3-{5-[1-(2-morpholin-4-ylethyl)-1H-pyrazol-4-yl]pyrimidin-2-yl}benzyl)-6-pyridin-4-yl-2H-pyridazin-3-one |
| "A183" | 6-(4-methanesulfonylphenyl)-2-(3-{5-[1-(2-morpholin-4-yl-ethyl)-1H-pyrazol-4-yl]pyrimidin-2-yl}benzyl)-2H-pyridazin-3-one |
| "A184" | 6-(5-methyloxazol-2-yl)-2-(3-{5-[1-(2-pyrrolidin-1-ylethyl)-1H-pyrazol-4-yl]pyrimidin-2-yl}benzyl)-2H-pyridazin-3-one |
| | 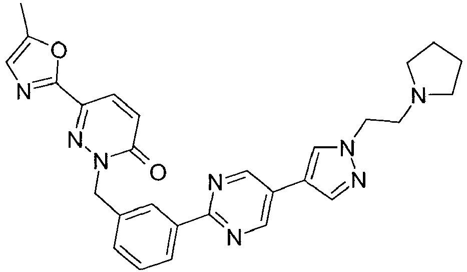 |
| "A185" | 6-(3-fluorophenyl)-2-(3-{5-[1-(2-pyrrolidin-1-ylethyl)-1H-pyrazol-4-yl]pyrimidin-2-yl}benzyl)-2H-pyridazin-3-one |
| "A186" | 6-(1-propyl-1H-pyrazol-4-yl)-2-(3-{5-[1-(2-pyrrolidin-1-yl-ethyl)-1H-pyrazol-4-yl]pyrimidin-2-yl}benzyl)-2H-pyridazin-3-one |
| | 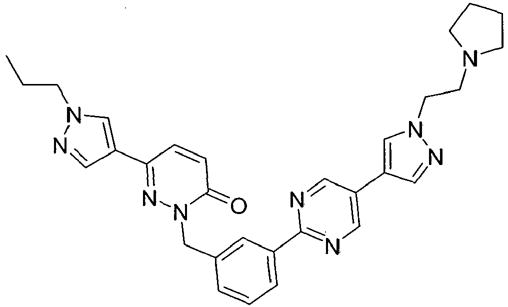 |
| "A187" | 2-(3-{5-[1-(2-pyrrolidin-1-ylethyl)-1H-pyrazol-4-yl]pyrimidin-2-yl}benzyl)-6-thiophen-3-yl-2H-pyridazin-3-one |
| | 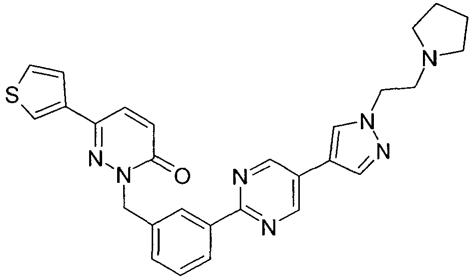 |
| "A188" | 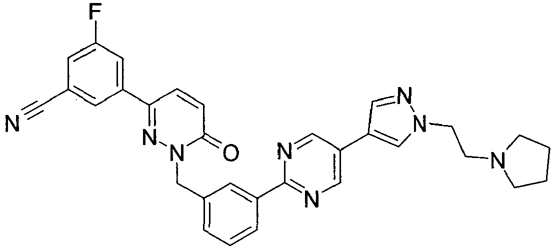 |
| "A188a" | 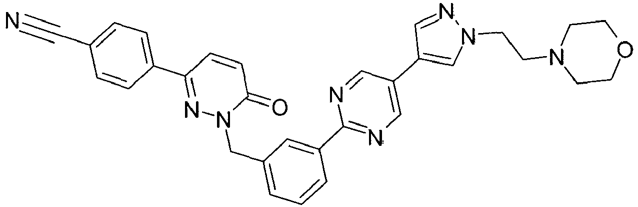 |
| "A189" | 3-(1-{3-[5-(3-dimethylaminopropoxy)pyrimidin-2-yl]benzyl}-6-oxo-1,6-dihydropyridazin-3-yl)benzonitrile |
| "A190" | 2-{3-[5-(3-dimethylaminopropoxy)pyrimidin-2-yl]benzyl}-6-(1-methyl-1 H-pyrazol-4-yl)-2H-pyridazin-3-one |
| | 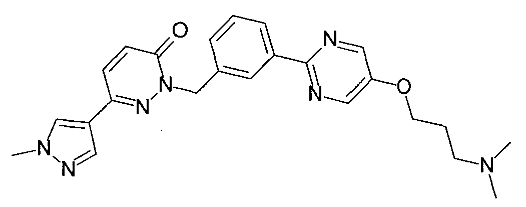 |
| "A191" | 2-{3-[5-(3-dimethylaminopropoxy)pyrimidin-2-yl]benzyl}-6-(3-fluorophenyl)-2H-pyridazin-3-one |
| "A192" | 2-{3-[5-(3-dimethylaminopropoxy)pyrimidin-2-yl]benzyl}-6-thiazol-2-yl-2H-pyridazin-3-one |
| "A193" | 2-{3-[5-(3-dimethylaminopropoxy)pyrimidin-2-yl]benzyl}-6-phenyl-2H-pyridazin-3-one |
| "A 194" | 4-(1-{3-[5-(3-dimethylaminopropoxy)pyrimidin-2-yl]benzyl}-6-oxo-1,6-dihydropyridazin-3-yl)benzonitrile |
| "A195" | 2-{3-[5-(3-dimethylaminopropoxy)pyrimidin-2-yl]benzyl}-6-p-tolyl-2H-pyridazin-3-one |
| "A 196" | 2-{3-[5-(3-dimethylaminopropoxy)pyrimidin-2-yl]benzyl}-6-(2H-pyrazol-3-yl)-2H-pyridazin-3-one |
| "A197" | 6-(3,4-difluorophenyl)-2-{3-[5-(3-dimethylaminopropoxy)-pyrimidin-2-yl]benzyl}-2H-pyridazin-3-one |
| "A198" | 2-{3-[5-(3-dimethylaminopropoxy)pyrimidin-2-yl]benzyl}-6-(4-methanesulfonylphenyl)-2H-pyridazin-3-one |
| "A199" | 2-{3-[5-(3-dimethylaminopropoxy)pyrimidin-2-yl]benzyl}-6-[4-(5-methyl-1,2,4-oxadiazol-3-yl)phenyl]-2H-pyridazin-3-one |
| "A200" | 2-{3-[5-(3-dimethylaminopropoxy)pyrimidin-2-yl]benzyl}-6-pyridin-4-yl-2H-pyridazin-3-one |
| "A201" | 6-(3-bromophenyl)-2-{3-[5-(3-dimethylaminopropoxy)-pyrimidin-2-yl]benzyl}-2H-pyridazin-3-one |
| "A202" | 2-{3-[5-(3-dimethylaminopropoxy)pyrimidin-2-yl]benzyl}-6-(3,4,5-trifluorophenyl)-2H-pyridazin-3-one |
| "A203" | 6-(3,5-dimethoxyphenyl)-2-{3-[5-(3-dimethylaminopropoxy)-pyrimidin-2-yl]benzyl}-2H-pyridazin-3-one |
| "A204" | 2-{3-[5-(3-dimethylaminopropoxy)pyrimidin-2-yl]benzyl}-6-(3-fluoro-4-methoxyphenyl)-2H-pyridazin-3-one |
| "A205" | 2-{3-[5-(3-dimethylaminopropoxy)pyrimidin-2-yl]benzyl}-6-(4-methoxyphenyl)-2H-pyridazin-3-one |
| "A206" | 2-{3-[5-(3-dimethylaminopropoxy)pyrimidin-2-yl]benzyl}-6-(3-trifluoromethylphenyl)-2H-pyridazin-3-one |
| "A207" | 6-(3-chlorophenyl)-2-{3-[5-(3-dimethylaminopropoxy)-pyrimidin-2-yl]benzyl}-2H-pyridazin-3-one |
| "A208" | 2-{3-[5-(3-dimethylaminopropoxy)pyrimidin-2-yl]benzyl}-6-pyridin-3-yl-2H-pyridazin-3-one |
| "A209" | 2-{3-[5-(3-dimethylaminopropoxy)pyrimidin-2-yl]benzyl}-6-(1-methyl-1H-pyrazol-4-yl)-2H-pyridazin-3-one |
| "A210" | 6-(3-chloro-5-fluorophenyl)-2-{3-[5-(3-dimethylamino-propoxy)pyrimidin-2-yl]benzyl}-2H-pyridazin-3-one |
| "A211" | 2-{3-[5-(3-dimethylaminopropoxy)pyrimidin-2-yl]benzyl}-6-(4-fluoro-3-methoxyphenyl)-2H-pyridazin-3-one |
| "A212" | 6-(4-chlorophenyl)-2-{3-[5-(3-dimethylaminopropoxy)-pyrimidin-2-yl]benzyl}-2H-pyridazin-3-one |
| "A213" | 2-{3-[5-(3-dimethylaminopropoxy)pyrimidin-2-yl]benzyl}-6-(4-fluorophenyl)-2H-pyridazin-3-one |
| "A214" | 2-{3-[5-(3-dimethylaminopropoxy)pyrimidin-2-yl]benzyl}-6-thiophen-2-yl-2H-pyridazin-3-one |
| "A215" | N-[4-(1-{3-[5-(3-dimethylaminopropoxy)pyrimidin-2-yl]-benzyl}-6-oxo-1,6-dihydropyridazin-3-yl)phenyl]acetamide |
| | 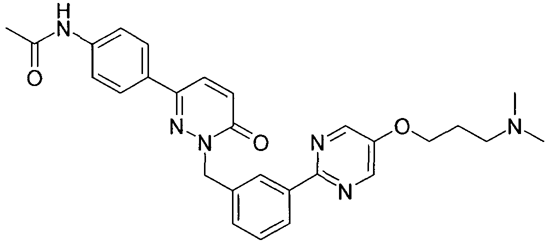 |
| "A216" | 6-(3,4-dimethoxyphenyl)-2-{3-[5-(3-dimethylaminopropoxy)-pyrimidin-2-yl]benzyl}-2H-pyridazin-3-one |
| "A217" | 6-benzo-1,2,5-thiadiazol-5-yl-2-{3-[5-(3-dimethylamino-propoxy)pyrimidin-2-yl]benzyl}-2H-pyridazin-3-one |
| | 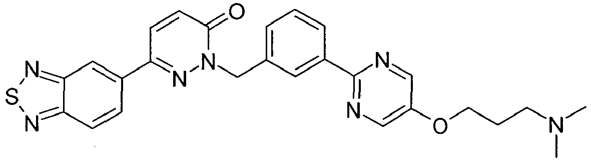 |
| "A218" | 2-{3-[5-(3-dimethylaminopropoxy)pyrimidin-2-yl]benzyl}-6-furan-3-yl-2H-pyridazin-3-one |
| "A219" | 2-{3-[5-(3-dimethylaminopropoxy)pyrimidin-2-yl]benzyl}-6-(5-methyl-1,2,4-oxadiazol-3-yl)-2H-pyridazin-3-one |
| "A220" | 4-(1-{3-[5-(1-methylpiperidin-4-yloxy)pyrimidin-2-yl]benzyl}-6-oxo-1,6-dihydropyridazin-3-yl)benzonitrile |
| "A221" | 3-(1-{3-[5-(1-methylpiperidin-4-yloxy)pyrimidin-2-yl]benzyl}-6-oxo-1,6-dihydropyridazin-3-yl)benzonitrile |
| "A222" | 3-(1-{3-[5-(2-morpholin-4-ylethoxy)pyrimidin-2-yl]benzyl}-6-oxo-1,6-dihydropyridazin-3-yl)benzonitrile |
| "A223" | 2-{3-[5-(1-methylpiperidin-4-yloxy)pyrimidin-2-yl]benzyl}-6-pyridin-4-yl-2H-pyridazin-3-one |
| "A224" | 6-(4-methanesulfonylphenyl)-2-{3-[5-(1-methylpiperidin-4-yloxy)pyrimidin-2-yl]benzyl}-2H-pyridazin-3-one |
| "A225" | methyl 5-(1-{3-[5-(1-methylpiperidin-4-yloxy)pyrimidin-2-yl]-benzyl}-6-oxo-1,6-dihydropyridazin-3-yl)thiophene-2-carboxylate |
| | 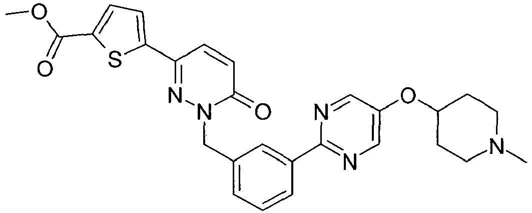 |
| "A226" | 2-{3-[5-(1-methylpiperidin-4-yloxy)pyrimidin-2-yl]benzyl}-6-(1-methyl-1H-pyrazol-4-yl)-2H-pyridazin-3-one |
| "A227" | 2-{3-[5-(3-dimethylaminopropoxy)pyrimidin-2-yl]benzyl}-6-(5-oxo-4,5-dihydro-1,2,4-oxadiazol-3-yl)-2H-pyridazin-3-one |
| | 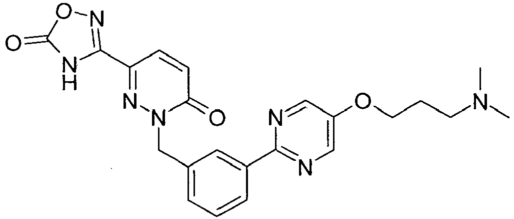 |
| "A228" | 2-{3-[5-(3-dimethylaminopropoxy)pyrimidin-2-yl]benzyl}-6-piperazin-1-yl-2H-pyridazin-3-one |
| | 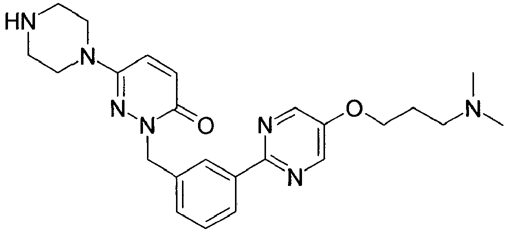 |
| "A229" | 6-(1-methyl-1H-pyrazol-4-yl)-2-{3-[5-(2-morpholin-4-yl-ethoxy)pyrimidin-2-yl]benzyl}-2H-pyridazin-3-one |
| "A235" | 6-(3-fluorophenyl)-2-{3-[5-(2-morpholin-4-ylethoxy)pyrimidin-2-yl]benzyl}-2H-pyridazin-3-one |
| "A237" | 2-{3-[5-(1-methylpiperidin-4-ylmethoxy)pyrimidin-2-yl]-benzyl}-6-(1-methyl-1H-pyrazol-4-yl)-2H-pyridazin-3-one |
| "A238" | 2-{3-[5-(1-methylpiperidin-4-yloxy)pyrimidin-2-yl]benzyl}-6-(1-methyl-1H-pyrazol-4-yl)-2H-pyridazin-3-one |
| "A240" | 2-{3-[5-(3-methoxypropoxy)pyrimidin-2-yl]benzyl}-6-(1-methyl-1 H-pyrazol-4-yl)-2H-pyridazin-3-one |
| "A241" | 2-{3-[5-(2-methoxyethoxy)pyrimidin-2-yl]benzyl}-6-(1-methyl-1H-pyrazol-4-yl)-2H-pyridazin-3-one |
| "A242" | 2-{3-[5-(2-morpholin-4-ylethoxy)pyrimidin-2-yl]benzyl}-6-(1-propyl-1 H-pyrazol-4-yl)-2H-pyridazin-3-one |
| "A243" | 2-(3-{5-[2-(4-methylpiperazin-1-yl)ethoxy]pyrimidin-2-yl}-benzyl)-6-(1-methyl-1H-pyrazol-4-yl)-2H-pyridazin-3-one |
| "A244" | 2-(3-{5-[2-(4-methyl-3-oxopiperazin-1-yl)ethoxy]pyrimidin-2-yl}benzyl)-6-(1-methyl-1H-pyrazol-4-yl)-2H-pyridazin-3-one |
| | 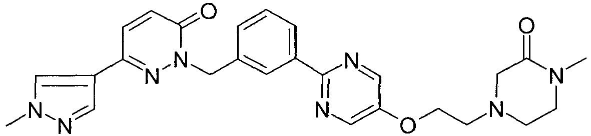 |
| "A245" | 6-(1-methyl-1H-pyrazol-4-yl)-2-{3-[5-(3-morpholin-4-yl-propoxy)pyrimidin-2-yl]benzyl}-2H-pyridazin-3-one |
| "A246" | 6-(1-methyl-1H-pyrazol-4-yl)-2-{3-[5-(2-morpholin-4-yl-propoxy)pyrimidin-2-yl]benzyl}-2H-pyridazin-3-one |
| | 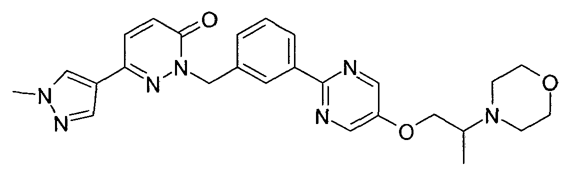 |
| "A247" | 2-{3-[5-(1-methyl-2-morpholin-4-ylethoxy)pyrimidin-2-yl]-benzyl}-6-(1-methyl-1H-pyrazol-4-yl)-2H-pyridazin-3-one |
| | 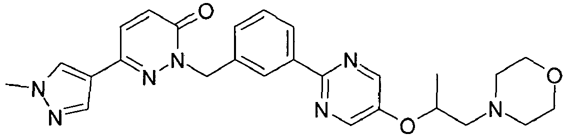 |
| "A248" | 2-{3-[5-(2-dimethylaminoethoxy)pyrimidin-2-yl]benzyl}-6-(1-methyl-1H-pyrazol-4-yl)-2H-pyridazin-3-one |
| "A249" | 2-{3-[5-(2-methyl-3-morpholin-4-ylpropoxy)pyrimidin-2-yl]-benzyl}-6-(1-methyl-1H-pyrazol-4-yl)-2H-pyridazin-3-one |
| | 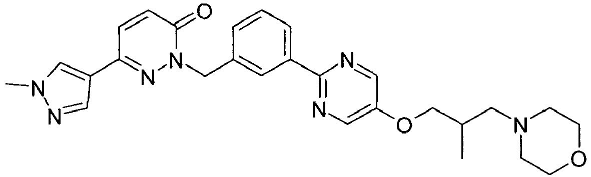 |
| "A250" | 6-(1-methyl-1H-pyrazol-4-yl)-2-{3-[5-(2-pyrrolidin-1-yl-ethoxy)pyrimidin-2-yl]benzyl}-2H-pyridazin-3-one |
| "A251" | 2-[3-(5-ethoxypyrimidin-2-yl)benzyl]-6-(1-methyl-1H-pyrazol-4-yl)-2H-pyridazin-3-one |
| "A252" | 6-(1-methyl-1H-pyrazol-4-yl)-2-{3-[5-(2-morpholin-4-yl-2-oxo-ethoxy)pyrimidin-2-yl]benzyl}-2H-pyridazin-3-one |
| | 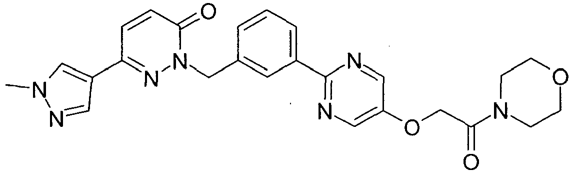 |
| "A253" | 6-(3-chlorophenyl)-2-{3-[5-(2-morpholin-4-ylethoxy)-pyrimidin-2-yl]benzyl}-2H-pyridazin-3-one |
| "A254" | 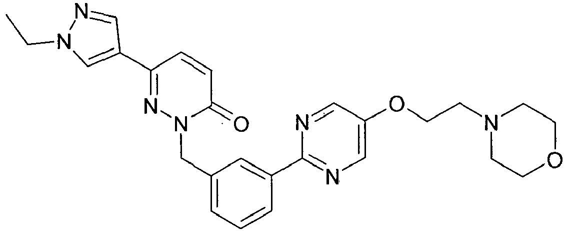 |
| "A255" | 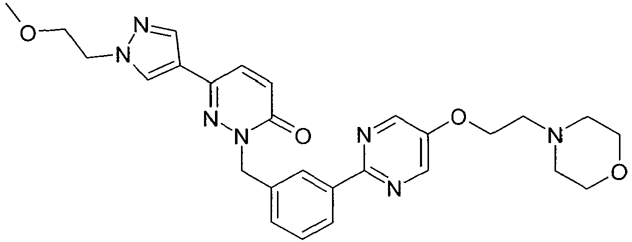 |
| "A256" | 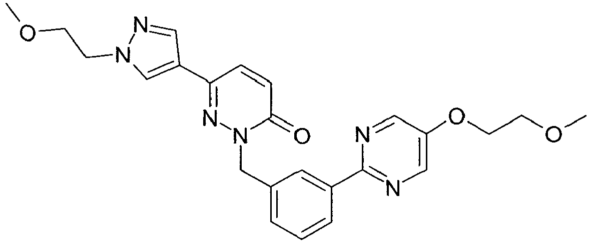 |
| "A257" | 3-(1-{3-[5-(1-methylpiperidin-4-ylmethoxy)pyrimidin-2-yl]-benzyl}-6-oxo-1,6-dihydropyridazin-3-yl)benzonitrile |
| "A259" | 6-(3,5-difluorophenyl)-2-{3-[5-(1-piperidin-4-yl-1H-pyrazol-4-yl)pyridin-2-yl]benzyl}-2H-pyridazin-3-one |
| "A260" | 3-(1-{3-[5-(3-dimethylaminopropoxy)pyrimidin-2-yl]benzyl}-6-oxo-1,6-dihydropyridazin-3-yl)benzamide |
| "A261" | 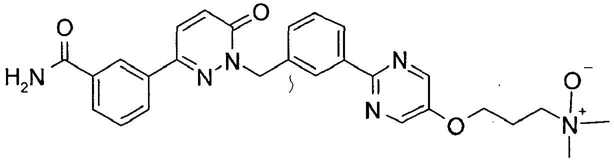 |
| "A264" | 3-(6-oxo-1-{3-[5-(piperidin-4-ylmethoxy)pyrimidin-2-yl]-benzyl}-1,6-dihydropyridazin-3-yl)benzonitrile |
| "A265" | tert-butyl 4-(2-{3-[3-(3-cyanophenyl)-6-oxo-6H-pyridazin-1-yl-methyl]phenyl}pyrimidin-5-yloxymethyl)piperidine-1-carboxylate |
| "A266" | 6-(1-methyl-1H-pyrazol-4-yl)-2-{3-[5-(piperidin-4-yloxy)-pyrimidin-2-yl]benzyl}-2H-pyridazin-3-one |
| "A267" | 6-(1-methyl-1H-pyrazol-4-yl)-2-{3-[5-(piperidin-4-ylmethoxy)-pyrimidin-2-yl]benzyl}-2H-pyridazin-3-one |
| "A268" | 3-(1-{3-[5-(3-methylaminopropoxy)pyrimidin-2-yl]benzyl}-6-oxo-1,6-dihydropyridazin-3-yl)benzonitrile |
| | 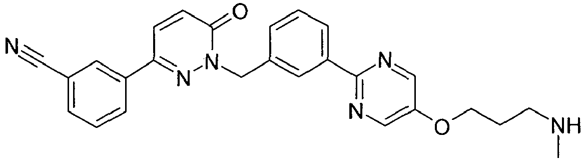 |
| "A269" | 3-[1-(3-{5-[2-(4-methyl-3-oxopiperazin-1-yl)ethoxy]pyrimidin-2-yl}benzyl)-6-oxo-1,6-dihydropyridazin-3-yl]benzonitrile |
| | 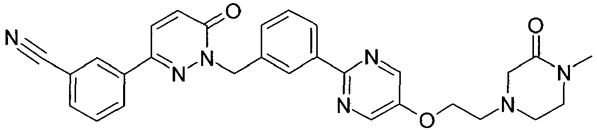 |
| "A270" | 3-[1-(3-{5-[2-(4-methylpiperazin-1-yl)ethoxy]pyrimidin-2-yl}-benzyl)-6-oxo-1,6-dihydropyridazin-3-yl]benzonitrile |
| "A271" | 3-(1-{3-[5-(2-methoxyethoxy)pyrimidin-2-yl]benzyl}-6-oxo-1,6-dihydropyridazin-3-yl)benzonitrile |
| "A272" | 3-(1-{3-[5-(3-methoxypropoxy)pyrimidin-2-yl]benzyl}-6-oxo-1,6-dihydropyridazin-3-yl)benzonitrile |
| "A273" | 6-(3-fluorophenyl)-2-{3-[5-(1-methylpiperidin-4-ylmethoxy)-pyrimidin-2-yl]benzyl}-2H-pyridazin-3-one |
| "A274" | 2-{3-[5-(1-methylpiperidin-4-yloxy)pyrimidin-2-yl]benzyl}-6-(1-propyl-1H-pyrazol-4-yl)-2H-pyridazin-3-one |
| "A275" | 6-(3-chlorophenyl)-2-{3-[5-(1-methylpiperidin-4-ylmethoxy)-pyrimidin-2-yl]benzyl}-2H-pyridazin-3-one |
| "A276" | 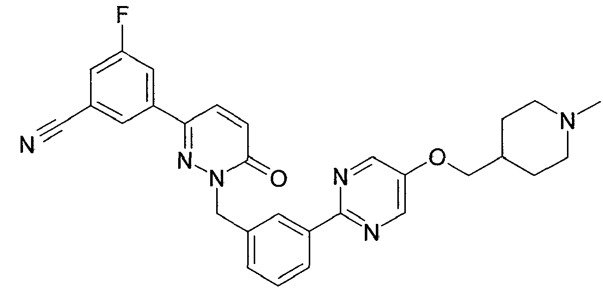 |
| "A276a" | 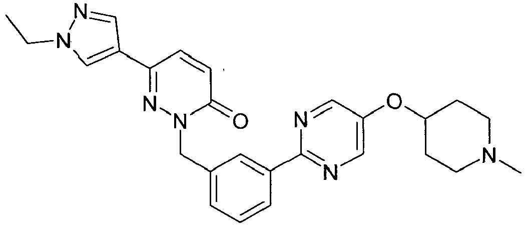 |
| "A277" | 5-(1-{3-[5-(1-methylpiperidin-4-yloxy)pyrimidin-2-yl]benzyl}-6-oxo-1,6-dihydropyridazin-3-yl)thiophene-2-carboxylic acid |
| "A278" | 5-(1-{3-[5-(1-methylpiperidin-4-yloxy)pyrimidin-2-yl]benzyl}-6-oxo-1,6-dihydropyridazin-3-yl)thiophene-2-carboxamide |
| "A279" | N-methyl-5-(1-{3-[5-(1-methylpiperidin-4-yloxy)pyrimidin-2-yl]benzyl}-6-oxo-1,6-dihydropyridazin-3-yl)thiophene-2-carboxamide |
| "A280" | 2-[3-(5-aminopyrazin-2-yl)benzyl]-6-(3,5-difluorophenyl)-2H-pyridazin-3-one |
| "A282" | 2-[3-(6-aminopyridazin-3-yl)benzyl]-6-(3,5-diffuorophenyl)-2H-pyridazin-3-one |
| "A283" | methyl (E)-3-(2-{3-[6-oxo-3-(3,4,5-trifluorophenyl)-6H-pyridazin-1-ylmethyl]phenyl}pyrimidin-5-yl)acrylate |
| "A284" | 2-{3-[5-((E)-3-aminopropenyl)pyrimidin-2-yl]benzyl}-6-(3,4,5-trifluorophenyl)-2H-pyridazin-3-one |
| "A285" | 2-{3-[5-(3-aminopropyl)pyrimidin-2-yl]benzyl}-6-(3,4,5-trifluorophenyl)-2H-pyridazin-3-one |
| "A286" | 2-{3-[5-(4-methylpiperazin-1-yl)pyrimidin-2-yl]benzyl}-6-(1-methyl-1 H-pyrazol-4-yl)-2H-pyridazin-3-one |
| "A287" | 3-(1-{3-[5-(4-methylpiperazin-1-yl)pyrimidin-2-yl]benzyl}-6-oxo-1,6-dihydropyridazin-3-yl)benzonitrile |
| "A288" | 3-{6-oxo-1-[3-(5-piperazin-1-ylpyrimidin-2-yl)benzyl]-1,6-dihydropyridazin-3-yl}benzonitrile |
| "A289" | 6-(4-methanesulfonylphenyl)-2-[3-(5-piperazin-1-ylpyrimidin-2-yl)benzyl]-2H-pyridazin-3-one |
| "A290" | 4-{1-[3-(5-aminopyrimidin-2-yl)benzyl]-6-oxo-1,6-dihydro-pyridazin-3-yl}benzonitrile |
| "A291" | 3-{1-[3-(5-aminopyrimidin-2-yl)benzyl]-6-oxo-1,6-dihydro-pyridazin-3-yl}benzonitrile |
| "A292" | 6-(1-methyl-1H-pyrazol-4-yl)-2-[3-(5-piperazin-1-ylpyrimidin-2-yl)benzyl]-2H-pyridazin-3-one |
| "A293" | 2-[3-(5-aminopyrimidin-2-yl)benzyl]-6-(1-methyl-1H-pyrazol-4-yl)-2H-pyridazin-3-one |
| "A294" | 2-{3-[5-(2-hydroxyethoxy)pyrimidin-2-yl]benzyl}-6-(1-methyl-1 H-pyrazol-4-yl)-2H-pyridazin-3-one |
| "A295" | 3-(1-{3-[5-(3-hydroxypropoxy)pyrimidin-2-yl]benzyl}-6-oxo-1,6-dihydropyridazin-3-yl)benzonitrile |
| "A296" | 3-(1-{3-[5-(2-hydroxyethoxy)pyrimidin-2-yl]benzyl}-6-oxo-1,6-dihydropyridazin-3-yl)benzonitrile |
| "A297" | 2-{3-[5-(3-hydroxypropoxy)pyrimidin-2-yl]benzyl}-6-(1-methyl-1H-pyrazol-4-yl)-2H-pyridazin-3-one |
| "A298" | 3-[1-(1-{3-[5-(3-dimethylaminopropoxy)pyrimidin-2-yl]-phenyl}ethyl)-6-oxo-1,6-dihydropyridazin-3-yl]benzonitrile |
| "A299" | 6-(3,5-difluorophenyl)-2-(1-{3-[5-(3-dimethylaminopropoxy)-pyrimidin-2-yl]phenyl}ethyl)-2H-pyridazin-3-one |
| "A300" | 6-(3,5-difluorophenyl)-2-((R)-1-{3-[5-(3-dimethylamino-propoxy)pyrimidin-2-yl]phenyl}ethyl)-2H-pyridazin-3-one |
| "A301" | 6-(3,5-difluorophenyl)-2-((S)-1-{3-[5-(3-dimethylamino-propoxy)pyrimidin-2-yl]phenyl}ethyl)-2H-pyridazin-3-one |
| "A302" | 3-(1-{3-[5-(1-methyl-1-oxypiperidin-4-ylmethoxy)pyrimidin-2-yl]benzyl}-6-oxo-1,6-dihydropyridazin-3-yl)benzonitrile |
| "A303" | 3-(1-{3-[5-(1-formylpiperidin-4-ylmethoxy)pyrimidin-2-yl]-benzyl}-6-oxo-1,6-dihydropyridazin-3-yl)benzonitrile |
| "A304" | 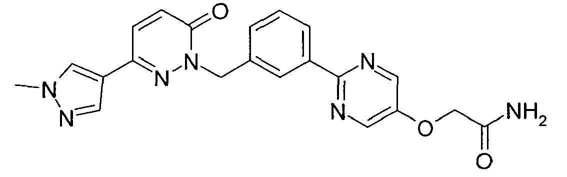 |
| "A305" | 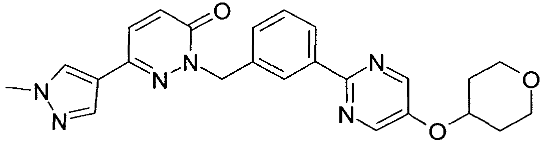 |
| "A306" | 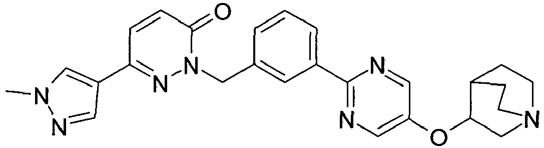 |
| "A307" | 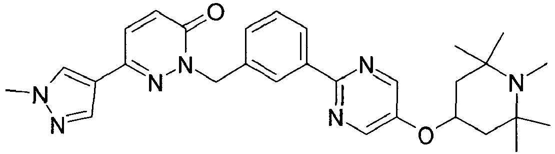 |
| "A308" | 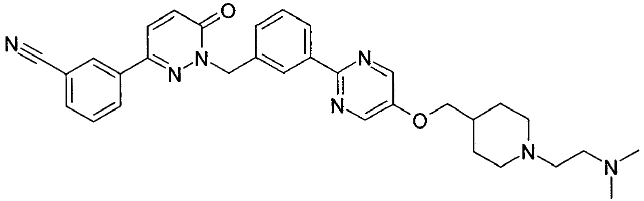 |
| "A309" | 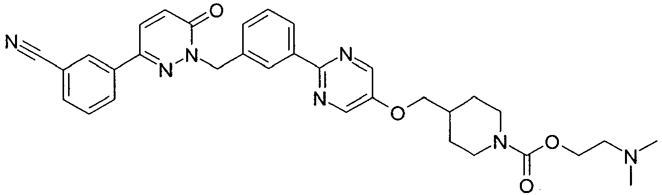 |
| "A310" | 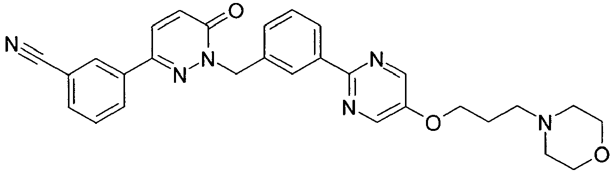 |
| "A311" | 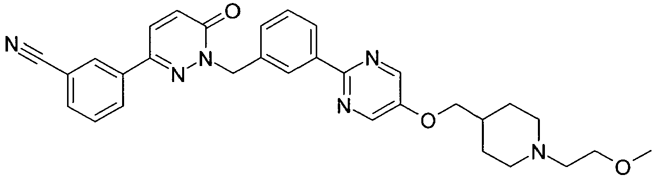 |
| "A312" | 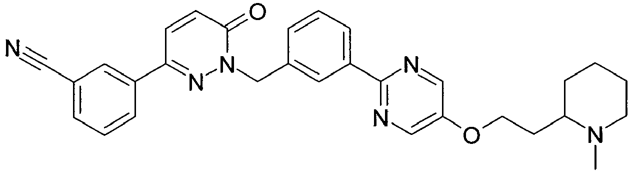 |
| "A313" | 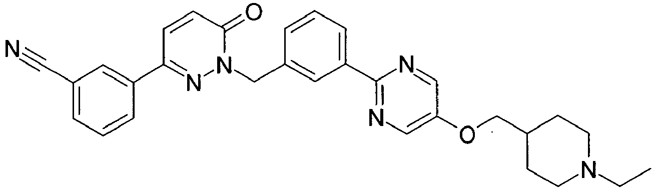 |
| "A314" | 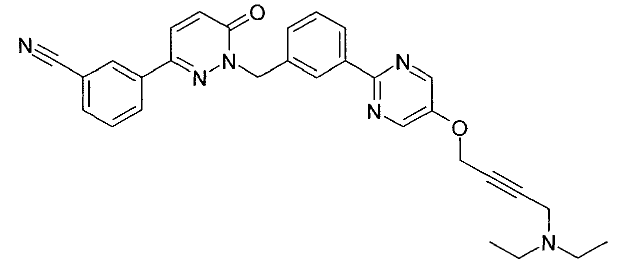 |

15. Medicament according to one or more of Claims 1-14, where 1 mg to 700 mg of a compound of the formula I is present.

16. Medicament according to Claim 15, where 5 mg to 100 mg of a compound of the formula I is present.

## Revendications

1. Médicament comprenant au moins un composé de formule I 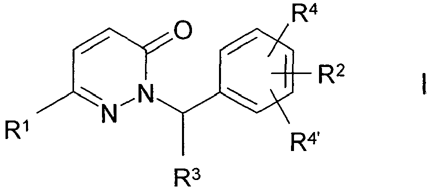 dans laquelle
R¹ désigne Ar ou Hét,
R² désigne un hétérocycle insaturé, saturé ou aromatique de 6 chaînons ayant de 1 à 4 atomes de N, O et/ou S, qui est mono-, di- ou trisubstitué par N=CR³N(R³)₂, SR³, NO₂, CN, COOR³, CON(R³)₂, NR³COA, NR³SO₂A, SO₂N(R³)₂, S(O)mA, [C(R³)₂]ₙN(R³)2, [C(R³)₂]ₙHét, O[C(R³)₂]ₚOR³, O[C(R³)₂]ₙN(R³)₂, O[C(R³)₂]ₙC≡C[C(R³)₂]ₙN(R³)₂, O[C(R³)₂]ₙN⁺O⁻(R³)2, O[C(R³)₂]ₙHét, S[C(R³)₂]ₙN(R³)₂, S[C(R³)₂]ₙHét, NR³[C(R³)₂]ₙN(R³)₂, NR³[C(R³)₂]ₙHét, NHCON(R³)₂, NHCONH[C(R³)₂]ₙN(R³)₂, NHCONH[C(R³)₂]ₙHét, [C(R³)₂]ₙNHCON[C(R³)₂]ₙ(R³)_{2,} [C(R³)₂]ₙNHCON[C(R³)₂]ₙHét, CON(R³)₂, CONR³[C(R³)₂]ₙN(R³)₂, CONR³[C(R³)₂]ₙNR³COOA, CONR³[C(R³)₂]ₙOR³, CONR³[C(R³)₂]ₙHét, COHét, COA, CH=CH-COOR³ et/ou CH=CH-N(R³)₂,
R³ désigne H ou A,
R⁴, R^{4'} désignent chacun, indépendamment l'un de l'autre, H, Hal, A, OR³, CN, COOR³, CON(R³)₂, NR³COA, NR³SO₂A, SO₂N(R³)₂ ou S(O)ₘA,
Ar désigne phényle, naphtyle ou biphényle, chacun d'entre eux étant non substitué ou mono-, di- ou trisubstitué par Hal, A, [C(R³)₂]ₙOR³, [C(R³)₂]ₙN(R³)₂, SR³, NO₂, CN, COOR³, CON(R³)₂, NR³COA, NR³SO₂A, SO₂N(R³)₂, S(O)mA, CO-Hét, Hét, O[C(R³)₂]ₙN(R³)₂, O[C(R³)₂]ₙHét, NHCOOA, NHCON(R³)₂, NHCOO[C(R³)₂]ₙN(R³)₂, NHCOO[C(R³)₂]ₙHét, NHCONH[C(R³)₂]ₙN(R³)₂, NHCONH[C(R³)₂]ₙHét, OCONH[C(R³)₂]ₙN(R³)₂, OCONH[C(R³)₂]ₙHét, CONR³[C(R³)₂]ₙN(R³)₂, CONR³[C(R³)₂]ₙHét et/ou COA,
Hét désigne un hétérocycle mono-, bi- ou tricyclique saturé, insaturé ou aromatique ayant de 1 à 4 atomes de N, O et/ou S, pouvant être non substitué ou mono-, di-, tri-, tétra- ou pentasubstitué par Hal, A, [C(R³)₂]ₙOR³, [C(R³)₂]ₙN(R³)₂, SR³, NO₂, CN, COOR³, CON(R³)₂, NR³COA, NR³SO₂A, SO₂N(R³)₂, S(O)mA, CO-Hét¹, [C(R³)₂]ₙHét¹, O[C(R³)₂]ₙN(R³)₂, O[C(R³)₂]ₙHét¹, NHCOOA, NHCON(R³)₂, NHCOO[C(R³)₂]ₙN(R³)₂, NHCOO[C(R³)₂]ₙHét¹, NHCONH[C(R³)₂]ₙN(R³)₂; NHCONH[C(R³)₂]ₙHét¹, OCONH[C(R³)₂]ₙN(R³)₂, OCONH[C(R³)₂]ₙHét¹, CO-Hét¹, CHO, COA, =S, =NH, =NA et/ou =O (oxygène du carbonyle), et où un azote de cycle peut être oxydé,
Hét¹ désigne un hétérocycle monocyclique saturé ayant de 1 à 2 atomes de N et/ou O, pouvant être mono- ou disubstitué par A, OA, OH, Hal et/ou =O (oxygène du carbonyle),
A désigne alkyle non ramifié ou ramifié ayant 1-10 atomes de C, où 1-7 atomes de H peuvent être remplacés par F et/ou où un ou deux groupements CH₂ non adjacents peuvent être remplacés par O, NH, S, SO, SO₂ et/ou par des groupements CH=CH, ou alkyle cyclique ayant 3-7 atomes de C,
Hal désigne F, Cl, Br ou I,
m désigne 0, 1 ou 2,
n désigne 0, 1, 2, 3 ou 4,
p désigne 1, 2, 3 ou 4,
et/ou les solvats, sels, tautomères et stéréoisomères pharmaceutiquement utilisables de ceux-ci, y compris des mélanges de ceux-ci en toutes proportions, et éventuellement des excipients et/ou des adjuvants, dans laquelle 0,5 mg à 1 mg d'un composé de formule I est présent.

2. Médicament selon la revendication 1, dans lesquels
R² désigne un hétérocycle insaturé, saturé ou aromatique de 6 chaînons ayant de 1 à 4 atomes de N et/ou O, qui est mono-, di- ou trisubstitué par N=CR³N(R³)₂, CN, COOR³, [C(R³)₂]ₙN(R³)₂, [C(R³)₂]ₙHét, O[C(R³)₂]ₚOR³, O[C(R³)₂]ₙN(R³)₂, O[C(R³)₂]ₙC≡C[C(R³)₂]ₙN(R³)₂, O[C(R³)₂]nN⁺O⁻(R³)₂, O[C(R³)₂]ₙHét, NR³[C(R³)₂]ₙN(R³)₂, NR³[C(R³)₂]ₙHét, [C(R³)₂]ₙNHCO[C(R³)₂]ₙN(R³)₂, [C(R³)₂]ₙNHCO[C(R³)₂]ₙHét, CONR³[C(R³)₂]ₙN(R³)₂, CONR³[C(R³)₂]ₙNR³COOA, CONR³[C(R³)₂]ₙOR³, CONR³[C(R³)₂]ₙHét, COHét, CH=CH-COOR³ et/ou CH=CH-N(R³)₂.

3. Médicament selon la revendication 1 ou 2, dans lesquels
Ar désigne phényle, naphtyle ou biphényle, chacun d'entre eux étant non substitué ou mono-, di- ou trisubstitué par A, Hal, CN, S(O)ₘA, NR³COA, CON(R³)₂, O[C(R³)₂]ₙN(R³)₂, [C(R³)₂]ₙOR³, CONR³[C(R³)₂]ₙN(R³)₂ et/ou CONR³[C(R³)₂]ₙHét,
et les dérivés, solvats, sels, tautomères et stéréoisomères pharmaceutiquement utilisables de ceux-ci, y compris des mélanges de ceux-ci en toutes proportions.

4. Médicament selon l'une ou plusieurs parmi les revendications 1-3,
dans lesquels
R⁴, R^{4'} désignent H.

5. Médicament selon l'une ou plusieurs parmi les revendications 1-4,
dans lesquels
Hét désigne un hétérocycle mono-, bi- ou tricyclique saturé, insaturé ou aromatique ayant de 1 à 4 atomes de N, O et/ou S, pouvant être non substitué ou mono-, di-, tri-, tétra- ou pentasubstitué par A, CHO, COOR³, CON(R³)₂, [C(R³)₂]ₙHét¹, [C(R³)₂]ₙOR³, [C(R³)₂]ₙN(R³)₂, O[C(R³)₂]ₙHét¹ et/ou =O (oxygène du carbonyle), et où un azote de cycle peut être oxydé.

6. Médicament selon l'une ou plusieurs parmi les revendications 1-5,
dans lesquels
Hét¹ désigne un hétérocycle monocyclique saturé ayant de 1 à 2 atomes de N et/ou O, pouvant être mono- ou disubstitué par A et/ou =O (oxygène du carbonyle).

7. Médicament selon l'une ou plusieurs parmi les revendications 1-6,
dans lesquels
A désigne alkyle non ramifié ou ramifié ayant 1-8 atomes de C, où 1-7 atomes de H peuvent être remplacés par F.

8. Médicament selon l'une ou plusieurs parmi les revendications 1-7,
dans lesquels
R¹ désigne Ar ou benzo-2,1,3-thiadiazolyle.

9. Médicament selon l'une ou plusieurs parmi les revendications 1-8,
dans lesquels
R³ désigne H, méthyle, éthyle ou propyle.

10. Médicament selon l'une ou plusieurs parmi les revendications 1-9,
dans lesquels
R² désigne pyrimidinyle, pyridazinyle, pyridinyle, 1,3-oxazinanyle, morpholinyle, pipéridinyle ou pipérazinyle, chacun d'entre eux étant mono-, di- ou trisubstitué par N=CR³N(R³)₂, CN, COOR³, [C(R³)₂]ₙN(R³)₂, [C(R³)₂]ₙHét, O[C(R³)₂]ₚOR³, O[C(R³)₂]ₙN(R³)₂, O[C(R³)₂]ₙC≡C[C(R³)₂]ₙN(R³)₂, O[C(R³)₂]ₙN⁺O⁻(R³)₂, O[C(R³)₂]ₙHét, NR³[C(R³)₂]ₙN(R³)₂, NR³[C(R³)₂]ₙHét, [C(R³)₂]ₙNHCO[C(R³)₂]ₙN(R³)₂, [C(R³)₂]ₙNHCO[C(R³)₂]ₙHét, CONR³[C(R³)₂]ₙN(R³)₂, CONR³[C(R³)₂]ₙNR³COOA, CONR³[C(R³)₂]ₙOR³, CONR³[C(R³)₂]ₙHét, COHét, CH=CH-COOR³ et/ou CH=CH-N(R³)₂.

11. Médicament selon l'une ou plusieurs parmi les revendications 1-10,
dans lesquels
Hét désigne pipéridinyle, pipérazinyle, pyrrolidinyle, morpholinyle, furyle, thiényle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle, benzimidazolyle, benzotriazolyle, indolyle, benzo-1,3-dioxolyle, indazolyle, azabicyclo[3.2.1]octyle, azabicyclo[2.2.2]octyle, imidazolidinyle, azépanyle ou benzo-2,1,3-thiadiazolyle, chacun d'entre eux étant non substitué ou mono-, di-, tri-, tétra- ou pentasubstitué par A, CHO, COOR³, CON(R³)₂, [C(R³)₂]ₙHét¹, [C(R³)₂]ₙOR³, [C(R³)₂]ₙN(R³)₂, O[C(R³)₂]ₙHét¹ et/ou =O (oxygène du carbonyle), et où un azote de cycle peut être oxydé.

12. Médicament selon l'une ou plusieurs parmi les revendications 1-11,
dans lesquels
Hét¹ désigne pyrrolidinyle, pipéridinyle, pipérazinyle ou morpholinyle, chacun d'entre eux étant non substitué ou mono- ou disubstitué par A et/ou =O (oxygène du carbonyle).

13. Médicament selon l'une ou plusieurs parmi les revendications 1-12,
dans lesquels
R¹ désigne Ar ou Hét,
R² désigne pyrimidinyle, pyridazinyle, pyridinyle, 1,3-oxazinanyle, morpholinyle, pipéridinyle ou pipérazinyle, chacun d'entre eux étant mono-, di- ou trisubstitué par N=CR³N(R³)₂, CN, COOR³, [C(R³)₂]ₙN(R³)₂, [C(R³)₂]ₙHét, O[C(R³)₂]ₚOR³, O[C(R³)₂]ₙN(R³)₂, O[C(R³)₂]ₙC≡C[C(R³)₂]ₙN(R³)₂, O[C(R³)₂]ₙN⁺O⁻(R³)₂, O[C(R³)₂]ₙHét, NR³[C(R³)₂]ₙN(R³)₂, NR³[C(R³)₂]ₙHét, [C(R³)₂]ₙNHCO[C(R³)₂]ₙN(R³)₂, [C(R³)₂]ₙNHCO[C(R³)₂]ₙHét, CONR³[C(R³)₂]ₙN(R³)₂, CONR³[C(R³)₂]ₙNR³COOA, CONR³[C(R³)₂]ₙOR³, CONR³[C(R³)₂]ₙHét, COHét, CH=CH-COOR³ et/ou CH=CH-N(R³)₂,
R³ désigne H, méthyle, éthyle ou propyle,
R⁴, R^{4'} désignent H,
Ar désigne phényle, naphtyle ou biphényle, chacun d'entre eux étant non substitué ou mono-, di- ou trisubstitué par A, Hal, CN, S(O)mA, NR³COA, CON(R³)₂, O[C(R³)₂]ₙN(R³)₂, [C(R³)₂]ₙOR³, CONR³[C(R³)₂]ₙN(R³)₂ et/ou CONR³[C(R³)₂]ₙHét,
Hét désigne pipéridinyle, pipérazinyle, pyrrolidinyle, morpholinyle, furyle, thiényle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle, benzimidazolyle, benzotriazolyle, indolyle, benzo-1,3-dioxolyle, indazolyle, azabicyclo[3.2.1]octyle, azabicyclo[2.2.2]octyle, imidazolidinyle, azépanyle ou benzo-2,1,3-thiadiazolyle, chacun d'entre eux étant non substitué ou mono-, di-, tri-, tétra- ou pentasubstitué par A, CHO, COOR³, CON(R³)₂, [C(R³)₂]ₙHét¹, [C(R³)₂]ₙOR³, [C(R³)₂]ₙN(R³)₂, O[C(R³)₂]ₙHét¹ et/ou =O (oxygène du carbonyle), et où un azote de cycle peut être oxydé,
Hét¹ désigne pyrrolidine, pipéridine, pipérazine ou morpholine, chacun d'entre eux étant non substitué ou mono- ou disubstitué par A et/ou =O (oxygène du carbonyle),
A désigne alkyle non ramifié ou ramifié ayant 1-8 atomes de C, où 1-7 atomes de H peuvent être remplacés par F,
Hal désigne F, Cl, Br ou I,
m désigne 0, 1 ou 2,
n désigne 0, 1, 2, 3 ou 4,
p désigne 1, 2, 3 ou 4.

14. Médicament selon la revendication 1, comprenant au moins un composé de formule I choisis dans le groupe constitué par
| n° | Nom et/ou structure |
|---|---|
| "A11" | 2-[3-(5-aminopyridin-2-yl)benzyl]-6-(3,5-difluorophényl)-2H-pyridazin-3-one |
| "A12" | 6-(3,5-difluorophényl)-2-{3-[5-(4-méthylpipérazin-1-yl)pyridin-2-yl]benzyl}-2H-pyridazin-3-one |
| "A13" | 6-(3,5-difluorophényl)-2-[3-(4-pipérazin-1-ylpyrimidin-2-yl)benzyl]-2H-pyridazin-3-one |
| "A14" | 6-(3,5-difluorophényl)-2-{3-[5-(4-méthylpipérazin-1-yl-méthyl)pyrimidin-2-yl]benzyl}-2H-pyridazin-3-one |
| "A16" | N'-(2-{3-[3-(3,5-difluorophényl)-6-oxo-6H-pyridazin-1-yl-méthyl]phényl}pyrimidin-5-yl)-N,N-diméthylformamidine |
| "A17" | 2-[3-(5-aminopyrimidin-2-yl)benzyl]-6-(3,5-difluorophényl)-2H-pyridazin-3-one |
| "A18" | 6-(3,5-difluorophényl)-2-{3-[5-(4-méthylpipérazin-1-yl)pyrimidin-2-yl]benzyl}-2H-pyridazin-3-one |
| "A19" | 6-(3,5-difluorophényl)-2-[3-(5-pipérazin-1-ylpyrimidin-2-yl)-benzyl]-2H-pyridazin-3-one |
| "A20" | 2-{3-[5-(4-méthylpipérazin-1-yl)pyrimidin-2-yl]benzyl}-6-(3,4,5-trifluorophényl)-2H-pyridazin-3-one |
| "A22" | 6-(3,5-difluorophényl)-2-{3-[5-(3-diméthylamino-propoxy)pyrimidin-2-yl]benzyl}-2H-pyridazin-3-one |
| "A23" | 6-(3,5-difluorophényl)-2-{3-[5-(1-méthylpipéridin-4-yloxy)-pyrimidin-2-yl]benzyl}-2H-pyridazin-3-one |
| | 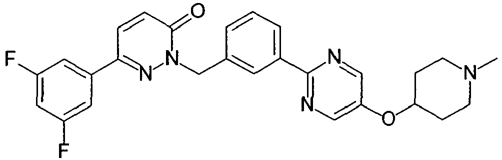 |
| "A24" | 6-(3,5-difluorophényl)-2-{3-[5-(3-diméthylamino-propoxy)pyridin-2-yl]benzyl}-2H-pyridazin-3-one |
| "A26" | 6-(3,5-difluorophényl)-2-{3-[5-(1-méthyl-1H-pyrazol-4-yl)-pyrimidin-2-yl]benzyl}-2H-pyridazin-3-one |
| "A27" | 6-(3,5-difluorophényl)-2-{3-[6-(4-méthylpipérazin-1-yl)-pyridazin-3-yl]benzyl}-2H-pyridazin-3-one |
| "A28" | 6-(3,5-difluorophényl)-2-{3-[6-(3-diméthylamino-propoxy)pyridazin-3-yl]benzyl}-2H-pyridazin-3-one |
| "A29" | 2-{3-[6-oxo-3-(3,4,5-trifluorophényl)-6H-pyridazin-1-yl-méthyl]phényl}pyrimidine-5-carboxylate d'éthyle |
| "A30" | 2-{3-[3-(3,5-difluorophényl)-6-oxo-6H-pyridazin-1-yl-méthyl]phényl}pyrimidine-5-carboxylate d'éthyle |
| | 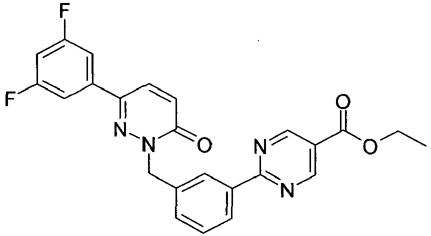 |
| "A31" | acide 2-{3-[6-oxo-3-(3,4,5-trifluorophényl)-6H-pyridazin-1-yl-méthyl]phényl}pyrimidine-5-carboxylique |
| "A32" | acide 2-{3-[3-(3,5-difluorophényl)-6-oxo-6H-pyridazin-1-yl-méthyl]phényl}pyrimidine-5-carboxylique |
| "A33" | N-(2-diméthylaminoéthyl)-2-{3-[6-oxo-3-(3,4,5-trifluoro-phényl)-6H-pyridazin-1-ylméthyl]phényl}pyrimidine-5-carboxamide |
| "A34" | 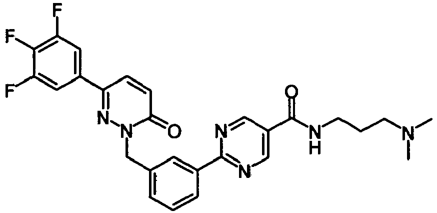 |
| "A35" | 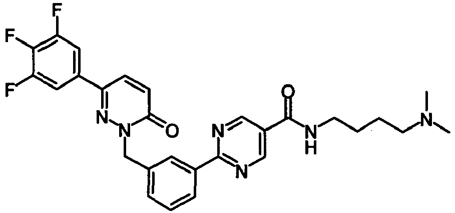 |
| "A36" | 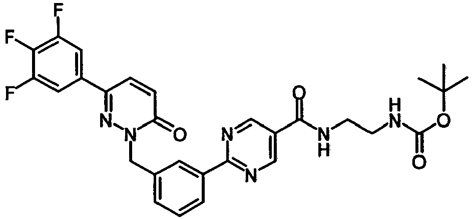 |
| "A37" | 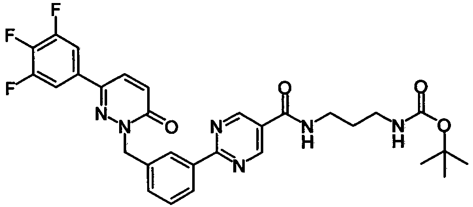 |
| "A38" | 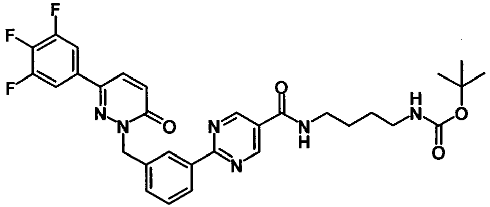 |
| "A39" | 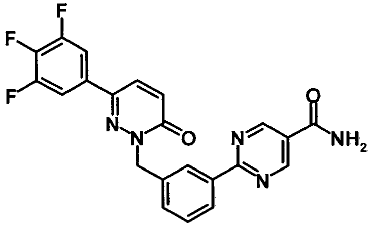 |
| "A40" | 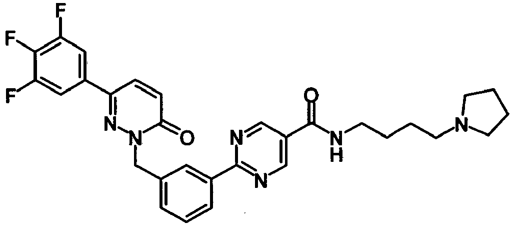 |
| "A41" | 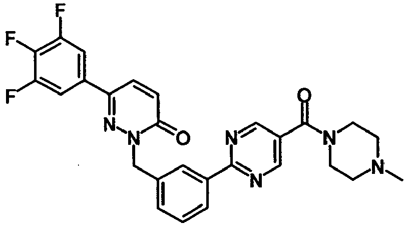 |
| "A42" | 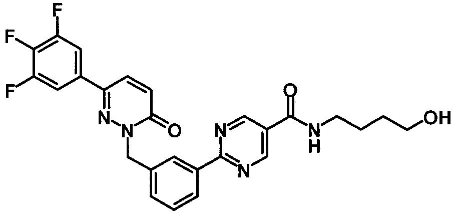 |
| "A43" | 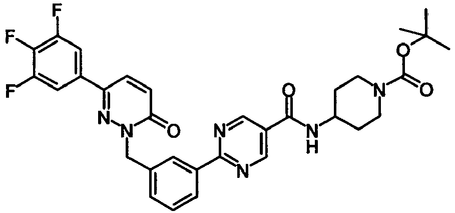 |
| "A44" | 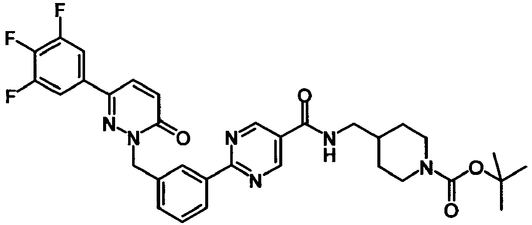 |
| "A44a" | 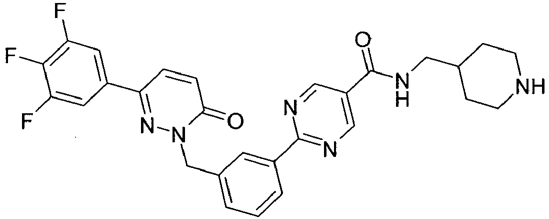 |
| "A45" | 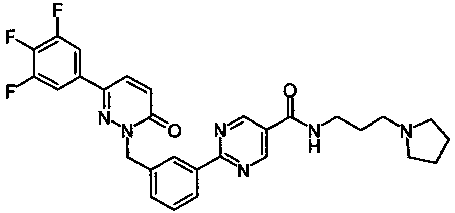 |
| "A46" | 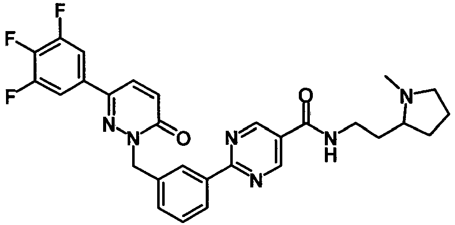 |
| "A47" | 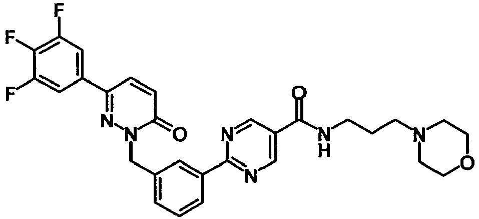 |
| "A48" | 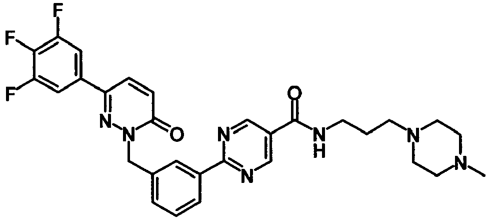 |
| "A49" | 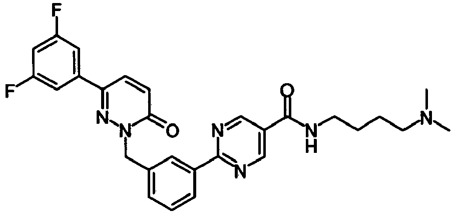 |
| "A50" | 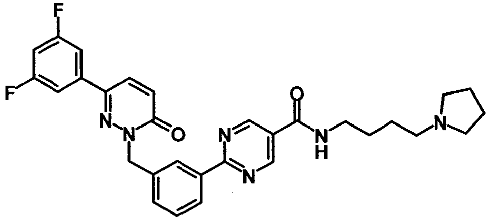 |
| "A51" | N-[2-(1H-imidazol-4-yl)éthyl]-2-{3-[6-oxo-3-(3,4,5-trifluoro-phényl)-6H-pyridazin-1-ylméthyl]phényl}pyrimidine-5-carboxamide |
| "A52" | 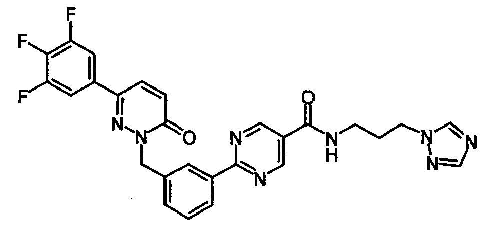 |
| "A53" | 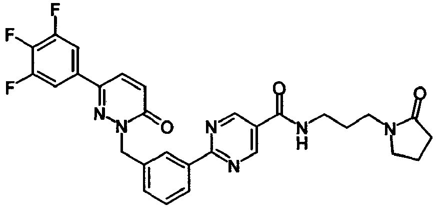 |
| "A54" | 2-[3-(5-chloropyrimidin-2-yl)benzyl]-6-(3,4,5-trifluorophényl)-2H-pyridazin-3-one |
| "A55" | 4-{1-[3-(5-méthylpyrimidin-2-yl)benzyl]-6-oxo-1,6-dihydro-pyridazin-3-yl}-N-(3-pipéridin-1-ylpropyl)benzamide |
| "A56" | 6-(3,5-difluorophényl)-2-{3-[5-(3-pyrrolidin-1-yl-propoxy)pyrimidin-2-yl]benzyl}-2H-pyridazin-3-one |
| "A57" | 6-(3,5-difluorophényl)-2-(3-{5-[2-(4-méthylpipérazin-1-yl)-éthoxy]pyrimidin-2-yl}benzyl)-2H-pyridazin-3-one |
| "A58" | 6-(3,5-difluorophényl)-2-[3-(5-diméthylaminométhyl-pyrimidin-2-yl)benzyl]-2H-pyridazin-3-one |
| "A59" | 6-(3,5-difluorophényl)-2-{3-[4-(méthylpipéridin-4-yl-amino)pyrimidin-2-yl]benzyl}-2H-pyridazin-3-one |
| | 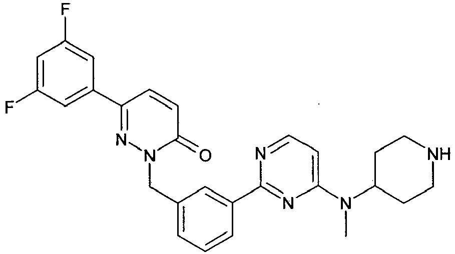 |
| "A60" | 2-{3-[5-(3-diméthylaminopropoxy)pyrimidin-2-yl]benzyl}-6-(3,4,5-trifluorophényl)-2H-pyridazin-3-one |
| | |
| | |
| "A63" | 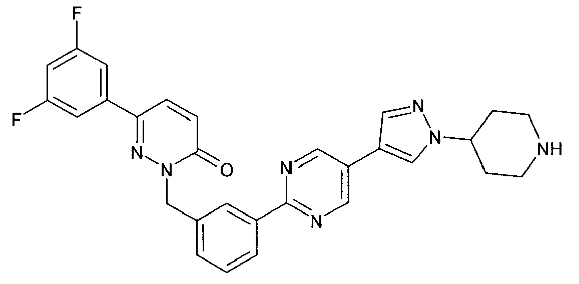 |
| "A64" | 2-{3-[5-(2-diméthylaminoéthoxy)pyrimidin-2-yl]benzyl}-6-(3,5-difluorophényl)-2H-pyridazin-3-one |
| "A65" | 2-{3-[5-(pipérazin-1-yl)pyrimidin-2-yl]benzyl}-6-(3,4,5-trifluorophényl)-2H-pyridazin-3-one |
| "A66" | 6-(3,5-difluorophényl)-2-{3-[5-(1-méthylpipéridin-4-yl-méthoxy)pyrimidin-2-yl]benzyl}-2H-pyridazin-3-one |
| "A67" | 6-(3,5-difluorophényl)-2-{3-[6-(3-diméthylaminopropyl-amino)pyridazin-3-yl]benzyl}pyridazin-3-one |
| "A68" | 6-(3,5-difluorophényl)-2-{3-[6-(2-diméthylaminoéthyl-amino)pyridazin-3-yl]benzyl}pyridazin-3-one |
| "A69" | 6-(3,5-difluorophényl)-2-{3-[6-(4-diméthylaminobutyl-amino)pyridazin-3-yl]benzyl}pyridazin-3-one |
| "A70" | 6-(3,5-difluorophényl)-2-{3-[6-(1-méthylpipéridin-4-yl-amino)pyridazin-3-yl]benzyl}pyridazin-3-one |
| "A71" | 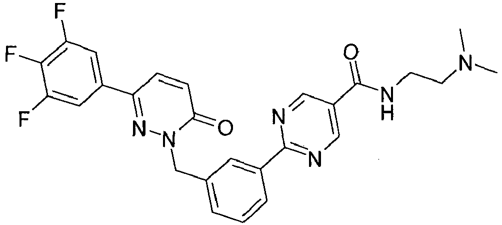 |
| "A72" | 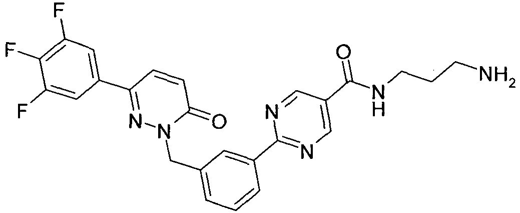 |
| "A73" | 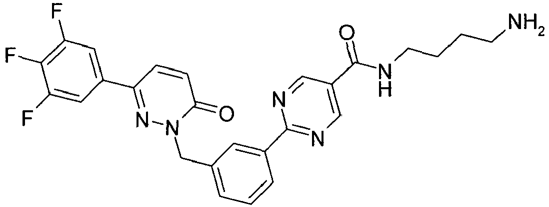 |
| "A74" | 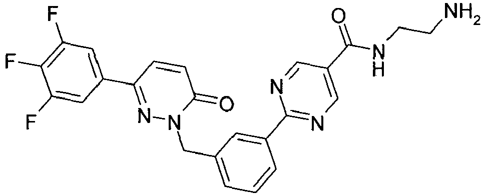 |
| "A75" | 4-(2-{3-[3-(3,5-difluorophényl)-6-oxo-6H-pyridazin-1-yl-méthyl]phényl}pyrimidin-5-yl)morpholin-3-one |
| | 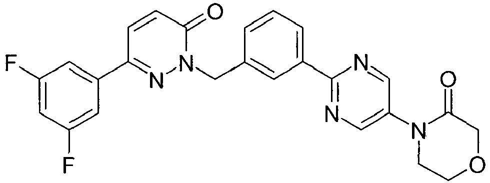 |
| "A76" | N'-(2-{3-[3-(3,4,5-trifluorophényl)-6-oxo-6H-pyridazin-1-yl-méthyl]phényl}pyrimidin-5-yl)-N,N-diméthylformamidine |
| "A77" | 2-{3-[6-oxo-3-(3,4,5-trifluorophényl)-6H-pyridazin-1-yl-méthyl]phényl}pyrimidine-5-carbonitrile |
| | 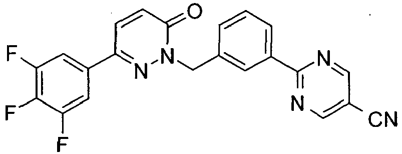 |
| "A81" | 6-(3,5-difluorophényl)-2-{3-[4-(3-diméthylamino-propoxy)pyrimidin-2-yl]benzyl}-2H-pyridazin-3-one |
| "A82" | 2-[3-(5-aminopyrimidin-2-yl)benzyl]-6-(3,4,5-trifluorophényl)-2H-pyridazin-3-one |
| "A84" | 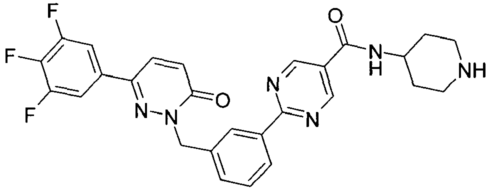 |
| "A85" | 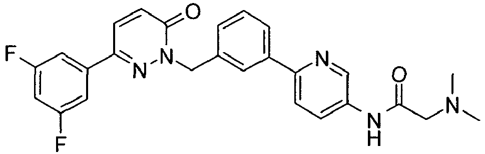 N-(2-{3-[3-(3,5-difluorophényl)-6-oxo-6H-pyridazin-1-yl-méthyl]phényl}pyrimidin-5-yl)-2-diméthylaminoacétamide |
| "A87" | N-(2-{3-[3-(3,5-difluorophényl)-6-oxo-6H-pyridazin-1-yl-méthyl]phényl}pyrimidin-5-yl)-4-diméthylaminobutyramide |
| | 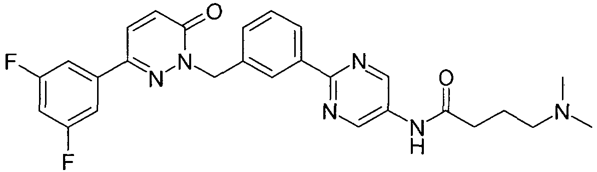 |
| "A88" | 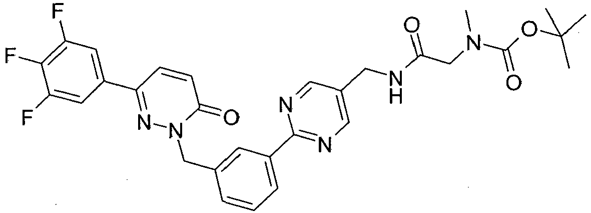 |
| "A89" | 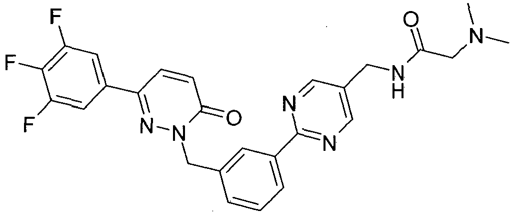 |
| "A90" | 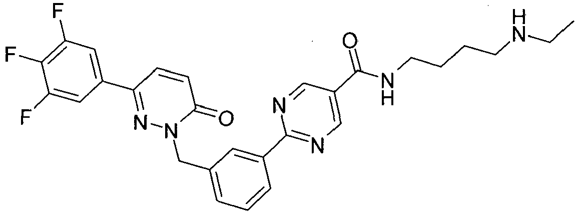 |
| "A92" | 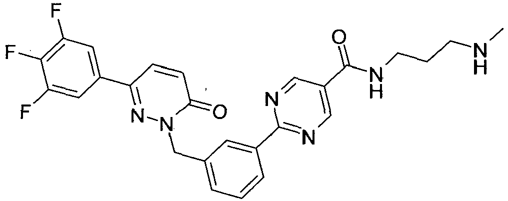 |
| "A93" | 2-[3-(5-aminométhylpyrimidin-2-yl)benzyl]-6-(3,4,5-trifluoro-phényl)-2H-pyridazin-3-one |
| "A95" | N-(2-{3-[3-(3,5-difluorophényl)-6-oxo-6H-pyridazin-1-yl-méthyl]phényl}pyrimidin-5-yl)-3-diméthylaminopropion-amide |
| | 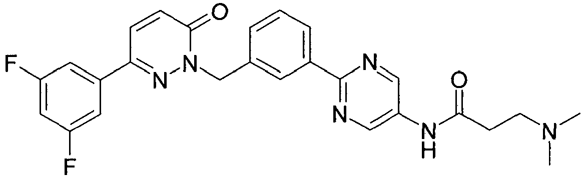 |
| "A96" | 3-(4-méthylpipérazin-1-yl)-N-(2-{3-[6-oxo-3-(3,4,5-trifluoro-phényl)-6H-pyridazin-1-ylméthyl]phényl}pyrimidin-5-yl-méthyl)propionamide |
| | 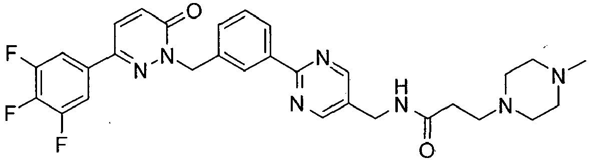 |
| "A97" | 2-(4-méthylpipérazin-1-yl)-N-(2-{3-[6-oxo-3-(3,4,5-trifluoro-phényl)-6H-pyridazin-1-ylméthyl]phényl}pyrimidin-5-yl-méthyl)acétamide |
| | 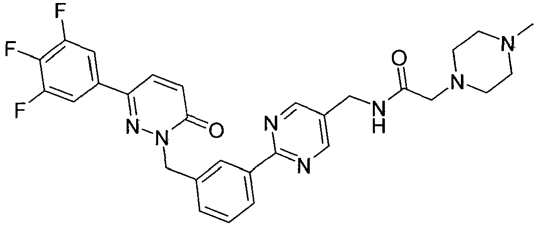 |
| "A98" | 2-méthylamino-N-(2-{3-[6-oxo-3-(3,4,5-trifluorophényl)-6H-pyridazin-1-ylméthyl]phényl}pyrimidin-5-ylméthyl)acétamide |
| | 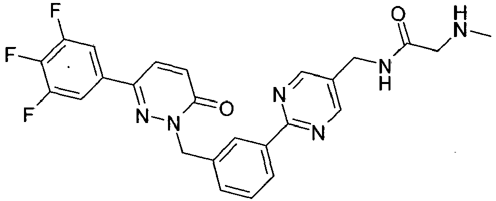 |
| "A99" | 3-diméthylamino-N-(2-{3-[6-oxo-3-(3,4,5-trifluorophényl)-6H-pyridazin-1-ylméthyl]phényl}pyrimidin-5-ylméthyl)-propionamide |
| | 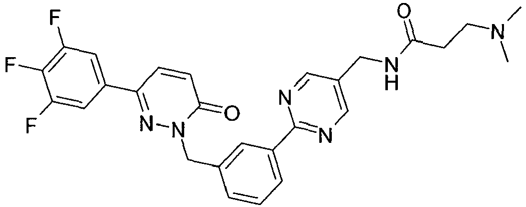 |
| "A101" | 6-(3,5-difluorophényl)-2-[3-(5-hydroxyméthylpyrimidin-2-yl)-benzyl]-2H-pyridazin-3-one |
| "A102" | 6-(3,5-diftuorophényl)-2-{3-[5-(pipéridin-4-yloxy)pyrimidin-2-yl]benzyl}-2H-pyridazin-3-one |
| "A103" | 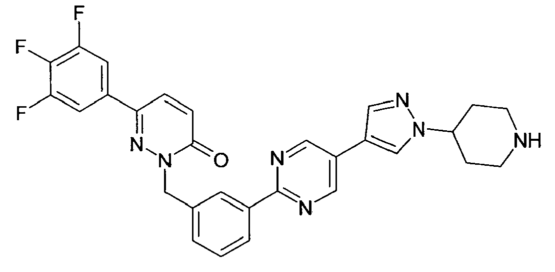 |
| "A104" | 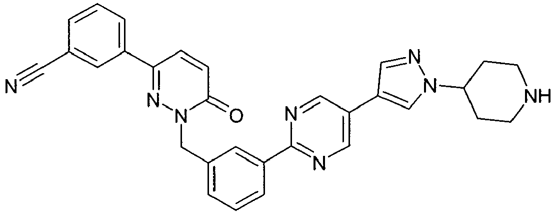 |
| "A105" | 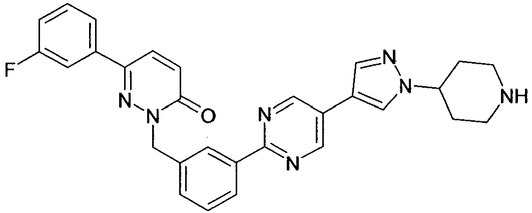 |
| "A106" | 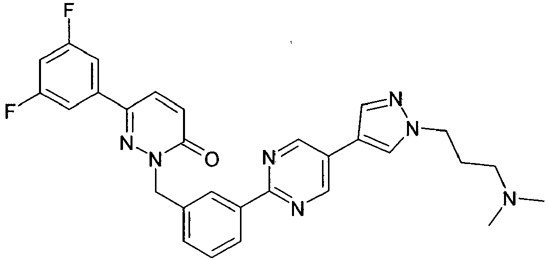 |
| "A107" | 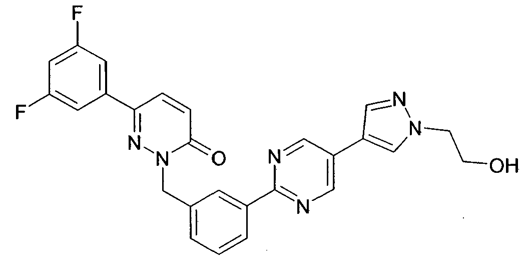 |
| "A108" | 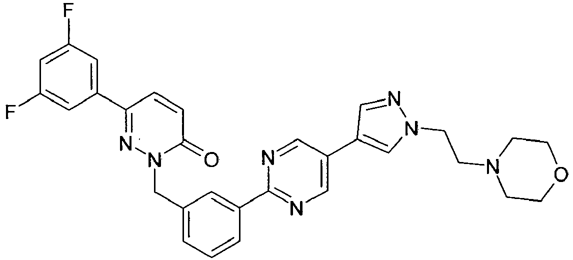 |
| "A109" | 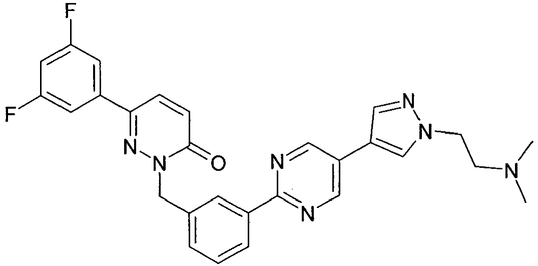 |
| "A110" | 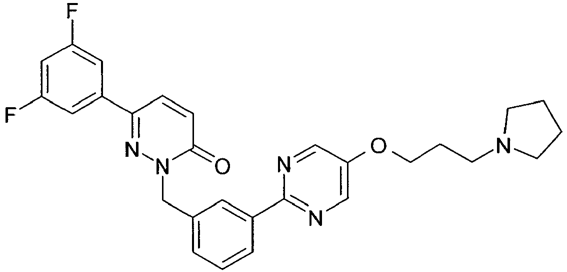 |
| "A111" | 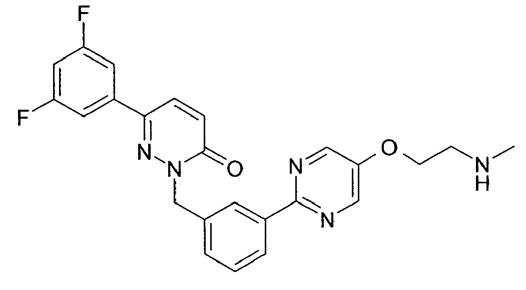 |
| "A114" | 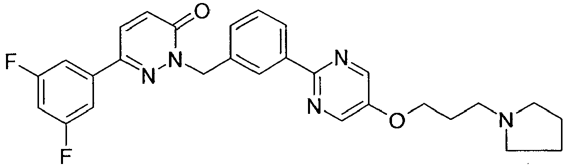 |
| "A115" | 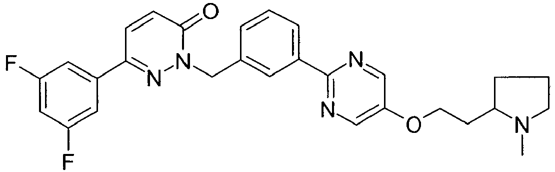 |
| "A116" | 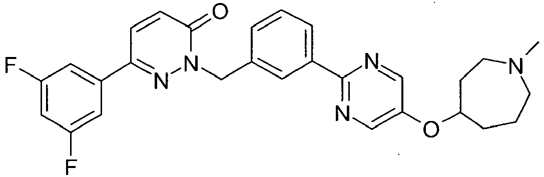 |
| "A117" | 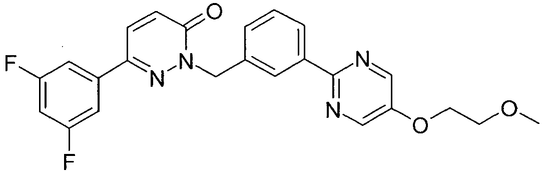 |
| "A118" | 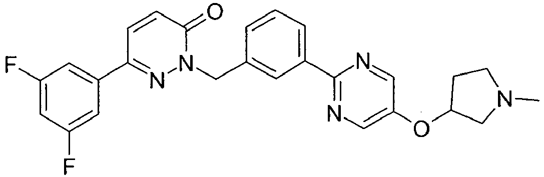 |
| "A119" | 6-(3,5-difluorophényl)-2-{3-[5-(8-méthyl-8-azabicyclo[3.2.1]-oct-3-yloxy)pyrimidin-2-yl]benzyl}-2H-pyridazin-3-one |
| | 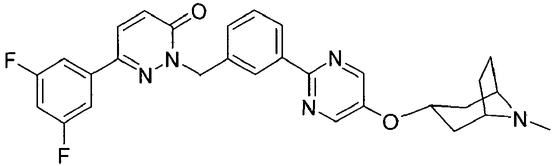 |
| "A120" | 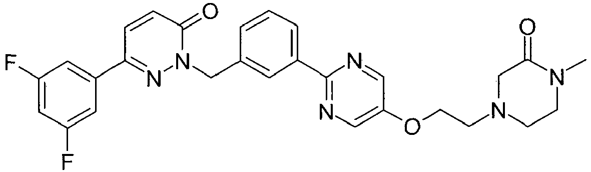 |
| "A121" | 6-(3,5-difluorophényl)-2-{3-[5-((S)-1-méthylpyrrolidin-3-yl-oxy)pyrimidin-2-yl]benzyl}-2H-pyridazin-3-one |
| | 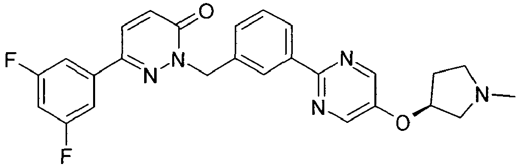 |
| "A122" | 6-(3,5-difluorophényl)-2-{3-[5-((R)-1-méthylpyrrolidin-3-yl-oxy)pyrimidin-2-yl]benzyl}-2H-pyridazin-3-one |
| "A123" | 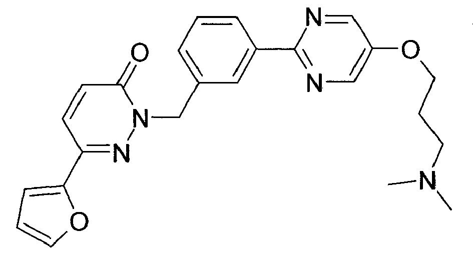 |
| "A124" | 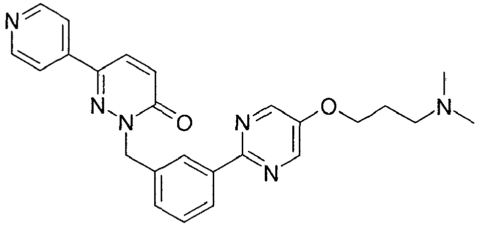 |
| "A125" | 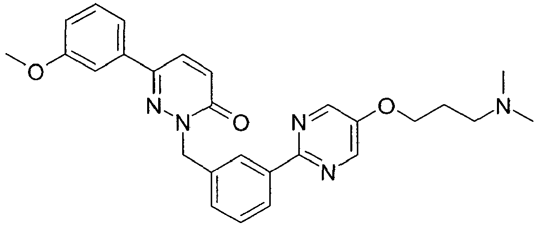 |
| "A126" | 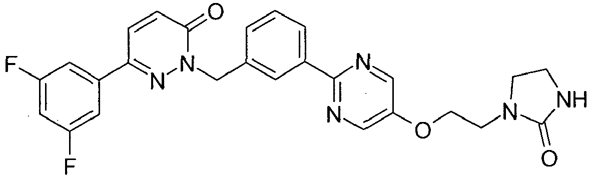 |
| "A127" | 6-(3,5-difluorophényl)-2-{3-[5-(2-pyrrolidin-1-yl-éthoxy)pyrimidin-2-yl]benzyl}-2H-pyridazin-3-one |
| "A128" | 6-(3,5-difluorophényl)-2-{3-[5-(3-morpholin-4-yl-propoxy)pyrimidin-2-yl]benzyl}-2H-pyridazin-3-one |
| "A129" | 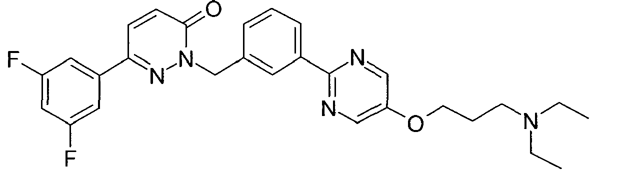 |
| "A130" | 6-(3,5-difluorophényl)-2-{3-[5-(2-morpholin-4-yl-éthoxy)pyrimidin-2-yl]benzyl}-2H-pyridazin-3-one, chlorhydrate |
| "A131" | 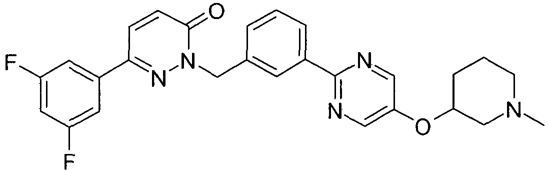 |
| "A132" | 6-(3,5-difluorophényl)-2-{3-[5-(4-méthylamino-butoxy)pyrimidin-2-yl]benzyl}-2H-pyridazin-3-one |
| "A133" | 6-(3,5-difluorophényl)-2-{3-[5-(3-méthylamino-propoxy)pyrimidin-2-yl]benzyl}-2H-pyridazin-3-one |
| "A134" | 6-(3,5-difluorophényl)-2-{3-[5-(pyrrolidin-3-ylméthoxy)-pyrimidin-2-yl]benzyl}-2H-pyridazin-3-one |
| "A135" | 6-(3,5-difluorophényl)-2-{3-[5-(3-éthylaminopropoxy)-pyrimidin-2-yl]benzyl}-2H-pyridazin-3-one |
| "A136" | 2-{3-[5-(2-aminoéthoxy)pyrimidin-2-yl]benzyl}-6-(3,5-difluorophényl)-2H-pyridazin-3-one |
| "A 137" | 6-(3,5-difluorophényl)-2-{3-[5-(pipéridin-3-yloxy)pyrimidin-2-yl]benzyl}-2H-pyridazin-3-one |
| "A138" | 6-(3,5-difluorophényl)-2-{3-[5-(pipéridin-4-ylméthoxy)-pyrimidin-2-yl]benzyl}-2H-pyridazin-3-one |
| "A139" | 6-(3,5-difluorophényl)-2-{3-[5-(pyrrolidin-3-yloxy)pyrimidin-2-yl]benzyl}-2H-pyridazin-3-one |
| "A140" | 6-(3,5-difluorophényl)-2-{3-[5-((S)-pyrrolidin-3-yloxy)-pyrimidin-2-yl]benzyl}-2H-pyridazin-3-one |
| "A141" | 6-(3,5-difluorophényl)-2-{3-[5-((R)-pyrrolidin-3-yloxy)-pyrimidin-2-yl]benzyl}-2H-pyridazin-3-one |
| "A142" | 2-{3-[5-(pipéridin-4-yloxy)pyrimidin-2-yl]benzyl}-6-pyridin-4-yl-2H-pyridazin-3-one |
| | 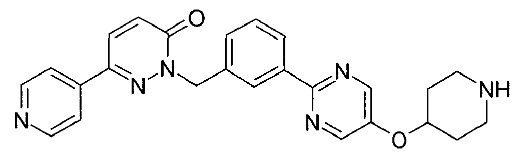 |
| "A143" | 4-(6-oxo-1-{3-[5-(pipéridin-4-yloxy)pyrimidin-2-yl]benzyl}-1,6-dihydropyridazin-3-yl)benzonitrile |
| | 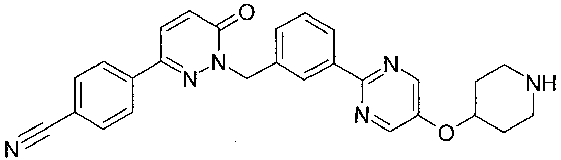 |
| "A144" | 3-(6-oxo-1-{3-[5-(pipéridin-4-yloxy)pyrimidin-2-yl]benzyl}-1,6-dihydropyridazin-3-yl)benzonitrile |
| "A145" | 6-(3,5-difluorophényl)-2-{3-[5-(2-pipérazin-1-yléthoxy)-pyrimidin-2-yl]benzyl}-2H-pyridazin-3-one |
| "A146" | 6-(3,5-difluorophényl)-2-{3-[5-(pipéridin-4-yloxy)pyrimidin-2-yl]benzyl}-2H-pyridazin-3-one |
| "A147" | 3-(6-oxo-1-{3-[5-(2-pipérazin-1-yléthoxy)pyrimidin-2-yl]-benzyl}-1,6-dihydropyridazin-3-yl)benzonitrile |
| "A148" | 6-(3-fluorophényl)-2-{3-[5-(pipéridin-4-ylméthoxy)pyrimidin-2-yl]benzyl}-2H-pyridazin-3-one |
| "A149" | 2-{3-[5-(1-pipéridin-4-yl-1H-pyrazol-4-yl)pyrimidin-2-yl]-benzyl}-6-(3,4,5-trifluorophényl)-2H-pyridazin-3-one |
| | 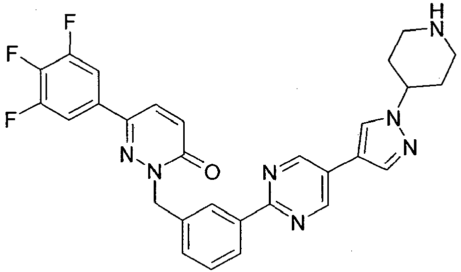 |
| "A150" | 3-(6-oxo-1-{3-[5-(1-pipéridin-4-yl-1H-pyrazol-4-yl)pyrimidin-2-yl]benzyl}-1,6-dihydropyridazin-3-yl)benzonitrile |
| | 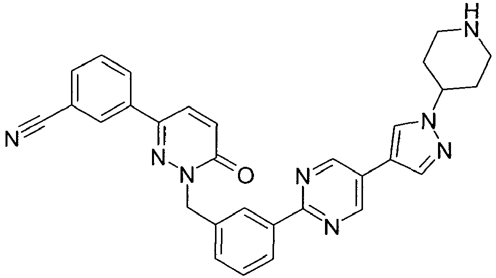 |
| "A151" | 6-(1-méthyl-1H-pyrazol-4-yl)-2-{3-[5-(1-pipéridin-4-yl-1H-pyrazol-4-yl)pyrimidin-2-yl]benzyl}-2H-pyridazin-3-one |
| | 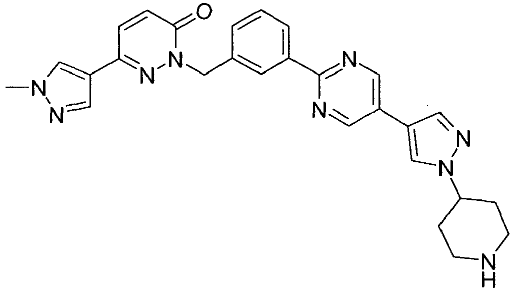 |
| "A152" | 6-(3-méthoxyphényl)-2-{3-[5-(1-pipéridin-4-yl-1H-pyrazol-4-yl)pyrimidin-2-yl]benzyl}-2H-pyridazin-3-one |
| "A153" | 6-(3-fluorophényl)-2-{3-[5-(1-pipéridin-4-yl-1H-pyrazol-4-yl)-pyrimidin-2-yl]benzyl}-2H-pyridazin-3-one |
| "A154" | 6-(3,5-difluorophényl)-2-{3-[5-(1H-pyrazol-4-yl)pyrimidin-2-yl]benzyl}-2H-pyridazin-3-one |
| | 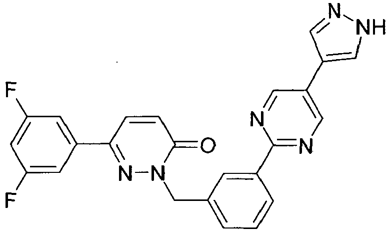 |
| "A155" | 6-(3,5-difluorophényl)-2-(3-{5-[1-(2-méthylaminoéthyl)-1H-pyrazol-4-yl]pyrimidin-2-yl}benzyl)-2H-pyridazin-3-one |
| | 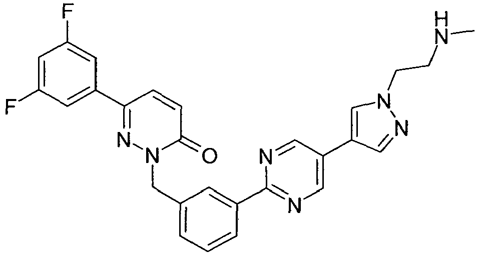 |
| "A156" | 6-(3-chlorophényl)-2-{3-[5-(1-pipéridin-4-yl-1H-pyrazol-4-yl)-pyrimidin-2-yl]benzyl}-2H-pyridazin-3-one |
| | 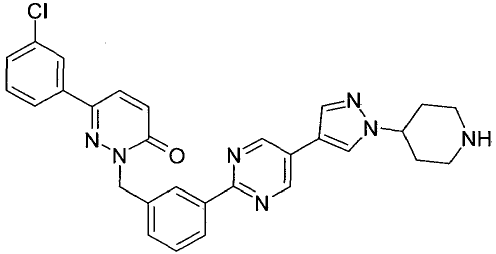 |
| "A157" | 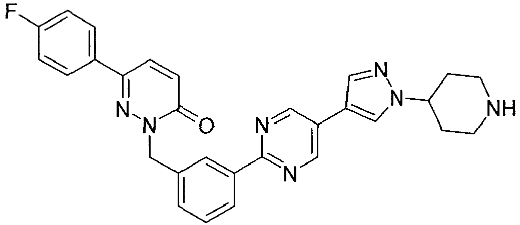 |
| "A158" | 6-(3,5-difluorophényl)-2-(3-{5-[1-(3-méthylaminopropyl)-1H-pyrazol-4-yl]pyrimidin-2-yl}benzyl)-2H-pyridazin-3-one |
| | 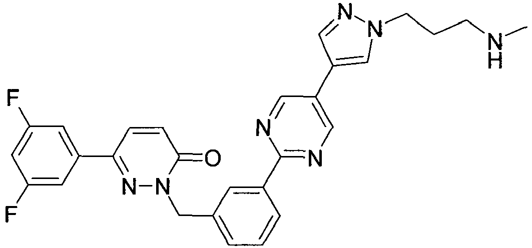 |
| "A159" | 3-[1-(3-{5-[1-(2-méthylaminoéthyl)-1H-pyrazol-4-yl]pyrimidin-2-yl}benzyl)-6-oxo-1,6-dihydropyridazin-3-yl]benzonitrile |
| | 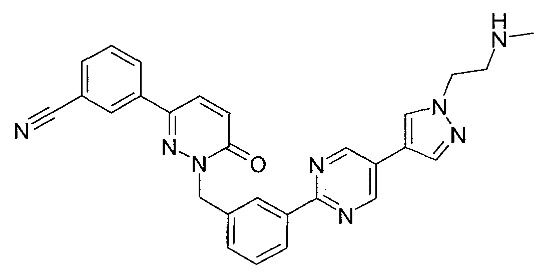 |
| "A160" | 6-(3,5-difluorophényl)-2-(3-{5-[1-(2-pipérazin-1-yléthyl)-1H-pyrazol-4-yl]pyrimidin-2-yl}benzyl)-2H-pyridazin-3-one |
| | 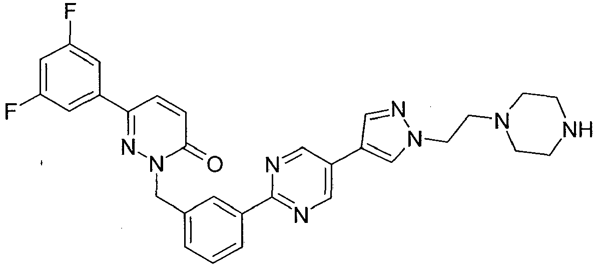 |
| "A161" | 3-(6-oxo-1-{3-[5-(1H-pyrazol-4-yl)pyrimidin-2-yl]benzyl}-1,6-dihydropyridazin-3-yl)benzonitrile |
| | 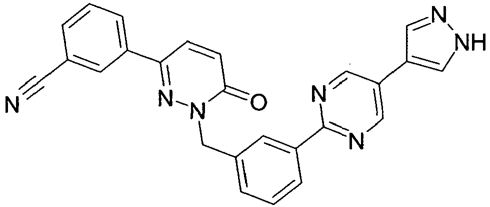 |
| "A162" | 3-[6-oxo-1-(3-{5-[1-(2-pipérazin-1-yléthyl)-1H-pyrazol-4-yl]-pyrimidin-2-yl}benzyl)-1,6-dihydropyridazin-3-yl]benzonitrile |
| | 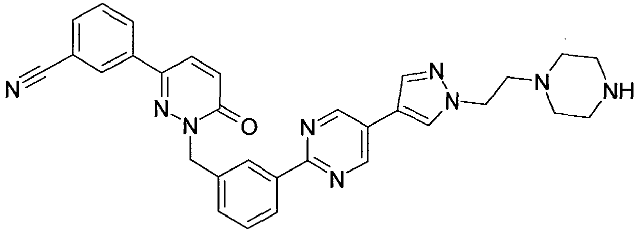 |
| "A163" | N-(4-diméthylaminobutyl)-2-[3-(6-oxo-3-pyridin-4-yl-6H-pyridazin-1-ylméthyl)phényl]pyrimidine-5-carboxamide |
| | 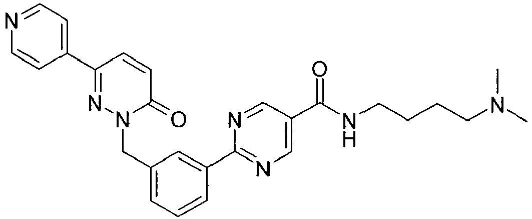 |
| "A164" | N-(4-diméthylaminobutyl)-2-{3-[3-(4-cyanophényl)-6-oxo-6H-pyridazin-1-ylméthyl]phényl}pyrimidine-5-carboxamide |
| | 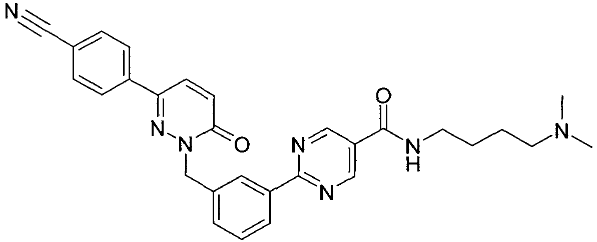 |
| "A165" | 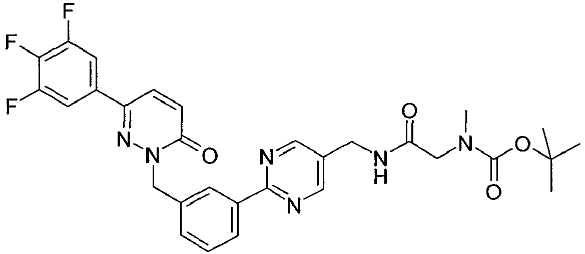 |
| "A166" | 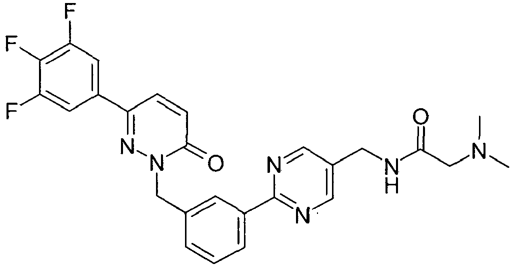 |
| "A167" | 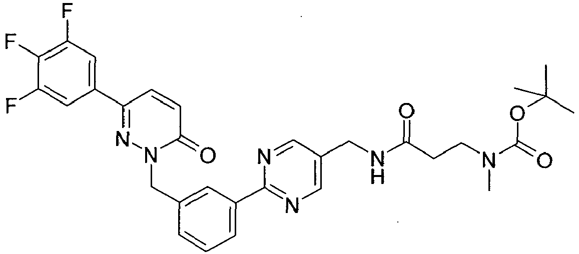 |
| "A168" | 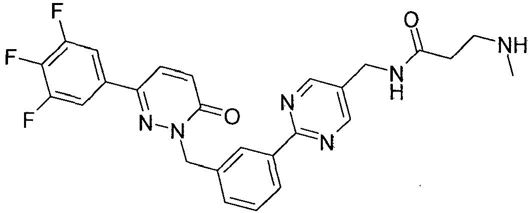 |
| "A169" | 6-(1-méthyl-1H-pyrazol-4-yl)-2-(3-{5-[1-(2-pyrrolidin-1-yl-éthyl)-1H-pyrazol-4-yl]pyrimidin-2-yl}benzyl)-2H-pyridazin-3-one |
| | 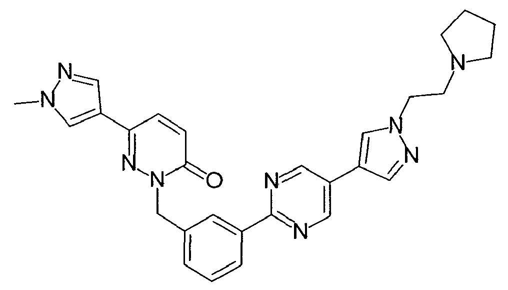 |
| "A170" | 6-(3,5-difluorophényl)-2-(3-{5-[1-(2-morpholin-4-yléthyl)-1H-pyrazol-4-yl]pyrimidin-2-yl}benzyl)-2H-pyridazin-3-one |
| | 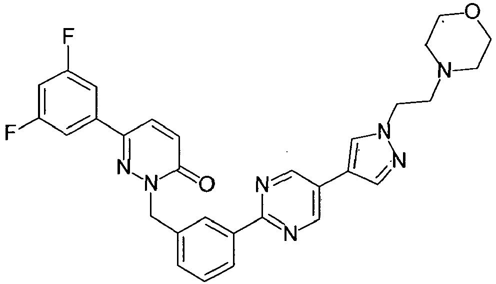 |
| "A171" | 6-(3,5-difluorophényl)-2-(3-{5-[1-(2-diméthylaminoéthyl)-1H-pyrazol-4-yl]pyrimidin-2-yl}benzyl)-2H-pyridazin-3-one |
| | 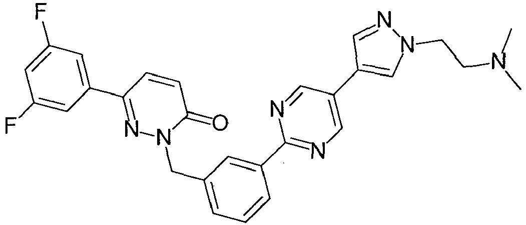 |
| "A172" | 6-(3,5-difluorophényl)-2-(3-{5-[1-(3-diméthylaminopropyl)-1H-pyrazol-4-yl]pyrimidin-2-yl}benzyl)-2H-pyridazin-3-one |
| "A173" | 6-(3,5-difluorophényl)-2-(3-{5-[1-(2-pyrrolidin-1-yléthyl)-1H-pyrazol-4-yl]pyrimidin-2-yl}benzyl)-2H-pyridazin-3-one |
| "A174" | 3-[1-(3-{5-[1-(2-morpholin-4-yléthyl)-1H-pyrazol-4-yl]-pyrimidin-2-yl}benzyl)-6-oxo-1,6-dihydropyridazin-3-yl]-benzonitrile |
| | 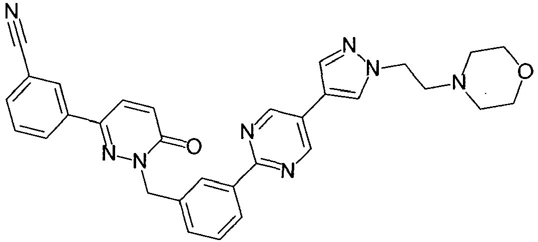 |
| "A175" | 2-(3-{5-[1-(2-morpholin-4-yléthyl)-1H-pyrazol-4-yl]pyrimidin-2-yl}benzyl)-6-pyridin-3-yl-2H-pyridazin-3-one |
| | 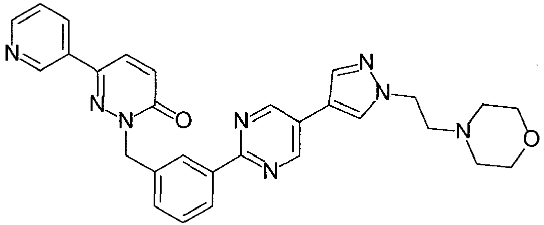 |
| "A176" | 6-(1-méthyl-1H-pyrazol-4-yl)-2-(3-{5-[1-(2-morpholin-4-yl-éthyl)-1H-pyrazol-4-yl]pyrimidin-2-yl}benzyl)-2H-pyridazin-3-one |
| | 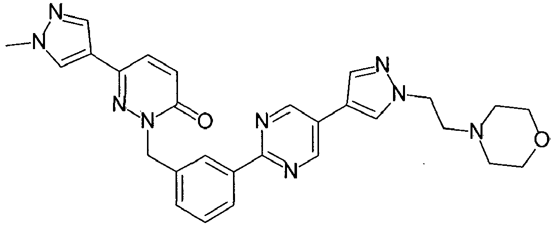 |
| "A177" | 2-(3-{5-[1-(2-morpholin-4-yléthyl)-1H-pyrazol-4-yl]pyrimidin-2-yl}benzyl)-6-pyridin-4-yl-2H-pyridazin-3-one |
| "A178" | 6-(4-méthanesulfonylphényl)-2-(3-{5-[1-(2-morpholin-4-yl-éthyl)-1H-pyrazol-4-yl]pyrimidin-2-yl}benzyl)-2H-pyridazin-3-one |
| "A179" | 6-pyridin-4-yl-2-(3-{5-[1-(2-pyrrolidin-1-yléthyl)-1H-pyrazol-4-yl]pyrimidin-2-yl}benzyl)-2H-pyridazin-3-one |
| "A180" | 4-[6-oxo-1-(3-{5-[1-(2-pyrrolidin-1-yléthyl)-1H-pyrazol-4-yl]-pyrimidin-2-yl}benzyl)-1,6-dihydropyridazin-3-yl]benzonitrile |
| | 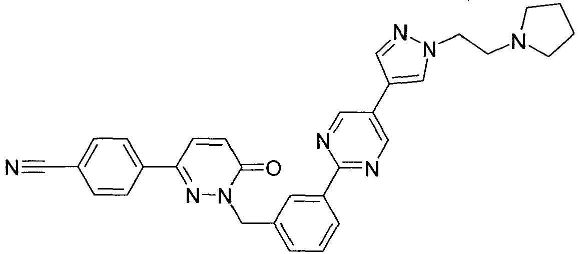 |
| "A181" | 2-(3-{5-[1-(2-morpholin-4-yléthyl)-1H-pyrazol-4-yl]pyrimidin-2-yl}benzyl)-6-pyridin-4-yl-2H-pyridazin-3-one |
| "A183" | 6-(4-méthanesulfonylphényl)-2-(3-{5-[1-(2-morpholin-4-yl-éthyl)-1H-pyrazol-4-yl]pyrimidin-2-yl}benzyl)-2H-pyridazin-3-one |
| "A184" | 6-(5-méthyloxazol-2-yl)-2-(3-{5-[1-(2-pyrrolidin-1-yléthyl)-1H-pyrazol-4-yl]pyrimidin-2-yl}benzyl)-2H-pyridazin-3-one |
| | 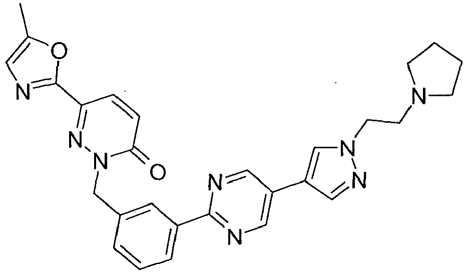 |
| "A185" | 6-(3-fluorophényl)-2-(3-{5-[1-(2-pyrrolidin-1-yléthyl)-1H-pyrazol-4-yl]pyrimidin-2-yl)benzyl)-2H-pyridazin-3-one |
| "A186" | 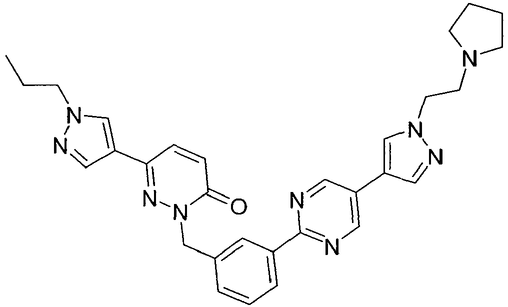 6-(1-propyl-1H-pyrazol-4-yl)-2-(3-{5-[1-(2-pyrrolidin-1-yl-éthyl)-1H-pyrazol-4-yl]pyrimidin-2-yl}benzyl)-2H-pyridazin-3-one |
| "A187" | 2-(3-{5-[1-(2-pyrrolidin-1-yléthyl)-1H-pyrazol-4-yl]pyrimidin-2-yl}benzyl)-6-thiophén-3-yl-2H-pyridazin-3-one |
| | 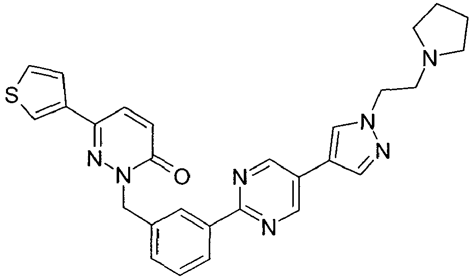 |
| "A188" | 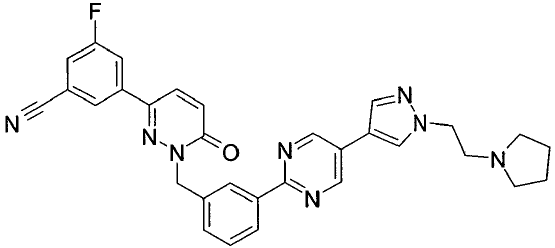 |
| "A188a" | 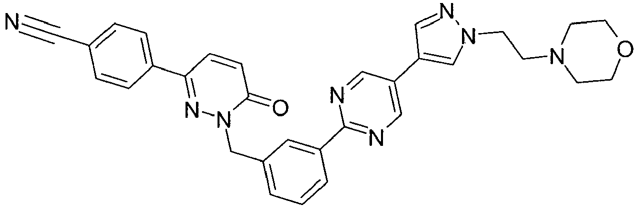 |
| "A189" | 3-(1-{3-[5-(3-diméthy)aminopropoxy)pyrimidin-2-yl]benzyl}-6-oxo-1,6-dihydropyridazin-3-yl)benzonitrile |
| "A190" | 2-{3-[5-(3-diméthylaminopropoxy)pyrimidin-2-yl]benzyl}-6-(1-méthyl-1 H-pyrazol-4-yl)-2H-pyridazin-3-one |
| | 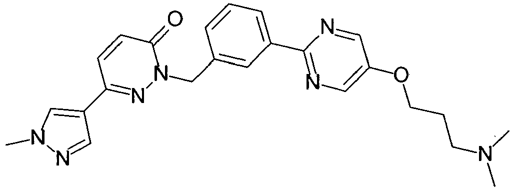 |
| "A191" | 2-{3-[5-(3-diméthylaminopropoxy)pyrimidin-2-yl]benzyl}-6-(3-fluorophényl)-2H-pyridazin-3-one |
| "A192" | 2-{3-[5-(3-diméthylaminopropoxy)pyrimidin-2-yl]benzyl}-6-thiazol-2-yl-2H-pyridazin-3-one |
| "A193" | 2-{3-[5-(3-diméthylaminopropoxy)pyrimidin-2-yl]benzyl}-6-phényl-2H-pyridazin-3-one |
| "A 194" | 4-(1-{3-[5-(3-diméthylaminopropoxy)pyrimidin-2-yl]benzyl}-6-oxo-1,6-dihydropyridazin-3-yl)benzonitrile |
| "A195" | 2-{3-[5-(3-diméthylaminopropoxy)pyrimidin-2-yl]benzyl}-6-p-tolyl-2H-pyridazin-3-one |
| "A196" | 2-{3-[5-(3-diméthylaminopropoxy)pyrimidin-2-yl]benzyl}-6-(2H-pyrazol-3-yl)-2H-pyridazin-3-one |
| "A197" | 6-(3,4-difluorophényl)-2-{3-[5-(3-diméthylamino-propoxy)pyrimidin-2-yl]benzyl}-2H-pyridazin-3-one |
| "A 198" | 2-{3-[5-(3-diméthylaminopropoxy)pyrimidin-2-yl]benzyl}-6-(4-méthanesulfonylphényl)-2H-pyridazin-3-one |
| "A199" | 2-{3-[5-(3-diméthylaminopropoxy)pyrimidin-2-yl]benzyl}-6-[4-(5-méthyl-1,2,4-oxadiazol-3-yl)phényl]-2H-pyridazin-3-one |
| "A200" | 2-{3-[5-(3-diméthylaminopropoxy)pyrimidin-2-yl]benzyl}-6-pyridin-4-yl-2H-pyridazin-3-one |
| "A201" | 6-(3-bromophényl)-2-{3-[5-(3-diméthylamino-propoxy)pyrimidin-2-yl]benzyl}-2H-pyridazin-3-one |
| "A202" | 2-{3-[5-(3-diméthylaminopropoxy)pyrimidin-2-yl]benzyl}-6-(3,4,5-trifluorophényl)-2H-pyridazin-3-one |
| "A203" | 6-(3,5-diméthoxyphényl)-2-{3-[5-(3-diméthylamino-propoxy)pyrimidin-2-yl]benzyl}-2H-pyridazin-3-one |
| "A204" | 2-{3-[5-(3-diméthylaminopropoxy)pyrimidin-2-yl]benzyl}-6-(3-fluoro-4-méthoxyphényl)-2H-pyridazin-3-one |
| "A205" | 2-{3-[5-(3-diméthylaminopropoxy)pyrimidin-2-yl]benzyl}-6-(4-méthoxyphényl)-2H-pyridazin-3-one |
| "A206" | 2-{3-[5-(3-diméthylaminopropoxy)pyrimidin-2-yl]benzyl}-6-(3-trifluorométhylphényl)-2H-pyridazin-3-one |
| "A207" | 6-(3-chlorophényl)-2-{3-[5-(3-diméthylamino-propoxy)pyrimidin-2-yl]benzyl}-2H-pyridazin-3-one |
| "A208" | 2-{3-[5-(3-diméthylaminopropoxy)pyrimidin-2-yl]benzyl}-6-pyridin-3-yl-2H-pyridazin-3-one |
| "A209" | 2-{3-[5-(3-diméthylaminopropoxy)pyrimidin-2-yl]benzyl}-6-(1-méthyl-1H-pyrazol-4-yl)-2H-pyridazin-3-one |
| "A210" | 6-(3-chloro-5-fluorophényl)-2-{3-[5-(3-diméthylamino-propoxy)pyrimidin-2-yl]benzyl}-2H-pyridazin-3-one |
| "A211" | 2-{3-[5-(3-diméthylaminopropoxy)pyrimidin-2-yl]benzyl}-6-(4-fluoro-3-méthoxyphényl)-2H-pyridazin-3-one |
| "A212" | 6-(4-chlorophényl)-2-{3-[5-(3-diméthylaminopropoxy)pyrimidin-2-yl]benzyl}-2H-pyridazin-3-one |
| "A213" | 2-{3-[5-(3-diméthylaminopropoxy)pyrimidin-2-yl]benzyl}-6-(4-fluorophényl)-2H-pyridazin-3-one |
| "A214" | 2-{3-[5-(3-diméthylaminopropoxy)pyrimidin-2-yl]benzyl}-6-thiophén-2-yl-2H-pyridazin-3-one |
| "A215" | N-[4-(1-{3-[5-(3-diméthylaminopropoxy)pyrimidin-2-yl]-benzyl}-6-oxo-1,6-dihydropyridazin-3-yl)phényl]acétamide |
| | 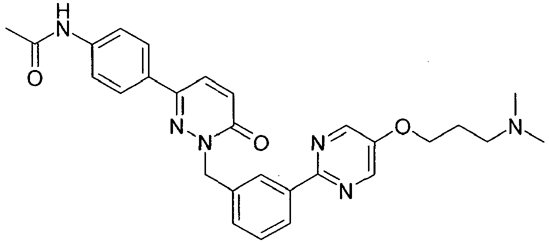 |
| "A216" | 6-(3,4-diméthoxyphényl)-2-{3-[5-(3-diméthylamino-propoxy)pyrimidin-2-yl]benzyl}-2H-pyridazin-3-one |
| "A217" | 6-benzo-1,2,5-thiadiazol-5-yl-2-{3-[5-(3-diméthylamino-propoxy)pyrimidin-2-yl]benzyl}-2H-pyridazin-3-one |
| | 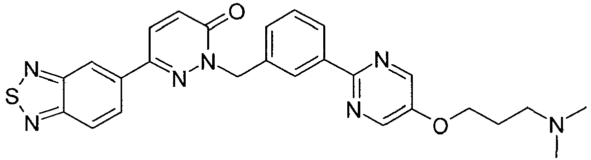 |
| "A218" | 2-{3-[5-(3-diméthylaminopropoxy)pyrimidin-2-yl]benzyl}-6-furan-3-yl-2H-pyridazin-3-one |
| "A219" | 2-{3-[5-(3-diméthylaminopropoxy)pyrimidin-2-yl]benzyl}-6-(5-méthyl-1,2,4-oxadiazol-3-yl)-2H-pyridazin-3-one |
| "A220" | 4-(1-{3-[5-(1-méthylpipéridin-4-yloxy)pyrimidin-2-yl]benzyl}-6-oxo-1,6-dihydropyridazin-3-yl)benzonitrile |
| "A221" | 3-(1-{3-[5-(1-méthylpipéridin-4-yloxy)pyrimidin-2-yl]benzyl}-6-oxo-1,6-dihydropyridazin-3-yl)benzonitrile |
| "A222" | 3-(1-{3-[5-(2-morpholin-4-yléthoxy)pyrimidin-2-yl]benzyl}-6-oxo-1,6-dihydropyridazin-3-yl)benzonitrile |
| "A223" | 2-{3-[5-(1-méthylpipéridin-4-yloxy)pyrimidin-2-yl]benzyl}-6-pyridin-4-yl-2H-pyridazin-3-one |
| "A224" | 6-(4-méthanesulfonylphényl)-2-{3-[5-(1-méthylpipéridin-4-yl-oxy)pyrimidin-2-yl]benzyl}-2H-pyridazin-3-one |
| "A225" | 5-(1-{3-[5-(1-méthylpipéridin-4-yloxy)pyrimidin-2-yl]benzyl}-6-oxo-1,6-dihydropyridazin-3-yl)thiophène-2-carboxylate de méthyle |
| | 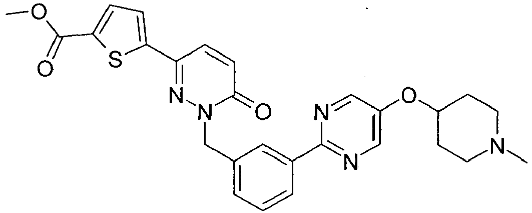 |
| "A226" | 2-{3-[5-(1-méthylpipéridin-4-yloxy)pyrimidin-2-yl]benzyl}-6-(1-méthyl-1H-pyrazol-4-yl)-2H-pyridazin-3-one |
| "A227" | 2-{3-[5-(3-diméthylaminopropoxy)pyrimidin-2-yl]benzyl}-6-(5-oxo-4,5-dihydro-1,2,4-oxadiazol-3-yl)-2H-pyridazin-3-one |
| | 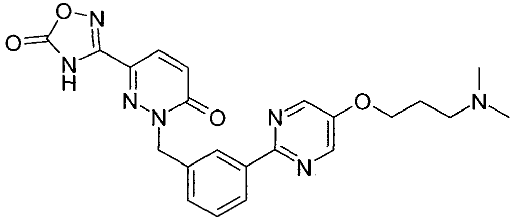 |
| "A228" | 2-{3-[5-(3-diméthylaminopropoxy)pyrimidin-2-yl]benzyl}-6-pipérazin-1-yl-2H-pyridazin-3-one |
| | 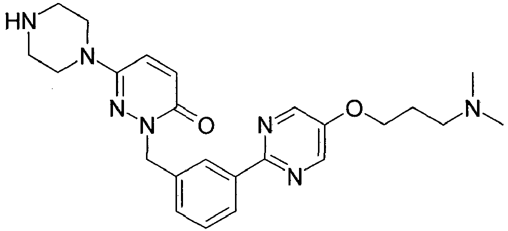 |
| "A229" | 6-(1-méthyl-1H-pyrazol-4-yl)-2-{3-[5-(2-morpholin-4-yl-éthoxy)pyrimidin-2-yl]benzyl}-2H-pyridazin-3-one |
| "A235" | 6-(3-fluorophényl)-2-{3-[5-(2-morpholin-4-yléthoxy)pyrimidin-2-yl]benzyl}-2H-pyridazin-3-one |
| "A237" | 2-{3-[5-(1-méthylpipéridin-4-ylméthoxy)pyrimidin-2-yl]-benzyl}-6-(1-méthyl-1H-pyrazol-4-yl)-2H-pyridazin-3-one |
| "A238" | 2-{3-[5-(1-méthylpipéridin-4-yloxy)pyrimidin-2-yl]benzyl}-6-(1-méthyl-1 H-pyrazol-4-yl)-2H-pyridazin-3-one |
| "A240" | 2-{3-[5-(3-méthoxypropoxy)pyrimidin-2-yl]benzyl}-6-(1-méthyl-1 H-pyrazol-4-yl)-2H-pyridazin-3-one |
| "A241" | 2-{3-[5-(2-méthoxyéthoxy)pyrimidin-2-yl]benzyl}-6-(1-méthyl-1 H-pyrazol-4-yl)-2H-pyridazin-3-one |
| "A242" | 2-{3-[5-(2-morpholin-4-yléthoxy)pyrimidin-2-yl]benzyl}-6-(1-propyl-1 H-pyrazol-4-yl)-2H-pyridazin-3-one |
| "A243" | 2-(3-{5-[2-(4-méthylpipérazin-1-yl)éthoxy]pyrimidin-2-yl}-benzyl)-6-(1-méthyl-1H-pyrazol-4-yl)-2H-pyridazin-3-one |
| "A244" | 2-(3-{5-[2-(4-méthyl-3-oxopipérazin-1-yl)éthoxy]pyrimidin-2-yl}benzyl)-6-(1-méthyl-1H-pyrazol-4-yl)-2H-pyridazin-3-one |
| | 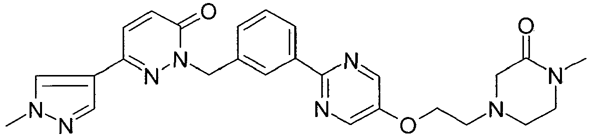 |
| "A245" | 6-(1-méthyl-1H-pyrazol-4-yl)-2-{3-[5-(3-morpholin-4-yl-propoxy)pyrimidin-2-yl]benzyl}-2H-pyridazin-3-one |
| "A246" | 6-(1-méthyl-1H-pyrazol-4-yl)-2-{3-[5-(2-morpholin-4-yl-propoxy)pyrimidin-2-yl)benzyl}-2H-pyridazin-3-one |
| | 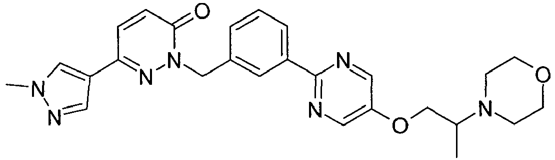 |
| "A247" | 2-{3-[5-(1-méthyl-2-morpholin-4-yléthoxy)pyrimidin-2-yl]-benzyl}-6-(1-méthyl-1H-pyrazol-4-yl)-2H-pyridazin-3-one |
| | 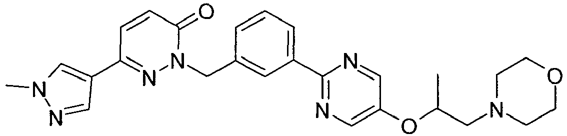 |
| "A248" | 2-{3-[5-(2-diméthylaminoéthoxy)pyrimidin-2-yl]benzyl}-6-(1-méthyl-1 H-pyrazol-4-yl)-2H-pyridazin-3-one |
| "A249" | 2-{3-[5-(2-méthyl-3-morpholin-4-ylpropoxy)pyrimidin-2-yl]-benzyl}-6-(1-méthyl-1H-pyrazol-4-yl)-2H-pyridazin-3-one |
| | 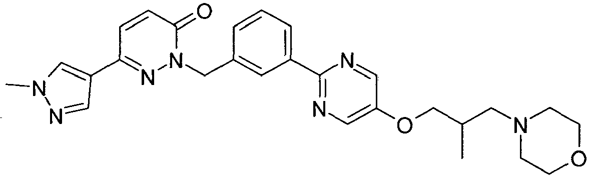 |
| "A250" | 6-(1-méthyl-1H-pyrazol-4-yl)-2-{3-[5-(2-pyrrolidin-1-yl-éthoxy)pyrimidin-2-yl]benzyl}-2H-pyridazin-3-one |
| "A251" | 2-[3-(5-éthoxypyrimidin-2-yl)benzyl]-6-(1-méthyl-1H-pyrazol-4-yl)-2H-pyridazin-3-one |
| "A252" | 6-(1-méthyl-1H-pyrazol-4-yl)-2-{3-[5-(2-morpholin-4-yl-2-oxoéthoxy)pyrimidin-2-yl]benzyl}-2H-pyridazin-3-one |
| | 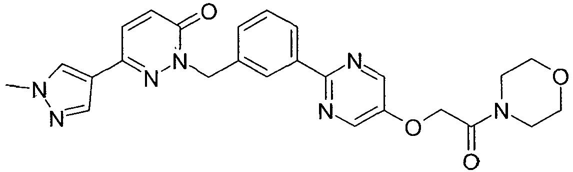 |
| "A253" | 6-(3-chlorophényl)-2-{3-[5-(2-morpholin-4-yl-éthoxy)pyrimidin-2-yl]benzyl}-2H-pyridazin-3-one |
| "A254" | 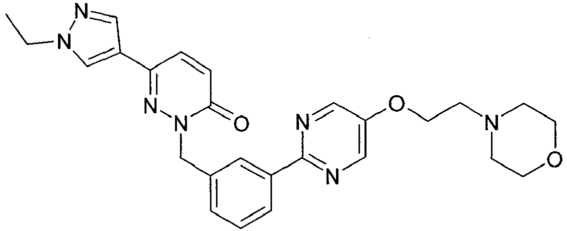 |
| "A255" | 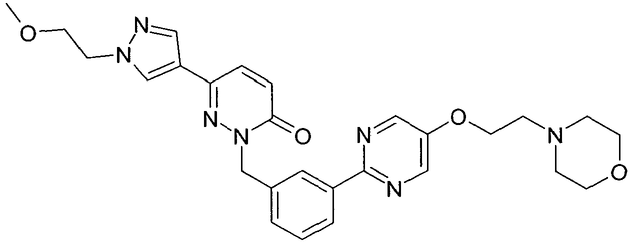 |
| "A256" | 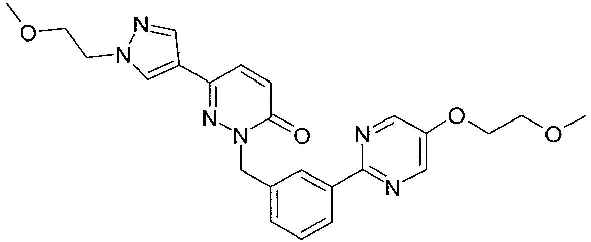 |
| "A257" | 3-(1-{3-[5-(1-méthylpipéridin-4-ylméthoxy)pyrimidin-2-yl]-benzyl}-6-oxo-1,6-dihydropyridazin-3-yl)benzonitrile |
| "A259" | 6-(3,5-difluorophényl)-2-{3-[5-(1-pipéridin-4-yl-1H-pyrazol-4-yl)pyridin-2-yl]benzyl}-2H-pyridazin-3-one |
| "A260" | 3-(1-{3-[5-(3-diméthylaminopropoxy)pyrimidin-2-yl]benzyl}-6-oxo-1,6-dihydropyridazin-3-yl)benzamide |
| "A261" | 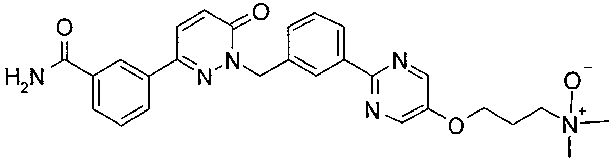 |
| "A264" | 3-(6-oxo-1-{3-[5-(pipéridin-4-ylméthoxy)pyrimidin-2-yl]-benzyl}-1,6-dihydropyridazin-3-yl)benzonitrile |
| "A265" | 4-(2-{3-[3-(3-cyanophényl)-6-oxo-6H-pyridazin-1-yl-méthyl]phényl}pyrimidin-5-yloxyméthyl)pipéridine-1-carboxylate de tertio-butyle |
| "A266" | 6-(1-méthyl-1H-pyrazol-4-yl)-2-{3-[5-(pipéridin-4-yloxy)-pyrimidin-2-yl]benzyl}-2H-pyridazin-3-one |
| "A267" | 6-(1-méthyl-1H-pyrazol-4-yl)-2-{3-[5-(pipéridin-4-yl-méthoxy)pyrimidin-2-yl]benzyl}-2H-pyridazin-3-one |
| "A268" | 3-(1-{3-[5-(3-méthylaminopropoxy)pyrimidin-2-yl]benzyl}-6-oxo-1,6-dihydropyridazin-3-yl)benzonitrile |
| | 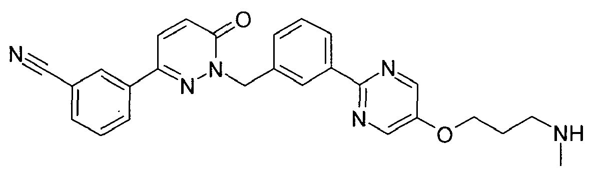 |
| "A269" | 3-[1-(3-{5-[2-(4-méthyl-3-oxopipérazin-1-yl)éthoxy]pyrimidin-2-yl}benzyl)-6-oxo-1,6-dihydropyridazin-3-yl]benzonitrile |
| | 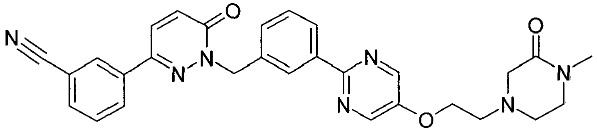 |
| "A270" | 3-[1-(3-{5-[2-(4-méthylpipérazin-1-yl)éthoxy]pyrimidin-2-yl}-benzyl)-6-oxo-1,6-dihydropyridazin-3-yl]benzonitrile |
| "A271" | 3-(1-{3-[5-(2-méthoxyéthoxy)pyrimidin-2-yl]benzyl}-6-oxo-1,6-dihydropyridazin-3-yl)benzonitrile |
| "A272" | 3-(1-{3-[5-(3-méthoxypropoxy)pyrimidin-2-yl]benzyl}-6-oxo-1,6-dihydropyridazin-3-yl)benzonitrile |
| "A273" | 6-(3-fluorophényl)-2-{3-[5-(1-méthylpipéridin-4-yl-méthoxy)pyrimidin-2-yl]benzyl}-2H-pyridazin-3-one |
| "A274" | 2-{3-[5-(1-méthylpipéridin-4-yloxy)pyrimidin-2-yl]benzyl}-6-(1-propyl-1H-pyrazol-4-yl)-2H-pyridazin-3-one |
| "A275" | 6-(3-chlorophényl)-2-{3-[5-(1-méthylpipéridin-4-yl-méthoxy)pyrimidin-2-yl]benzyl}-2H-pyridazin-3-one |
| "A276" | 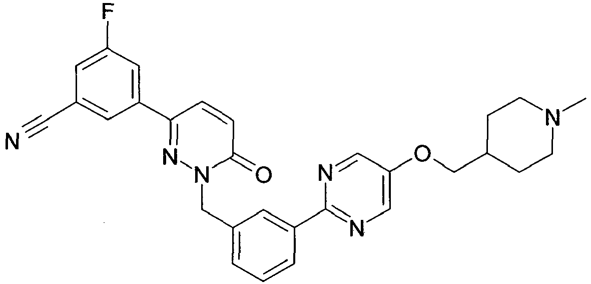 |
| "A276a" | 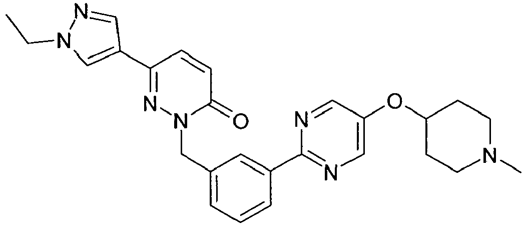 |
| "A277" | acide 5-(1-{3-[5-(1-méthylpipéridin-4-yloxy)pyrimidin-2-yl]-benzyl}-6-oxo-1,6-dihydropyridazin-3-yl)thiophène-2-carboxylique |
| "A278" | 5-(1-{3-[5-(1-méthylpipéridin-4-yloxy)pyrimidin-2-yl]benzyl}-6-oxo-1,6-dihydropyridazin-3-yl)thiophène-2-carboxamide |
| "A279" | N-méthyl-5-(1-{3-[5-(1-méthylpipéridin-4-yloxy)pyrimidin-2-yl]benzyl}-6-oxo-1,6-dihydropyridazin-3-yl)thiophène-2-carboxamide |
| "A280" | 2-[3-(5-aminopyrazin-2-yl)benzyl]-6-(3,5-difluorophényl)-2H-pyridazin-3-one |
| "A282" | 2-[3-(6-aminopyridazin-3-yl)benzyl]-6-(3,5-difluorophényl)-2H-pyridazin-3-one |
| "A283" | (E)-3-(2-{3-[6-oxo-3-(3,4,5-trifluorophényl)-6H-pyridazin-1-ylméthyl]phényl}pyrimidin-5-yl)acrylate de méthyle |
| "A284" | 2-{3-[5-((E)-3-aminopropényl)pyrimidin-2-yl]benzyl}-6-(3,4,5-trifluorophényl)-2H-pyridazin-3-one |
| "A285" | 2-{3-[5-(3-aminopropyl)pyrimidin-2-yl]benzyl}-6-(3,4,5-trifluorophényl)-2H-pyridazin-3-one |
| "A286" | 2-{3-[5-(4-méthylpipérazin-1-yl)pyrimidin-2-yl]benzyl}-6-(1-méthyl-1 H-pyrazol-4-yl)-2H-pyridazin-3-one |
| "A287" | 3-(1-{3-[5-(4-méthylpipérazin-1-yl)pyrimidin-2-yl]benzyl}-6-oxo-1,6-dihydropyridazin-3-yl)benzonitrile |
| "A288" | 3-{6-oxo-1-[3-(5-pipérazin-1-ylpyrimidin-2-yl)benzyl]-1,6-dihydropyridazin-3-yl}benzonitrile |
| "A289" | 6-(4-méthanesulfonylphényl)-2-[3-(5-pipérazin-1-ylpyrimidin-2-yl)benzyl]-2H-pyridazin-3-one |
| "A290" | 4-{1-[3-(5-aminopyrimidin-2-yl)benzyl]-6-oxo-1,6-dihydro-pyridazin-3-yl}benzonitrile |
| "A291" | 3-{1-[3-(5-aminopyrimidin-2-yl)benzyl]-6-oxo-1,6-dihydro-pyridazin-3-yl}benzonitrile |
| "A292" | 6-(1-méthyl-1H-pyrazol-4-yl)-2-[3-(5-pipérazin-1-ylpyrimidin-2-yl)benzyl]-2H-pyridazin-3-one |
| "A293" | 2-[3-(5-aminopyrimidin-2-yl)benzyl]-6-(1-méthyl-1H-pyrazol-4-yl)-2H-pyridazin-3-one |
| "A294" | 2-{3-[5-(2-hydroxyéthoxy)pyrimidin-2-yl]benzyl}-6-(1-méthyl-1 H-pyrazol-4-yl)-2H-pyridazin-3-one |
| "A295" | 3-(1-{3-[5-(3-hydroxypropoxy)pyrimidin-2-yl]benzyl}-6-oxo-1,6-dihydropyridazin-3-yl)benzonitrile |
| "A296" | 3-(1-{3-[5-(2-hydroxyéthoxy)pyrimidin-2-yl]benzyl}-6-oxo-1,6-dihydropyridazin-3-yl)benzonitrile |
| "A297" | 2-{3-[5-(3-hydroxypropoxy)pyrimidin-2-yl]benzyl}-6-(1-méthyl-1 H-pyrazol-4-yl)-2H-pyridazin-3-one |
| "A298" | 3-[1-(1-{3-[5-(3-diméthylaminopropoxy)pyrimidin-2-yl]-phényl}éthyl)-6-oxo-1,6-dihydropyridazin-3-yl]benzonitrile |
| "A299" | 6-(3,5-difluorophényl)-2-(1-{3-[5-(3-diméthylamino-propoxy)pyrimidin-2-yl]phényl}éthyl)-2H-pyridazin-3-one |
| "A300" | 6-(3,5-difluorophényl)-2-((R)-1-{3-[5-(3-diméthylamino-propoxy)pyrimidin-2-yl]phényl}éthyl)-2H-pyridazin-3-one |
| "A301" | 6-(3,5-difluorophényl)-2-((S)-1-{3-[5-(3-diméthylamino-propoxy)pyrimidin-2-yl]phényl}éthyl)-2H-pyridazin-3-one |
| "A302" | 3-(1-{3-[5-(1-méthyl-1-oxypipéridin-4-ylméthoxy)pyrimidin-2-yl]benzyl}-6-oxo-1,6-dihydropyridazin-3-yl)benzonitrile |
| "A303" | 3-(1-{3-[5-(1-formylpipéridin-4-ylméthoxy)pyrimidin-2-yl]-benzyl}-6-oxo-1,6-dihydropyridazin-3-yl)benzonitrile |
| "A304" | 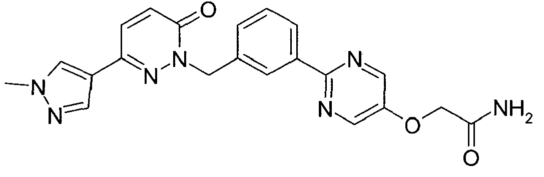 |
| "A305" | 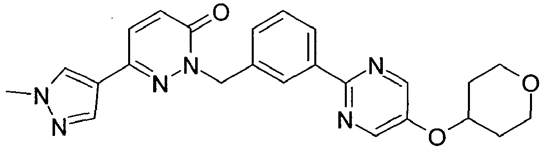 |
| "A306" | 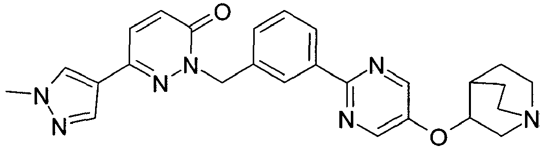 |
| "A307" | 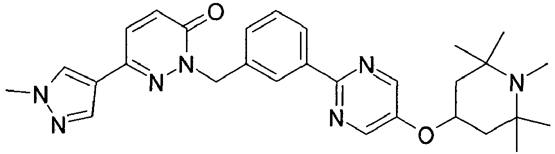 |
| "A308" | 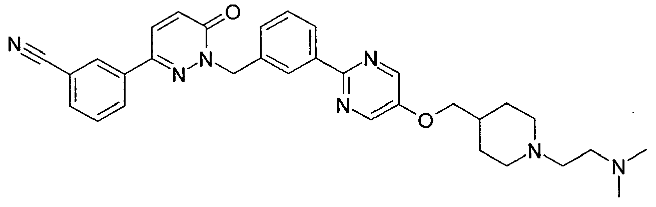 |
| "A309" | 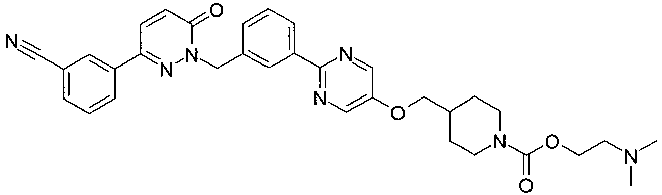 |
| "A310" | 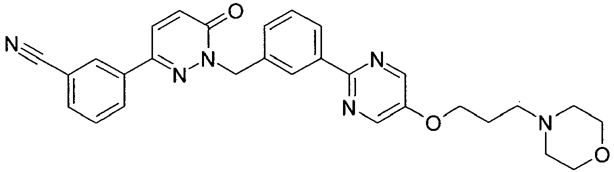 |
| "A311" | 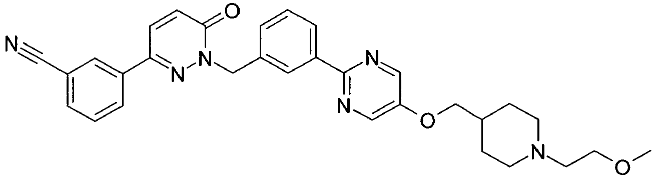 |
| "A312" | 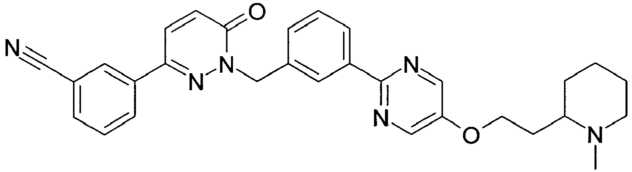 |
| "A313" | 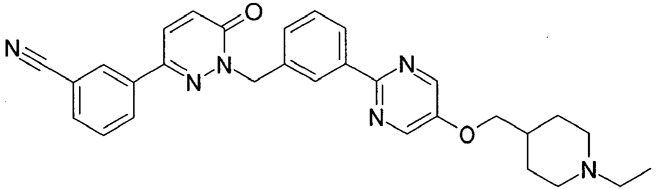 |
| "A314" | 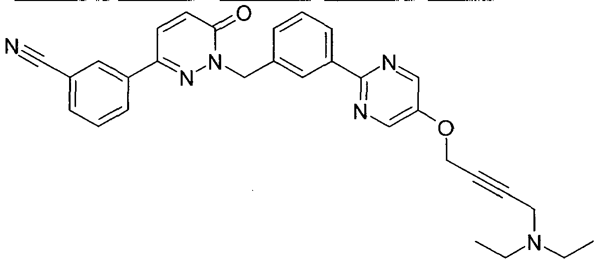 |

15. Médicament selon l'une ou plusieurs parmi les revendications 1-14, dans laquelle 1 mg à 700 mg d'un composé de formule I est présent.

16. Médicament selon la revendication 15, dans laquelle 5 mg à 100 mg d'un composé de formule I est présent.
